# EUROPEAN PATENT SPECIFICATION

(11) **EP 3 908 579 B1**
(45) Date of publication and mention of the grant of the patent: **27.12.2023**
(21) Application number: 20702357.3
(22) Date of filing: 10.01.2020
(51) Int. Cl.: C07D 401/04, C07D 401/14, C07D 405/14, C07D 413/14, C07D 417/14, A61P 35/02, A61K 31/455

(54) **DIHYDROOROTATE DEHYDROGENASE INHIBITORS**
DIHYDROOROTAT-DEHYDROGENASE-INHIBITOREN
INHIBITEURS DE DIHYDROOROTATE DÉSHYDROGÉNASE

(30) Priority: 11.01.2019 US 201962791057 P; 11.06.2019 US 201962859851 P
(43) Date of publication of application: 17.11.2021
(73) Proprietor: Janssen Biotech, Inc., Horsham, PA 19044 (US)
(72) Inventor: CISAR, Justin, Spring House, Pennsylvania 19477 (US); KUDUK, Scott, Spring House, Pennsylvania 19477 (US); WANG, Chao-yuan, Spring House, Pennsylvania 19477 (US); SIMONNET, Yvan Rene Ferdinand, Spring House, Pennsylvania 19477 (US); KEOHANE, Colleen Elizabeth, Spring House, Pennsylvania 19477 (US); JACOBY, Edgar, 2340 Beerse (BE)
(74) Representative: Carpmaels & Ransford LLP
(86) International application number: PCT/IB2020/050180
(87) International publication number: WO 2020/144638

(56) References cited:
- WO-A1-2018/077923
- MUNIER-LEHMANN ET AL.: "On Dihydroorotate Dehydrogenases and Their Inhibitors and Uses", J. MED. CHEM., vol. 56, no. 8, 25 April 2013 (2013-04-25) , pages 3148-3167, XP055148746, ISSN: 0022-2623, DOI: 10.1021/jm301848w

## Description

### FIELD OF THE INVENTION

The present invention relates to novel compounds that are dihydroorotate dehydrogenase (DHODH) inhibitors. These compounds may be useful for the treatment of a disease, disorder, or medical condition where there is an advantage in inhibiting DHODH. The invention also relates to pharmaceutical compositions comprising one or more of such compounds, and to such compounds or pharmaceutical compositions for use in the treatment of cancer, and autoimmune and inflammatory diseases, syndromes, and disorders.

### BACKGROUND OF THE INVENTION

Acute myelogenous leukemia (AML) is a clonal disease of the blood and bone marrow resulting from mutations that occur in normal hematopoietic stem cells. AML is a heterogenous disease in that it presents with a range of cytogenetic, morphological and immunophenotypic features, and is characterized by an accumulation of clonal, abnormal myeloid progenitor cells, known as myeloblasts. These cells demonstrate disruption of normal myeloid differentiation and excessive proliferation, resulting in the decreased formation of hematopoietic cells. Disease remission can be achieved with standard induction chemotherapy, but refractory and relapsed disease remains a challenge due to persistence of leukemic stem cells. Therefore, AML represents an unmet medical need with >20,000 new cases per year in the US with 5-year overall survival below 30% (Stein ET et al., Health Qual Life Outcomes 16: 193, 2018).

Differentiation therapy is considered an attractive approach to AML treatment based on the knowledge that differentiation and loss of stem cell self-renewal are coupled in normal cells. Treatment of acute promyelocytic leukemia, which represents 10-15% of all AML, with all-trans retinoic acid is the paradigm for differentiation therapy. Retinoic acid targets the promyelocytic leukemia protein (PML)-retinoic acid receptor-α (RAR-α) fusion protein encoded by a t(15,17) chromosomal translocation. Targeting PML-RAR specifically lifts the transcriptionally mediated differentiation block induced by the fusion protein and early clinical trials with single agent ATRA demonstrated complete hematologic remission in all treated patients (McCulloch D et al. Onco Targets Ther 2017; 10: 1585-1601; Nowak D et al. Blood 113: 3655, 2009).

Although differentiation therapy is successful, it is only applicable to a small population of AML patients. Research efforts have aimed at identifying additional differentiation inducing agents, but with limited success. Recently dihydroorotate dehydrogenase (DHODH) emerged as a potentially more broadly applicable differentiation target in a phenotypic screen aimed at identifying small molecules that overcome blockade of the maturation of primary murine bone marrow cells expressing the homeobox protein HoxA9. This protein is a key transcription factor involved in balancing stem cell maintenance/differentiation and is normally expressed in hematopoietic progenitor cells and downregulated upon induction of differentiation and has been found to be widely overexpressed in AML (Sykes et al., Cell 167: 171, 2016).

DHODH is a flavin mononucleotide (FMN) flavoprotein located in the inner mitochondrial membrane that catalyzes the oxidation of dihydroorotate to orotate, the fourth step in the de novo pyrimidine biosynthesis pathway. Inhibition of DHODH leads to decreased pyrimidine synthesis important precursors for nucleotide synthesis, but also glycoprotein and phospholipid biosynthesis (Reis RAG et al., Archives Biochem Biophysics 632: 175, 2017; Vyas VK et al., Mini Rev Med Chem 11: 1039, 2011). DHODH is a validated target for the treatment of autoimmune diseases with the FDA approved small molecule DHODH inhibitors leflunomide and teriflunomide for rheumatoid arthritis and multiple sclerosis, respectively (Lolli ML et al., Recent patents on Anti-Cancer Drug Discovery 13: 86, 2018).

Since the first observation by Sykes et al. demonstrating that DHODH inhibition drives AML differentiation in vitro, as evidenced by upregulation of the differentiation markers CD11b and CD14, and results in dose dependent anti-leukemic effects, decreased leukemic stem cells and prolonged survival in vivo, additional evidence emerged demonstrating that small molecule DHODH inhibitors mediate antiproliferative activity against AML cells with concomitant cell cycle arrest, upregulation of CD 11b and CD14, and induction of apoptosis (Wu D et al.. Haematologica 103: 1472, 2018; Sainas S et al., J Med Chem 61: 6034, 2018; Cao Let al., Mol Cancer Ther, October 23rd Epub ahead of print). Moreover, preclinical solid tumor in vitro and in vivo models demonstrated effectiveness of DHODH inhibition and DHODH was identified as a synthetic lethality in PTEN and KRAS mutant solid tumors (Pharmacology and Therapeutics, Epub October 19th, 2018; Mathur D et al., Cancer Discovery 7: 1, 2017; Cell Chemical Biology 25: 1, 2018).

Thus, there remains a need for DHODH inhibitors that provide a therapeutic benefit to patients suffering from cancer and/or inflammatory and immunological diseases. Certain inhibitors of DHODH are disclosed in WO 2018/077923.

### SUMMARY OF THE INVENTION

Embodiments of the present invention relate to compounds, pharmaceutical compositions containing them, compounds and pharmaceutical compositions containing them for use in treating a subject suffering from or diagnosed with a disease, disorder, or medical condition comprising inhibiting or altering DHODH enzymatic activity, wherein the disease, disorder, or medical condition is an inflammatory disorder, an autoimmune disorder, or cancer, and compounds and pharmaceutical compositions containing them for use in the treatment of a subject suffering from or diagnosed with a disease, disorder, or medical condition such as autoimmune or inflammatory disorders, or diseases such as cancer.

Embodiments of this invention are compounds of Formula (I), 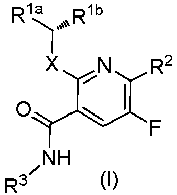 wherein
X is selected from the group consisting of: O, S and NR^{a};
   when X is NR^{a}; R^{a} is H or CH₃; or R^{a} and R^{1a} come together to form a heterocyclic ring selected from the group consisting of: 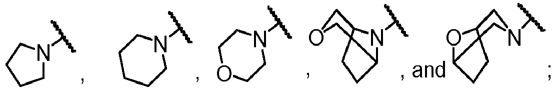 wherein the heterocyclic ring is substituted with one or two members each independently selected from the group consisting of: C₁₋₆alkyl, C₁₋₆haloalkyl, and OC₁₋₆alkyl;
R^{1a} is selected from the group consisting of: C₁₋₆alkyl; C₁₋₆alkyl substituted with OH, or OCH₃; C₁₋₆haloalkyl; C₁₋₆haloalkyl substituted with OH, or OCH₃; and C₃₋₆cycloalkyl;
R^{1b} is CH₃ or CHF₂; or R^{1a} and R^{1b} come together to form C₃₋₆cycloalkyl; C₃₋₆cycloalkyl independently substituted with one, two, three or four members each independently selected from the group consisting of: halo, OH, C₁₋₆alkyl, and C₁₋₆haloalkyl; oxetanyl; tetrahydrofuranyl; and tetrahydropyranyl;
R² is 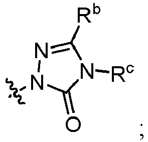 where
   R^{b} is C₁₋₆alkyl substituted with a member selected from the group consisting of: OH, halo, CN, OC₁₋₆alkyl, OC₁₋₆haloalkyl and OC₃₋₆cycloalkyl;
   R^{c} is selected from the group consisting of: C₁₋₆alkyl, C₁₋₆alkyl substituted with OH, allyl, C₁₋₆haloalkyl, and C₃₋₆cycloalkyl; and
R³ is selected from the group consisting of: 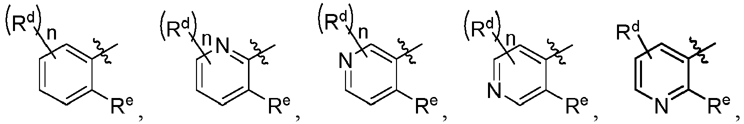 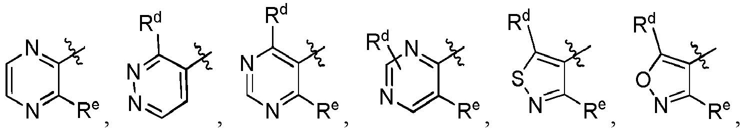 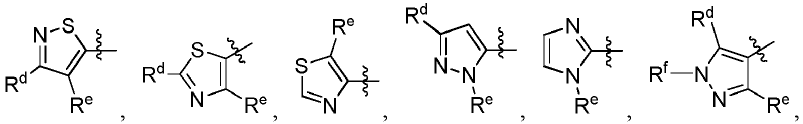 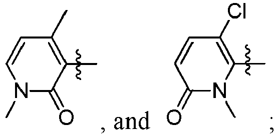
   R^{d} is selected from the group consisting of: H; halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; N(CH₃)₂; OH; CN and OC₁₋₆alkyl;
   R^{e} is selected from the group consisting of: halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; OH; OC₁₋₆alkyl; and C₃₋₆cycloalkyl;
   R^{f} is selected from the group consisting of: H; C₁₋₆alkyl; C₁₋₆alkyl substituted with a member selected from the group consisting of: OH, OCH₃, SCH₃, and OCF₃; C₁₋₆haloalkyl; and C₁₋₆haloalkyl substituted with a member selected from the group consisting of: OH, and OCH₃; and
   n is 1, or 2;
   or a pharmaceutically acceptable salt, isotope, N-oxide, solvate, or stereoisomer thereof.

The present invention further provides a compound of Formula (I) or a pharmaceutically acceptable salt, isotope, N-oxide, solvate, or stereoisomer thereof, for use in treating or ameliorating a disease, syndrome, condition, or disorder in a subject, including a mammal and/or human in which the disease, syndrome, condition, or disorder is affected by the inhibition of DHODH enzymatic activity, including but not limited to, cancer and/or inflammatory or immunological diseases.

Additional embodiments, features, and advantages of the invention will be apparent from the following detailed description and through practice of the invention.

### DETAILED DESCRIPTION OF THE INVENTION

Unless defined otherwise, all technical and scientific terms used herein have the same meaning as is commonly understood by one of ordinary skill in art. As used in the specification and the appended claims, unless specified to the contrary, the following terms have the meaning indicated in order to facilitate the understanding of the present invention.

The singular forms "a", "an" and "the" encompass plural references unless the context clearly indicates otherwise.

With reference to substituents, the term "independently" refers to the situation where when more than one substituent is possible, the substituents may be the same or different from each other.

The term "substituted" means that the specified group or moiety bears one or more substituents. The term "unsubstituted" means that the specified group bears no substituents. The term "optionally substituted" means that the specified group is unsubstituted or substituted by one or more substituents. Where the term "substituted" is used to describe a structural system, the substitution is meant to occur at any valency-allowed position on the system.

Unless qualified specifically in particular instances of use, the term "alkyl" refers to a straight- or branched-chain alkyl group having from 1 to 8 carbon atoms in the chain. Examples of alkyl groups include methyl (Me), ethyl (Et), n-propyl, isopropyl, butyl, isobutyl, sec-butyl, tert-butyl (tBu), pentyl, isopentyl, tert-pentyl, hexyl, isohexyl, and groups that in light of the ordinary skill in the art and the teachings provided herein would be considered equivalent to any one of the foregoing examples. "C₁-₆alkyl" refers to straight- or branched-chain alkyl group having from 1 to 6 carbon atoms in the chain. "C₁-₄alkyl" refers to straight- or branched-chain alkyl group having from 1 to 4 carbon atoms in the chain.

The term "cycloalkyl" refers to a saturated or partially saturated, monocyclic, fused polycyclic, or spiro polycyclic carbocycle having from 3 to 12 ring atoms per carbocycle. "C₃-₆cycloalkyl" refers to a carbocycle having from 3 to 6 ring atoms per carbocycle. Illustrative examples of cycloalkyl groups include the following entities, in the form of properly bonded moieties: 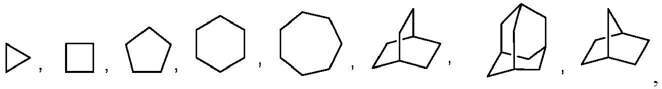 or

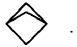

The term "halogen" or "halo" represents chlorine, fluorine, bromine, or iodine.

The term "haloalkyl" refers to a straight- or branched-chain alkyl group having from 1 to 6 carbon atoms in the chain optionally substituting hydrogens with halogens. The term "C₁-₆ haloalkyl" as used here refers to a straight- or branched-chain alkyl group having from 1 to 6 carbon atoms in the chain, optionally substituting hydrogens with halogens. The term "C₁-₄ haloalkyl" as used here refers to a straight- or branched-chain alkyl group having from 1 to 4 carbon atoms in the chain, optionally substituting hydrogens with halogens. Examples of "haloalkyl" groups include trifluoromethyl (CF₃), difluoromethyl (CF₂H), monofluoromethyl (CH₂F), pentafluoroethyl (CF₂CF₃), tetrafluoroethyl (CHFCF₃), monofluoroethyl (CH₂CH₂F), trifluoroethyl (CH₂CF₃), tetrafluorotrifluoromethylethyl (CF(CF₃)₂), and groups that in light of the ordinary skill in the art and the teachings provided herein would be considered equivalent to any one of the foregoing examples.

The terms "4- to 8-membered heterocycloalkyl" and "4- to 6-membered heterocycloalkyl" mean a monocyclic, bicyclic, or bridged, saturated heterocycle with 4, 5, 6, 7 or 8 or, respectively, 4, 5, or 6, ring atoms in total, which contains one or two identical or different ring heteroatoms from the series N, O and S being possible for said heterocycloalkyl group to be attached to the rest of the molecule via any one of the carbon atoms or, if present and not excluded otherwise, a nitrogen atom. Illustrative examples of heterocycloalkyl groups include the following entities, in the form of properly bonded moieties:

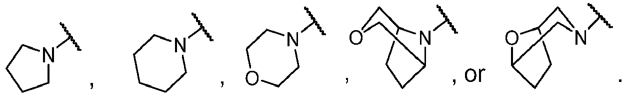

The term "aryl" refers to a monocyclic, aromatic carbocycle (ring structure having ring atoms that are all carbon) having 6 atoms per ring. (Carbon atoms in the aryl groups are sp2 hybridized.)

The term "phenyl" represents the following moiety:

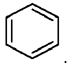

The term "pyridinyl" or "pyridyl" represents the following moiety:

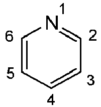

The pyridinyl or pyridyl moiety can be attached through any one of the 2-, 3-, 4-, 5-, or 6-position carbon atoms.

The term "pyrimidinyl" represents the following moiety:

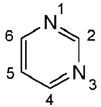

The pyrimidinyl moiety can be attached through any one of the 2-, 4-, 5-, or 6-position carbon atoms.

The term "pyridazinyl" represents the following moiety:

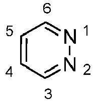

The pyridazinyl moiety can be attached through any one of the 3-, 4-, 5-, or 6-position carbon atoms.

The term "imidazolyl" represents the following moiety:

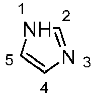

The imidazolyl moiety can be attached through any one of the 1-, 2-, 3-, 4-, or 5-position carbon atoms.

The term "heteroaryl" refers to a monocyclic or fused bicyclic heterocycle (ring structure having ring atoms selected from carbon atoms and up to four heteroatoms selected from nitrogen, oxygen, and sulfur) having from 3 to 9 ring atoms per heterocycle. Illustrative examples of heteroaryl groups include the following entities, in the form of properly bonded moieties:

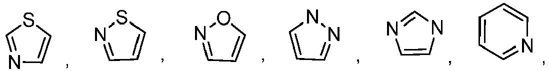

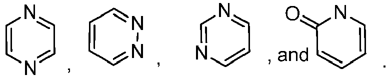

The term " tautomeric " or " tautomeric form " refers to structural isomers of different energies that are interconvertible through low energy barriers. For example, proton tautomers (also known as proton tautomers) include interconversions through the transfer of protons, such as keto-enol and imine-enamine isomerization. The valence tautomers include interconversions by restructuring some bond electrons.

For example, hydroxypyridine or the tautomeric pyridone is represented below.

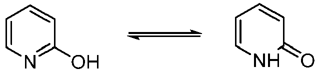

For example, pyrazole tautomers are represented below.

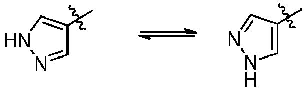

Those skilled in the art will recognize that the species of heterocycloalkyl, cycloalkyl, heteroaryl and aryl groups listed or illustrated above are not exhaustive, and that additional species within the scope of these defined terms may also be selected.

The term "variable point of attachment" means that a group is allowed to be attached at more than one alternative position in a structure. The attachment will always replace a hydrogen atom on one of the ring atoms. In other words, all permutations of bonding are represented by the single diagram, as shown in the illustrations below.

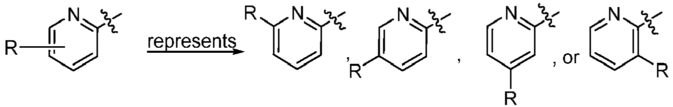

Those skilled in the art will recognize that that if more than one such substituent is present for a given ring, the bonding of each substituent is independent of all of the others. The groups listed or illustrated above are not exhaustive.

As used herein, the term "or" means "and/or" unless stated otherwise.

As used herein, the terms "including", "containing" and "comprising" are used in their open, non-limiting sense.

As used herein, the term "composition" is intended to encompass a product comprising the specified ingredients in the specified amounts, as well as any product which results, directly or indirectly, from combination of the specified ingredients in the specified amounts.

As used herein, the term "treat", "treating", or "treatment" of any disease, condition, syndrome or disorder refers, in one embodiment, to ameliorating the disease, condition, syndrome or disorder (i.e. slowing or arresting or reducing the development of the disease or at least one of the clinical symptoms thereof). In another embodiment, "treat", "treating", or "treatment" refers to alleviating or ameliorating at least one physiological or biochemical parameter associated with or causative of the disease, condition, syndrome or disorder, including those which may not be discernible by the patient. In a further embodiment, "treat", "treating", or "treatment" refers to modulating the disease, condition, syndrome or disorder either physically (*e.g.* stabilization of a discernible symptom), physiologically, (*e.g.* stabilization of a physical parameter), or both. In yet another embodiment, "treat", "treating", or "treatment" refers to preventing or delaying the onset or development or progression of the disease, condition, syndrome or disorder.

The terms "subject" and "patient" are used interchangeably herein and may refer to an animal, preferably a mammal, most preferably a human.

As used herein, the terms active compound, pharmaceutical agent and active ingredient are used interchangeably to refer to a pharmaceutically active compound. Other ingredients in a drug composition, such as carriers, diluents or excipients, may be substantially or completely pharmaceutically inert. A pharmaceutical composition (also referred to herein as a composition or formulation) may comprise the active ingredient in combination with one or more carriers and/or one or more excipients and/or one or more diluents.

The term "therapeutically effective amount" (used interchangeably herein with "effective amount") refers to an amount (e.g., of an active compound or pharmaceutical agent, such as a compound of the present invention), which elicits the biological or medicinal response in a tissue system, animal or human that is being sought by a researcher, veterinarian, medical doctor or other clinician, including reduction or inhibition of an enzyme or a protein activity, or ameliorating symptoms, alleviating conditions, slowing or delaying disease progression, or preventing a disease. Stated another way, the term therapeutically effective amount may refer to an amount that, when administered to a particular subject, achieves a therapeutic effect by inhibiting, alleviating or curing a disease, condition, syndrome or disorder in the subject or by prophylactically inhibiting, preventing or delaying the onset of a disease, condition, syndrome or disorder, or symptom(s) thereof. A therapeutically effective amount may be an amount which relieves to some extent one or more symptoms of a disease, condition, syndrome or disorder in a subject; and/or returns to normal either partially or completely one or more physiological or biochemical parameters associated with or causative of the disease, condition, syndrome or disorder; and/or reduces the likelihood of the onset of the disease, condition, syndrome or disorder, or symptom(s) thereof.

"Pharmaceutically acceptable" means that, which is useful in preparing a pharmaceutical composition that is generally safe, non-toxic and neither biologically nor otherwise undesirable and includes that which is acceptable for veterinary as well as human pharmaceutical use.

A "pharmaceutically acceptable salt" is intended to mean a salt of an acid or base of a compound represented by Formula (I) (as well as compounds of Formula (IA), (IB), and (IC)) that is non-toxic, biologically tolerable, or otherwise biologically suitable for administration to the subject. See, generally, S.M. Berge, et al., "Pharmaceutical Salts", J. Pharm. Sci., 1977, 66:1-19, and Handbook of Pharmaceutical Salts, Properties, Selection, and Use, Stahl and Wermuth, Eds., Wiley-VCH and VHCA, Zurich, 2002. Preferred pharmaceutically acceptable salts are those that are pharmacologically effective and suitable for contact with the tissues of patients without undue toxicity, irritation, or allergic response.

Non-limiting examples of pharmaceutically acceptable salts include sulfates, pyrosulfates, bisulfates, sulfites, bisulfites, phosphates, monohydrogen-phosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, γ-hydroxybutyrates, glycolates, tartrates, methane-sulfonates, propanesulfonates, naphthalene-1-sulfonates, naphthalene-2-sulfonates, and mandelates.

A compound of Formula (I) may possess a sufficiently acidic group, a sufficiently basic group, or both types of functional groups, and accordingly react with a number of inorganic or organic bases, and inorganic and organic acids, to form a pharmaceutically acceptable salt.

Compounds of Formula (I) may contain at least one nitrogen of basic character, so desired pharmaceutically acceptable salts may be prepared by any suitable method available in the art, for example, treatment of the free base with an inorganic acid, such as hydrochloric acid, hydrobromic acid, sulfuric acid, sulfamic acid, nitric acid, boric acid, phosphoric acid, and the like, or with an organic acid, such as acetic acid, phenylacetic acid, propionic acid, stearic acid, lactic acid, ascorbic acid, maleic acid, hydroxymaleic acid, isethionic acid, succinic acid, valeric acid, fumaric acid, malonic acid, pyruvic acid, oxalic acid, glycolic acid, salicylic acid, oleic acid, palmitic acid, lauric acid, a pyranosidyl acid, such as glucuronic acid or galacturonic acid, an alpha-hydroxy acid, such as mandelic acid, citric acid, or tartaric acid, an amino acid, such as aspartic acid or glutamic acid, an aromatic acid, such as benzoic acid, 2-acetoxybenzoic acid, naphthoic acid, or cinnamic acid, a sulfonic acid, such as laurylsulfonic acid, p-toluenesulfonic acid, methane sulfonic acid, ethanesulfonic acid, any compatible mixture of acids such as those given as examples herein, and any other acid and mixture thereof that are regarded as equivalents.

Compounds of Formula (I) may contain a carboxylic acid moiety, a desired pharmaceutically acceptable salt may be prepared by any suitable method, for example, treatment of the free acid with an inorganic or organic base, such as an amine (primary, secondary or tertiary), an alkali metal hydroxide, alkaline earth metal hydroxide, any compatible mixture of bases such as those given as examples herein, and any other base and mixture thereof that are regarded as equivalents or acceptable substitutes in light of the ordinary level of skill in this technology. Illustrative examples of suitable salts include organic salts derived from amino acids, such as glycine and arginine, ammonia, carbonates, bicarbonates, primary, secondary, and tertiary amines, and cyclic amines, such as benzylamines, pyrrolidines, piperidine, morpholine, piperazine, N-methylglucamine and tromethamine and inorganic salts derived from sodium, calcium, potassium, magnesium, manganese, iron, copper, zinc, aluminum, and lithium.

Each compound used herein may be discussed interchangeably with respect to its chemical formula, chemical name, abbreviation, etc.

Any formula given herein is intended to represent compounds having structures depicted by the structural formula as well as certain variations or forms. In particular, compounds of any formula given herein may have asymmetric centers and therefore exist in different enantiomeric forms. All optical isomers and stereoisomers of the compounds of the general formula, and mixtures thereof, are considered within the scope of such formula. The compounds of this invention may possess one or more asymmetric centers; such compounds can therefore be produced as individual (*R*)- or (*S*)-stereoisomers or as mixtures thereof. Thus, any formula given herein is intended to represent a racemate, one or more of its enantiomeric forms, one or more of its diastereomeric forms, and mixtures thereof. Additionally, any formula given herein is intended to refer also to any one of hydrates, solvates, polymorphs and of such compounds, and mixtures thereof, even if such forms are not listed explicitly.

The term "R" at a stereocenter designates that the stereocenter is purely of the *R-*configuration as defined in the art; likewise, the term "S" means that the stereocenter is purely of the *S*-configuration. As used herein, the term "RS" refers to a stereocenter that exists as a mixture of the *R*- and *S*-configurations.

Compounds containing one stereocenter drawn without a stereo bond designation are a mixture of 2 enantiomers. Compounds containing 2 stereocenters both drawn without stereo bond designations are a mixture of 4 diastereomers. Compounds with 2 stereocenters both labeled "RS" and drawn with stereo bond designations are a 2-component mixture with relative stereochemistry as drawn. Unlabeled stereocenters drawn without stereo bond designations are a mixture of the *R-* and *S*-configurations. For unlabeled stereocenters drawn with stereo bond designations, the absolute stereochemistry is as depicted.

Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art.

Reference to a compound herein stands for a reference to any one of: (a) the recited form of such compound, and (b) any of the forms of such compound in the medium in which the compound is being considered when named. For example, reference herein to a compound such as R-COOH, encompasses reference to any one of, for example, R-COOH(s), R-COOH(sol), and R-COO-(sol). In this example, R-COOH(s) refers to the solid compound, as it could be for example in a tablet or some other solid pharmaceutical composition or preparation; R-COOH(sol) refers to the undissociated form of the compound in a solvent; and R-COO-(sol) refers to the dissociated form of the compound in a solvent, such as the dissociated form of the compound in an aqueous environment, whether such dissociated form derives from R-COOH, from a salt thereof, or from any other entity that yields R-COO- upon dissociation in the medium being considered. In another example, an expression such as "exposing an entity to compound of formula R-COOH" refers to the exposure of such entity to the form, or forms, of the compound R-COOH that exists, or exist, in the medium in which such exposure takes place. In still another example, an expression such as "reacting an entity with a compound of formula R-COOH" refers to the reacting of (a) such entity in the chemically relevant form, or forms, of such entity that exists, or exist, in the medium in which such reacting takes place, with (b) the chemically relevant form, or forms, of the compound R-COOH that exists, or exist, in the medium in which such reacting takes place. In this regard, if such entity is for example in an aqueous environment, it is understood that the compound R-COOH is in such same medium, and therefore the entity is being exposed to species such as R-COOH(aq) and/or R-COO-(aq), where the subscript "(aq)" stands for "aqueous" according to its conventional meaning in chemistry and biochemistry. A carboxylic acid functional group has been chosen in these nomenclature examples; this choice is not intended, however, as a limitation but it is merely an illustration. It is understood that analogous examples can be provided in terms of other functional groups, including but not limited to hydroxyl, basic nitrogen members, such as those in amines, and any other group that interacts or transforms according to known manners in the medium that contains the compound. Such interactions and transformations include, but are not limited to, dissociation, association, tautomerism, solvolysis, including hydrolysis, solvation, including hydration, protonation, and deprotonation. No further examples in this regard are provided herein because these interactions and transformations in a given medium are known by any one of ordinary skill in the art.

Any formula given herein is also intended to represent unlabeled forms as well as isotopically labeled forms of the compounds. Isotopically labeled compounds have structures depicted by the formulas given herein except that one or more atoms are replaced by an atom having a selected atomic mass or mass number in an enriched form. Examples of isotopes that can be incorporated into compounds of the invention in a form that exceeds natural abundances include isotopes of hydrogen, carbon, nitrogen, oxygen, phosphorous, fluorine, chlorine, and iodine, such as ²H (or chemical symbol D), ³H (or chemical symbol T), ¹¹C, ¹³C, ¹⁴C, ¹⁵N, ¹⁸O, ¹⁷O, ³¹P, ³²P, ³⁵S, ¹⁸F, ³⁶Cl, and ¹²⁵I, respectively. Such isotopically labelled compounds are useful in metabolic studies (preferably with ¹⁴C), reaction kinetic studies (with, for example ²H or ³H), detection or imaging techniques [such as positron emission tomography (PET) or single-photon emission computed tomography (SPECT)] including drug or substrate tissue distribution assays, or in radioactive treatment of patients. In particular, an ¹⁸F or ¹¹C labeled compound may be particularly preferred for PET or SPECT studies. Further, substitution with heavier isotopes such as deuterium (i.e., ²H, or D) may afford certain therapeutic advantages resulting from greater metabolic stability, for example increased *in vivo* half-life or reduced dosage requirements. Isotopically labeled compounds of this invention can generally be prepared by carrying out the procedures disclosed in the schemes or in the examples and preparations described below by substituting a readily available isotopically labeled reagent for a non-isotopically labeled reagent.

The term Cₙ₋ₘ alkyl refers to an aliphatic chain, whether straight or branched, with a total number N of carbon members in the chain that satisfies n ≤ N ≤ m, with m > n.

When the same plurality of substituents is assigned to various groups, the specific individual substituent assignment to each of such groups is meant to be independently made with respect to the specific individual substituent assignments to the remaining groups. By way of illustration, but not as a limitation, if each of groups Q and R can be H or F, the choice of H or F for Q is made independently of the choice of H or F for R, so the choice of assignment for Q does not determine or condition the choice of assignment for R, or vice-versa, unless it is expressly indicated otherwise. Illustrative claim recitation in this regard would read as "each of Q and R is independently H or F", or "each of Q and R is independently selected from the group consisting of H and F".

In another example, a zwitterionic compound would be encompassed herein by referring to a compound that is known to form a zwitterion, even if it is not explicitly named in its zwitterionic form. Terms such as zwitterion, zwitterions, and their synonyms zwitterionic compound(s) are standard IUPAC-endorsed names that are well known and part of standard sets of defined scientific names. In this regard, the name zwitterion is assigned the name identification CHEBI:27369 by the Chemical Entities of Biological Interest (ChEBI) dictionary of molecular entities. As generally well known, a zwitterion or zwitterionic compound is a neutral compound that has formal unit charges of opposite sign. Sometimes these compounds are referred to by the term "inner salts". Other sources refer to these compounds as "dipolar ions", although the latter term is regarded by still other sources as a misnomer. As a specific example, aminoethanoic acid (the amino acid glycine) has the formula H₂NCH₂COOH, and it exists in some media (in this case in neutral media) in the form of the zwitterion ⁺H₃NCH₂COO⁻. Zwitterions, zwitterionic compounds, inner salts and dipolar ions in the known and well-established meanings of these terms are within the scope of this invention, as would in any case be so appreciated by those of ordinary skill in the art. Because there is no need to name each and every embodiment that would be recognized by those of ordinary skill in the art, no structures of the zwitterionic compounds that are associated with the compounds of this invention are given explicitly herein. They are, however, part of the embodiments of this invention. No further examples in this regard are provided herein because the interactions and transformations in a given medium that lead to the various forms of a given compound are known by any one of ordinary skill in the art.

When referring to any formula given herein, the selection of a particular moiety from a list of possible species for a specified variable is not intended to define the same choice of the species for the variable appearing elsewhere. In other words, where a variable appears more than once, the choice of the species from a specified list is independent of the choice of the species for the same variable elsewhere in the formula, unless stated otherwise.

By way of a first example on substituent terminology, if substituent S¹ₑₓₐₘₚₗₑ is one of S₁ and S₂, and substituent S²ₑₓₐₘₚₗₑ is one of S₃ and S₄, then these assignments refer to embodiments of this invention given according to the choices S¹ₑₓₐₘₚₗₑ is S₁ and S²ₑₓₐₘₚₗₑ is S₃; S¹ₑₓₐₘₚₗₑ is S₁ and S²ₑₓₐₘₚₗₑ is S₄; S¹ₑₓₐₘₚₗₑ is S₂ and S²ₑₓₐₘₚₗₑ is S₃; S¹ₑₓₐₘₚₗₑ is S₂ and S²ₑₓₐₘₚₗₑ is S₄; and equivalents of each one of such choices. The shorter terminology "S¹ₑₓₐₘₚₗₑ is one of S₁ and S₂, and S²ₑₓₐₘₚₗₑ is one of S₃ and S₄" is accordingly used herein for the sake of brevity, but not by way of limitation. The foregoing first example on substituent terminology, which is stated in generic terms, is meant to illustrate the various substituent assignments described herein.

Furthermore, when more than one assignment is given for any member or substituent, embodiments of this invention comprise the various groupings that can be made from the listed assignments, taken independently, and equivalents thereof. By way of a second example on substituent terminology, if it is herein described that substituent Sₑₓₐₘₚₗₑ is one of S₁, S₂, and S₃, this listing refers to embodiments of this invention for which Sₑₓₐₘₚₗₑ is S₁; Sₑₓₐₘₚₗₑ is S₂; Sₑₓₐₘₚₗₑ is S₃; Sₑₓₐₘₚₗₑ is one of S₁ and S₂; Sₑₓₐₘₚₗₑ is one of S₁ and S₃; Sₑₓₐₘₚₗₑ is one of S₂ and S₃; Sₑₓₐₘₚₗₑ is one of S₁, S₂ and S₃; and Sₑₓₐₘₚₗₑ is any equivalent of each one of these choices. The shorter terminology "Sₑₓₐₘₚₗₑ is one of S₁, S₂, and S₃" is accordingly used herein for the sake of brevity, but not by way of limitation. The foregoing second example on substituent terminology, which is stated in generic terms, is meant to illustrate the various substituent assignments described herein.

The nomenclature "Cᵢ-Cⱼ" with j > i, when applied herein to a class of substituents, is meant to refer to embodiments of this invention for which each and every one of the number of carbon members, from i to j including i and j, is independently realized. By way of example, the term C₁-C₃ refers independently to embodiments that have one carbon member (C₁), embodiments that have two carbon members (C₂), and embodiments that have three carbon members (C₃).

Embodiments of this invention include compounds of Formula (I), 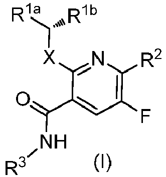 wherein
X is selected from the group consisting of: O, S and NR^{a};
   when X is NR^{a}; R^{a} is H or CH₃; or R^{a} and R^{1a} come together to form a heterocyclic ring selected from the group consisting of: 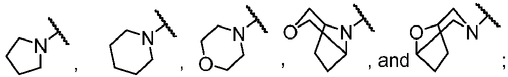 wherein the heterocyclic ring is substituted with one or two members each independently selected from the group consisting of: C₁₋₆alkyl, C₁₋₆haloalkyl, and OC₁₋₆alkyl;
R^{1a} is selected from the group consisting of: C₁₋₆alkyl; C₁₋₆alkyl substituted with OH, or OCH₃; C₁₋₆haloalkyl; C₁₋₆haloalkyl substituted with OH, or OCH₃; and C₃₋₆cycloalkyl;
R^{1b} is CH₃ or CHF₂; or R^{1a} and R^{1b} come together to form C₃₋₆cycloalkyl; C₃₋₆cycloalkyl independently substituted with one, two, three or four members each independently selected from the group consisting of: halo, OH, C₁₋₆alkyl, and C₁₋₆haloalkyl; oxetanyl; tetrahydrofuranyl; and tetrahydropyranyl;
R² is 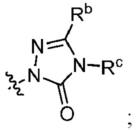 where
   R^{b} is C₁₋₆alkyl substituted with a member selected from the group consisting of: OH, halo, CN, OC₁₋₆alkyl, OC₁₋₆haloalkyl and OC₃₋₆cycloalkyl;
   R^{c} is selected from the group consisting of: C₁₋₆alkyl, C₁₋₆alkyl substituted with OH, allyl, C₁₋₆haloalkyl, and C₃₋₆cycloalkyl; and
R³ is selected from the group consisting of: 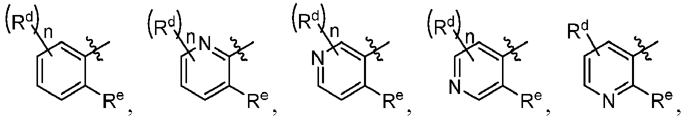 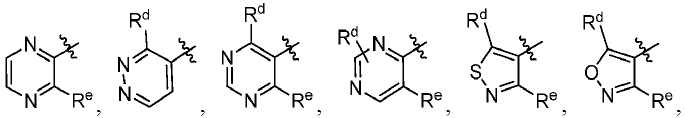 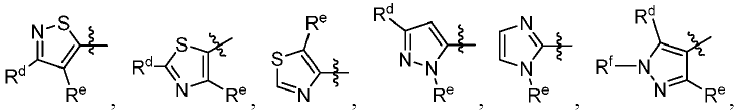 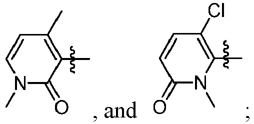
   R^{d} is selected from the group consisting of: H; halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; N(CH₃)₂; OH; CN and OC₁₋₆alkyl;
   R^{e} is selected from the group consisting of: halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; OH; OC₁₋₆alkyl; and C₃₋₆cycloalkyl;
   R^{f} is selected from the group consisting of: H; C₁₋₆alkyl; C₁₋₆alkyl substituted with a member selected from the group consisting of: OH, OCH₃, SCH₃, and OCF₃; C₁₋₆haloalkyl; and C₁₋₆haloalkyl substituted with a member selected from the group consisting of: OH, and OCH₃; and
   n is 1, or 2;
or pharmaceutically acceptable salts, isotopes, tautomers, N-oxides, solvates, or stereoisomers thereof.

An additional embodiment of the invention is a compound of Formula (I) wherein XisO.

An additional embodiment of the invention is a compound of Formula (I) wherein X is S.

An additional embodiment of the invention is a compound of Formula (I) wherein X is NR^{a}, and R^{a} is H.

An additional embodiment of the invention is a compound of Formula (I) wherein X is NR^{a}, and R^{a} is CH₃.

An additional embodiment of the invention is a compound of Formula (I) wherein X is NR^{a}, where R^{a} and R^{1a} come together to form a heterocyclic ring selected from: 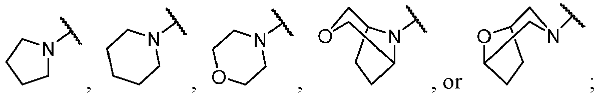 wherein the heterocyclic ring is substituted with one or two members each independently selected from the group consisting of: C₁₋₄alkyl, C₁₋₄haloalkyl, and OC₁₋₄alkyl.

An additional embodiment of the invention is a compound of Formula (I) wherein R^{1a} is C₁₋₄alkyl; C₁₋₄alkyl substituted with OH, or OCH₃; C₁₋₄haloalkyl; C₁₋₄haloalkyl substituted with OH, or OCH₃; or C₃₋₆cycloalkyl.

An additional embodiment of the invention is a compound of Formula (I) wherein R^{1a} is CF₃.

An additional embodiment of the invention is a compound of Formula (I) wherein R^{1b} is CH₃ or CHF₂.

An additional embodiment of the invention is a compound of Formula (I) wherein R^{1b} is CH₃.

An additional embodiment of the invention is a compound of Formula (I) wherein R^{1a} and R^{1b} come together to form cyclopropyl, cyclobutyl, cyclopentyl, or cyclohexyl; cyclopropyl, cyclobutyl, cyclopentyl, or cyclohexyl each independently substituted with one, two, three or four members selected from the group consisting of: halo, OH, C₁₋₄alkyl, and C₁₋₄haloalkyl; oxetanyl; tetrahydrofuranyl; and tetrahydropyranyl.

An additional embodiment of the invention is a compound of Formula (I) wherein R² is 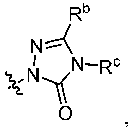 where
R^{b} is C₁₋₄alkyl substituted with OH, halo, CN, OC₁₋₄alkyl, OC₁₋₄haloalkyl or OC₃₋₆cycloalkyl; and
R^{c} is C₁₋₄alkyl, C₁₋₄haloalkyl, or C₃₋₆cycloalkyl.

An additional embodiment of the invention is a compound of Formula (I) wherein R² is

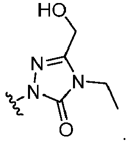

An additional embodiment of the invention is a compound of Formula (I) wherein R³ is 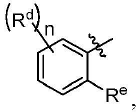 where
R^{d} is H; halo; C₁₋₄alkyl; C₁₋₄alkyl substituted with OH, OCH₃, SCH₃, or OCF₃; C₁₋₄haloalkyl; C₁₋₄haloalkyl substituted with OH, or OCH₃; CN; or OC₁₋₄alkyl;
R^{e} is halo; C₁₋₄alkyl; C₁₋₄alkyl substituted with OH, OCH₃, SCH₃, or OCF₃; C₁₋₄haloalkyl; or C₁₋₄haloalkyl substituted with OH, or OCH₃; and
n is 1 or 2.

An additional embodiment of the invention is a compound of Formula (I) wherein R³ is:

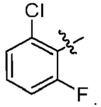

An additional embodiment of the invention is a compound of Formula (I) wherein R³ is: 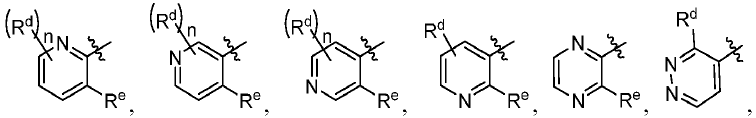 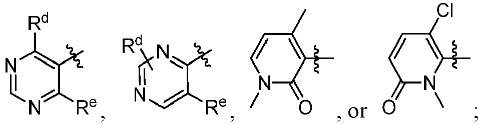 where
R^{d} is H; halo; C₁₋₄alkyl; C₁₋₄alkyl substituted with OH, OCH₃, SCH₃, or OCF₃; C₁₋₄haloalkyl; C₁₋₄haloalkyl substituted with OH, or OCH₃; N(CH₃)₂; OH; CN; or OC₁₋₄alkyl;
R^{e} is halo; OH; C₁₋₄alkyl; C₁₋₄alkyl substituted with OH, OCH₃, SCH₃, or OCF₃; C₁₋₄haloalkyl; OC₁₋₄alkyl; or C₁₋₄haloalkyl substituted with OH, or OCH₃; and n is 1 or 2.

An additional embodiment of the invention is a compound of Formula (I) wherein R³ is 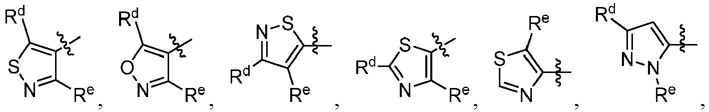 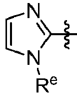 , where
R^{d} is H; halo; C₁₋₄alkyl; C₁₋₄alkyl substituted with OH, OCH₃, SCH₃, or OCF₃; C₁₋₄haloalkyl; C₁₋₄haloalkyl substituted with OH, or OCH₃; or OC₁₋₄alkyl;
R^{e} is halo; C₁₋₄alkyl; C₁₋₄alkyl substituted with OH, OCH₃, SCH₃, or OCF₃; C₁₋₄haloalkyl; C₃₋₆cycloalkyl; or C₁₋₄haloalkyl substituted with OH, or OCH₃; and
R^{f} is H; C₁₋₄alkyl; C₁₋₄alkyl substituted with OH, OCH₃, SCH₃, or OCF₃; C₁₋₄haloalkyl; or C₁₋₄haloalkyl substituted with OH, or OCH₃.

An additional embodiment of the current invention is a compound selected from the compounds shown below in Table 1, and pharmaceutically acceptable salts, isotopes, N-oxides, solvates, and stereoisomers thereof:

**Table 1**

| Example # | Compound Name |
|---|---|
| 1 | (S)-N-(2-Chloro-6-fluorophenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 2 | (S)-6-(4-Ethyl-3-(hydroxymethyl)-5-oxo-4,5-dihydro-1H-1,2,4-triazol-1-yl)-5-fluoro-N-(2-fluorophenyl)-2-((1,1,1-trifluoropropan-2-yl)oxy)nicotinamide; |
| 3 | (S)-N-(2-Chloro-6-fluorophenyl)-6-(4-ethyl-3-(fluoromethyl)-5-oxo-4,5-dihydro-1H-1,2,4-triazol-1-yl)-5-fluoro-2-((1,1,1-trifluoropropan-2-yl)oxy)nicotinamide; |
| 4 | (S)-N-(2-Chloropyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 5 | (S)-N-(2-Chloro-4-methylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 6 | (S)-N-(2-Chloro-6-fluorophenyl)-6-(3-(chloromethyl)-4-ethyl-5-oxo-4,5-dihydro-1H-1,2,4-triazol-1-yl)-5-fluoro-2-((1,1,1-trifluoropropan-2-yl)oxy)nicotinamide; |
| 7 | N-(2-Chloro-6-fluorophenyl)-6-(4-ethyl-3-(hydroxymethyl)-5-oxo-4,5-dihydro-1H-1,2,4-triazol-1-yl)-5-fluoro-2-isopropoxynicotinamide; |
| 8 | N-(2-Chloro-6-fluorophenyl)-6-(4-ethyl-3-(hydroxymethyl)-5-oxo-4,5-dihydro-1H-1,2,4-triazol-1-yl)-5-fluoro-2-(2,2,3,3-tetrafluorocyclobutoxy)nicotinamide; |
| 9 | Racemic-N-(2-Chloro-6-fluorophenyl)-6-(4-ethyl-3-(hydroxymethyl)-5-oxo-4,5-dihydro-1H-1,2,4-triazol-1-yl)-5-fluoro-2-((tetrahydrofuran-3-yl)oxy)nicotinamide; |
| 10 | (S)-N-(3-Chloropyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 11 | (S)-6-(4-Ethyl-3-(hydroxymethyl)-5-oxo-4,5-dihydro-1H-1,2,4-triazol-1-yl)-5-fluoro-N-(2-fluoro-5-methylphenyl)-2-((1,1,1-trifluoropropan-2-yl)oxy)nicotinamide; |
| 13 | (S)-N-(2-(Difluoromethyl)-4-methylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 14 | (S)-N-(2-Chloro-6-fluorophenyl)-6-(4-ethyl-3-(methoxymethyl)-5-oxo-4,5-dihydro-1H-1,2,4-triazol-1-yl)-5-fluoro-2-((1,1,1-trifluoropropan-2-yl)oxy)nicotinamide; |
| 16 | (S)-N-(3-Chloropyridazin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 17 | (S)-6-(4-Ethyl-3-(hydroxymethyl)-5-oxo-4,5-dihydro-1H-1,2,4-triazol-1-yl)-5-fluoro-N-(3-methylisothiazol-4-yl)-2-((1,1,1-trifluoropropan-2-yl)oxy)nicotinamide; |
| 18 | (S)-N-(2,4-Dimethylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 19 | (S)-N-(3-Chloropyrazin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 21 | (S)-N-(3-chloro-6-methoxypyridin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 24 | (S)-N-(3-Chloro-2-methoxypyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 25 | (S)-N-(5-Chloro-3-methyl-1H-pyrazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 26 | (S)-N-(4-Chloro-3-methylisothiazol-5-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 27 | (S)-N-(4-Chloropyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 28 | (S)-N-(3-Chloro-5-fluoropyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 29 | (S)-N-(3-Cyano-5-fluoropyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 30 | (S)-N-(3-Chloro-5-fluoro-2-methoxypyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 31 | (S)-N-(4-Bromo-3-methylisothiazol-5-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 32 | (S)-6-(4-Ethyl-3-(hydroxymethyl)-5-oxo-4,5-dihydro-1H-1,2,4-triazol-1-yl)-5-fluoro-N-(5-methylthiazol-4-yl)-2-((1,1,1-trifluoropropan-2-yl)oxy)nicotinamide; |
| 33 | (S)-N-(5-Chloro-2-methoxypyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 34 | (S)-6-(4-Ethyl-3-(hydroxymethyl)-5-oxo-4,5-dihydro-1H-1,2,4-triazol-1-yl)-5-fluoro-N-(3-methoxypyrazin-2-yl)-2-((1,1,1-trifluoropropan-2-yl)oxy)nicotinamide; |
| 35 | (S)-N-(3,5-Dimethylisothiazol-4-yl)-6-(4-ethyl-3-(hydroxymethyl)-5-oxo-4,5-dihydro-1H-1,2,4-triazol-1-yl)-5-fluoro-2-((1, 1, 1-trifluoropropan-2-yl)oxy)nicotinamide; |
| 36 | (S)-6-(4-Ethyl-3-(hydroxymethyl)-5-oxo-4,5-dihydro-1H-1,2,4-triazol-1-yl)-5-fluoro-N-(2-fluoro-5-methylpyridin-3-yl)-2-((1,1,1-trifluoropropan-2-yl)oxy)nicotinamide; |
| 37 | (S)-6-(4-Ethyl-3-(hydroxymethyl)-5-oxo-4,5-dihydro-1H-1,2,4-triazol-1-yl)-5-fluoro-N-(5-fluoro-2-methylpyridin-4-yl)-2-((1,1,1-trifluoropropan-2-yl)oxy)nicotinamide; |
| 38 | (S)-N-(4-Chloro-6-methylpyrimidin-5-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 39 | (S)-6-(4-Ethyl-3-(hydroxymethyl)-5-oxo-4,5-dihydro-1H-1,2,4-triazol-1-yl)-5-fluoro-N-(3-fluoro-6-methylpyridin-2-yl)-2-((1,1,1-trifluoropropan-2-yl)oxy)nicotinamide; |
| 40 | (S)-N-(3-Chloro-2-methoxy-5-methylpyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 41 | (S)-6-(4-Ethyl-3-(hydroxymethyl)-5-oxo-4,5-dihydro-1H-1,2,4-triazol-1-yl)-5-fluoro-N-(2-methoxy-5-methylpyridin-3-yl)-2-((1,1,1-trifluoropropan-2-yl)oxy)nicotinamide; |
| 42 | (S)-6-(4-Ethyl-3-(hydroxymethyl)-5-oxo-4,5-dihydro-1H-1,2,4-triazol-1-yl)-5-fluoro-N-(2-fluoro-4-methylpyridin-3-yl)-2-((1,1,1-trifluoropropan-2-yl)oxy)nicotinamide; |
| 43 | (S)-N-(5,6-Dimethylpyrimidin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 44 | (S)-N-(2-Chloro-4-methoxypyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 45 | (S)-6-(4-Ethyl-3-(hydroxymethyl)-5-oxo-4,5-dihydro-1H-1,2,4-triazol-1-yl)-5-fluoro-N-(3-methylisoxazol-4-yl)-2-((1,1,1-trifluoropropan-2-yl)oxy)nicotinamide; |
| 46 | (S)-N-(3,5-Dimethylisoxazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 47 | (S)-6-(4-Ethyl-3-(hydroxymethyl)-5-oxo-4,5-dihydro-1H-1,2,4-triazol-1-yl)-5-fluoro-N-(2-fluoro-5-methoxyphenyl)-2-((1,1,1-trifluoropropan-2-yl)oxy)nicotinamide; |
| 48 | (S)-N-(2-Chloro-5-methylphenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 49 | (S)-N-(3,5-Dimethyl-1H-pyrazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 50 | (S)-N-(4-Chloro-6-methoxypyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 51 | (S)-N-(2,5-Dimethylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 52 | (S)-N-(5-Chloro-2-methoxypyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 53 | N-(1,4-Dimethyl-2-oxo-2,3-dihydro-1l4-pyridin-3-yl)-6-(4-ethyl-3-(hydroxymethyl)-5-oxo-4, 5-dihydro-1H-1,2,4-triazol-1-yl)-5-fluoro-2-(((S)-1,1,1-trifluoropropan-2-yl)oxy)nicotinamide; |
| 54 | (S)-N-(3-Chloropyridin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 55 | (S)-6-(4-Ethyl-3-(hydroxymethyl)-5-oxo-4,5-dihydro-1H-1,2,4-triazol-1-yl)-5-fluoro-2-((1,1,1-trifluoropropan-2-yl)oxy)-N-(1,3,5-trimethyl-1H-pyrazol-4-yl)nicotinamide; |
| 56 | (S)-6-(4-Ethyl-3-(hydroxymethyl)-5-oxo-4,5-dihydro-1H-1,2,4-triazol-1-yl)-5-fluoro-N-(3-fluoro-6-methoxypyridin-2-yl)-2-((1,1,1-trifluoropropan-2-yl)oxy)nicotinamide; |
| 57 | (S)-6-(4-Ethyl-3-(hydroxymethyl)-5-oxo-4, 5-dihydro-1H-1,2,4-triazol-1-yl)-5-fluoro-N-(3-methyl-1H-pyrazol-4-yl)-2-((1,1,1-trifluoropropan-2-yl)oxy)nicotinamide; |
| 58 | (S)-6-(4-Ethyl-3-(hydroxymethyl)-5-oxo-4,5-dihydro-1H-1,2,4-triazol-1-yl)-5-fluoro-N-(3-methoxy-1H-pyrazol-4-yl)-2-((1,1,1-trifluoropropan-2-yl)oxy)nicotinamide; |
| 59 | (S)-6-(4-Ethyl-3-(hydroxymethyl)-5-oxo-4, 5-dihydro-1H-1,2,4-triazol-1-yl)-5-fluoro-N-(3-isopropyl-1-methyl-1H-pyrazol-5-yl)-2-((1,1,1-trifluoropropan-2-yl)oxy)nicotinamide; |
| 60 | (S)-N-(5-Chloro-6-methylpyrimidin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 61 | (S)-6-(4-Ethyl-3-(hydroxymethyl)-5-oxo-4,5-dihydro-1H-1,2,4-triazol-1-yl)-5-fluoro-N-(3-(methoxymethyl)-1-methyl-1H-pyrazol-4-yl)-2-((1,1,1-trifluoropropan-2-yl)oxy)nicotinamide; |
| 62 | (S)-N-(3-Cyclopropyl-1H-pyrazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 64 | (S)-N-(3-Chloro-1-methyl-6-oxo-1,6-dihydropyridin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 65 | (S)-6-(4-Ethyl-3-(hydroxymethyl)-5-oxo-4,5-dihydro-1H-1,2,4-triazol-1-yl)-5-fluoro-N-(5-fluoro-2-methoxypyridin-4-yl)-2-((1,1,1-trifluoropropan-2-yl)oxy)nicotinamide; |
| 66 | (S)-6-(4-ethyl-3-(hydroxymethyl)-5-oxo-4,5-dihydro-1H-1,2,4-triazol-1-yl)-5-fluoro-N-(2-methoxy-5-methylpyridin-4-yl)-2-((1,1,1-trifluoropropan-2-yl)oxy)nicotinamide; |
| 67 | (S)-N-(2-Chloro-5-methylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 68 | (S)-6-(4-Ethyl-3-(hydroxymethyl)-5-oxo-4,5-dihydro-1H-1,2,4-triazol-1-yl)-5-fluoro-N-(2-methoxy-4-methylpyridin-3-yl)-2-((1,1,1-trifluoropropan-2-yl)oxy)nicotinamide; |
| 69 | (S)-N-(2,4-Dichloropyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 70 | (S)-N-(2-Ethoxy-5-fluoropyrimidin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 71 | (S)-6-(4-Ethyl-3-(hydroxymethyl)-5-oxo-4,5-dihydro-1H-1,2,4-triazol-1-yl)-5-fluoro-N-(3-fluoro-5-methylpyridin-4-yl)-2-((1,1,1-trifluoropropan-2-yl)oxy)nicotinamide; |
| 72 | (S)-N-(2-Chloro-4,6-dimethylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 73 | (S)-N-(1-Cyclopropyl-1H-imidazol-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 74 | (S)-N-(2-Chloro-5-cyanophenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 75 | (S)-N-(2-Chloro-5-cyanopyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 76 | (S)-N-(4-(Dimethylamino)-2-methoxypyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; |
| 77 | N-(2-Chloro-6-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 78 | N-(2-Chloro-5-fluoro-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 79 | N-(4-Chloro-2-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 80 | N-(3-Chloro-1-methyl-pyrazol-4-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3- carboxamide; |
| 81 | N-[2-(Difluoromethyl)-5-fluoro-4-pyridyl]-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 82 | 6-[4-Ethyl-3-(hydroxymethyl)-5-oxo-1,2,4-triazol-1-yl]-5-fluoro-N-(5-fluoro-2-methoxy-pyrimidin-4-yl)-2-[(1S)-2,2,2-trifluoro-1-methyl-ethoxy]pyridine-3-carboxamide; |
| 83 | N-(6-Chloro-2,3-dimethyl-phenyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 84 | N-(2-Chloro-4-methyl-3-pyridyl)-5-fluoro-6-[3-(hydroxymethyl)-5-oxo-4-propyl-1,2,4-triazol-1-yl]-2-[(1S)-2,2,2-trifluoro-1-methyl-ethoxy]pyridine-3-carboxamide; and |
| 85 | 6-[4-Ethyl-3-(hydroxymethyl)-5-oxo-1,2,4-triazol-1-yl]-5-fluoro-N-(2-methoxy-3,5-dimethyl-4-pyridyl)-2-[(1S)-2,2,2-trifluoro-1-methyl-ethoxy]pyridine-3-carboxamide; |
| 86 | (S)-2-(5-(((2-Chloro-4-methylpyridin-3-yl)-l2-azaneyl)carbonyl)-3-fluoro-6-((1,1,1-trifluoropropan-2-yl)oxy)pyridin-2-yl)-5-(fluoromethyl)-4-isopropyl-2,4-dihydro-3H-1,2,4-triazol-3 -one; |
| 87 | N-(2-Chloro-4-methyl-3-pyridyl)-6-[4-ethyl-3-(fluoromethyl)-5-oxo-1,2,4-triazol-1-yl]-5-fluoro-2-[(1S)-2,2,2-trifluoro-1-methyl-ethoxy]pyridine-3-carboxamide; |
| 88 | N-(3-Chloro-2-methoxy-5-methyl-4-pyridyl)-6-[4-ethyl-3-(hydroxymethyl)-5-oxo-1,2,4-triazol-1-yl]-5-fluoro-2-isopropoxy-pyridine-3-carboxamide; |
| 89 | N-(2-Chloro-4-methyl-3-pyridyl)-5-fluoro-6-[3-(hydroxymethyl)-4-isopropyl-5-oxo-1,2,4-triazol-1-yl]-2-[(1S)-2,2,2-trifluoro-1-methyl-ethoxy]pyridine-3-carboxamide; |
| 90 | N-(2-Chloro-4-methyl-3-pyridyl)-6-[4-(2,2-difluoroethyl)-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 91 | N-(5-Chloro-3-methyl-1H-pyrazol-4-yl)-5-fluoro-6-[3-(hydroxymethyl)-5-oxo-4-propyl-1,2,4-triazol-1-yl]-2-[(1S)-2,2,2-trifluoro-1-methyl-ethoxy]pyridine-3-carboxamide; |
| 93 | N-(2,4-Dimethoxy-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 94 | N-(4,6-Dimethoxypyrimidin-5-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 95 | 6-[4-Ethyl-3-(hydroxymethyl)-5-oxo-1,2,4-triazol-1-yl]-5-fluoro-2-[(1S)-2,2,2-trifluoro-1-methyl-ethoxy]-N-[3-(trifluoromethyl)-1H-pyrazol-4-yl]pyridine-3-carboxamide; |
| 96 | N-(5-Chloro-3-methyl-1H-pyrazol-4-yl)-6-[4-ethyl-3-(fluoromethyl)-5-oxo-1,2,4-triazol-1-yl]-5-fluoro-2-[(1S)-2,2,2-trifluoro-1-methyl-ethoxy]pyridine-3-carboxamide; |
| 97 | N-(2-Chloro-4-methyl-3-pyridyl)-6-[4-ethyl-3-(fluoromethyl)-5-oxo-1,2,4-triazol-1-yl]-5-fluoro-2-isopropoxy-pyridine-3-carboxamide; |
| 98 | N-(5-Chloro-2-methoxy-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 99 | N-(5-Chloro-3-methyl-1H-pyrazol-4-yl)-5-fluoro-6-[3-(fluoromethyl)-5-oxo-4-propyl-1,2,4-triazol-1-yl]-2-[(1S)-2,2,2-trifluoro-1-methyl-ethoxy]pyridine-3- carboxamide; |
| 100 | N-(3-Cyano-1H-pyrazol-4-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 101 | N-(2-Chloro-4-methyl-3-pyridyl)-5-fluoro-6-[3-(fluoromethyl)-4-(3-hydroxypropyl)-5-oxo-1,2,4-triazol-1-yl]-2-[(1S)-2,2,2-trifluoro-1-methyl-ethoxy]pyridine-3-carboxamide; |
| 102 | N-(2-chloro-4,5-dimethyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 103 | 6-[4-ethyl-3-(hydroxymethyl)-5-oxo-1,2,4-triazol-1-yl]-5-fluoro-N-(4-methoxy-2,6-dimethyl-3-pyridyl)-2-[(1S)-2,2,2-trifluoro-1-methyl-ethoxy]pyridine-3-carboxamide; |
| 104 | N-(3-Chloro-4-methoxy-2-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 105 | N-(3-Chloro-4-ethoxy-2-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 106 | N-(2-Chloro-6-methoxy-4-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 107 | N-(3-Chloro-5-methyl-1H-pyrazol-4-yl)-5-fluoro-6-[3-(fluoromethyl)-4-(3-hydroxypropyl)-5-oxo-1,2,4-triazol-1-yl]-2-[(1S)-2,2,2-trifluoro-1-methyl-ethoxy]pyridine-3-carboxamide; |
| 108 | N-(5-Chloro-3-methyl-1H-pyrazol-4-yl)-6-[4-ethyl-3-(fluoromethyl)-5-oxo-1,2,4-triazol-1-yl] -5 -fluoro-2-isopropoxy-pyridine-3 -carboxamide; |
| 109 | 6-[4-Ethyl-3-(hydroxymethyl)-5-oxo-1,2,4-triazol-1-yl]-5-fluoro-N-(3-methoxypyridazin-4-yl)-2-[(1S)-2,2,2-trifluoro-1-methyl-ethoxy]pyridine-3-carboxamide; |
| 110 | N-(5-Chloro-2-methoxy-3-methyl-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 111 | 6-[4-Ethyl-3-(hydroxymethyl)-5-oxo-1,2,4-triazol-1-yl]-5-fluoro-N-[3-methyl-5-(trifluoromethyl)-1H-pyrazol-4-yl]-2-[(1S)-2,2,2-trifluoro-1-methyl-ethoxy]pyridine -3 -carboxamide; |
| 112 | N-[3-Chloro-5-(trifluoromethyl)-1H-pyrazol-4-yl]-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 113 | N-(6-Chloro-2-methoxy-4-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 114 | 6-[4-Ethyl-3-(hydroxymethyl)-5-oxo-1,2,4-triazol-1-yl]-5-fluoro-N-(2-methoxy-4,6-dimethyl-3-pyridyl)-2-[(1S)-2,2,2-trifluoro-1-methyl-ethoxy]pyridine-3-carboxamide; |
| 115 | N-(5-Chloro-3-methyl-1H-pyrazol-4-yl)-5-fluoro-6-[3-(fluoromethyl)-4-(2-hydroxyethyl)-5-oxo-1,2,4-triazol-1-yl]-2-[(1S)-2,2,2-trifluoro-1-methyl-ethoxy]pyridine-3-carboxamide; |
| 116 | N-(2-Chloro-4-methyl-3-pyridyl)-5-fluoro-6-[3-(fluoromethyl)-4-(2-hydroxyethyl)-5-oxo-1,2,4-triazol-1-yl]-2-[(1S)-2,2,2-trifluoro-1-methyl-ethoxy]pyridine-3-carboxamide; |
| 117 | N-(3-Cyano-5-fluoro-2-methoxy-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 118 | N-(3-Chloro-5-methoxy-2-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 119 | N-(2,6-Dimethoxyphenyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 120 | 6-[4-Ethyl-3-(hydroxymethyl)-5-oxo-1,2,4-triazol-1-yl]-5-fluoro-N-(o-tolyl)-2-[(1S)-2,2,2-trifluoro-1-methyl-ethoxy]pyridine-3-carboxamide; |
| 121 | 6-[4-Ethyl-3-(hydroxymethyl)-5-oxo-1,2,4-triazol-1-yl]-5-fluoro-N-(3-methoxy-5-methyl-1H-pyrazol-4-yl)-2-[(1S)-2,2,2-trifluoro-1-methyl-ethoxy]pyridine-3-carboxamide; |
| 122 | N-(3,5-Dimethoxy-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 123 | N-(3,5-Dichloro-1H-pyrazol-4-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 124 | N- [3-(Difluoromethyl)-5-methyl-1H-pyrazol-4-yl] -6- [4-ethyl-3 - (hydroxymethyl)-5-oxo-1,2,4-triazol-1-yl]-5-fluoro-2-[(1S)-2,2,2-trifluoro-1-methyl-ethoxy]pyridine-3-carboxamide; |
| 125 | 6-[4-Ethyl-3-(hydroxymethyl)-5-oxo-1,2,4-triazol-1-yl]-5-fluoro-N-(4-fluoro-2-methoxy-3-pyridyl)-2-[(1S)-2,2,2-trifluoro-1-methyl-ethoxy]pyridine-3-carboxamide; |
| 126 | N-(4-Chloro-2-methoxy-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide; |
| 127 | (Racemic)-6-[4-Ethyl-3-(1-hydroxyethyl)-5-oxo-1,2,4-triazol-1-yl]-5-fluoro-N-[3-methyl-5-(trifluoromethyl)-1H-pyrazol-4-yl]-2-(2,2,2-trifluoro-1-methyl-ethoxy)pyridine-3-carboxamide; and |
| 128 | 6-[4-Ethyl-3-(1-hydroxy-1-methyl-ethyl)-5-oxo-1,2,4-triazol-1-yl]-5-fluoro-N-[3-methyl-5-(trifluoromethyl)-1H-pyrazol-4-yl]-2-[(1S)-2,2,2-trifluoro-1-methyl-ethoxy]pyridine-3-carboxamide. |

A further embodiment of the current invention is a compound selected from the group consisting of:
(S)-N-(2-Chloro-4-methylpyridin-3-yl)-6-(4-ethyl-3-(hydroxymethyl)-5-oxo-4,5-dihydro- 1H-1,2,4-triazol-1-yl)-5-fluoro-2-((1,1,1 -trifluoropropan-2-yl)oxy)nicotinamide;
(S)-N-(5-Chloro-3-methyl-1H-pyrazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Chloro-2-methoxy-5-methylpyridin-4-yl)-6-(4-ethyl-3-(hydroxymethyl)-5-oxo-4,5-dihydro-1H-1,2,4-triazol-1-yl)-5-fluoro-2-((1,1,1 -trifluoropropan-2-yl)oxy)nicotinamide;
(S)-6-(4-Ethyl-3-(hydroxymethyl)-5-oxo-4,5-dihydro-1H-1,2,4 triazol-1-yl)-5-fluoro-N-(2-methoxy-4-methylpyridin-3-yl)-2-(1, 1-trifluoropropan-2-yl)oxy)nicotinamide;
6-[4-Ethyl-3-(hydroxymethyl)-5-oxo-1,2,4-triazol-1-yl]-5-fluoro-N-[3-methyl-5-(trifluoromethyl)-1H-pyrazol-4-yl]-2-[(1S)-2,2,2-trifluoro-1-methyl-ethoxy]pyridine-3-carboxamide; and
6-[4-Ethyl-3-(hydroxymethyl)-5-oxo-1,2,4-triazol-1-yl]-5-fluoro-N-(2-methoxy-3,5-dimethyl-4-pyridyl)-2-[(1S)-2,2,2-trifluoro-1-methyl-ethoxy]pyridine-3-carboxamide;
or pharmaceutically acceptable salts, isotopes, tautomers, N-oxides, solvates, or stereoisomers thereof.

A further embodiment of the current invention is a compound selected from the group consisting of:
(S)-5-Fluoro-6-(3-(hydroxymethyl)-5-oxo-4,5-dihydro-1H-1,2,4-triazol-1-yl)-N-(5-methyl-3-(trifluoromethyl)-1H-pyrazol-4-yl)-2-((1,1,1 -trifluoropropan-2-yl)oxy)nicotinamide;
5-Fluoro-6-(3-(1-hydroxyethyl)-4-methyl-5-oxo-4,5-dihydro-1H-1,2,4-triazol-1-yl)-N-(5-methyl-3-(trifluoromethyl)-1H-pyrazol-4-yl)-2-(((S)-1,1,1-trifluoropropan-2-yl)oxy)nicotinamide; and
(S)-5-Fluoro-6-(3-(2-hydroxypropan-2-yl)-4-methyl-5-oxo-4,5-dihydro-1H-1,2,4-triazol-1-yl)-N-(5 -methyl-3-(trifluoromethyl)-1H-pyrazol-4-yl)-2-((1,1,1-trifluoropropan-2-yl)oxy)nicotinamide;
or pharmaceutically acceptable salts, isotopes, tautomers, N-oxides, solvates, or stereoisomers thereof.

An additional embodiment of the invention is a compound of Formula (I) wherein R² is 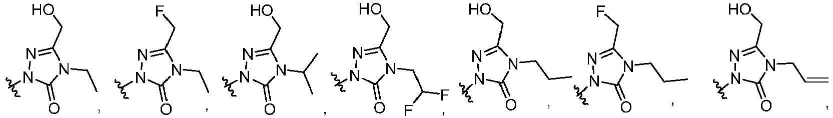 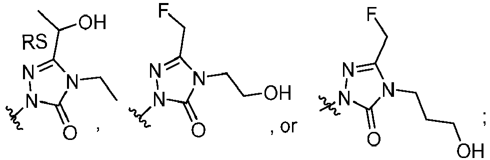 or pharmaceutically acceptable salts, isotopes, tautomers, N-oxides, solvates, or stereoisomers thereof.

An additional embodiment of the invention is a compound of Formula (I) wherein R^{1a} is CH₃; or a pharmaceutically acceptable salt, solvate, stereoisomer, tautomer, isotopic variant, or N-oxide thereof.

An additional embodiment of the invention is a compound of Formula (I) having the Formula (IA): 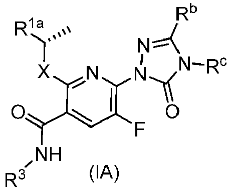 wherein
Xis O;
R^{1a} is selected from the group consisting of: C₁₋₆alkyl; C₁₋₆alkyl substituted with OH, or OCH₃; C₁₋₆haloalkyl; C₁₋₆haloalkyl substituted with OH, or OCH₃; and C₃₋₆cycloalkyl;
R^{b} is C₁₋₆alkyl substituted with a member selected from the group consisting of: OH, halo, CN, OC₁₋₆alkyl, OC₁₋₆haloalkyl and OC₃₋₆cycloalkyl;
R^{c} is selected from the group consisting of: C₁₋₆alkyl, C₁₋₆alkyl substituted with OH, allyl, C₁₋₆haloalkyl, and C₃₋₆cycloalkyl; and
R³ is selected from the group consisting of: 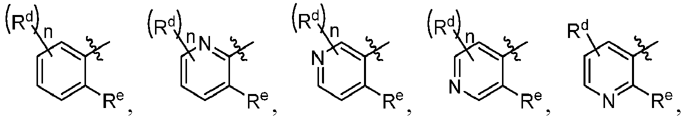 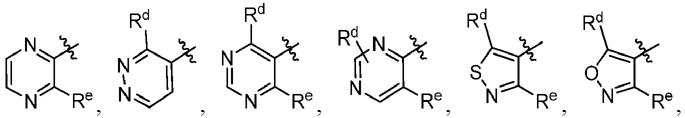 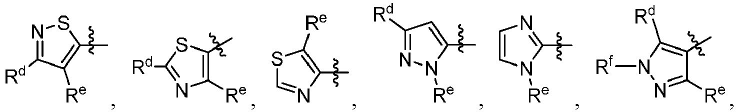 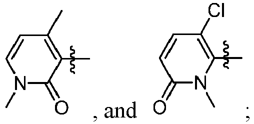
   R^{d} is selected from the group consisting of: H; halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; N(CH₃)₂; OH; CN and OC₁₋₆alkyl;
   R^{e} is selected from the group consisting of: halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; OH; OC₁₋₆alkyl; and C₃₋₆cycloalkyl;
   R^{f} is selected from the group consisting of: H; C₁₋₆alkyl; C₁₋₆alkyl substituted with a member selected from the group consisting of: OH, OCH₃, SCH₃, and OCF₃; C₁₋₆haloalkyl; and C₁₋₆haloalkyl substituted with a member selected from the group consisting of: OH, and OCH₃; and
   n is 1, or 2.

An additional embodiment of the invention is a compound of Formula (I) having the Formula (IB): 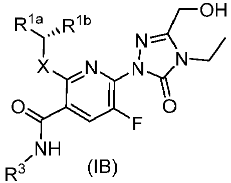 wherein
X is selected from the group consisting of: O, S and NR^{a};
when X is NR^{a}; R^{a} is H or CH₃; or R^{a} and R^{1a} come together to form a heterocyclic ring selected from the group consisting of: 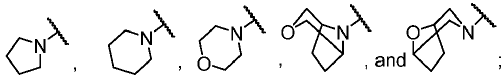 wherein the heterocyclic ring is substituted with one or two members each independently selected from the group consisting of: C₁₋₆alkyl, C₁₋₆haloalkyl, and OC₁₋₆alkyl;
R^{1a} is selected from the group consisting of: C₁₋₆alkyl; C₁₋₆alkyl substituted with OH, or OCH₃; C₁₋₆haloalkyl; C₁₋₆haloalkyl substituted with OH, or OCH₃; and C₃₋₆cycloalkyl;
R^{1b} is CH₃ or CHF₂; and
R³ is selected from the group consisting of: 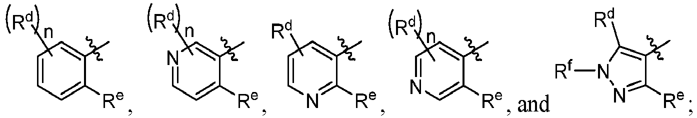
where
   R^{d} is selected from the group consisting of: H; halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; N(CH₃)₂; OH; CN and OC₁₋₆alkyl;
   R^{e} is selected from the group consisting of: halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; OH; OC₁₋₆alkyl; and C₃₋₆cycloalkyl;
   R^{f} is selected from the group consisting of: H; C₁₋₆alkyl; C₁₋₆alkyl substituted with a member selected from the group consisting of: OH, OCH₃, SCH₃, and OCF₃; C₁₋₆haloalkyl; and C₁₋₆haloalkyl substituted with a member selected from the group consisting of: OH, and OCH₃; and
n is 1, or 2.

An additional embodiment of the invention is a compound of Formula (I) having the Formula (IC): 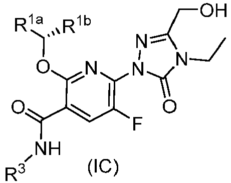 wherein
R^{1a} is selected from the group consisting of: C₁₋₆alkyl; C₁₋₆alkyl substituted with OH, or OCH₃; C₁₋₆haloalkyl; C₁₋₆haloalkyl substituted with OH, or OCH₃; and C₃₋₆cycloalkyl;
R^{1b} is CH₃ or CHF₂; or R^{1a} and R^{1b} come together to form C₃₋₆cycloalkyl; C₃₋₆cycloalkyl independently substituted with one, two, three or four members selected from the group consisting of: halo, OH, C₁₋₆alkyl, and C₁₋₆haloalkyl; oxetanyl; tetrahydrofuranyl; and tetrahydropyranyl; and
R³ is selected from the group consisting of: 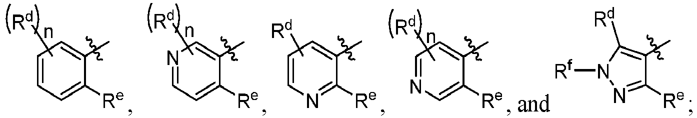
where
   R^{d} is selected from the group consisting of: H; halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; N(CH₃)₂; OH; CN and OC₁₋₆alkyl;
   R^{e} is selected from the group consisting of: halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; OH; OC₁₋₆alkyl; and C₃₋₆cycloalkyl;
   R^{f} is selected from the group consisting of: H; C₁₋₆alkyl; C₁₋₆alkyl substituted with a member selected from the group consisting of: OH, OCH₃, SCH₃, and OCF₃; C₁₋₆haloalkyl; and C₁₋₆haloalkyl substituted with a member selected from the group consisting of: OH, and OCH₃; and
n is 1, or 2.

An additional embodiment of the invention is a compound of Formula (I) having the Formula (IA), wherein R^{1a} is selected from the group consisting of: C₁₋₄alkyl; C₁₋₄alkyl substituted with OH, or OCH₃; C₁₋₄haloalkyl; C₁₋₄haloalkyl substituted with OH, or OCH₃; or C₃₋₆cycloalkyl.

An additional embodiment of the invention is a compound of Formula (I) having the Formula (IB), wherein R^{d} and R^{e} are each independently halo, C₁₋₄alkyl, and OC₁₋₄alkyl.

An additional embodiment of the invention is a compound of Formula (I) having the Formula (IC), wherein R^{1a} and R^{1b} come together to form C₃₋₆cycloalkyl; C₃₋₆cycloalkyl independently substituted with one, two, three or four members selected from the group consisting of: F, OH, C₁₋₆alkyl, and C₁₋₆haloalkyl; oxetanyl; tetrahydrofuranyl; or tetrahydropyranyl.

Also within the scope of the invention are enantiomers and diastereomers of the compounds of Formula (I) (as well as Formulas (IA), (IB), and (IC)). Also within the scope of the invention are pharmaceutically acceptable salts, N-oxides or solvates of the compounds of Formula (I) (as well as Formulas (IA), (IB), and (IC)).

Also within the scope of the invention are isotopic variations of compounds of Formula (I) (as well as Formulas (IA), (IB), and (IC)), such as, e.g., deuterated compounds of Formula (I). Also within the scope of the invention are the pharmaceutically acceptable salts, N-oxides or solvates of the isotopic variations of the compounds of Formula (I) (as well as Formulas (IA), (IB), and (IC))

Even though the compounds of embodiments of the present invention (including their pharmaceutically acceptable salts and pharmaceutically acceptable solvates) can be administered alone, they will generally be administered in admixture with a pharmaceutically acceptable carrier, a pharmaceutically acceptable excipient and/or a pharmaceutically acceptable diluent selected with regard to the intended route of administration and standard pharmaceutical or veterinary practice.

Thus, particular embodiments of the present invention are directed to pharmaceutical and veterinary compositions comprising compounds of Formula (I) and at least one pharmaceutically acceptable carrier, pharmaceutically acceptable excipient, and/or pharmaceutically acceptable diluent. By way of example, in the pharmaceutical compositions of embodiments of the present invention, the compounds of Formula (I) may be admixed with any suitable binder(s), lubricant(s), suspending agent(s), coating agent(s), solubilizing agent(s), and combinations thereof.

An embodiment of the invention relates to a pharmaceutical composition comprising an effective amount of at least one compound selected from compounds of Formula (I), and pharmaceutically acceptable salts, isotopes, tautomers, N-oxides, solvates, and stereoisomers thereof, in accordance with any embodiment described herein; and at least one pharmaceutically acceptable excipient.

An additional embodiment of the invention is a pharmaceutical composition comprising:
(A) an effective amount of at least one compound selected from compounds of 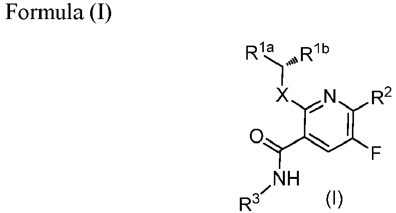 wherein
   X is selected from the group consisting of: O, S and NR^{a};
      when X is NR^{a}; R^{a} is H or CH₃; or R^{a} and R^{1a} come together to form a heterocyclic ring selected from the group consisting of: 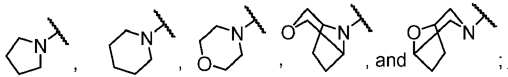 wherein the heterocyclic ring is substituted with one or two members each independently selected from the group consisting of: C₁₋₆alkyl, C₁₋₆haloalkyl, and OC₁₋₆alkyl;
   R^{1a} is selected from the group consisting of: C₁₋₆alkyl; C₁₋₆alkyl substituted with OH, or OCH₃; C₁₋₆haloalkyl; C₁₋₆haloalkyl substituted with OH, or OCH₃; and C₃₋₆cycloalkyl;
   R^{1b} is CH₃ or CHF₂; or R^{1a} and R^{1b} come together to form C₃₋₆cycloalkyl; C₃₋₆cycloalkyl independently substituted with one, two, three or four members each independently selected from the group consisting of: halo, OH, C₁₋₆alkyl, and C₁₋₆haloalkyl; oxetanyl; tetrahydrofuranyl; and tetrahydropyranyl;
   R² is 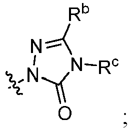 where
      R^{b} is C₁₋₆alkyl substituted with a member selected from the group consisting of: OH, halo, CN, OC₁₋₆alkyl, OC₁₋₆haloalkyl and OC₃₋₆cycloalkyl;
      R^{c} is selected from the group consisting of: C₁₋₆alkyl, C₁₋₆alkyl substituted with OH, allyl, C₁₋₆haloalkyl, and C₃₋₆cycloalkyl; and
   R³ is selected from the group consisting of: 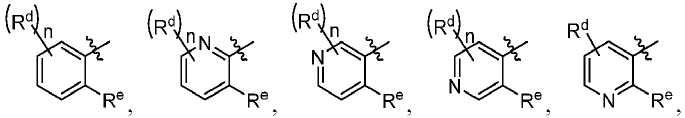 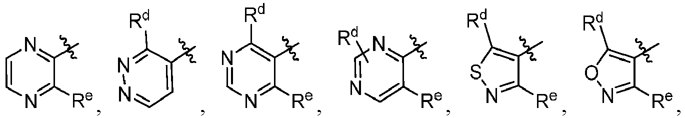 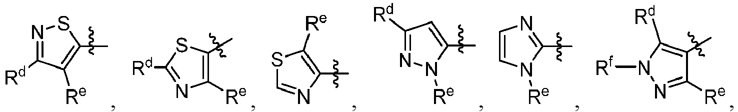 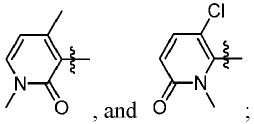
      R^{d} is selected from the group consisting of: H; halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; N(CH₃)₂; OH; CN and OC₁₋₆alkyl;
      R^{e} is selected from the group consisting of: halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; OH; OC₁₋₆alkyl; and C₃₋₆cycloalkyl;
      R^{f} is selected from the group consisting of: H; C₁₋₆alkyl; C₁₋₆alkyl substituted with a member selected from the group consisting of: OH, OCH₃, SCH₃, and OCF₃; C₁₋₆haloalkyl; and C₁₋₆haloalkyl substituted with a member selected from the group consisting of: OH, and OCH₃; and
      n is 1, or 2;
      or pharmaceutically acceptable salts, isotopes, tautomers, N-oxides, solvates, or stereoisomers of a compound of Formula (I);
and (B) at least one pharmaceutically acceptable excipient.

An additional embodiment of the invention is a pharmaceutical composition comprising an effective amount of a compound shown in Table 1 (*e.g.,* a compound selected from Examples 1-128), or a pharmaceutically acceptable salt, isotope, N-oxide, solvate, or stereoisomer of the compound of Table 1, a pharmaceutically acceptable prodrug of the compound of Table 1, or a pharmaceutically active metabolite of the compound of Table 1; and at least one pharmaceutically acceptable excipient.

Solid oral dosage forms such as, tablets or capsules, containing one or more compounds of the present invention may be administered in at least one dosage form at a time, as appropriate. It is also possible to administer the compounds in sustained release formulations.

Additional oral forms in which the present inventive compounds may be administered include elixirs, solutions, syrups, and suspensions; each optionally containing flavoring agents and coloring agents.

Alternatively, one or more compounds of Formula (I) can be administered by inhalation (intratracheal or intranasal) or in the form of a suppository or pessary, or they may be applied topically in the form of a lotion, solution, cream, ointment or dusting powder. For example, they can be incorporated into a cream comprising, consisting of, and/or consisting essentially of an aqueous emulsion of polyethylene glycols or liquid paraffin. They can also be incorporated, at a concentration of between about 1 % and about 10 % by weight of the cream, into an ointment comprising, consisting of, and/or consisting essentially of a wax or soft paraffin base together with any stabilizers and preservatives as may be required. An alternative means of administration includes transdermal administration by using a skin or transdermal patch.

The pharmaceutical compositions of the present invention (as well as the compounds of the present invention alone) can also be injected parenterally, for example, intracavernosally, intravenously, intramuscularly, subcutaneously, intradermally, or intrathecally. In this case, the compositions will also include at least one of a suitable carrier, a suitable excipient, and a suitable diluent.

For parenteral administration, the pharmaceutical compositions of the present invention are best used in the form of a sterile aqueous solution that may contain other substances, for example, enough salts and monosaccharides to make the solution isotonic with blood.

For buccal or sublingual administration, the pharmaceutical compositions of the present invention may be administered in the form of tablets or lozenges, which can be formulated in a conventional manner.

By way of further example, pharmaceutical compositions containing at least one of the compounds of Formula (I) as the active ingredient can be prepared by mixing the compound(s) with a pharmaceutically acceptable carrier, a pharmaceutically acceptable diluent, and/or a pharmaceutically acceptable excipient according to conventional pharmaceutical compounding techniques. The carrier, excipient, and diluent may take a wide variety of forms depending upon the desired route of administration (e.g., oral, parenteral, etc.). Thus, for liquid oral preparations such as, suspensions, syrups, elixirs and solutions, suitable carriers, excipients and diluents include water, glycols, oils, alcohols, flavoring agents, preservatives, stabilizers, coloring agents and the like; for solid oral preparations such as, powders, capsules, and tablets, suitable carriers, excipients and diluents include starches, sugars, diluents, granulating agents, lubricants, binders, disintegrating agents and the like. Solid oral preparations also may be optionally coated with substances such as, sugars, or be enterically coated so as to modulate the major site of absorption and disintegration. For parenteral administration, the carrier, excipient and diluent will usually include sterile water, and other ingredients may be added to increase solubility and preservation of the composition. Injectable suspensions or solutions may also be prepared utilizing aqueous carriers along with appropriate additives such as, solubilizers and preservatives.

According to particular embodiments, a therapeutically effective amount of a compound of Formula (I) or a pharmaceutical composition thereof may comprise a dose range from about 0.1 mg to about 3000 mg, or any particular amount or range therein, in particular from about 1 mg to about 1000 mg, or any particular amount or range therein, or, more particularly, from about 10 mg to about 500 mg, or any particular amount or range therein, of active ingredient in a regimen of about 1 to about (4x) per day for an average (70 kg) human; although, it is apparent to one skilled in the art that the therapeutically effective amount for a compound of Formula (I) will vary as will the diseases, syndromes, conditions, and disorders being treated.

For oral administration, a pharmaceutical composition may be provided in the form of one or more tablets containing about 1.0, about 10, about 50, about 100, about 150, about 200, about 250, or about 500 milligrams of a compound of Formula (I).

An embodiment of the present invention is directed to a pharmaceutical composition for oral administration, comprising a compound of Formula (I) in an amount of from about 1 mg to about 500 mg.

Advantageously, a compound of Formula (I) may be administered in a single daily dose, or the total daily dosage may be administered in divided doses of two, three and (4x) daily.

Optimal dosages of a compound of Formula (I) to be administered may be readily determined and will vary with the particular compound used, the mode of administration, the strength of the preparation, and the advancement of the disease, syndrome, condition or disorder. In addition, factors associated with the particular subject being treated, including subject gender, age, weight, diet and time of administration, will result in the need to adjust the dose to achieve an appropriate therapeutic level and desired therapeutic effect. The above dosages are thus exemplary of the average case. There can be, of course, individual instances wherein higher or lower dosage ranges are merited, and such are within the scope of this invention.

Compounds of Formula (I) may be administered in any of the foregoing compositions and dosage regimens or by means of those compositions and dosage regimens established in the art whenever use of a compound of Formula (I) is administered to a subject in need thereof.

According to particular embodiments, one or more compounds of Formula (I) are useful in methods for treating, ameliorating and / or preventing a disease, a syndrome, a condition or a disorder that is affected by the inhibition of DHODH enzymatic activity.

An additional embodiment of the invention relates to the use of compounds of Formula (I), *e.g.,* by inhibiting dihydroorotate oxygenase enzyme activity, in treating disorders like inflammatory disorders, autoimmune disorders, or cancer; 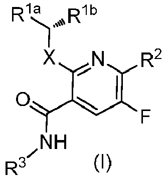 wherein
X is selected from the group consisting of: O, S and NR^{a};
   when X is NR^{a}; R^{a} is H or CH₃; or R^{a} and R^{1a} come together to form a heterocyclic ring selected from the group consisting of: 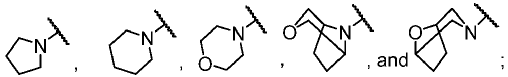 wherein the heterocyclic ring is substituted with one or two members each independently selected from the group consisting of: C₁₋₆alkyl, C₁₋₆haloalkyl, and OC₁₋₆alkyl;
R^{1a} is selected from the group consisting of: C₁₋₆alkyl; C₁₋₆alkyl substituted with OH, or OCH₃; C₁₋₆haloalkyl; C₁₋₆haloalkyl substituted with OH, or OCH₃; and C₃₋₆cycloalkyl;
R^{1b} is CH₃ or CHF₂; or R^{1a} and R^{1b} come together to form C₃₋₆cycloalkyl; C₃₋₆cycloalkyl independently substituted with one, two, three or four members each independently selected from the group consisting of: halo, OH, C₁₋₆alkyl, and C₁₋₆haloalkyl; oxetanyl; tetrahydrofuranyl; and tetrahydropyranyl;
R² is 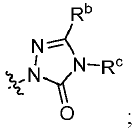 where
   R^{b} is C₁₋₆alkyl substituted with a member selected from the group consisting of: OH, halo, CN, OC₁₋₆alkyl, OC₁₋₆haloalkyl and OC₃₋₆cycloalkyl;
   R^{c} is selected from the group consisting of: C₁₋₆alkyl, C₁₋₆alkyl substituted with OH, allyl, C₁₋₆haloalkyl, and C₃₋₆cycloalkyl; and
R³ is selected from the group consisting of: 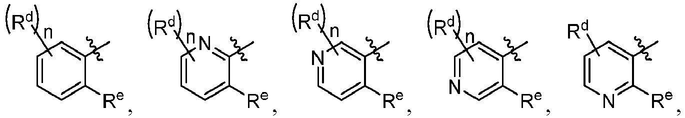 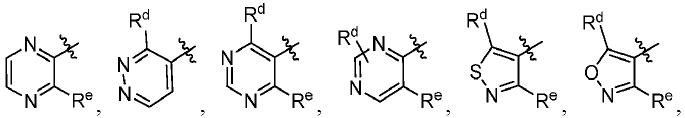 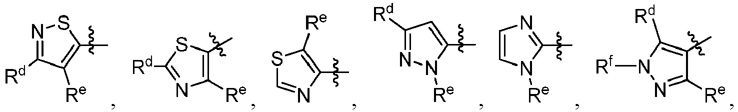 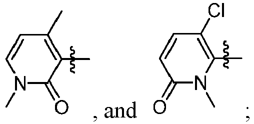
   R^{d} is selected from the group consisting of: H; halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; N(CH₃)₂; OH; CN and OC₁₋₆alkyl;
   R^{e} is selected from the group consisting of: halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; OH; OC₁₋₆alkyl; and C₃₋₆cycloalkyl;
   R^{f} is selected from the group consisting of: H; C₁₋₆alkyl; C₁₋₆alkyl substituted with a member selected from the group consisting of: OH, OCH₃, SCH₃, and OCF₃; C₁₋₆haloalkyl; and C₁₋₆haloalkyl substituted with a member selected from the group consisting of: OH, and OCH₃; and
   n is 1, or 2;
      or pharmaceutically acceptable salts, isotopes, tautomers, N-oxides, solvates, or stereoisomers thereof.

In a further aspect the present invention provides a compound of Formula (I) or a pharmaceutically acceptable salt, solvate, stereoisomer, tautomer, isotopic variant, or N-oxide thereof, or a sub-embodiment thereof, for use in treating a subject suffering from or diagnosed with a disease, disorder, or medical condition comprising inhibiting or altering Dihydroorotate Dehydrogenase (DHODH) enzymatic activity, and comprising contacting DHODH with said compound of Formula (I) or a pharmaceutically acceptable salt, solvate, stereoisomer, tautomer, isotopic variant, or N-oxide thereof, or a sub-embodiment thereof, thereby inhibiting or otherwise altering DHODH enzymatic activity, wherein the disease, disorder, or medical condition is an inflammatory disorder, an autoimmune disorder, or cancer.

An additional embodiment of the present invention provides a compound of Formula (I) for use in treating diseases, disorders, or medical conditions mediated or otherwise affected by dihydroorotate dehydrogenase (DHODH) enzyme activity comprising administering said compound of Formula (I) to a subject in need thereof.

As used herein, the term "DHODH inhibitor" may refer to an agent that inhibits or reduces DHODH activity.

In one embodiment, the term "therapeutically effective amount" (or "effective amount") refers to the amount of a compound of the present invention that, when administered to a subject, is effective to (1) at least partially alleviate, inhibit, prevent, and/ or ameliorate a condition, or a disorder or a disease (i) mediated by DHODH enzymatic activity; or (ii) associated with DHODH enzymatic activity; or (iii) characterized by activity (normal or abnormal) of DHODH enzyme; or (2) reduce or inhibit the activity of DHODH enzyme; or (3) reduce or inhibit the expression of DHODH; or (4) modify the protein levels of DHODH. Without being bound by a particular theory, DHODH inhibitors are believed to act by inhibiting nucleic acid synthesis, cell cycle arrest or altering post-translational glycosylation of proteins involved in regulating myeloid differentiation within progenitor tumor cells.

An additional embodiment of the invention is at least one compound selected from compounds of Formula (I) (as well as Formulas (IA), (IB), and (IC), such as a compound of Table 1), enantiomers and diastereomers of the compounds of Formula (I) (as well as Formulas (IA), (IB), and (IC) , such as a compound of Table 1), isotopic variations of the compounds of Formula (I) (as well as Formulas (IA), (IB), and (IC) , such as a compound of Table 1), and pharmaceutically acceptable salts of all of the foregoing, for use in treating a subject suffering from or diagnosed with a disease, disorder, or medical condition mediated or otherwise affected by DHODH enzymatic activity, comprising administering to a subject in need of such treatment an effective amount of at least one compound selected from said compounds of Formula (I) (as well as Formulas (IA), (IB), and (IC), such as a compound of Table 1), enantiomers and diastereomers of the compounds of Formula (I) (as well as Formulas (IA), (IB), and (IC) , such as a compound of Table 1), isotopic variations of the compounds of Formula (I) (as well as Formulas (IA), (IB), and (IC) , such as a compound of Table 1), and pharmaceutically acceptable salts of all of the foregoing, wherein the disease, disorder, or medical condition is an inflammatory disorder, an autoimmune disorder, or cancer. Stated another way, according to an embodiment, at least one compound selected from compounds of Formula (I) (as well as Formulas (IA), (IB), and (IC), such as a compound of Table 1), for use in treating a subject suffering from or diagnosed with a disease, disorder, or medical condition comprises inhibiting or otherwise altering dihydroorotate oxygenase enzyme activity in the subject by administering to the subject an effective amount of at least one compound selected from said compounds of Formula (I) (as well as Formulas (IA), (IB), and (IC), such as a compound of Table 1), wherein the disease, disorder, or medical condition is an inflammatory disorder, an autoimmune disorder, or cancer.

In another embodiment, inhibitors of DHODH of the present invention may be used for the treatment of immunological diseases including, but not limited to, autoimmune and inflammatory disorders, e.g. arthritis, inflammatory bowel disease, gastritis, ankylosing spondylitis, ulcerative colitis, pancreatitis, Crohn's disease, celiac disease, multiple sclerosis, systemic lupus erythematosus, lupus nephritis, rheumatic fever, gout, organ or transplant rejection, chronic allograft rejection, acute or chronic graft-versus-host disease, dermatitis including atopic, dermatomyositis, psoriasis, Behcet's diseases, uveitis, myasthenia gravis, Grave's disease, Hashimoto thyroiditis, Sjogren's syndrome, blistering disorders, antibody-mediated vasculitis syndromes, immune-complex vasculitides, allergic disorders, asthma, bronchitis, chronic obstructive pulmonary disease (COPD), cystic fibrosis, pneumonia, pulmonary diseases including edema, embolism, fibrosis, sarcoidosis, hypertension and emphysema, silicosis, respiratory failure, acute respiratory distress syndrome, BENTA disease, berylliosis, and polymyositis.

As used herein, unless otherwise noted, the term "affect" or "affected" (when referring to a disease, disorder, or medical condition that is affected by the inhibition or alteration of DHODH enzymatic activity) includes a reduction in the frequency and / or severity of one or more symptoms or manifestations of said disease, syndrome, condition or disorder; and / or includes the prevention of the development of one or more symptoms or manifestations of said disease, syndrome, condition or disorder or the development of the disease, condition, syndrome or disorder.

An additional embodiment of the invention provides a compound of Formula (I), or a pharmaceutically acceptable salt, isotope, N-oxide, solvate, or stereoisomer thereof, for use in treating cancer comprising administering to a subject in need thereof, a therapeutically effective amount of said compound of Formula (I), or a pharmaceutically acceptable salt, isotope, N-oxide, solvate, or stereoisomer thereof.

According to an embodiment, the cancer is selected from but not limited to, lymphomas, leukemias, carcinomas, and sarcomas.

An additional embodiment of the description provides the use of a compound of Formula (I), or a pharmaceutically acceptable salt, isotope, N-oxide, solvate, or stereoisomer thereof, for the treatment of one or more cancer types.

According to particular embodiments, the compound of Formula (I), or a pharmaceutically acceptable salt, isotope, N-oxide, solvate, or stereoisomer thereof for use in treatment described herein is directed to the treatment of cancer, wherein the cancer is selected from but not limited to:
leukemias including but not limited to acute lymphoblastic leukemia (ALL), acute myeloid leukemia (AML), (acute) T-cell leukemia, acute monocytic leukemia, acute promyelocytic leukemia (APL), bisphenotypic B myelomonocytic leukemia, chronic myeloid leukemia (CML), chronic myelomonocytic leukemia (CMML), large granular lymphocytic leukemia, plasma cell leukemia, and also myelodysplastic syndrome (MDS), which can develop into an acute myeloid leukemia,
lymphomas including but not limited to AIDS-related lymphoma, Hodgkin lymphoma, non-Hodgkin's lymphoma (NHL), T-non-Hodgkin lymphoma (T-NHL), subtypes of NHL such as Diffuse Large Cell Lymphoma (DLBCL), activated B-cell DLBCL, germinal center B-cell DLBCL, double-hit lymphoma and double-expressor lymphoma; anaplastic large cell lymphoma, marginal B cell lymphoma and primary mediastinal B-cell lymphoma, immunoblastic large cell lymphoma, Burkitt lymphoma, follicular lymphoma, hairy cell leukemia, Hodgkin's disease, mantle cell lymphoma (MCL), lymphoplasmatic lymphoma, precursor B -lymphoblastic lymphoma, lymphoma of the central nervous system, small lymphocytic lymphoma (SLL) and chronic lymphocytic leukemia (CLL); T-cell NHL such as precursor T-lymphoblastic lymphomalleukemia, peripheral T-cell lymphoma (PTCL), cutaneous T-cell lymphoma (CTCL), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma
sarcomas including but not limited to sarcoma of the soft tissue, gliosarcoma, osteosarcoma, malignant fibrous histiocytoma, lymphosarcoma, and rhabdomyosarcoma;
   and
other cancers, such as solid tumors, including but not limited to breast cancer, colorectal carcinoma, gastric cancer, gliosarcoma, head & neck cancer, hepatocellular carcinoma, lung cancer, multiple myeloma, neuroblastoma, ovarian cancer, pancreatic cancer, prostate cancer, renal cell carcinoma and sarcoma.

In an embodiment, cancers that may benefit from a treatment with inhibitors of DHODH of the present invention include, but are not limited to, lymphomas, leukemias, carcinomas, and sarcomas, e.g. non-Hodgkin's lymphoma, diffuse large B-cell lymphoma (DLBCL), mantle cell lymphoma (MCL), follicular lymphoma (FL), marginal zone lymphoma, T-cell lymphoma, Hodgkin's lymphoma, Burkitt's lymphoma, multiple myeloma, brain (gliomas), glioblastomas, breast cancer, colorectal/colon cancer, prostate cancer, lung cancer including non-small-cell, gastric cancer, endometrial cancer, melanoma, pancreatic cancer, liver cancer, kidney cancer, squamous cell carcinoma, ovarian cancer, sarcoma, osteosarcoma, thyroid cancer, bladder cancer, head & neck cancer, testicular cancer, Ewing's sarcoma, rhabdomyosarcoma, medulloblastoma, neuroblastoma, cervical cancer, renal cancer, urothelial cancer, vulval cancer, esophageal cancer, salivary gland cancer, nasopharangeal cancer, buccal cancer, cancer of the mouth, and GIST (gastrointestinal stromal tumor).

In another embodiment of the present invention, the compounds of the present invention may be employed in combination with one or more other medicinal agents, more particularly with one or more anti-cancer agents, e.g. chemotherapeutic, antiproliferative or immunomodulating agents, or with adjuvants in cancer therapy, e.g. immunosuppressive or anti-inflammatory agents. Additional non-limiting examples of anti-cancer agents that may be administered in combination with a compound of the present invention include biologic compounds, such as monoclonal antibodies (e.g., that mediate effector function upon binding to cancer cell-associated antigens, or block interaction of a receptor expressed on cancer cells with a soluble or cell bound ligand), bispecific antibodies that mediate immune cell redirection, etc. According to an embodiment, a compound Formula (I) or a pharmaceutically acceptable salt, solvate, stereoisomer, tautomer, isotopic variant, or N-oxide thereof, and an effective amount of one or more additional anti-cancer agents is used in treating cancer, wherein the compound of the present invention and the additional anti-cancer agent(s) are administered either simultaneously (e.g., as part of the same pharmaceutical composition) or sequentially. According to an embodiment, a pharmaceutical composition comprises an effective amount of a compound of the present invention (e.g., selected from compounds of Formula (I), such as a compound shown in Table 1, pharmaceutically acceptable salts, isotopes, tautomers, N-oxides, solvates, and stereoisomers thereof), an effective amount of one or more additional anti-cancer agents, and optionally one or more excipients.

An additional embodiment of the description provides the use of a compound of Formula (I), or pharmaceutically acceptable salts, isotopes, tautomers, N-oxides, solvates, or stereoisomers thereof, as part of chemotherapeutic regimens for the treatment of cancers, lymphomas and leukemias alone or in combination with classic antitumoral compounds well known by the one skilled in the art.

### GENERAL SYNTHETIC METHODS

Exemplary compounds useful in methods of the invention will now be described by reference to the illustrative synthetic schemes for their general preparation below and the specific examples that follow. Artisans will recognize that, to obtain the various compounds herein, starting materials may be suitably selected so that the ultimately desired substituents will be carried through the reaction scheme with or without protection as appropriate to yield the desired product. Alternatively, it may be necessary or desirable to employ, in the place of the ultimately desired substituent, a suitable group that may be carried through the reaction scheme and replaced as appropriate with the desired substituent. Unless otherwise specified, the variables are as defined above in reference to Formula (I). Reactions may be performed between the melting point and the reflux temperature of the solvent, and preferably between 0 °C and the reflux temperature of the solvent. Reactions may be heated employing conventional heating or microwave heating. Reactions may also be conducted in sealed pressure vessels above the normal reflux temperature of the solvent.

Abbreviations used in the instant specification, particularly the schemes and examples, are as follows:
- ACN: acetonitrile
- AcOH: glacial acetic acid
- aq.: aqueous
- Bn or Bzl: benzyl
- Boc: tert-butyloxycarbonyl
- conc.: concentrated
- DCC: *N*,*N*'-dicyclohexyl-carbodiimide
- DCM: dichloromethane
- DIPEA or DIEA: diisopropyl-ethyl amine
- DMA: dimethylaniline
- DMAP: 4-dimethylaminopyridine
- DME: dimethoxyethane
- DMF: *N*,*N-*dimethylformamide
- DMSO: dimethylsulfoxide
- EA: ethyl acetate
- EDCI: 1-ethyl-3-(3-dimethylaminopropyl) carbodiimide
- ESI: electrospray ionization
- EtOAc or EA: ethyl acetate
- EtOH: ethanol
- GCMS: gas chromatography-mass spectrometry
- h or hr(s): hour or hours
- HPLC: high performance liquid chromatography
- KHMDS: Potassium bis(trimethylsilyl)amide
- LiHMDS: Lithium bis(trimethylsilyl)amide
- MeOH: methanol
- MHz: megahertz
- min: minute or minutes
- MS: mass spectrometry
- NaHMDS: Sodium bis(trimethylsilyl)amide
- NMR: nuclear magnetic resonance
- PE: petrolum ether
- RP: reverse-phase
- rt or RT: room temperature
- Rₜ: retention time
- Sec: second or seconds
- TBDPS: *tert*-Butyldiphenylchlorosilane
- TBS: tert-Butyldimethylsilyl
- TES: triethylsilane
- TIPS: triisopropylsilane
- TEA or Et₃N: triethylamine
- TFA: trifluoroacetic acid
- THF: tetrahydrofuran
- TLC: thin layer chromatography
- TMED: Tetramethylethylenediamine

### PREPARATIVE EXAMPLES

Exemplary compounds useful in methods of the invention will now be described by reference to the illustrative synthetic schemes for their general preparation below and the specific examples to follow.

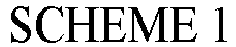

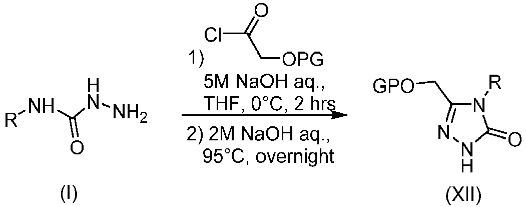

According to SCHEME 1, hydrazine carboxamides (I), where R is allyl, alkyl, or haloalkyl, can be reacted with a suitably protected 2-(hydroxy)acetyl chloride, where the hydroxy is protected with a suitable protecting group (PG) such as benzyl, in the presence of a suitable base such as NaOH, LiOH, TEA, N,N-diisopropylethylamine, and the like, at a temperature of about 0 °C. Hydrazine carboxamides (I) can be prepared by reaction of amines, H₂N-R, with carbamyltating reagents such as phosgene, 4-nitrophenyl chloroformate and subsequent combination with hydrazine.Subsequent cyclization is achieved in the presence of a suitable base such as NaOH, LiOH, TEA, N,N-diisopropylethylamine, and the like; at a temperature of about 95 °C; for a period of about 18 to 24 hrs; to provide 4-allyl-5-((benzyloxy)methyl)-2,4-dihydro-3H-1,2,4-triazol-3-one.

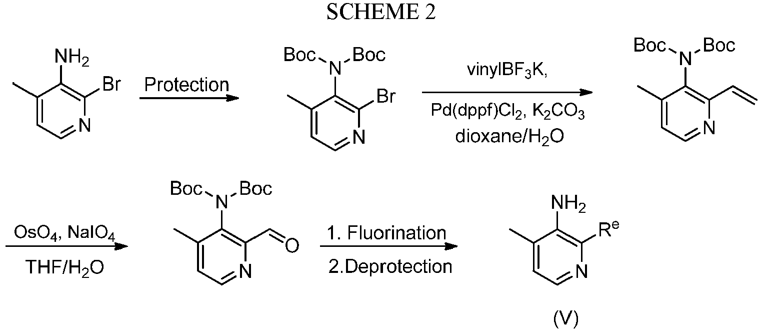

According to SCHEME 2, Boc protection of 2-bromo-4-methylpyridin-3-amine, by treatment with Boc anhydride using 4-dimethylaminopyridine as an additive, in a suitable solvent such as EtOAc, DCM, THF and the like, provides 3-[di(tert-butoxycarbonyl)amino] -2-bromo-4-methylpyridine. 3 -[Di(tert-butoxycarbonyl)amino] -2-bromo-4-methylpyridine is treated with vinyl boronate, and is subjected to Suzuki cross coupling conditions such as catalytic Pd(dppf)Cl₂( [1,1'-bis(diphenylphosphino)ferrocene]dichloropalladium(II) ), tetrakis(triphenylphosphine)palladium(0), 2nd Generation XPhos Precatalyst, chloro(2-dicyclohexylphosphino-2',4',6'-triisopropyl-1,1'-biphenyl)[2-(2'-amino-1,1'-biphenyl)]palladium(II) or the like, preferably Pd(dppf)Ch, and a base additive such as K₂CO₃ Cs₂CO₃, K₃PO₄, NaOH, TEA or the like, preferably K₂CO₃, in a suitable solvent such as water, dioxane, THF, or mixture thereof, to provide 3-[di(tert-butoxycarbonyl)amino]-2-vinyl-4-methylpyridine. 3-[Di(tert-butoxycarbonyl)amino]-2-vinyl-4-methylpyridine is oxidized by treatment with osmium tetroxide and NaIO₄, in a suitable solvent such as THF, water, or a mixture thereof, to provide 3-[di(tert-butoxycarbonyl)amino] -2-formyl-4-methylpyridine. 3 -[Di(tert-butoxycarbonyl)amino] -2-formyl-4-methylpyridine is reacted with an electrophilic fluorinating agent such as diethylaminosulfur trifluoride, in the presence of ethanol, in a suitable solvent such as DCM, THF, and the like, to provide the difluoromethyl intermediate which is subsequently treated with an acid such as HCl, in a solvent such as dioxane, to provide a compound of the formula (V), where R^{e} is CF₂H.

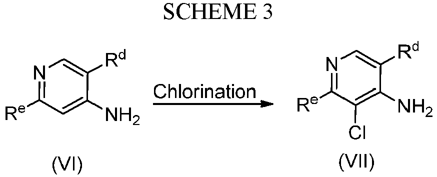

According to SCHEME 3, an amine compound of formula (VI) is chlorinated with N-chlorosuccinimide; in a suitable solvent such as DMF, MeCN, and the like; for a period of 30 minutes to 18 hours; to provide a compound of formula (VII) where R^{e} is methoxy or chloro, and R^{d} is hydrogen, fluoro, or chloro.

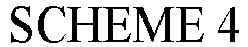

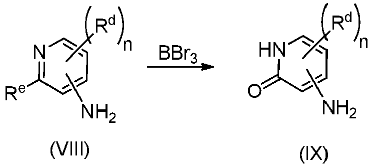

According to SCHEME 4, an amine compound of formula (VIII); where R^{d} is independently hydrogen or chloro, R^{e} is OCH₃, and n is 1 or 2; is demethylated using BBr₃ in a suitable solvent such as dichloroethane, dichloromethane, THF, and the like, for a period of 18 hr; at 0 °C to 80 °C to provide a compound of formula (IX).

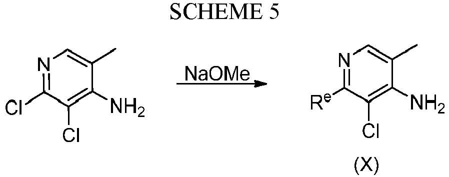

According to SCHEME 5 , 2,3-dichloro-5-methylpyridin-4-amine is treated with sodium methoxide, in a solvent such as toluene, at a temperature such as 120 °C, to provide a compound of the formula (X) where R^{e} is OCH₃.

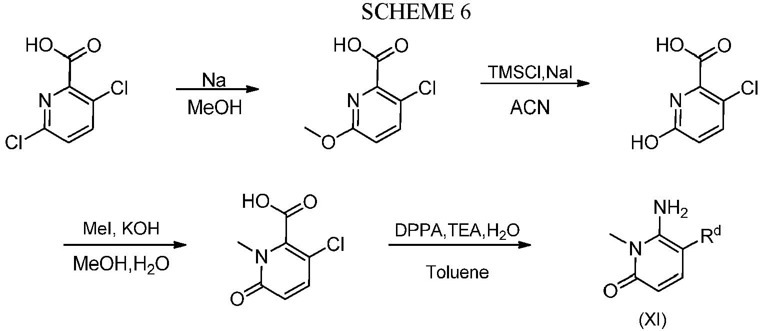

According to SCHEME 6, 3,6-dichloropicolinic acid is reacted with sodium methoxide, in a suitable solvent such as MeOH, and the like, for a period of 36 hr, at a temperature such as 100 °C, to afford 3-chloro-6-methoxypicolinic acid. 3-Chloro-6-methoxypicolinic acid is reacted with a demethylating agent such as chlorotrimethylsilane and sodium iodide, in a suitable solvent such as MeCN, THF, and the like, for a period of 12 hr, at a temperature of about 80 °C, to afford 3-chloro-6-hydroxypicolinic acid. 3-Chloro-6-hydroxypicolinic acid is N-methylated using a reagent such as methyl iodide, a base such as potassium hydroxide, and the like, in a suitable solvent such as MeOH, water, or a mixture thereof, at a temperature of 80 °C, for a period of 1 hr, to afford 3-chloro-1-methyl-6-oxo-1,6-dihydropyridine-2-carboxylic acid. 3-Chloro-1-methyl-6-oxo-1,6-dihydropyridine-2-carboxylic acid is treated with diphenylphosphoryl azide, a base such as triethylamine, in a suitable solvent such as toluene, at a temperature such as 80 °C, to afford a compound of formula (XI), where R^{d} is Cl.

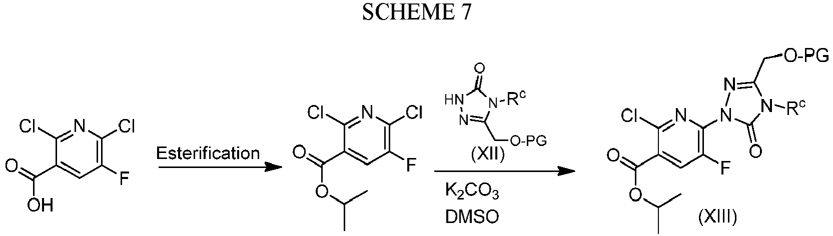

According to SCHEME 7, 2,6-dichloro-5-fluoronicotinoyl chloride is prepared from commercially available or synthetically accessible 2,6-dichloro-5-fluoronicotinic acid, by using oxalyl chloride or thionyl chloride, in the presence of a catalytic amount of DMF, in a suitable solvent such as an aprotic non polar solvent such as dichloromethane (DCM), tetrahydrofuran (THF), acetonitrile (ACN, MeCN), toluene, and the like, at a temperatures ranging from 0 °C to room temperature. Subsequent reaction of 2,6-dichloro-5-fluoronicotinoyl chloride with an alcohol such as isopropanol, methanol, or ethanol, preferably isopropanol; in a suitable solvent such as THF, dimethylformamide (DMF), or ACN affords isopropyl 2,6-dichloronicotinate.

Reaction of isopropyl 2,6-dichloronicotinate with a commercially available or synthetically accessible nucleophile compounds of formula (XII); such as suitably protected triazolones, where PG is selected from: benzyl, 4-methoxy benzyl, or an alkyl or aryl silane such as TBDPS, TBS, TES, or TIPS; in the presence of a base such as K₂CO₃, Cs₂CO₃, NaHCO₃, triethylamine, and the like; in a suitable solvent such as dimethylsulfoxide (DMSO), DMF, THF, ACN, and the like; affords a compound of formula (XIII). In a preferred method, PG is benzyl, and R^{c} is C₁₋₆alkyl.

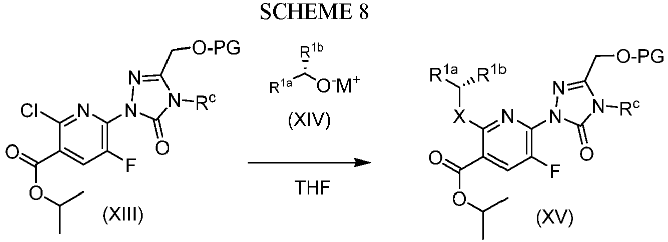

According to SCHEME 8, an alkoxide salt of formula (XIV) is prepared by treatment of a suitable alcohol such as (*S*)-1,1,1-trifluoropropan-2-ol, (*R*)-1,1,1-trifluoropropan-2-ol, isopropanol, cyclobutanol, preferably (*S*)-1,1,1-trifluoropropan-2-ol; with a suitable base such as NaH, KHMDS, LiHMDS, or NaHMDS, preferably KHMDS; in a suitable solvent such as THF, DMF, or ACN, preferably THF. Subsequent reaction of an alkoxide salt of formula (XIV), with a compound of formula (XIII), in a suitable solvent such as in a solvent such as THF, DMF, or ACN, preferably THF; provides a compound of formula (XV), where X is O, PG is benzyl, R^{1a} is C₁₋₆alkyl or C₁₋₆haloalkyl, R^{1b} is C₁₋₆alkyl, and R^{c} is C₁₋₆alkyl. A compound of formula (XV), where X is O, and R^{1a} is C₁₋₆alkyl substituted with OH, or OCH₃; C₁₋₆haloalkyl substituted with OH, or OCH₃; and C₃₋₆cycloalkyl; R^{1b} is CH₃ or CHF₂; or R^{1a} and R^{1b} come together to form C₃₋₆cycloalkyl; C₃₋₆cycloalkyl independently substituted with one or two members each selected from the group consisting of: halo, OH, C₁₋₆alkyl, and C₁₋₆haloalkyl; oxetanyl; tetrahydrofuranyl; and tetrahydropyranyl; and R^{c} is C₁₋₆alkyl, C₁₋₆haloalkyl, and C₃₋₆cycloalkyl may be prepared in a manner analogous to previously described methods from a compound of formula (XIII).

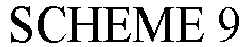

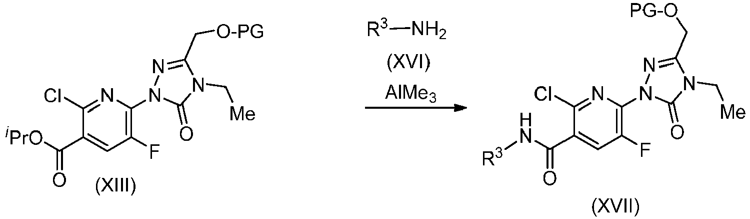

According to SCHEME 9, a compound of the formula (XVI) where R³ is 2-chloro-6-fluorophenyl; is reacted with trimethyl aluminum; in a suitable solvent such as dichloromethane, toluene, or a mixture thereof; the resulting solution is combined with a compound of the formula (XIII); to provide a compound of the formula (XVII). Compounds of formula (XVII), may also be prepared from amines of formula (XVI), where R³ is as defined in claim 1, employing the procedures as described previously.

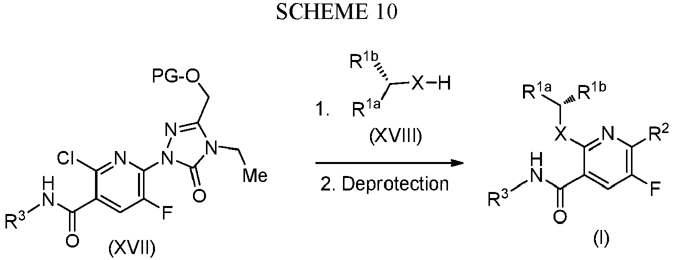

According to SCHEME 10, a compound of formula (XVII) is reacted with a commercially available or synthetically accessible nucleophile of formula (XVIII), where X is O, S, or NR^{a}, and R^{1a} and R^{1b} are as defined in claim 1; in the presence or absence of suitable base such as LiHMDS, NaHMDS, KHMDS, K₂CO₃, Cs₂CO₃, NaHCO₃, triethylamine, and the like; in a suitable solvent such as THF, DMSO, DMF, DMA, N-methyl-2-pyrrolidone (NMP), ACN, and the like; followed by subsequent deprotection of the protecting group PG, employing established methodologies, such as those described in T. W. Greene and P. G. M. Wuts, "Protective Groups in Organic Synthesis," 3 ed., John Wiley & Sons, 1999; to afford a compound of Formula (I).

A compound of Formula (I), where R² is 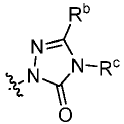 and R^{b} is CH₂OH, is fluorinated with a fluorinating agent such as diethylaminosulfur trifluoride (DAST), and the like, in a suitable solvent such as DCM, and the like, at temperatures ranging from 0 °C to 50 °C, for a period of 2-16 h, to provide a compound of Formula (I) where R² is 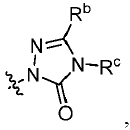 and R^{b} is CH₂F.

A compound of Formula (I), where R² is 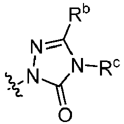 and R^{b} is CH₂OH, is chlorinated employing thionyl chloride, in a suitable solvent such as benzene, and the like, at temperatures ranging from 0 °C to 50 °C, for a period of 2-16 h, to provide a compound of Formula (I) where R² is 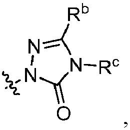 and R^{b} is CH₂Cl.

A compound of Formula (I), where R² is 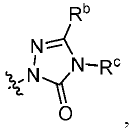 and R^{b} is CH₂OH is reacted with sodium methoxide, in a suitable solvent such as MeOH, at a temperature ranging from rt to 65 °C, to provide a compound of Formula (I) where R² is 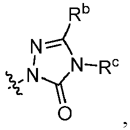 and R^{b} is CH₂OCH₃.

A compound of Formula (I), where R² is 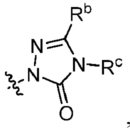 and R^{b} is CH₂OH is reacted with an oxidizing agent such as KMnO₄, MnO₂, Des Martin periodinane, and the like; in a suitable solvent such as acetone, DCM, DMF, and the like; at temperatures ranging from 0 °C to room temperature; for a period of about 5 minutes to 16 hours to provide an acid derivative. The acid is converted to the Weinreb amide, by methods known to one skilled in the art, employing N,O-dimethylhydroxylamine hydrochloride, base such as triethylamine or the like, and an amidating agent such as HATU, or the like, to provide a Weinreb amide derivative. Furthermore, the Weinreb amide can be treated with a Grignard reagent, such as methylmagnesium bromide, for example, to produce a ketone which is subsequently reduced with a suitable reducing agent, such as NaBH₄, or the like; in a suitable solvent, such as MeOH, EtOH, or the like; to produce the secondary alcohol of the Formula (I), where R² is 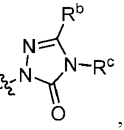 and R^{b} is CH(CH₃)OH. Alternatively, the ketone can be subsequently treated with another Grignard reagent, such as MeMgBr, to provide the tertiary alcohol of Formula (I), where R² is 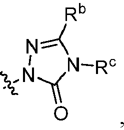 and R^{b} is C(CH₃)₂OH.

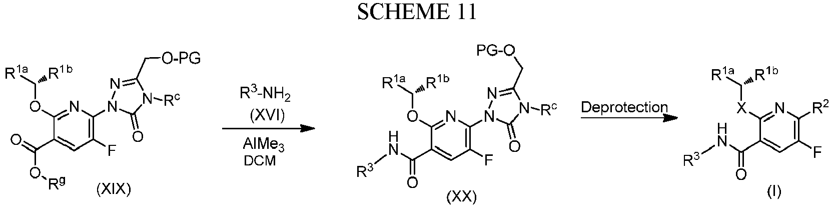

According to SCHEME 11, an amine compound of formula (XVI) where R³ is as defined in claim 1, is combined with trimethylaluminum; in a solvent such as toluene, dichloromethane, and the like; for a period of 15 minutes to 4 hours, preferably 30 min; and then subsequently combined with a compound of formula (XIX), where R^{g} is C₁₋₄alkyl, to provide a compound of formula (XX), where PG is benzyl; R^{1a} is C₁₋₆alkyl or C₁₋₆haloalkyl; R^{1b} is C₁₋₆alkyl or C₁₋₆haloalkyl; and R² is as described in Claim 1.

Alternatively, a compound of formula (XIX), where R^{g} is C₁₋₄alkl is hydrolyzed to the acid compound of formula (XIX), where R^{g} is H; using a suitable base such as NaOH, LiOH, KOH, and the like; in a suitable solvent such as MeOH, EtOH, THF, MeCN, H₂O, or a mixture thereof; and subsequently reacted with an amine compound of formula (XVI) (including compounds of formula (V), (VI), (VII), (IX), (X), and (XI)); using conventional amide bond forming techniques such as coupling reactions which are well known to those skilled in the art. For example, reaction of an amine compound of formula (XVI) where R³ is as defined in claim 1; with the acid compound of formula (XIX), where R^{g} is H, where the acid is first activated with an appropriate activating reagent, for example a carbodiimide, such as DCC or EDCI optionally in the presence of hydroxybenzotriazole (HOBt) and/or a catalyst such as DMAP; a halotrisaminophosphonium salt such as benzotriazol-1-yloxy)tris(dimethylamino)phosphonium hexafluorophosphate (BOP), or benzotriazol-1-yl-oxytripyrrolidinophosphonium hexafluorophosphate (PyBroP); a suitable pyridinium salt such as 2-chloro-1-methyl pyridinium chloride; or another suitable coupling agent such as 2-(1H-benzotriazol-1-yl)-1,1,3,3-tetramethyluronium hexafluorophosphate (HBTU), 1-[bis(dimethylamino)methylene]-1*H-*1,2,3-triazolo[4,5-b]pyridinium 3-oxid hexafluorophosphate (HATU), 2,4,6-tripropyl-1,3,5,2,4,6-trioxatriphosphorinane-2,4,6-trioxide (T₃P^{®}) and the like. Coupling reactions are conducted in a suitable solvent such as DCM, THF, DMF and the like, optionally in the presence of a tertiary amine such as N-methylmorpholine, N-ethyldiisopropylamine (DIEA, DIPEA), or triethylamine (TEA), at a temperature ranging from about 0 °C to rt, to provide compound a of formula (XX).

Alternatively, a compound of formula (XIX), where R^{g} is C₁₋₄alkyl is hydrolyzed to the acid compound of formula (XIX), where R^{g} is H, employing conditions previously described. Subsequent reaction of the acid compound of formula (XIX), where R^{g} is H; with a chlorinating agent such as thionyl chloride or oxalyl chloride; in a suitable solvent such as THF, DCM, and the like; with a catalytic amount of DMF; followed by reaction with an amine compound of formula (XVI), R³-NH₂; at temperatures ranging from 0 °C to room temperature; for a period of about 2-4 hrs; provides a compound of formula (XX).

Cleavage of the benzyl protecting group on a compound of formula (XX) is achieved according to procedures known to one skilled in the art and employing established methodologies, such as those described in T. W. Greene and P. G. M. Wuts, "Protective Groups in Organic Synthesis," 3 ed., John Wiley & Sons, 1999. For example, when PG is benzyl, deprotection is achieved employing Pd/C; under an H₂; in a suitable solvent such as EtOH, MeOH, EtOAc, or a mixture thereof, preferably EtOH; with or without the presence HCl, preferably 0.75 equiv for 4 to 72 hr, to provide a compound of Formula (I). Additionally, when PG is benzyl, deprotection can be employed using trifluoroacetic acid as solvent. In an alternate method, debenzylation is also achieved employing BCl₃, in a suitable solvent such as DCM, at a temperature of about -78 °C, to afford a compound of Formula (I).

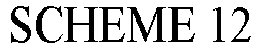

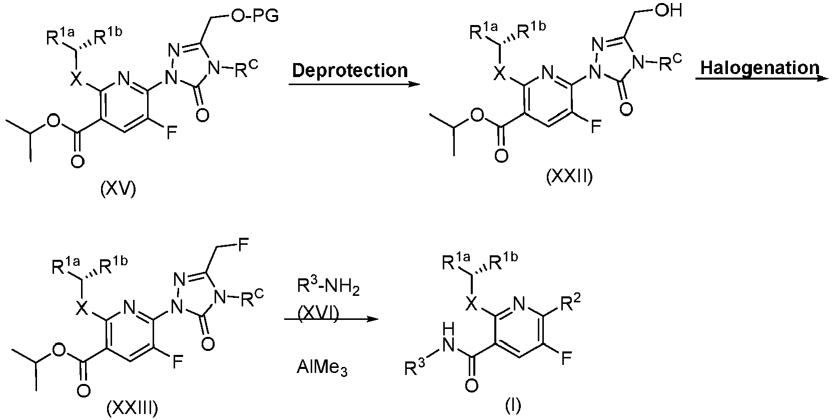

According to SCHEME 12, a compound of formula (XV) where X is O, PG is benzyl, R^{1a} is C₁₋₆alkyl or C₁₋₆haloalkyl, R^{1b} is C₁₋₆alkyl, and R^{c} is C₁₋₆alkyl is as described in claim 1, is combined with an acid such as TFA, HCl, TsOH and the like; for a period of 1 to 24 hours, at a temperature of 70 °C; to provide a compound of formula (XXII). A compound of formula (XXII) is reacted with an electrophilic fluorinating agent such as diethylaminosulfur trifluoride (DAST), in a suitable solvent such as DCM, THF, and the like, to provide a fluoromethyl compound of formula (XXIII). A compound of formula (XXIII) is reacted with an amine of the formula (XVI), where R³ is an appropriately substituted commercially available or synthetically accessible aryl, or 5 or 6 membered heteroaryl as defined in claim 1, employing amidation conditions previously described to afford a compound of Formula (I), where R^{1a} is C₁₋₆alkyl or C₁₋₆haloalkyl, R^{1b} is C₁₋₆alkyl.

Alternatively, a compound of formula (XXIII) where R^{c} is allyl, is dehydroxylated, oxidatively cleaved and reduced using an osmium tetroxide-sodium periodinate-sodium borohydride sequence, known to one skilled in the art, to provide compound of formula (XXIII) where R^{c} is CH₂CH₂OH. A compound of formula (XXIII) where R^{c} is CH₂CH₂OH is amidated with an amine of formula R³NH₂, employing conditions known to one skilled in the art or as previously described, to afford a compound of Formula (I), where R^{c} is CH₂CH₂OH and R^{1a} is C₁₋₆alkyl or C₁₋₆haloalkyl, R^{1b} is C₁₋₆alkyl, and R³ is an aryl, or 5 or 6 membered heteroaryl as defined in claim 1.

Alternatively, a compound (XXIII) where R^{c} is allyl, can be hydroborated using 9-borabicyclo(3.3.1)nonane (9-BBN) and subsequently oxidized using hydrogen peroxide, under conditions known to one skilled in the art, to afford a compound of formula (XXIII) where R^{c} is CH₂CH₂CH₂OH. A compound of formula (XXIII), where R^{c} is CH₂CH₂CH₂OH, is amidated employing conditions previously described with an amine of formula R³NH₂, to afford a compound of Formula (I) where R^{c} is CH₂CH₂CH₂OH, R^{1a} is C₁₋₆alkyl or C₁₋₆haloalkyl, R^{1b} is C₁₋₆alkyl, and R³ is an aryl, or 5 or 6 membered heteroaryl as defined in claim 1.

Compounds of Formula (I) may be converted to their corresponding salts using methods known to one of ordinary skill in the art. For example, an amine of Formula (I) is treated with trifluoroacetic acid, HCl, or citric acid in a solvent such as Et₂O, CH₂Cl₂, THF, MeOH, chloroform, or isopropanol to provide the corresponding salt form. Alternately, trifluoroacetic acid or formic acid salts are obtained as a result of reverse phase HPLC purification conditions. Cyrstalline forms of pharmaceutically acceptable salts of compounds of Formula (I) may be obtained in crystalline form by recrystallization from polar solvents (including mixtures of polar solvents and aqueous mixtures of polar solvents) or from non-polar solvents (including mixtures of non-polar solvents).

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention.

Compounds prepared according to the schemes described above may be obtained as single forms, such as single enantiomers, by form-specific synthesis, or by resolution. Compounds prepared according to the schemes above may alternately be obtained as mixtures of various forms, such as racemic (1:1) or non-racemic (not 1:1) mixtures. Where racemic and non-racemic mixtures of enantiomers are obtained, single enantiomers may be isolated using conventional separation methods known to one of ordinary skill in the art, such as chiral chromatography, recrystallization, diastereomeric salt formation, derivatization into diastereomeric adducts, biotransformation, or enzymatic transformation. Where regioisomeric or diastereomeric mixtures are obtained, as applicable, single isomers may be separated using conventional methods such as chromatography or crystallization.

The following specific examples are provided to further illustrate the invention and various preferred embodiments.

### EXAMPLES

In obtaining the compounds described in the examples below and the corresponding analytical data, the following experimental and analytical protocols were followed unless otherwise indicated.

Unless otherwise stated, reaction mixtures were magnetically stirred at room temperature (rt) under a nitrogen atmosphere. Where solutions were "dried," they were generally dried over a drying agent such as Na₂SO₄ or MgSO₄. Where mixtures, solutions, and extracts were "concentrated", they were typically concentrated on a rotary evaporator under reduced pressure.

Normal-phase silica gel chromatography (FCC) was performed on silica gel (SiO₂) using prepacked cartridges.

Preparative reverse-phase high performance liquid chromatography (RP HPLC) was performed on either:
METHOD A. A Gilson GX-281 semi-prep-HPLC with Phenomenex Synergi C18(10µm, 150 × 25mm), or Boston Green ODS C18(5µm, 150 × 30mm), and mobile phase of 5-99% ACN in water (with 0.225%FA) over 10 min and then hold at 100% ACN for 2 min, at a flow rate of 25 mL/min.
   or
METHOD B. A Gilson GX-281 semi-prep-HPLC with Phenomenex Synergi C18(10µm, 150 × 25mm), or Boston Green ODS C18(5µm, 150 × 30mm), and mobile phase of 5-99% ACN in water(0.1%TFA) over 10 min and then hold at 100% ACN for 2 min, at a flow rate of 25 mL/min.
   or
METHOD C. A Gilson GX-281 semi-prep-HPLC with Phenomenex Synergi C18(10µm, 150 × 25mm), or Boston Green ODS C18(5µm, 150 × 30mm), and mobile phase of 5-99% ACN in water(0.05%HCl) over 10 min and then hold at 100% ACN for 2 min, at a flow rate of 25 mL/min.
   or
METHOD D. a Gilson GX-281 semi-prep-HPLC with Phenomenex Gemini C18 (10µm, 150 × 25mm), AD(10µm, 250mm × 30mm), or Waters XBridge C18 column (5µm, 150 × 30mm), mobile phase of 0-99% ACN in water (with 0.05% ammonia hydroxide v/v) over 10 min and then hold at 100% ACN for 2 min, at a flow rate of 25 mL/min.
   or
METHOD E. a Gilson GX-281 semi-prep-HPLC with Phenomenex Gemini C18 (10µm, 150 × 25mm), or Waters XBridge C18 column (5µm, 150 × 30mm), mobile phase of 5-99% ACN in water(10mM NH₄HCO₃) over 10 min and then hold at 100% ACN for 2 min, at a flow rate of 25 mL/min.
   or
METHOD F. Teledyne ISCO ACCQPrep HP150 semi-prep-HPLC with Phenomenex Gemini-NX C18 (5 µm, 150 × 30 mm), mobile phase of 10-100 % ACN in water(10mM NH₄OH) over 10 min and then hold at 100% ACN for 2 min, at a flow rate of 30 mL/min.

Preparative supercritical fluid high performance liquid chromatography (SFC) was performed either on a Thar 80 Prep-SFC system, or Waters 80Q Prep-SFC system from Waters. The ABPR was set to 100bar to keep the CO2 in SF conditions, and the flow rate may verify according to the compound characteristics, with a flow rate ranging from 50g/min to 70g/min. The column temperature was ambient temperature

Mass spectra (MS) were obtained on a SHIMADZU LCMS-2020 MSD or Agilent 1200\G6110A MSD using electrospray ionization (ESI) in positive mode unless otherwise indicated. Calculated (calcd.) mass corresponds to the exact mass.

Nuclear magnetic resonance (NMR) spectra were obtained on Bruker model AVIII 400 spectrometers. Definitions for multiplicity are as follows: s = singlet, d = doublet, t= triplet, q = quartet, m = multiplet, br = broad. It will be understood that for compounds comprising an exchangeable proton, said proton may or may not be visible on an NMR spectrum depending on the choice of solvent used for running the NMR spectrum and the concentration of the compound in the solution.

Chemical names were generated using ChemDraw Ultra 17.1 (Cambridge Soft Corp., Cambridge, MA) or OEMetaChem V1.4.0.4 (Open Eye).

Compounds designated as R* or S* are enantiopure compounds where the absolute configuration was not determined.

### Intermediate 1: 4-Amino-3-chloropyridin-2(1H)-one.

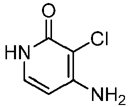

Step A: 3-Chloro-2-methoxypyridin-4-amine. To a mixture of 2-methoxypyridin-4-amine (20 g, 161.1 mmol) in DMF (200 mL) was added N-chlorosuccinimide (NCS) (21.5 g, 161.1 mmol) at 15 °C under N₂. The mixture was stirred at 30 °C for 36 hours. The aqueous phase was extracted with ethyl acetate (200 mL ×2). The combined organic phase was washed with brine (50 mL ×2), dried with anhydrous Na₂SO₄, filtered and concentrated in vacuo. The residue was purified by silica gel chromatography (petroleum ether/ethyl acetate= 15/1 to 3/1) to give the title compound (24 g, 149.8 mmol, 93% yield, 99% purity) as yellow oil. MS (ESI): mass calcd. for C₆H₇ClN₂O, 158.0; m/z found, 159.1 [M+H]⁺. ¹H NMR (400 MHz, CDCl₃) δ = 7.72 (d, *J=* 5.5 Hz, 1H), 6.32 (d, *J=* 5.6 Hz, 1H), 4.52 (br s, 2H), 3.99 (s, 3H).

Step B. 4-Amino-3-chloropyridin-2(1H)-one. To a solution of 3-chloro-2-methoxy-pyridin-4-amine (1 g, 6.31 mmol) in dichloroethane (DCE) (20 mL) was added BBr₃ (7.90 g, 31.5 mmol, 3.0 mL) at 0 °C. The mixture was stirred at 80 °C for 12 hr. The reaction mixture was quenched by addition water (100 mL×2) at 0° C, and then extracted with ethyl acetate (150 mL ×8). The combined organic layer was washed with brine (300 mL), dried over Na₂SO₄, filtered and concentrated under reduced pressure. The residue was purified by column chromatography (SiO₂, dichloromethane / methanol= 1/1 to 5/1) to give the title compound (550 mg, 3.80 mmol, 60% yield) as a brown solid. MS (ESI): mass calcd. for C₅H₅ClN₂O, 144.0; m/z found, 144.9 [M+H]⁺. ¹H NMR (400 MHz, CD₃OD) δ = 7.12 (d, *J* = 7.2 Hz, 1H), 6.06 (d, *J* = 7.2 Hz, 1H).

### Intermediate 2: 4-Amino-3,5-dichloropyridin-2(1H)-one.

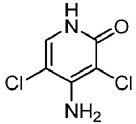

Step A: 3,5-Dichloro-2-methoxypyridin-4-amine. To a solution of 2-methoxypyridin-4-amine (3 g, 24 mmol) in acetonitrile (ACN) (100 mL) was added NCS (12.9 g, 96.7 mmol) portions. The reaction mixture was stirred at 25 °C for 72 h. The reaction mixture was diluted with water (500 mL) and extracted with ethyl acetate (500 mL×2). The combined organic layers were dried over Na₂SO₄, filtered and concentrated under vacuum. The residue was purified by silica gel chromatography to give title compound (3.7 g, 76% yield, 96% purity) as yellow solid. MS (ESI): mass calcd. For C₆H₅N₂Cl₂O, 192.0; m/z found, 193.2 [M+H]⁺. ¹H NMR (400 MHz, CDCl₃) δ = 7.83 (s, 1H), 4.92 (br s, 1H), 3.98 (s, 1H).

Step B: 4-Amino-3,5-dichloropyridin-2(1H)-one. To a solution of 3,5-dichloro-2-methoxypyridin-4-amine (1.2 g, 6.22 mmol) in DCM (25 mL) was added BBr₃ (7.79 g, 31.08 mmol, 3.00 mL) at 0 °C under N₂. The reaction mixture was stirred at 80 °C for 12 h. The reaction mixture was slowly added into methanol (100 mL) at 0 °C. The mixture was adjusted to pH~8 with solid potassium carbonate. Then the mixture was concentrated under reduced pressure in vacuo. The residue was purified by silica gel chromatography (SiO₂, dichloromethane : methanol= 100:1-10:1) to give title compound (566 mg, 3.13 mmol, 50% yield, 99% purity) as yellow solid. MS (ESI): mass calcd. for C₅H₄N₂OCl₂,178.0; m/z found, 179.2 [M+H]⁺. ¹H NMR (400 MHz, DMSO-*d*₆) δ = 11.34 (br s, 1H), 7.43 (s, 1H), 6.45 (s, 1H).

### Intermediate 3: 6-Amino-5-chloropyridin-2(1H)-one.

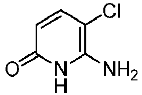

To a mixture of 3-chloro-6-methoxypyridin-2-amine (650 mg, 4.10 mmol in DCE (15 mL) was added BBr₃ (5.13 g, 20.49 mmol, 1.97 mL) in one portion at 0°C under N₂. The mixture was stirred at 80 °C for 13 hours. The mixture was poured into water (100 mL). The aqueous phase was extracted with ethyl acetate (30 mL×15). The combined organic phase was dried with anhydrous Na₂SO₄, filtered and concentrated in vacuo. The residue was purified by silica gel chromatography (1000 mesh silica gel, dichloromethane : methanol= 100/1 to 20/1) to give title compound (585 mg, 3.84 mmol, 94% yield, 95% purity) as brown solid. MS (ESI): mass calcd. for C₅H₅ClN₂O,144.0; m/z found, 145.2 [M+H]⁺.¹H NMR (400 MHz, CD₃OD) δ = 7.37 (d, *J* = 9.2 Hz, 1H), 5.72 (d, *J* = 9.2 Hz, 1H).

### Intermediate 4: 3-Chloro-5-fluoro-2-methoxypyridin-4-amine.

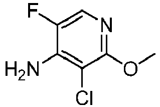

To a solution of 5-fluoro-2-methoxy-pyridin-4-amine (500 mg, 3.52 mmol) in DMF (10 mL) was added NCS (1.41 g, 10.55 mmol). The mixture was stirred at 25 °C for 12 hr. The reaction mixture was diluted with water (50 mL) and extracted with ethyl acetate (50 mL×3). The organic layer was washed with brine (20 mL×5), dried over Na₂SO₄, filtered and concentrated under vacuum. The residue was purified by column chromatography (SiO₂, petroleum ether / ethyl acetate = 50/1 to 3/1) to give the title compound (485 mg, 2.72 mmol, 77 % yield, 99% purity) as brown solid. MS (ESI): mass calcd. For C₆H₆N₂ClFO, 176.0; m/z found, 177.1 [M+H]⁺. ¹H NMR (400 MHz, CDCl₃) δ = 7.72 (s, 1H), 4.63 (br s, 2H), 3.96 (s, 3H).

### Intermediate 5: 3-Chloro-2-methoxy-5-methylpyridin-4-amine.

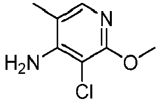

Step A. 2,3-Dichloro-5-methylpyridin-4-amine. To a solution of 2-chloro-5-methyl-pyridin-4-amine (6.5 g, 45.59 mmol) in ACN (130 mL) was added N- N-chlorosuccinimide (24.35 g, 182.35 mmol). The mixture was stirred at 40 °C for 3.5 hrs. The reaction mixture was concentrated in vacuo. The resulting residue was poured into water (100 mL) and extracted with ethyl acetate (100 mL×2).The combined organic phase was washed with brine (100 mL×2), dried with anhydrous Na₂SO₄, filtered and concentrated in vacuo. The residue was purified by column chromatography (SiO₂, 0.1% NH₃•H₂O, petroleum ether/ethyl acetate= 10/1 to 1/1) to give 15 g product with 54.5% purity. 11 g product was further purified by reversed-phase HPLC (Column: Welch Ultimate XB_C18 57*235mm 20/40um; 120 Å; A:water (0.1% FA) B:acetonitrile; Gradient B%: 0%-50%, 25 min; 50%, 30 min) to give the title compound (3.2 g, 97.5% purity) as a white solid. MS (ESI): mass calcd. for C₆H₆Cl₂N₂, 176.0; m/z found, 177.5 [M+H]⁺.¹H NMR (400 MHz, CDCl₃) δ = 7.83 (s, 1H), 4.69 (br s, 2H), 2.14 (s, 3H).

Step B. 3-Chloro-2-methoxy-5-methylpyridin-4-amine. To a stirred solution of methanol (MeOH) (30 mL) was added Na metal (2.15 g, 93.37 mmol) slowly. The reaction was stirred at 16 °C for 2 hrs. Then the mixture was concentrated in vacuo. The residue was dissolved into toluene (60 mL) and added 2,3-dichloro-5-methylpyridin-4-amine (1.9 g, 10.73 mmol). The mixture was stirred at 120 °C for 100 hrs. The mixture was poured into water (100 mL) and extracted with DCM (100 mL×2).The combined organic phase was washed with brine (150 mL×2), dried with anhydrous Na₂SO₄, filtered and concentrated in vacuo. The residue was purified by column chromatography (SiO₂, petroleum ether/ethyl acetate= 60/1 to 5/1) to give the title compound (1.12 g, 6.44 mmol, 60% yield, 99.7% purity) was obtained as a white solid. MS (ESI): mass calcd. for C₇H₉ClN₂O, 172.0; m/z found, 173.8 [M+H]⁺. ¹H NMR (400 MHz, CDCl₃) δ = 7.61 (s, 1H), 4.50 (br s, 2H), 3.97 (s, 3H), 2.09 (s, 3H).

### Intermediate 6: 3-Isopropyl-1-methyl-1H-pyrazol-5-amine.

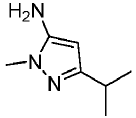

The title compound was prepared according to procedure as described in J. Med. Chem., 2016, 59 (18), pp 8293-8305. MS (ESI): mass calcd. For C₇H₁₃N₃, 139.1; m/z found, 140.0 [M+H]⁺. ¹H NMR (400MHz, DMSO-*d*₆) δ = 5.09 (s, 1H), 4.97 (s, 2H), 3.41 (s, 3H), 2.65-2.61 (m, 1H), 1.09 (d, *J* =7.0 Hz, 6H).

### Intermediate 7: 6-Amino-5-chloro-1-methylpyridin-2(1H)-one.

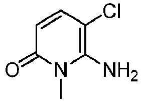

Step A: 3-Chloro-6-methoxypicolinic acid. Sodium (15.31 g, 665.81 mmol) was added into MeOH (350 mL) which was stirred at 25 °C for 1 hr. Then 3,6-dichloropyridine-2-carboxylic acid (18.5 g, 96.35 mmol) was added and the mixture was warmed to 100 °C. The mixture was stirred at 100 °C for 36 h. The mixture was concentrated under reduced pressure in vacuo. Aqueous hydrochloric acid (1 M, 800 mL) was added to the residue and the aqueous phase was extracted with ethyl acetate (500 mL ×3). The combined organic phase was washed with brine (100 mL), dried over MgSO₄ and concentrated in vacuo to give the title compound as white solid (17.5 g, 83.96 mmol, 87.14% yield, 90% purity). MS (ESI): mass calcd. for C₇H₆ClNO₃, 187.0; m/z found, 188.6 [M+H]⁺.

Step B: 3-Chloro-6-hydroxypicolinic acid. To a solution of 3-chloro-6-methoxypicolinic acid (5 g, 26.66 mmol) and trimethylsilyl chloride (TMSCI) (5.79 g, 53.31 mmol, 6.77 mL) in MeCN (65 mL) was added NaI (7.99 g, 53.31 mmol). The reaction mixture was stirred at 80 °C for 12 h. The reaction mixture was concentrated in vacuo. The residue was triturated with water (50 mL) and filtered to collect the filter cake. The filter cake was triturated with dichloromethane (50 mL) at 25 °C and then filtered. The filter cake was dried to give the title compound as white solid (2.8 g, 15.81 mmol, 59% yield, 98% purity). MS (ESI): mass calcd. for C₆H₄ClNO₃, 173.0; m/z found, 174.6 [M+H]⁺.

Step C: 3-Chloro-1-methyl-6-oxo-1,6-dihydropyridine-2-carboxylic acid. To a mixture of 3-chloro-6-hydroxypicolinic acid (2.8 g, 16.13 mmol) and KOH (1.81 g, 32.27 mmol) in MeOH (30 mL) and H₂O (0.3 mL) was added CH₃I (2.98 g, 20.97 mmol, 1.31 mL) at 0 °C under N₂. Then the mixture was warmed to 80 °C and stirred for 12 hr. The reaction mixture a combined with another two batches (200 mg scale and 300 mg scale) was concentrated in vacuo. The residue was diluted with water (100mL) and washed with dichloromethane (150 mL × 3). The aqueous phase was concentrated in vacuo. The residue was dissolved with (dichloromethane/ methyl alcohol= 10/1, 80 mL) and stirred for 12 h, then filtered and concentrated in vacuo to give title compound as yellow solid (2.12 g, 11.30 mmol, 100% purity). MS (ESI): mass calcd. for C₇H₆ClNO₃, 187.0; m/z found, 188.6 [M+H]⁺. ¹H NMR (400 MHz, DMSO-*d*₆) δ = 7.42 (d, *J* = 9.7 Hz, 1H), 6.30 (d, *J* = 9.7 Hz, 1H), 3.35 (s, 3H).

Step D: 6-Amino-5-chloro-1-methylpyridin-2(1H)-one. To a solution of 3-chloro-1-methyl-6-oxo-pyridine-2-carboxylic acid (1 g, 5.33 mmol) in toluene (40 mL) was added TEA (1.08 g, 10.66 mmol, 1.48 mL). The mixture was warmed to 50 °C. Then diphenylphosphoryl azide (DPPA) (2.20 g, 8.00 mmol, 1.73 mL) was added and the mixture was warmed to 80 °C and stirred for 6 h. Then water (0.1 mL) was added. The mixture was stirred for 6 h at 80 °C. The reaction mixture was concentrated under reduced pressure to remove solvent. The residue was purified by column chromatography (SiO₂, dichloromethane/ methanol= 100/1 to 30/1), following by RP HPLC (condition A) to afford the title compound (100 mg, 630.58 µmol, 12% yield, 100% purity). MS (ESI): mass calcd. for C₆H₇ClN₂O, 158.0; m/z found, 159.1 [M+H]⁺. ¹H NMR (400 MHz, CD₃OD) δ = 7.35 (d, *J* = 9.3 Hz, 1H), 5.79 (d, *J* = 9.3 Hz, 1H), 3.52 (s, 3H).

### Intermediate 8: 2-(Difluoromethyl)-4-methylpyridin-3-amine.

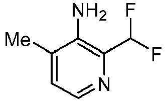

### Step A. tert-Butyl N-(2-bromo-4-methyl-3-pyridyl)-N-tert-butoxycarbonyl-carbamate.

To a stirred solution of 2-bromo-4-methylpyridin-3-amine (4 g, 21 mmol) and DMAP (2.35 g, 19.2 mmol) in ethyl acetate (80 mL) was added Boc₂O (14.00 g, 64.16 mmol, 14.74 mL) in ethyl acetate (12 mL) dropwise at 30 °C. The mixture was stirred at 30 °C for 15 hrs. The mixture was poured into ice-water (150 mL) and extracted with ethyl acetate (100 mL×2).The combined organic phase was washed with 0.5 N HCl (100 mL) and brine (150 mL×2), dried with anhydrous Na₂SO₄, filtered and concentrated in vacuo. The residue was purified by column chromatography (SiO₂, petroleum ether/ethyl acetate=15/1) to give the title compound (7.3 g, 18.85 mmol, 88 % yield, 100% purity) as a white solid. MS (ESI): mass calcd. for C₁₆H₂₃BrN₂O₄, 386.1/388.1; m/z found, 387.3/389.3 [M+H]⁺. ¹H NMR (400 MHz, CDCl₃) δ = 8.19 (d, *J* = 4.9 Hz, 1H), 7.15 (d, *J* = 4.9 Hz, 1H), 2.29 (s, 3H), 1.42 (s, 18H).

### Step B. tert-Butvl N-tert-butoxycarbonyl-N-(4-methyl-2-vinyl-3-pyridyl)carbamate.

To a mixture of tert-butyl N-(2-bromo-4-methyl-3-pyridyl)-N-tert-butoxycarbonyl-carbamate (8 g, 20.66 mmol), vinyl BF₃K (4.98 g, 37.2 mmol), K₂CO₃ (7.14 g, 51.6 mmol) in dioxane (100 mL) and H₂O (20 mL) was added Pd(dppf)Cl₂ (3.02 g, 4.13 mmol) under N₂. The mixture was stirred at 70 °C for 16 hr under N₂ atmosphere. The mixture was poured into water (200 mL) and extracted with ethyl acetate (300 mL×2). The combined organic phase was washed with brine (400 mL×2), dried with anhydrous Na₂SO₄, filtered and concentrated in vacuo. The residue was purified by column chromatography (SiO₂, petroleum ether/ ethyl acetate=20/1) to give the title compound (5.73 g, 17.0 mmol, 82 % yield, 99.1% purity) as a white solid. MS (ESI): mass calcd. for C₁₈H₂₆N₂O₄, 334.2; m/z found, 335.5 [M+H]⁺. ¹H NMR (400 MHz, CDCl₃) δ = 8.39 (d, *J* = 4.8 Hz, 1H), 7.07 (d, *J* = 4.9 Hz, 1H), 6.83 (dd, *J* = 10.8, 17.0 Hz, 1H), 6.40 (dd, *J* = 1.7, 17.0 Hz, 1H), 5.52 (dd, *J* = 1.8, 10.8 Hz, 1H), 2.21 (s, 3H), 1.36 (s, 18H).

### Step C. tert-Butyl N-tert-butoxycarbonyl-N-(2-formyl-4-methyl-3-pyridyl)carbamate.

To a solution of tert-butyl N-tert-butoxycarbonyl-N-(4-methyl-2-vinyl-3-pyridyl)carbamate (4.7 g, 14.1 mmol,) in THF (120 mL) and H₂O (40 mL) was added OsO₄ (714 mg, 2.81 mmol) and NaIO₄ (18.04 g, 84.33 mmol) at 0 °C. The mixture was stirred at 16 °C for 5 hr. The mixture was poured into saturated aqueous Na₂SO₃ (600 mL) and extracted with ethyl acetate (300 mL×2). The combined organic phase was washed with brine (500 mL×2), dried with anhydrous Na₂SO₄, filtered and concentrated in vacuum. The residue was purified by column chromatography (SiO₂, petroleum ether/ethyl acetate=9/1) to give the title compound (4.12 g, 11.6 mmol, 83 % yield, 95% purity) was obtained as a white solid. MS (ESI): mass calcd. for C₁₇H₂₄N₂O₅, 336.2; m/z found, 337.1 [M+H]⁺. ¹H NMR (400 MHz, CDCl₃) δ = 10.09 (s, 1H), 8.61 (d, *J* = 4.8 Hz, 1H), 7.40 (d, *J* = 4.8 Hz, 1H), 2.30 (s, 3H), 1.38 (s, 18H)

Step D. *tert*-Butyl N-tert-butoxycarbonyl-N-[2-(difluoromethyl)-4-methyl-3-pyridyl]carbamate. To a solution of tert-butyl N-tert-butoxycarbonyl-N-(2-formyl-4-methyl-3-pyridyl)carbamate (3.9 g, 11.6 mmol) in DCM (50 mL) was added DAST (9.34 g, 58.0 mmol, 7.66 mL) at 0 °C under N₂. To the mixture was added EtOH (53.41 mg, 1.16 mmol, 67.78 µL) at 0 °C. The mixture was stirred at 16 °C for 15 hrs. The mixture was poured into saturated aqueous NaHCO₃ (500 mL) and extracted with DCM (200 mL×2).The combined organic phase was washed with brine (300 mL×2), dried with anhydrous Na₂SO₄, filtered and concentrated in vacuo. The residue was purified by RP HPLC (condition A) to give the title compound (1.9 g, 4.77 mmol, 41 % yield, 90% purity) as a white solid. MS (ESI): mass calcd. for C₁₇H₂₄F₂N₂O₄, 358.2; m/z found, 359.4 [M+H]⁺. ¹H NMR (400 MHz, CDCl₃) δ = 8.48 (d, *J* = 4.9 Hz, 1H), 7.32 (d, *J* = 4.8 Hz, 1H), 6.65 (t, *J* = 54.0 Hz, 1H), 2.28 (s, 3H), 1.38 (s, 18H)

Step E. 2-(Difluoromethyl)-4-methylpyridin-3-amine. A solution of tert-butyl N-tert-butoxycarbonyl-N-[2-(difluoromethyl)-4-methyl-3-pyridyl]carbamate (1.8 g, 4.5 mmol) in HCl/dioxane (20 mL) was stirred at 16 °C for 1 hr. The mixture was concentrated in vacuo. The residue was poured into saturated aqueous NaHCO₃ (50 mL) and extracted with ethyl acetate (50 mL×2). The combined organic phase was washed with brine (70 mL×2), dried with anhydrous Na₂SO₄, filtered and concentrated in vacuo. The residue was purified by column chromatography (SiO₂, petroleum ether/ethyl acetate=50/1) to give the title compound (536 mg, 3.39 mmol, 75% yield, 100% purity) was obtained as a white solid. MS (ESI): mass calcd. for C₇H₈F₂N₂, 158.1; m/z found, 159.1 [M+H]⁺. ¹H NMR (400 MHz, CDCl₃) δ = 7.90 (d, *J* = 4.6 Hz, 1H), 7.08 (d, *J* = 4.5 Hz, 1H), 6.70 (t, *J* = 54.0 Hz, 1H), 4.22 (br s, 2H), 2.22 (s, 3H).

### Intermediate 9: 4-Allyl-5-((benzyloxy)methyl)-2,4-dihydro-3H-1,2,4-triazol-3-one.

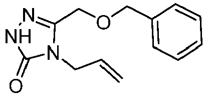

Step A: N-Allylhydrazinecarboxamide. To a solution of NH₂NH₂.H₂O (4.2 mL, 84 mmol, 98%) in DCM (70 mL) was added allyl isocyanate (7 g, 84 mmol) at -60 °C. The mixture was stirred at room temperature for 4 hours. Then the reaction mixture was cooled in ice for 30 minutes and filtered. The solid was washed with DCM. The filtrate dried under reduced pressure to give the title compound as white solid (8.2 g, yield: 84%) which was used crude in the next step without purification. ¹H NMR (400 MHz, DMSO-*d*₆) δ ppm 6.98 (s, 1H), 6.41 (s, 1H), 5.72 - 5.89 (m, 1H), 4.96 - 5.15 (m, 2H), 4.10 (s, 2H), 3.58 - 3.70 (m, 2 H).

Step B: 4-Allyl-5-((benzyloxy)methyl)-2,4-dihydro-3H-1,2,4-triazol-3-one. To a solution of N-allylhydrazinecarboxamide (8.2 g, 71 mmol) in THF (80 mL) was added benzyloxyacetyl chloride (13.1 g, 71 mmol). The mixture was cooled to 0 °C. Then 5 M aqueous NaOH solution (14 mL) was added and the mixture was stirred at 0 °C for 2 hours. The mixture was evaporated under reduced pressure. The residue was suspended in 2 M aqueous NaOH solution (114 mL), then stirred and refluxed at 95 °C overnight. The mixture was cooled room temperature. Then neutralized by the dropwise addition of 6 M aqueous HCl solution. The mixture was filtered and the filter cake was evaporated under reduced pressure to afford the title compound as white solid (10.7 g, yield: 62%).

### Intermediate 10: 5-((Benzyloxy)methyl)-4-isopropyl-2,4-dihydro-3H-1,2,4-triazol-3-one.

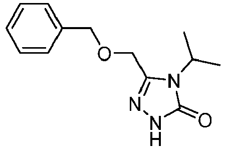

To a solution of N-isopropylhydrazinecarboxamide (12.5 g, 106.7 mmol) in 100 mL THF was added 2-(benzyloxy)acetyl chloride (19.7 g, 106.7 mmol). The mixture was cooled to 0°C, 5M NaOH (21.3 mL, 106.71 mmol) was added, and reaction was stirred at 0°C for 2 hours. The mixture was warmed to room temperature and concentrated under reduced pressure. The residue was suspended in 2M NaOH (320.1 mmol) and stirred for 12 hours at 95°C. Following heating, the reaction was cooled to 0°C and neutralized to pH 7 with 6M HCl. Upon neutralization, white solids crashed out. The solids were filtered and the filter cake dried under vacuum to provide the title compound as a white solid (13.1 g, 51.6 mmol, 48.3% yield). ¹H NMR (400 MHz, CDCl₃) δ = 1.49 - 1.50 (m, 3 H) 1.51 - 1.52 (m, 3 H) 4.30 - 4.38 (m, 1 H) 4.40 - 4.43 (m, 2 H) 4.52 - 4.56 (m, 2 H) 7.32 - 7.41 (m, 5 H) 8.61 (br s, 1 H).

### Intermediate 11: 5-((Benzyloxy)methyl)-4-(2,2-difluoroethyl)-2,4-dihydro-3H-1,2,4-triazol-3-one.

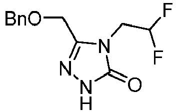

Step A: 4-Nitrophenyl (2,2-difluoroethyl)carbamate. 4-nitrophenyl chloroformate ([7693-46-1], 15 g, 74 mmol) was added to the solution of 2,2-difluoroethylamine (5 g, 62 mmol) and pyridine (9.8 g, 123 mmol) in THF (250 mL) at 0 °C. The mixture was stirred at room temperature for 1.5 hours. The reaction mixture was filtered. The filtrate was concentrated under reduced pressure to afford the title compound as white solid (20 g, crude), which was used directly in the next step.

Step B: *N*-(2,2-Difluoroethyl)hydrazinecarboxamide. To a solution of 4-nitrophenyl (2,2-difluoroethyl)carbamate (20 g crude) in THF (150 mL) was added hydrazine (98%, 3.3 mL, 67 mmol). The mixture was stirred at room temperature overnight. The reaction mixture was concentrated under reduced pressure to afford the title compound as red brown oil (21 g, crude), which was used directly in the next step.

### Step C: 5-((Benzyloxy)methyl)-4-(2,2-difluoroethyl)-2,4-dihydro-3H-1,2,4-triazol-3-one.

To a solution of N-(2,2-difluoroethyl)hydrazinecarboxamide (21 g crude) in THF (210 mL) was added benzyloxyacetyl chloride (24 mL, 151 mmol). The mixture was cooled to 0 °C, NaOH (5 M aqueous solution, 30 mL, 150 mmol) was added. The mixture was stirred at 0 °C for 2 hours. The mixture was concentrated under reduced pressure. The residue was dissolved in NaOH (2 M aqueous solution, 129 mL, 258 mmol), then stirred and refluxed overnight at 95 °C. The mixture was cooled to room temperature and filtered. The filtrate was neutralized by the drop wise addition of 6 M HCl aqueous solution. The mixture was extracted with DCM, and the organic layer was concentrated under reduced pressure. The crude was purified by flash column chromatography (SiO₂, gradient elution: 0 - 100% EtOAc in petroleum ether), then further purified by preparative reversed phase HPLC (Stationary phase: YMC-Triart Prep C18, 10 µm, 250 × 50 mm; Mobile phase: water (0.225% formic acid v/v) (A) - MeCN (B), gradient elution: 25 - 45% B in A over 20 min, flow rate: 120 mL/min) to give the title compound as a yellow solid (9.1 g, yield of 3 steps: 18%). MS (ESI): mass calcd. for C₁₂H₁₃F₂N₃O₂, 269.10; m/z found, 270.1 [M+H]⁺. ¹H NMR (400 MHz, CDCl₃) δ = 9.87 (br s, 1H), 7.30 - 7.42 (m, 5H), 5.86 - 6.19 (m, 1H), 4.55 (s, 2H), 4.48 (s, 2H), 4.14 (td, *J* = 13.8, 4.2 Hz, 2 H); ¹⁹F NMR (376 MHz, CDCl₃) δ -122.56 (t, *J* = 13.6 Hz, 1 F), -122.70 (t, *J* = 13.9 Hz, 1 F).

### Intermediate 12: 3-Chloro-4-methoxypyridin-2-amine.

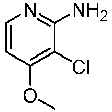

To a solution of 4-methoxypyridin-2-amine (250 mg, 2014 µmol) in chloroform (CDCl₃) (10 mL) was added portionwise 2-chloro-1,3-bis(methoxycarbonyl)guanidine (Palau'Chlor^{®})(506 mg, 2.417 mmol) at 0°C, The resulting mixture was stirred at rt for 1 hr. The reaction mixture was diluted with DCM. The combined organic layers were washed with brine, dried over MgSO₄, filtered and concentrated under reduced pressure. The residue was purified by silica gel chromatography (Isco-normal phase, 24g, SiO₂ from Heptane/Ethyl acetate=100/0 to Heptane/Ethyl acetate= 0/100) to afford the title compound (62 mg, 19% yield). MS (ESI): mass calcd. for C₆H₇ClN₂O, 158.0. ¹H NMR (400 MHz, CDCl₃) δ = 7.90 (d, *J* = 5.87 Hz, 1H), 6.34 (d, *J* = 5.87 Hz, 1H), 4.83 (br s, 2H), 3.92 (s, 3H).

### Intermediate 13: 3-Chloro-4-methoxypyridin-2-amine.

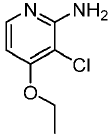

The title compound was prepared according to the representative procedure of Intermediate 12, except using 4-ethoxypyridin-2-amine instead of 4-methoxypyridin-2-amine in Step A. (106 mg, yield 17.0%). ¹H NMR (400 MHz, CDCl₃) δ = 7.87 (d, *J* = 5.87 Hz, 1H), 6.31 (d, *J* = 5.87 Hz, 1H), 4.82 (br s, 2H), 4.07 - 4.18 (m, 2H), 1.47 (t, *J* = 6.85 Hz, 4 H).

### Intermediate 14: 2-Chloro-6-methoxy-4-methylpyridin-3-amine.

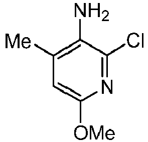

To a solution of 2,6-dichloro-4-methyl-3-nitro-pyridine (3 g, 14.49 mmol) in MeOH (20 mL) was added K₂CO₃ (3.00 g, 21.74 mmol). The mixture was stirred at 20 °C for 16 h. TLC showed the starting material was consumed completely and two new spots formed.

The mixture was poured into water (100 mL). The aqueous phase was extracted with EtOAc (100 mL×2).The combined organic layers were washed with brine (10 mL×2), dried over Na₂SO₄ filtered and concentrated in vacuo to give 3 g crude product as a yellow oil. To the 3 g crude product in AcOH (20 mL) was added Fe (8.27 g, 148.08 mmol). The mixture was stirred at 20 °C for 3 h. LCMS showed two peaks with desired mass were detected. The mixture was diluted with H₂O/EtOAc (300 mL/300 mL), and filtered. The filtrate was extracted with EtOAc (300 mL × 2). The combined organic layers were washed with NaHCO₃ (30 mL × 2) and brine (30 mL × 2), dried over Na₂SO₄, filtered and concentrated in vacuo. The crude product was purified by RP HPLC (METHOD D) to give 2-chloro-6-methoxy-4-methylpyridin-3-amine and 6-chloro-2-methoxy-4-methylpyridin-3-amine as separate fractions. The 2-chloro-6-methoxy-4-methylpyridin-3-amine was dissolved in EtOAc (5 mL). EtOAc/HCl (4 M, 5 mL) was added and the mixture was stirred at 25 °C for 10 min. The suspension was concentrated in vacuo to give the title compound (821 mg, 3.93 mmol, 100% purity, HCl salt) as a yellow solid. MS (ESI): mass calcd. For C₇H₉ClN₂O, 172.0; m/z found, 173.0 [M+H]⁺. ¹H NMR (400MHz, DMSO-*d*₆) δ = 6.64 (s, 1H), 6.46 (br s, 4H), 3.74 (s, 3H), 2.23 (s, 3H).

### Intermediate 15: 5-Chloro-2-methoxy-3-methylpyridin-4-amine.

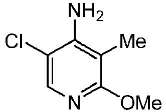

Step A. 3-Bromo-2-methoxypyridin-4-amine. To a solution of 2-methoxypyridin-4-amine (5.0 g, 40.28 mmol) in DCM (10 mL) was added NBS (7.17 g, 40.28 mmol) at 0°C. Then the reaction mixture was stirred at 25°C for 4 hrs. The solvent was removed under reduced pressure. The residue was purified by column chromatography (SiO₂, Petroleum ether/EtOAc=5/1 to 2/1) to give the title compound (7.9 g, 38.91 mmol, 96.60% yield) as a yellow oil. ¹H NMR (400 MHz, CDCl₃) δ = 7.67 (d, *J* = 5.6 Hz, 1H), 6.26 (d, *J* = 5.6 Hz, 1H), 3.90 (s, 3H).

Step B, 2-Methoxy-3-methylpyridin-4-amine. To a mixture of 3-bromo-2-methoxypyridin-4-amine (7.9 g, 38.91 mmol), 2,4,6-trimethyl-1,3,5,2,4,6-trioxatriborinane (39.08 g, 155.64 mmol) in dioxane (100 mL) and H₂O (10 mL) was added Na₂CO₃ (18.56 g, 175.09 mmol) followed by Pd(dppf)Cl₂ (2.85 g, 3.89 mmol) under N₂. Then the reaction mixture was heated to 80 °C and stirred under N₂ for 12 hrs. The reaction mixture was cooled to 25 °C and filtered through Celite^{®}. The solvent was removed under reduced pressure. The residue was purified by column chromatography (SiO₂, Petroleum ether/EttOAc=3/1 to 1/1) to give the title compound (5.00 g, 36.19 mmol, 93.01% yield) as a yellow oil. MS (ESI): mass calcd. for C₇H₁₀N₂O, 138.1; m/z found, 139.1 [M+H]⁺. ¹H NMR (400 MHz, CDCl₃) δ = 7.66 (d, *J* = 5.6 Hz, 1H), 6.24 (d, *J* = 5.6 Hz, 1H), 3.90 (s, 3H), 1.97 (m, 3H).

Step C. 5-Chloro-2-methoxy-3-methylpyridin-4-amine. To a solution of 2-methoxy-3-methylpyridin-4-amine (1.0 g, 7.24 mmol) in MeCN (15 mL) was added NCS (966.46 mg, 7.24 mmol) in MeCN (15 mL). Then the reaction mixture was stirred at 75 °C for 24 hrs. The reaction mixture was poured into H₂O (150 mL). The aqueous layer was extracted with EtOAc (50 mL × 3). The combined organic phases were dried over anhydrous Na₂SO₄, filtered and concentrated under reduced pressure. The residue was purified by RP HPLC (METHOD D). The resulting product was converted into HCl salt by treating with HCl/EtOAc (4 M) to give the title compounds (210 mg, 938.14 umol, 6.48% yield, 93.4% purity, HCl salt) as gray solid. MS (ESI): mass calcd. for C₇H₉ClN₂O, 172.0; m/z found, 173.1 [M+H]⁺. ¹H NMR (400 MHz, DMSO-*d*₆) δ = 7.85 (s, 1H), 3.90 (s, 3H), 1.97 (m, 3H).

### Intermediate 16: 6-Chloro-2-methoxy-4-methylpyridin-3-amine.

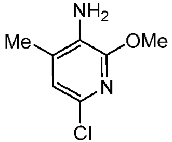

The title compound obtained as described in Intermediate was further purified by RP HPLC (method D). The resulting product was dissolved in EtOAc (5 mL). EtOAc/HCl(4 M, 5 mL) was added and the mixture was stirred at 25 °C for 10 min.

The suspension was concentrated in vacuo to give the tiltle compound (711.41 mg, 3.40 mmol, 100% purity, HCl salt) as a yellow solid. MS (ESI): mass calcd. For C₇H₉ClN₂O, 172.0; m/z found, 173.0 [M+H]⁺. ¹H NMR (400MHz, DMSO-*d*₆) δ = 7.29 (br s, 3H), 6.88 (s, 1H), 3.88 (s, 3H), 2.19 (s, 3H).

### Intermediate 17: 4-Amino-5-fluoro-2-methoxynicotinonitrile.

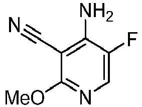

Step A. 3-Bromo-5-fluoro-2-methoxypyridin-4-amine. To a mixture of 5-fluoro-2-methoxypyridin-4-amine (1 g, 7.04 mmol) in DCM (45 mL) was added NBS (1.31 g, 7.39 mmol) in one portion at 0°C under N₂. The mixture was stirred at 30 °C for 1 hour. The mixture was poured into water (40 mL). The aqueous phase was extracted with DCM (30 mL × 2). The combined organic phase was washed with brine (20 mL × 2), dried with anhydrous Na₂SO₄, filtered and concentrated in vacuum. The residue was purified by silica gel chromatography (1000 mesh silica gel, petroleum ether/EtOAc=20/1, 5/1) to give the title compound (1.48 g, 6.62 mmol, 94% yield, 98.8% purity) as yellow solid. MS (ESI): mass calcd. for C₆H₆BrFN₂O, 219.9; m/z found, 221.0 [M+H]⁺. ¹H NMR (400 MHz, CDCl₃) δ = 7.73 (d, *J* = 2.0 Hz, 1H), 4.76 - 4.65 (m, 2H), 3.99 - 3.92 (m, 3H).

Step B. 4-Amino-5-fluoro-2-methoxynicotinonitrile. To a mixture of 3-bromo-5-fluoro-2-methoxypyridin-4-amine (1.4 g, 6.33 mmol) in NMP (15 mL) was added CuCN (1.13 g, 12.67 mmol) in one portion under N₂. The mixture was stirred at 145 °C for 24 hours. The mixture was poured into water (50 mL). The aqueous phase was extracted with EtOAc (50 mL × 3). The combined organic phase was washed with brine (15 mL × 2), dried with anhydrous Na₂SO₄, filtered and concentrated in vacuum. The residue was purified by silica gel chromatography (1000 mesh silica gel, Petroleum ether/EtOAc=20/1, 3/1) to give the title compound (1 g, 5.95 mmol, 93.99% yield, 99.5% purity) as yellow solid. MS (ESI): mass calcd. for C₇H₆FN₃O, 167.0; m/z found, 168.1 [M+H]⁺. ¹H NMR (400 MHz, CDCl₃) δ = 7.85 (d, *J* = 2.8 Hz, 1H), 5.15 - 4.91 (m, 2H), 4.02 - 3.91 (m, 3H).

### Intermediate 18: 4-Fluoro-2-methoxypyridin-3-amine.

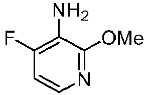

Step A: *tert*-Butyl (4-fluoro-2-methoxypyridin-3-yl)carbamate. *tert*-VButyl (2-methoxypyridin-3-yl) carbamate ([161117-83-5], 4 g, 17.8 mmol) was dissolved in TEMED (5.4 mL, 35.7 mmol) and THF (80 mL) under N₂. Then *n*-butyllithium (2.5 M in hexane, 28.5 mL, 71.3 mmol) was added to the mixture at -15 °C. After 1.5 hours at - 15 °C, NFSI ([133745-75-2], 6.2 g, 19.6 mmol) in THF (28 mL) was added to the mixture. The mixture was allowed slowly to warm to room temperature overnight. The mixture was added to saturated aqueous NH₄Cl solution and concentrated under vacuum. The residue was extracted with EtOAc. The organic layer was dried with Na₂SO₄, filtered and concentrated under reduced pressure. The residue was purified by flash column chromatography (SiO₂, gradient elution: 0 ~ 20% EtOAc in petroleum ether) to give the title compound as a yellow solid (1.75 g, yield: 36%). MS (ESI): mass calcd. for C₁₁H₁₅FN₂O₃, 242.11; m/z found, 243.0 [M+H]⁺. ¹H NMR (400 MHz, CDCl₃) δ = 7.94 (dd, *J* = 7.7, 5.9 Hz, 1H), 6.74 (dd, *J* = 8.8, 5.8 Hz, 1H), 5.92 (br s, 1H), 4.00 (s, 3H), 1.51 (s, 9 H); ¹⁹F NMR (376 MHz, CDCl₃) δ = -108.16 (br s, 1 F).

Step B: 4-Fluoro-2-methoxypyridin-3-amine. A solution of *tert*-butyl (4-fluoro-2-methoxypyridin-3-yl)carbamate (1.4 g, 5.78 mmol) in toluene (20 mL) was added to silica gel (6.9 g, 116 mmol). The mixture was stirred at 100 °C overnight then cooled down to room temperature. The mixture was concentrated under reduced pressure (below 25 °C). The residue was purified by flash column chromatography (SiO₂, gradient elution: 0 - 15% EtOAc in petroleum ether) to give the title compound as a yellow oil (700 mg, yield: 85%). MS (ESI): mass calcd. for C₆H₇FN₂O, 142.05; m/z found, 143.1 [M+H]⁺. ¹H NMR (400 MHz, CDCl₃) δ =7.52 (dd, *J* = 7.9, 5.7 Hz, 1H), 6.65 (dd, *J* = 9.5, 5.7 Hz, 1H), 3.99 - 4.05 (s, 3H), 3.69 (br s, 2 H); ¹⁹F NMR (376 MHz, CDCl₃) δ = - 127.93 - -127.06 (m, 1 F).

### Intermediate 19: 4-Chloro-2-methoxypyridin-3-amine.

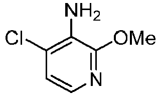

To a solution of 4-chloro-2-methoxy-3-nitropyridine ([934180-48-0], 300 mg, 1.6 mmol) in MeOH/THF/H₂O (v/v/v, 1/1/1, 9 mL) was added iron powder (444 mg, 8.0 mmol) and NH₄Cl (426 mg, 8.0 mmol). The reaction mixture was stirred at 70 °C for 1.5 hours. The reaction mixture was filtered through a pad of Celite^{®}. The solid was rinsed with EtOAc. The filtrate was concentrated under reduced pressure. Then, the residue was diluted with EtOAc and washed with saturated aqueous NaHCO₃ solution. The aqueous layer was extracted with EtOAc. The organic layer was dried with Na₂SO₄, filtered and concentrated under reduced pressure. The crude product was purified by flash column chromatography (SiO₂, gradient elution: 0 - 10% EtOAc in petroleum ether) to give the title compound as a brown semi-solid (160 mg, yield: 63%). MS (ESI): mass calcd. for C₆H₇ClN₂O, 158.02; m/z found, 159.0 [M+H]⁺. ¹H NMR (400 MHz, DMSO-*d*₆) δ = 7.34 (d, *J* = 5.5 Hz, 1H), 6.89 (d, *J* = 5.5 Hz, 1H), 5.17 (br s, 2H), 3.89 (s, 3 H).

### Intermediate 20: 6-(3-((Benzyloxy)methyl)-4-ethyl-5-oxo-4,5-dihydro-1H-1,2,4-triazol-1 -yl)-5 -fluoro-2-isopropoxynicotinic acid

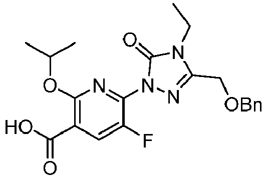

Step A: Isopropyl 6-(3-((benzyloxy)methyl)-4-ethyl-5-oxo-4,5-dihydro-1H-1,2,4-triazol-1-yl)-5-fluoro-2-isopropoxynicotinate. The title compound was prepared in a manner analogous to Example 1, Steps A - D except substituting isopropanol for (2S)-1,1,1-trifluoropropan-2-ol in Step D. MS (ESI): mass calcd. for C₂₄H₂₉FN₄O₅, 472.2; m/z found, 473 [M+H]⁺. ¹H NMR (CDCl₃, 400 MHz) δ 8.03 (d, 1H, *J* = 9.3 Hz), 7.3-7.4 (m, 5H), 5.3-5.4 (m, 1H), 5.2-5.3 (m, 1H), 4.6-4.6 (m, 2H), 4.5-4.5 (m, 2H), 3.8-3.9 (m, 2H), 1.3-1.4 (m, 15H).

Step B: 6-(3-((Benzyloxy)methyl)-4-ethyl-5-oxo-4,5-dihydro-1H-1,2,4-triazol-1-yl)-5-fluoro-2-isopropoxynicotinic acid. To a vial was added Isopropyl 6-(3-((benzyloxy)methyl)-4-ethyl-5-oxo-4,5-dihydro-1H-1,2,4-triazol-1-yl)-5-fluoro-2-isopropoxynicotinate (240 mg, 0.508 mmol), lithium hydroxide (243 mg, 10.2 mmol), 1,4-dioxane (5.4 mL) and water (0.96 mL). The reaction was heated to 100 °C for 2 hr. The mixture was concentrated and diluted in EtOAc (100 mL) and water (25 mL) and 1N HCl was added to reach pH=1. The aqueous layer was extracted with EtOAc (3 × 100 mL). The organics were dried, filtered, and concentrated and yielded the crude title compound as a white solid. MS (ESI): mass calcd. for C₂₁H₂₃FN₄O₅, 430.2; m/z found, 431 [M+H]⁺. ¹H NMR (CDCl₃, 400 MHz) δ 8.34 (d, 1H, *J* = 9.3 Hz), 7.3-7.4 (m, 5H), 5.5-5.7 (m, 1H), 4.61 (s, 2H), 4.52 (s, 2H), 3.86 (q, 2H, *J* = 7.3 Hz), 1.50 (d, 6H, *J* = 5.9 Hz), 1.36 (t, 4H, *J* = 7.1 Hz).

### Intermediate 21: Isopropyl 6-(4-ethyl-3-(hydroxymethyl)-5-oxo-4,5-dihydro-1H-1,2,4-triazol-1 -yl)-5 -fluoro-2-isopropoxynicotinate.

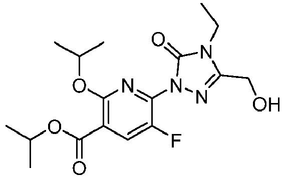

Isopropyl 6-(3-((benzyloxy)methyl)-4-ethyl-5-oxo-4,5-dihydro-1H-1,2,4-triazol-1-yl)-5-fluoro-2-isopropoxynicotinate (Intermediate 20, Step A, 150 mg, 0.317mmol) was dissolved in methanol (3 mL) and Pd-C (30% on carbon, 30 mg) was added. The reaction was stirred at rt under a atmosphere of hydrogen gas for 1.5 hr. The reaction was filtered and concentrated and yielded the title compound, which was used without further purification. MS (ESI): mass calcd. for C₁₇H₂₃FN₄O₅, 382.2; m/z found, 383 [M+H]⁺. ¹H NMR (CHLOROFORM-d, 400 MHz) δ 8.03 (d, 1H, *J*=9.3 Hz), 5.3-5.4 (m, 1H), 5.2-5.3 (m, 1H), 4.66 (s, 2H), 3.89 (q, 2H, *J*=7.3 Hz), 1.3-1.4 (m, 15H)

### Example 1: (S)-N-(2-Chloro-6-fluorophenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

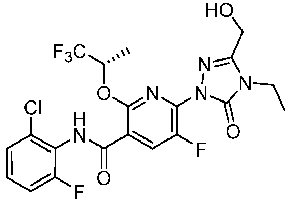

Step A: 2,6-Dichloro-5-fluoronicotinoyl chloride. To a solution of 2,6-dichloro-5-fluoronicotinic acid (20 g, 95.24 mmol) in tetrahydrofuran (THF) (200 mL) was added (COCl)₂ (12.69 g, 100.00 mmol, 8.75 mL) and dimethylformamide (DMF) (69.62 mg, 952.43 µmol, 73.28 µL) at 0 °C dropwise. The mixture was stirred at 0 °C for 30 min, then warmed to 25 °C, and stirred for 1 hour. The mixture was filtered and concentrated under reduced pressure to afford desired product (21.7 g, crude) as a colorless oil, which was used crude in the next step without further purification.

Step B: Isopropyl 2.6-dichloro-5-fluoronicotinate. To a mixture of propan-2-ol (8.56 g, 142.49 mmol, 10.91 mL) and pyridine (9.02 g, 113.99 mmol, 9.20 mL) in THF (200 mL) was added 2,6-dichloro-5-fluoronicotinoyl chloride (21.7 g, 95.2 mmol) in THF (50 mL) at 0 °C. The mixture was stirred at 25 °C for 1 h. The mixture was poured into water (300 mL). The aqueous phase was extracted with ethyl acetate (EtOAc) (300 mL). The combined organic phase was dried with anhydrous Na₂SO₄, filtered and concentrated in vacuum. The residue was purified by column chromatography (SiO₂, Petroleum ether (PE)/Ethyl acetate= 1/1 to 10: 1) to afford the title compound (21 g, 87% yield, 99% purity). MS (ESI): mass calcd. for C₉H₈Cl₂FNO₂, 250.1; m/z found, 252.0 [M+H]⁺. ¹H NMR (400MHz, CDCl₃) δ = 7.975-7.957 (d, *J* = 7.2 Hz, 1H), 5.321 - 5.259 (m, 1H), 1.587-1.395 (m, 6H).

Step C: Isopropyl 6-(3-((benzyloxy)methyl)-4-ethyl-5-oxo-4,5-dihydro-1H-1,2,4-triazol-1-yl)-2-chloro-5-fluoronicotinate. To a mixture of isopropyl 2,6-dichloro-5-fluoronicotinate (3 g, 11.90 mmol) in dimethylsulfoxide (DMSO) (30 mL) was added 3-((benzyloxy)methyl)-4-ethyl-1H-1,2,4-triazol-5(4H)-one (2.92 g, 12.50 mmol) and K₂CO₃ (2.47 g, 17.85 mmol). The mixture was stirred at 80 °C for 3 hours. The mixture was diluted with H₂O (30 mL) and extracted with EtOAc (60 mL*3). The combined organic layers were washed with brine (50 mL), dried over Na₂SO₄, filtered and concentrated under reduced pressure. The residue was purified by column chromatography (SiO₂, Petroleum ether/Ethyl acetate= 1/0 to 1:1) to afford the title compound (4 g, 75% yield). MS (ESI): mass calcd. for C₂₁H₂₂ClFN₄O₄, 448.1; m/z found, 449.2 [M+H]⁺. ¹H NMR (400MHz, CDCl₃) δ = 8.02 - 8.00 (d, *J* = 8.8 Hz, 1H), 7.29 - 7.18 (m, 5H), 5.23-5.20 (m, 1H), 4.52-4.45 (d, *J* = 27.7 Hz, 4H), 3.78-3.73 (m, 2H), 1.33-1.32 (d, *J* = 6.4 Hz, 6H), 1.273-1.237 (t, *J* = 7.2 Hz, 3H).

Step D: (S)-Isopropyl 6-(3-((benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinate. To a solution of (2S)-1,1,1-trifluoropropan-2-ol (55.91 mg, 490.11 µmol, 51.16 µL) in THF (1.5 mL) was added potassium bis(trimethylsilyl)amide (KHMDS) (1 M, 534.67 µL) at -10°C under N₂. The mixture was stirred at -10 °C for 30 min, then isopropyl 6-(3-((benzyloxy)methyl)-4-ethyl-5-oxo-4,5-dihydro-1H-1,2,4-triazol-1-yl)-2-chloro-5-fluoronicotinate (200 mg, 445.56 µmol) in THF (0.5 mL) was added to the mixture at - 10 °C, then stirred at -10 °C for 4 hours. The mixture was poured into 1N HCl (10 mL). The aqueous phase was extracted with ethyl acetate (15 mL*2). The combined organic phase was washed with brine (15 mL*2), dried with anhydrous Na₂SO₄, filtered and concentrated in vacuum. The residue was purified by silica gel chromatography (1000 mesh silica gel, Petroleum ether/Ethyl acetate= 15/1, 3/1) to afford the title compound (80 mg, 34% yield) as yellow solid. MS (ESI): mass calcd. for C₂₄H₂₆F₄N₄O₅, 526.2; m/z found, 527.3.

### Step E: (S)-6-(3-((Benzyloxy)methyl)-4-ethyl-5-oxo-4,5-dihydro-1H-1,2,4-triazol-1-yl)-N-(2-chloro-6-fluorophenyl)-5-fluoro-2-((1,1,1-trifluoropropan-2-yl)oxy)nicotinamide.

To a solution of 2-chloro-6-fluoroaniline (49.77 mg, 341.89 µmol) in dichloromethane (DCM) (2 mL) was added dropwise trimethylaluminium (AlMe₃) (2 M, 341.89 µL) at 25 °C for 0.5 hour, then added solution of (S)-isopropyl 6-(3-((benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinate (90 mg, 170.95 µmol) in DCM (1mL), The resulting mixture was stirred at 50 °C for 12 hr. The reaction mixture was diluted with H₂O (15 mL) and extracted with DCM (20 mL * 3). The combined organic layers were washed with brine (20 mL), dried over Na₂SO₄, filtered and concentrated under reduced pressure. The residue was purified by prep-TLC (SiO₂, PE: EtOAc = 1:1) to afford the title compound (57 mg, 53% yield, 97.9% purity) as a yellow solid. MS (ESI): mass calcd. for C₂₇H₂₃ClF₅N₅O₄, 611.1; m/z found, 612.2.

### Step F: (S)-N-(2-Chloro-6-fluorophenyl)-6-(4-ethyl-3-(hydroxymethyl)-5-oxo-4,5-dihydro-1H-1,2,4-triazol-1-yl)-5 -fluoro-2-((1,1,1-trifluoropropan-2-yl)oxy)nicotinamide.

A mixture of (S)-6-(3-((benzyloxy)methyl)-4-ethyl-5-oxo-4,5-dihydro-1H-1,2,4-triazol-1-yl)-N-(2-chloro-6-fluorophenyl)-5-fluoro-2-((1,1,1 -trifluoropropan-2-yl)oxy)nicotinamide (20 mg, 32.68 µmol), Pd/C (2 mg, 10% purity) and HCl (12 M, 2.04 µL, 0) in EtOH (2 mL) was degassed and purged with H₂ for 3 times. The mixture was stirred at 25 °C for 12 hr under H₂ atmosphere (15 psi). The reaction mixture was filtered. Another batch of Pd/C (2 mg, 10% purity) was added into the filtrate. The resulting mixture was stirred under H₂ atmosphere (15 psi) at 25°C for another 32 hr. The reaction mixture was filtered and concentrated under reduced pressure. The residue was purified by prep-HPLC (Method A) to afford the title compound (7.32 mg, 43% yield, 99.7% purity) as a white solid. MS (ESI): mass calcd. for C₂₀H₁₇ClF₅N₅O₄, 521.1; m/z found, 522.1 [M+H]⁺. ¹H NMR (400MHz, CDCl₃) δ = 9.115 (s, 1H), 8.578-8.554 (d, *J* = 9.6 Hz, 1H), 7.298-7.236 (m, 1H), 7.15 (m, 1H), 5.97 (m, 1H), 4.712-4.697 (d, *J* = 6 Hz, 2H), 3.951-3.851 (m, 2H), 2.090 (s, 1H), 1.695-1.678 (d, *J* = 6.8 Hz, 3H), 1.458-1.422 (t, *J* = 7.2 Hz, 3H).

### Example 2: (S)-6-(4-Ethyl-3-(hydroxymethyl)-5-oxo-4,5-dihydro-1H-1,2,4-triazol-1-yl)-5-fluoro-N-(2-fluorophenyl)-2-((1,1,1-trifluoropropan-2-yl)oxy)nicotinamide.

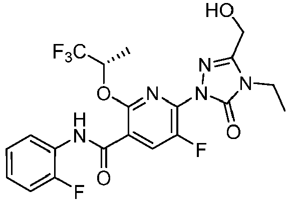

The title compound was prepared according to the representative procedure of Example 1, Steps E and F, except using 2-fluoroaniline instead of 2-chloro-6-fluoroaniline in Step E. MS (ESI): mass calcd. for C₂₀H₁₈F₅N₅O₄, 487.1; m/z found 488.1. ¹H NMR (400 MHz, CDCl₃) δ = 9.89 - 9.79 (m, 1H), 8.64 - 8.47 (m, 2H), 7.23 - 7.11 (m, 3H), 6.08 - 5.96 (m, 1H), 4.70 (s, 2H), 3.97 - 3.87 (m, 2H), 1.73 - 1.66 (m, 3H), 1.46 - 1.42 (m, 3H).

### Example 3: (S)-N-(2-Chloro-6-fluorophenyl)-6-(4-ethyl-3-(fluoromethyl)-5-oxo-4,5-dihvdro-1H-1,2,4-triazol-1-yl)-5-fluoro-2-((1,1,1-trifluoropropan-2-yl)oxy)nicotinamide.

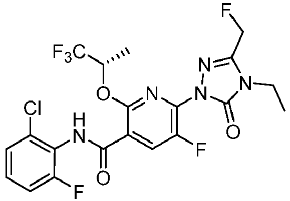

To a solution of (S)-N-(2-chloro-6-fluorophenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide (Example 1, 110 mg, 0.21 mmol) in dichloromethane (DCM) (3.5 mL) was added dropwise diethylaminosulfur trifluoride (DAST) (0.14 mL, 1.05 mmol) at 0 °C. The reaction mixture was stirred at room temperature (RT, rt) for 12 h and quenched with sat aq. NaHCO₃ (20 mL), extracted with DCM (30 mL), dried (Na₂SO₄) and concentrated under reduced pressure. The residue was purified by silica gel column chromatography (35-50% EtOAc/Heptane) to afford the title compound (18 mg, 16 % yield) as a white solid. MS (ESI): mass calcd. for C₂₀H₁₆ClF₆N₅O₃, 523.1; m/z found, 524 [M+H]⁺. ¹H NMR (400MHz, CDCl₃) δ = 9.12 (s, 1H), 8.58 (d, *J* = 9.4 Hz, 1H), 7.27 (m, 2H), 7.14 (t, *J* = 8.7 Hz, 1H), 5.94 (m, 1H), 5.44 (d, *J* = 1.1 Hz, 1H), 5.32 (d, *J* = 1.1 Hz, 1H), 3.91 (q, *J* = 7.2 Hz, 2H), 1.68 (d, *J* = 6.5 Hz, 3H), 1.44 (t, *J* = 7.2 Hz, 3H).

### Example 4: (S)-N-(2-Chloropyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

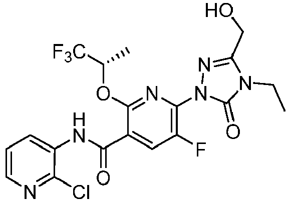

Step A: (S)-6-(3-((Benzyloxy)methyl)-4-ethyl-5-oxo-4,5-dihydro-1H-1,2,4-triazol-1-yl)-N-(2-chloropyridin-3-yl)-5-fluoro-2-((1,1,1-trifluoropropan-2-yl)oxy)nicotinamide. To a solution of 3-amino-2-chloropyridine (146.26 mg, 1138 µmol) in dichloromethane (DCM) (2 mL) was added dropwise trimethylaluminum (AlMe₃) (2 M, 950 µL) at 0 °C, then added solution of (S)-isopropyl 6-(3-((benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinate (Example 1, product from Step D, 200 mg, 380 µmol) in DCM (1mL), The resulting mixture was stirred at 50 °C for 12 hr. The reaction mixture was quenched with MeOH (1 mL) and diluted with DCM (30 mL). The combined organic layers were washed with brine (20 mL * 3), dried over Na₂SO₄, filtered and concentrated under reduced pressure to afford the title compound as a yellow solid and used without purification. MS (ESI): mass calcd. for C₂₇H₂₃ClF₅N₅O₄, 594.9; m/z found, 595.

Step B: (S)-N-(2-Chloropyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide. A mixture of (S)-6-(3-((benzyloxy)methyl)-4-ethyl-5-oxo-4,5-dihydro-1H-1,2,4-triazol-1-yl)-N-(2-chloropyridin-3-yl)-5-fluoro-2-((1,1,1-trifluoropropan-2-yl)oxy)nicotinamide (68 mg, 114 µmol) and trifluoroacetic acid (TFA) (3 mL, 0.6 mmol) was stirred at 70 °C for 12 h. The reaction mixture was filtered and concentrated under reduced pressure.

The residue was purified by prep-HPLC (Method F) to afford the title compound (20 mg, 35% yield) as a white solid. MS (ESI): mass calcd. for C₁₉H₁₇ClF₄N₆O₄, 504.8; m/z found, 505 [M+H]⁺. ¹H NMR (400MHz, CDCl₃) δ = 9.84 (s, 1H), 8.87 (d, *J* = 8.2 Hz, 1H), 8.54 (dd, *J* = 9.6, 1.3 Hz, 1H), 8.20 (dd, *J* = 4.7, 1.6 Hz, 1H), 7.33 (ddd, *J* = 8.3, 4.7, 1.1 Hz, 1H), 6.17 (h, *J* = 6.5 Hz, 1H), 4.70 (dd, *J* = 6.3, 1.8 Hz, 2H), 4.00 - 3.85 (m, 2H), 1.70 (d, *J* = 6.6 Hz, 3H), 1.54 - 1.35 (m, 3H).

### Example 5: (S)-N-(2-Chloro-4-methylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

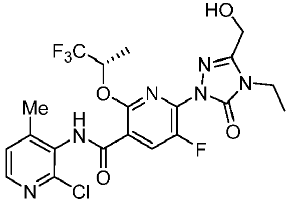

The title compound was prepared according to the representative procedure of Example 4, Steps A and B, except substituting 3-amino-2-chloro-4-methylpyridine for 3-amino-2-chloropyridine in Step A. MS (ESI): mass calcd. for C₂₀H₁₉ClF₄N₆O₄, 518.1; m/z found, 519 [M+H]⁺. ¹H NMR (400MHz, CDCl₃) δ = 9.21 (s, 1H), 8.53 (d, *J* = 9.5 Hz, 1H), 8.23 (d, *J* = 4.9 Hz, 1H), 7.21 (d, *J* = 4.9 Hz, 1H), 5.98 (m, 1H), 4.69 (s, 2H), 3.92 (q, *J* = 7.2 Hz, 2H), 2.35 (s, 3H), 1.69 (d, *J* = 6.4 Hz, 3H), 1.43 (t, *J* = 7.2 Hz, 3H).

### Example 6: (S)-N-(2-Chloro-6-fluorophenyl)-6-(3-(chloromethyl)-4-ethyl-5-oxo-4,5-dihydro-1H-1,2,4-triazol-1-yl)-5-fluoro-2-((1,1,1 -trifluoropropan-2-yl)oxy)nicotinamide.

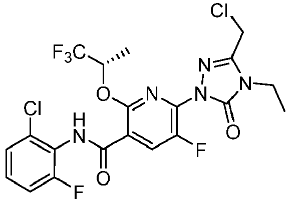

To a solution of (S)-N-(2-chloro-6-fluorophenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide (Example 1, 23 mg, 0.044 mmol) in benzene (2 mL) was added dropwise thionyl chloride (6.5 mL, 0.088 mmol) at 0 °C. The reaction mixture was stirred at 50 °C for 12 h and quenched with sat aq. NaHCO₃ (20 mL), extracted with DCM (30 mL), dried (Na₂SO₄) and concentrated under reduced pressure. The residue was purified by silica gel column chromatography (35-50% EtOAc/Heptane) to afford the title compound (8 mg, 33 % yield, 99% purity) as a white solid. MS (ESI): mass calcd. for C₂₀H₁₆Cl₂E₅N₅O₃, 540.3; m/z found, 541.2 [M+H]⁺. ¹H NMR (400MHz, CDCl₃) δ = 9.13 (s, 1H), 8.58 (d, *J* = 9.5 Hz, 1H), 7.42 - 7.03 (m, 3H), 6.10 - 5.85 (m, 1H), 4.55 (s, 2H), 3.94 (q, *J* = 7.2 Hz, 2H), 1.68 (d, *J* = 6.5 Hz, 3H), 1.47 (t, *J* = 7.2 Hz, 3H).

### Example 7: N-(2-Chloro-6- fluorophenyl)-6-(4-ethyl-3-(hydroxymethyl)-5-oxo-4,5-dihydro-1H-1,2,4-triazol-1-yl)-5-fluoro-2-isopropoxynicotinamide.

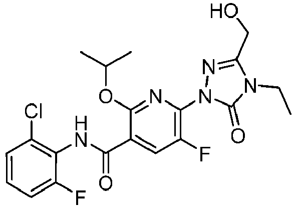

The title compound was prepared according to the representative procedure of Example 1, Steps A to F except substituting isopropanol for (2S)-1,1,1-trifluoropropan-2-ol in Step D. MS (ESI): mass calcd. for C₂₀H₂₀ClF₂N₅O₄, 467.1; m/z found, 468 [M+H]⁺. ¹H NMR (400 MHz, CDCl₃) δ 9.72 (s, 1H), 8.50 (d, *J* = 9.78 Hz, 1H), 7.28-7.32 (m, 1H), 7.20-7.25 (m, 1H), 7.14 (dt, *J* = 1.47, 8.80 Hz, 1H), 5.61 (td, *J* = 6.30, 12.35 Hz, 1H), 4.69 (d, *J* = 6.36 Hz, 2H), 3.91 (q, *J* = 7.34 Hz, 2H), 2.15 (t, *J* = 6.36 Hz, 1H), 1.51 (d, *J* = 5.87 Hz, 6H), 1.42 (t, *J* = 7.09 Hz, 3H).

### Example 8: Racemic-N-(2-Chloro-6-fluorophenyl)-6-(4-ethyl-3-(hydroxymethyl)-5-oxo-4,5-dihydro-1H-1,2,4-triazol-1-yl)-5-fluoro-2-(2,2,3,3-tetrafluorocyclobutoxy)nicotinamide.

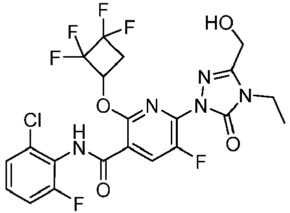

The title compound was prepared according to the representative procedure of Example 1, Steps A to F, except substituting 2,2,3,3-tetrafluorocyclobutan-1-ol for (2S)-1,1,1-trifluoropropan-2-ol in Step D. MS (ESI): mass calcd. for C₂₁H₁₆ClF₆N₅O₄, 551.1; m/z found, 574 [M+Na]⁺. ¹H NMR (400MHz, CDCl₃) δ = 9.09 (s, 1H), 8.56 (d, *J* = 9.5 Hz, 1H), 7.31 (d, *J* = 7.1 Hz, 2H), 7.20 - 7.12 (m, 1H), 5.79 - 5.60 (m, 1H), 4.70 (s, 2H), 3.92 (q, *J* = 7.2 Hz, 2H), 3.37 (s, 1H), 2.72 (dq, *J* = 14.2, 6.9 Hz, 1H), 1.43 (t, *J* = 7.2 Hz, 3H).

### Example 9: Racemic-N-(2-Chloro-6-fluorophenyl)-6-(4-ethyl-3-(hydroxymethyl)-5-oxo-4,5-dihydro-1H-1,2,4-triazol-1-yl)-5-fluoro-2-((tetrahydrofuran-3-yl)oxy)nicotinamide.

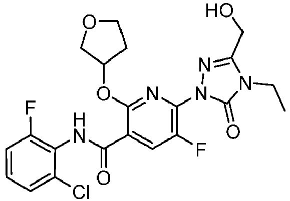

The title compound was prepared according to the representative procedure of Example 1, Steps A to F except substituting *racemic-* tetrahydrofuran-3-ol for (2S)-1,1,1-trifluoropropan-2-ol in Step D. MS (ESI): mass calcd. for C₂₁H₂₀ClF₂N₅O₅, 495.1; m/z found, 496 [M+H]⁺. ¹H NMR (400 MHz, CDCl₃) δ 9.44 (s, 1H), 8.51 (d, *J* = 9.78 Hz, 1H), 7.26-7.34 (m, 2H), 7.14 (m, 1H), 5.82-5.93 (m, 1H), 4.68 (d, *J* = 6.36 Hz, 2H), 3.99-4.17 (m, 3H), 3.84-3.98 (m, 3H), 2.25-2.47 (m, 3H), 1.42 (t, *J* = 7.09 Hz, 3H).

### Example 10: (S)-N-(3-Chloropyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

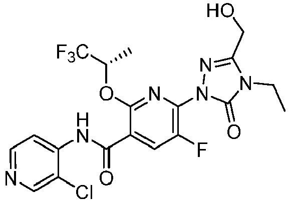

Step A: (S)-6-(3-((Benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinic acid. To a solution of (S)-isopropyl 6-(3-((benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinate (5.6 g, 10.6 mmol, Example 1, Step D) in 1,4-dioxane/ H₂O (1: 1, 45 mL) was added LiOH (5.1 g, 212.7 mmol). The resulting mixture was stirred at 100 °C for 16 h, after which time it was concentrated then diluted with EtOAc and water. The mixture was acidified with 1N HCl then the organics were extracted with EtOAc (3x 50 mL). The combined organics were washed with brine, dried over sodium sulfate, filtered and concentrated. The crude product was used without further purification. MS (ESI): mass calcd. for C₂₁H₂₀F₄N₄O₅, 484.4; m/z found, 485.

Step B: (S)-6-(3-((Benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinoyl chloride. To a solution of (S)-6-(3-((benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinic acid (300 mg, 0.62 mmol) in DCM (1 mL) was added oxalyl chloride (0.08 mL, 1 mmol) and DMF (5 µL, 62 µmol) at 0 °C. The mixture was stirred at RT for 2 h, after which time it was concentrated to provide the title compound and used without further purification. MS (ESI): mass calcd. for C₂₁H₁₉ClF₄N₄O₄, 502.9.

Step C: (S)-6-(3-((Benzyloxy)methyl)-4-ethyl-5-oxo-4,5-dihydro-1H-1,2,4-triazol-1-yl)-N-(3-chloropyridin-4-yl)-5-fluoro-2-((1,1,1-trifluoropropan-2-yl)oxy)nicotinamide. A mixture of 4-amino-3-chloropyridine (38.3 mg, 0.3 mmol), (S)-6-(3-((benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinoyl chloride (100 mg, 0.2 mmol), and triethylamine (55.3 µL, 398 µmol) in DCM (1 mL) was stirred at RT for 16 h, after which time it was concentrated under reduced pressure. The crude reaction product was used directly in the next step without further purification.

Step D: (S)-N-(3-Chloropyridin-4-yl)-6-(4-ethyl-3-(hydroxymethyl)-5-oxo-4,5-dihydro-1H-1,2,4-triazol-1-yl)-5-fluoro-2-((1,1,1 -trifluoropropan-2-yl)oxy)nicotinamide. (S)-6-(3-((Benzyloxy)methyl)-4-ethyl-5-oxo-4,5-dihydro-1H-1,2,4-triazol-1-yl)-N-(3-chloropyridin-4-yl)-5-fluoro-2-((1,1,1-trifluoropropan-2-yl)oxy)nicotinamide (crude from step C) was dissolved in TFA and stirred at 70 °C for 16 h. The reaction mixture was filtered and concentrated under reduced pressure. The residue was purified by prep-HPLC (Method F) to afford the title compound (65 mg, 65% yield, 99% purity) as a white solid. MS (ESI): mass calcd. for C₁₉H₁₇ClF₄N₆O₄, 504.8; m/z found, 505 [M+H]+. ¹H NMR (400MHz, CDCl₃) δ = 9.95 (s, 1H), 8.74 - 8.37 (m, 4H), 6.20 (hept, *J* = 6.5 Hz, 1H), 4.70 (d, *J* = 5.2 Hz, 2H), 3.92 (q, *J* = 7.2 Hz, 2H), 1.69 (d, *J* = 6.6 Hz, 3H), 1.43 (t, *J* = 7.2 Hz, 3H).

### Example 11: (S)-6-(4-Ethyl-3-(hydroxymethyl)-5-oxo-4,5-dihydro-1H-1,2,4-triazol-1-yl)-5-fluoro-N-(2-fluoro-5-methylphenyl)-2-((1,1,1-trifluoropropan-2-yl)oxy)nicotinamide.

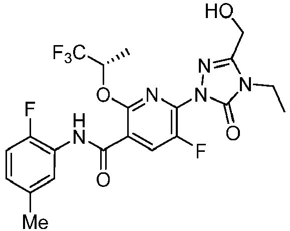

The title compound was prepared according to the representative procedure of Example 10, Steps A to D, except substituting 2-fluoro-5-methylaniline for 4-amino-3-chloropyridine in Step C. MS (ESI): mass calcd. for C₂₁H₂₀F₅N₅O₄, 501.1; m/z found, 502 [M+H]⁺. ¹H NMR (400MHz, CDCl₃) δ = 9.16 (s, 1H), 8.58 (d, *J* = 9.7 Hz, 1H), 7.94 (dd, *J* = 10.9, 2.7 Hz, 1H), 7.18 (dd, *J* = 8.4, 6.3 Hz, 1H), 6.84 (td, *J* = 8.2, 2.7 Hz, 1H), 6.09 (m, 1H), 4.70 (d, *J* = 5.7 Hz, 2H), 3.92 (q, *J* = 7.2 Hz, 2H), 2.30 (s, 3H), 2.15 (t, *J*= 6.2 Hz, 1H), 1.67 (d, *J* = 6.6 Hz, 3H), 1.43 (t, *J* = 7.2 Hz, 3H).

### Example 13: (S)-N-(2-(Difluoromethyl)-4-methylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

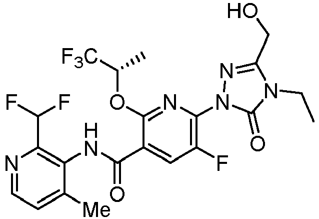

The title compound was prepared according to the representative procedure of Example 10, Step A to D, except substituting 2-(difluoromethyl)-4-methylpyridin-3-amine (Intermediate 8) for 4-amino-3-chloropyridine in Step C. MS (ESI): mass calcd. for C₂₁H₂₀F₆N₆O₄, 534.1; m/z found, 535 [M+H]⁺. ¹H NMR (400MHz, CDCl₃) δ = 9.37 (s, 1H), 8.50 (dd, *J* = 30.4, 7.3 Hz, 2H), 7.40 (d, *J* = 4.9 Hz, 1H), 6.70 (t, *J* = 54.3 Hz, 1H), 6.01 (m, 1H), 4.69 (s, 2H), 3.92 (q, *J* = 7.2 Hz, 2H), 2.35 (s, 3H), 1.66 (d, *J* = 6.6 Hz, 3H), 1.43 (t, *J* = 7.2 Hz, 3H).

### Example 14: (S)-N-(2-Chloro-6-fluorophenyl)-6-(4-ethyl-3-(methoxymethyl)-5-oxo-4,5-dihydro- 1H-1,2,4-triazol-1-yl)-5-fluoro-2-((1,1,1-trifluoropropan-2-yl)oxy)nicotinamide.

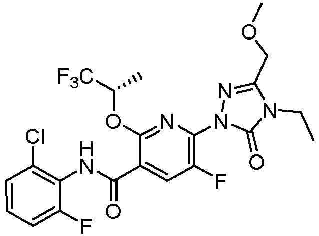

To a solution of (S)-N-(2-chloro-6-fluorophenyl)-6-(3-(chloromethyl)-4-ethyl-5-oxo-4,5-dihydro- 1H-1,2,4-triazol-1-yl)-5-fluoro-2-((1,1,1-trifluoropropan-2-yl)oxy)nicotinamide (Example 6, 30 mg, 55.5 µmol) in MeOH (0.5 mL) was added sodium methoxide solution in MeOH (0.5 M, 333 µL, 167 µmol) at RT. The reaction mixture was stirred at 65 °C for 16 h. The reaction mixture was concentrated under reduced pressure. The residue was purified by prep-HPLC (Method F) to afford the title compound (6 mg, 20% yield, 99% purity) as a white solid. MS (ESI): mass calcd. for C₂₁H₁₉ClF₅N₅O₄, 535.1; m/z found, 536 [M+H]+. ¹H NMR (400MHz, CDCl₃) δ = 9.12 (s, 1H), 8.56 (d, *J* = 9.6 Hz, 1H), 7.36 - 7.23 (m, 2H), 7.19 - 7.08 (m, 1H), 5.95 (s, 1H), 4.47 (s, 2H), 3.88 (q, *J* = 7.2 Hz, 2H), 3.46 (s, 3H), 1.68 (d, *J* = 6.5 Hz, 3H), 1.40 (t, *J* = 7.2 Hz, 3H).

### Example 15 (not claimed by the invention but provided because it is referred to later in the synthesis of other claimed compounds): (S)-N-(5-Bromoisothiazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

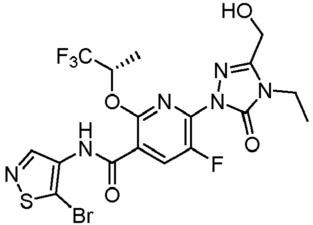

Step A: (S)-6-(3-((Benzyloxy)methyl)-4-ethyl-5-oxo-4,5-dihydro-1H-1,2,4-triazol-1-yl)-N-(5-bromoisothiazol-4-yl)-5-fluoro-2-((1,1,1-trifluoropropan-2-yl)oxy)nicotinamide. To a solution of (S)-6-(3-((benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinic acid (54 mg, 111 µmοl, Example 10, Step A) in THF (0.5 mL) was added triethylamine (TEA) (77.5 µL, 557 µmοl) and propylphosphonic anhydride (T₃P) (50% weight solution in THF) (0.15 mL, 223 µmol) respectively. After 1 min, the activated carboxylic acid derived intermediate was added into the solution of 5-bromoisothiazol-4-amine (30 mg, 167 µmol) in THF (0.5 mL). The reaction mixture was stirred at 90 °C for 24 h, after which time it was concentrated then diluted with EtOAc and water. The mixture was extracted with EtOAc (3x 5 mL) and washed with brine, dried over sodium sulfate, filtered and concentrated to provide the brown solid. The crude product was used in the next step without further purification.

Step B: (S)-N-(5-Bromoisothiazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide. A solution of (S)-6-(3-((benzyloxy)methyl)-4-ethyl-5-oxo-4,5-dihydro-1H-1,2,4-triazol-1-yl)-N-(5-bromoisothiazol-4-yl)-5-fluoro-2-((1,1,1-trifluoropropan-2-yl)oxy)nicotinamide (crude from Step A) was dissolved in TFA (5.6 mL, 558 µmol) and stirred at 70 °C for 16 h. The reaction mixture was filtered and concentrated under reduced pressure. The residue was purified by prep-HPLC (Method F) to afford the title compound (25 mg, 40.3% yield, 99% purity) as a white solid. MS (ESI): mass calcd. for C₁₇H₁₅BrF₄N₆O₄S, 555.3; m/z found, 555 [M+H]+. ¹H NMR (400MHz, CDCl₃) δ = 9.50 (s, 1H), 9.29 (s, 1H), 8.56 (d, *J* = 9.6 Hz, 1H), 6.12 (m, 1H), 4.70 (s, 2H), 3.92 (q, *J* = 7.3 Hz, 2H), 2.21 (s, 1H), 1.71 (d, *J* = 6.6 Hz, 3H), 1.43 (t, *J* = 7.2 Hz, 3H).

### Example 16: (S)-N-(3-Chloropyridazin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

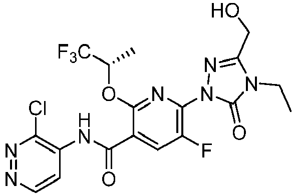

The title compound was prepared according to the representative procedure of Example 15, except substituting 3-chloropyridazin-4-amine for 5-bromoisothiazol-4-amine in Step A. MS (ESI): mass calcd. for C₁₈H₁₆ClF₄N₇O₄, 505.1; m/z found, 506 [M+H]⁺. ¹H NMR (400MHz, CDCl₃) δ = 10.02 (s, 1H), 9.06 (d, *J* = 5.7 Hz, 1H), 8.72 (d, *J* = 5.7 Hz, 1H), 8.5 1 (d, *J* = 9.6 Hz, 1H), 6.22 (h, *J* = 6.6 Hz, 1H), 4.71 (s, 2H), 3.93 (q, *J* = 7.2 Hz, 2H), 2.10 (s, 1H), 1.71 (d, *J* = 6.7 Hz, 3H), 1.43 (t, *J* = 7.2 Hz, 3H).

### Example 17: (S)-6-(4-Ethyl-3-(hydroxymethyl)-5-oxo-4,5-dihydro-1H-1,2,4-triazol-1-yl)-5-fluoro-N-(3-methylisothiazol-4-yl)-2-((1,1,1-trifluoropropan-2-yl)oxy)nicotinamide.

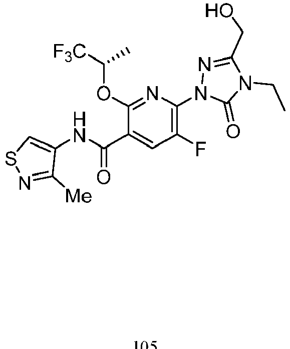

The title compound was prepared according to the representative procedure of Example 15, except substituting 3-methylisothiazol-4-amine for 5-bromoisothiazol-4-amine in Step A. MS (ESI): mass calcd. for C₁₈H₁₈F₄N₆O₄S, 490.1; m/z found, 491 [M+H]⁺. ¹H NMR (400MHz, CDCl₃) δ = 9.46 (s, 1H), 9.23 (s, 1H), 8.58 (d, *J* = 9.6 Hz, 1H), 6.07 (m, 1H), 4.70 (s, 2H), 3.92 (q, *J* = 7.2 Hz, 2H), 2.53 (s, 3H), 1.69 (d, *J* = 6.5 Hz, 3H), 1.43 (t, *J* = 7.2 Hz, 3H).

### Example 18: (S)-N-(2,4-Dimethylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

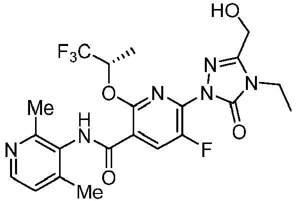

The title compound was prepared according to the representative procedure of Example 15, except substituting 2,4-dimethylpyridin-3-amine for 5-bromoisothiazol-4-amine in Step A. MS (ESI): mass calcd. for C₂₁H₂₂F₄N₆O₄, 498.1; m/z found, 499 [M+H]⁺. ¹H NMR (400MHz, CDCl₃) δ = 8.90 (s, 1H), 8.54 (d, *J* = 9.5 Hz, 1H), 8.34 (d, *J* = 5.0 Hz, 1H), 7.09 (d, *J* = 5.0 Hz, 1H), 5.93 (hept, *J* = 6.7 Hz, 1H), 4.70 (d, *J* = 5.5 Hz, 2H), 3.92 (q, *J* = 7.2 Hz, 2H), 2.51 (s, 3H), 2.28 (s, 3H), 1.68 (d, *J* = 6.5 Hz, 3H), 1.44 (t, *J* = 7.2 Hz, 3H).

### Example 19: (S)-N-(3-Chloropyrazin-2-yl)-6-(4-ethyl-3-(hydromethyl)-5-oxo-4,5-dihydro-1H-1,2,4-triazol-1-yl)-5 -fluoro-2-((1,1,1-trifluoropropan-2-yl)oxy)nicotinamide.

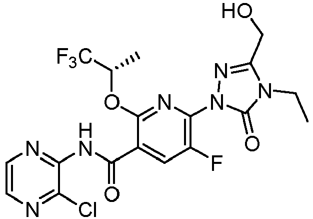

The title compound was prepared according to the representative procedure of Example 15, except substituting 3-chloropyrazin-2-amine for 5-bromoisothiazol-4-amine in Step A. MS (ESI): mass calcd. for C₁₈H₁₆ClF₄N₇O₄, 505.1; m/z found, 506 [M+H]⁺. ¹H NMR (400MHz, CDCl₃) δ = 10.08 (s, 1H), 8.57 (d, *J* = 9.6 Hz, 1H), 8.47 (d, *J* = 2.5 Hz, 1H), 8.23 (d, *J* = 2.5 Hz, 1H), 6.12 (m, 1H), 4.70 (s, 2H), 3.92 (q, *J* = 7.2 Hz, 2H), 1.69 (d, *J* = 6.6 Hz, 3H), 1.43 (t, *J* = 7.2 Hz, 3H).

### Example 21: (S)-N-(3-chloro-6-methoxypyridin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

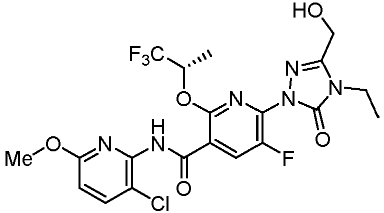

The title compound was prepared according to the representative procedure of Example 15, except substituting 3-chloro-6-methoxypyridin-2-amine for 5-bromoisothiazol-4-amine in Step A. MS (ESI): mass calcd. for C₂₀H₁₉ClF₄N₆O₅, 534.1; m/z found, 535 [M+H]⁺. ¹H NMR (400MHz, CDCl₃) δ = 9.69 (s, 1H), 8.54 (d, *J* = 9.6 Hz, 1H), 7.61 (d, *J* = 8.6 Hz, 1H), 6.60 (d, *J* = 8.7 Hz, 1H), 6.01 (m, 1H), 4.68 (s, 2H), 4.07 - 3.74 (m, 5H), 1.64 (d, *J* = 6.6 Hz, 3H), 1.42 (t, *J* = 7.2 Hz, 3H).

### Example 24: (S)-N-(3-Chloro-2-methoxypyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

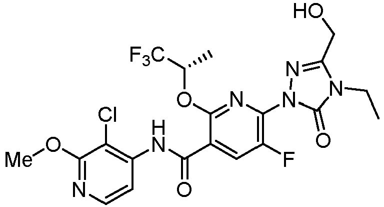

The title compound was prepared according to the representative procedure of Example 15, except substituting 3-chloro-2-methoxypyridin-4-amine for 5-bromoisothiazol-4-amine in Step A. MS (ESI): mass calcd. for C₂₀H₁₉ClF₄N₆O₅, 534.1; m/z found, 535 [M+H]⁺. ¹H NMR (400MHz, CDCl₃) δ = 9.97 (s, 1H), 8.54 (d, *J* = 9.7 Hz, 1H), 8.17 (d, *J* = 5.8 Hz, 1H), 8.06 (d, *J* = 5.7 Hz, 1H), 6.19 (m, 1H), 4.70 (d, *J* = 6.1 Hz, 2H), 4.05 (s, 3H), 3.92 (q, *J* = 7.2 Hz, 2H), 2.28 - 2.16 (m, 1H), 1.69 (d, *J* = 6.6 Hz, 3H), 1.43 (t, *J* = 7.2 Hz, 3H).

### Example 25: (S)-N-(5-Chloro-3-methyl-1H-pyrazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

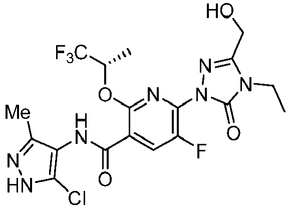

The title compound was prepared according to the representative procedure of Example 15, except substituting 5-chloro-3-methyl-1H-pyrazol-4-amine for 5-bromoisothiazol-4-amine in Step A. MS (ESI): mass calcd. for C₁₈H₁₈ClF₄N₇O₄, 507.1; m/z found, 508 [M+H]⁺. ¹H NMR (400MHz, CDCl₃) δ = 10.07 (s, 1H), 8.83 (s, 1H), 8.52 (d, *J* = 9.6 Hz, 1H), 6.02 - 5.81 (m, 1H), 4.69 (s, 2H), 3.92 (q, *J* = 7.2 Hz, 2H), 2.33 (s, 3H), 1.68 (d, *J* = 6.5 Hz, 3H), 1.43 (t, *J* = 7.2 Hz, 3H).

### Example 26: (S)-N-(4-Chloro-3-methylisothiazol-5-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

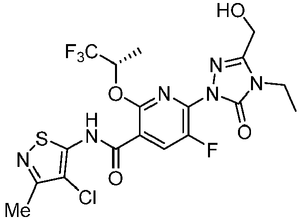

The title compound was prepared according to the representative procedure of Example 15, except substituting 4-chloro-3-methylisothiazol-5-amine for 5-bromoisothiazol-4-amine in Step A. MS (ESI): mass calcd. for C₁₈H₁₇ClF₄N₆O₄S, 524.1; m/z found, 525 [M+H]⁺. ¹H NMR (400MHz, CDCl₃) δ = 10.36 (s, 1H), 8.54 (d, *J* = 9.4 Hz, 1H), 6.08 (hept, *J* = 6.5 Hz, 1H), 4.70 (d, *J* = 3.1 Hz, 2H), 3.92 (d, *J* = 7.2 Hz, 2H), 2.47 (s, 3H), 1.72 (d, *J* = 6.5 Hz, 3H), 1.43 (t, *J* = 7.2 Hz, 3H).

### Example 27: (S)-N-(4-Chloropyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

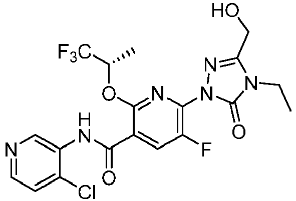

The title compound was prepared according to the representative procedure of Example 15, except substituting 4-chloropyridin-3-amine for 5-bromoisothiazol-4-amine in Step A. MS (ESI): mass calcd. for C₁₉H₁₇ClF₄N₆O₄, 504.1; m/z found, 505 [M+H]⁺. ¹H NMR (400MHz, CDCl₃) δ = 9.66 (d, *J* = 19.0 Hz, 2H), 8.47 - 8.22 (m, 2H), 7.43 (d, *J* = 5.3 Hz, 1H), 6.16 (h, *J* = 6.5 Hz, 1H), 4.69 (s, 2H), 3.94 (q, *J* = 7.2 Hz, 2H), 1.76 - 1.61 (m, 4H), 1.43 (t, *J* = 7.2 Hz, 3H).

### Example 28: (S)-N-(3-Chloro-5-fluoropyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-vl)oxy)nicotinamide.

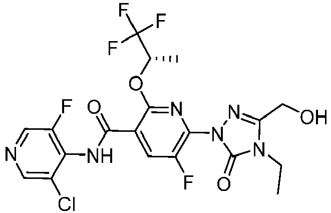

The title compound was prepared according to the representative procedures of Example 15 except substituting 3-chloro-5-fluoropyridin-4-amine for 5-bromoisothiazol-4-amine in Step A. MS (ESI): mass calcd. for C₁₉H₁₆ClF₅N₆O₄, 522.1; m/z found, 523.2 [M+H]⁺. O5, 522.1; m/z found, 535.2 [M+H]⁺. ¹H NMR (400 MHz, CDCl₃) δ ppm 9.33 (s, 1H) 8.45 - 8.64 (m, 2H) 5.98 (s, 1H) 4.70 (br d, *J* = 3.91 Hz, 2H) 3.92 (d, *J* = 7.34 Hz, 2H) 1.69 (d, *J* = 6.36 Hz, 3H) 1.32 - 1.45 (m, 3H).

### Example 29: (S)-N-(3-Cyano-5-fluoropyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

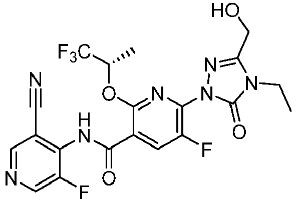

Step A: (S)-6-(3-((Benzyloxy)methyl)-4-ethyl-5-oxo-4,5-dihydro-1H-1,2,4-triazol-1-yl)-N-(3-cyano-5-fluoropyridin-4-yl)-5-fluoro-2-((1,1,1-trifluoropropan-2-vl)oxy)nicotinamide. The title compound was prepared according to the representative procedure of Example 15, Step A, except substituting 4-amino-5-fluoronicotinonitrile for 5-bromoisothiazol-4-amine. MS (ESI): mass calcd. for C₂₇H₂₂F₅N₇O₄, 603.5; m/z found, 603 [M+H]⁺.

Step B: (S)-N-(3-Cyano-5-fluoropyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide. To a mixture of (S)-6-(3-((benzyloxy)methyl)-4-ethyl-5-oxo-4,5-dihydro-1H-1,2,4-triazol-1-yl)-N-(3-cyano-5-fluoropyridin-4-yl)-5-fluoro-2-((1,1,1-trifluoropropan-2-yl)oxy)nicotinamide (60 mg, 0.0994 mmol) in methylene chloride anhydrous (DCM) (0.815 mL) was added dropwise boron trichloride (1M in methylene chloride (0.298 mL) at -78 °C under nitrogen. The mixture was stirred at -78 °C for 1 h. Few drops of methanol (MeOH) were to the mixture. The mixture was diluted with methylene chloride (DCM) and water. The aqueous phase was extracted with methylene chloride (DCM). The combined organic phase was dried with anhydrous MgSO₄, filtered and concentrated in vacuum. The residue was purified by column chromatography (Isco, 4g silica column, mobile phase Gradient from 100% heptane to 70% heptane, 30% ethyl acetate) to afford desired product (18 mg, yield 35%). MS (ESI): mass calcd. for C₂₀H₁₆F₅N₇O₄, 513.4; m/z found, 513.2 [M+H]⁺. ¹H NMR (400 MHz, CDCl₃) δ = 9.47 (s, 1H) 8.58 (d, *J* = 9.29 Hz, 1H) 7.55 (d, *J* = 7.83 Hz, 1H) 7.36 - 7.49 (m, 2H) 5.95 (s, 1H) 4.70 (d, *J* = 6.36 Hz, 2H) 3.80 - 3.99 (m, 2H) 1.70 (d, *J* = 6.36 Hz, 3H) 1.43 (t, *J* = 7.09 Hz, 3H).

### Example 30: (S)-N-(3-Chloro-5-fluoro-2-methoxypyridin-4-yl)-6-(4-ethyl-3-(hdyroxymethyl)-5-oxo-4,5-dihydro-1H-1,2,4triazol-1-yl)-5-fluoro-2-((1,1,1-trifluoropropan-2-yl)oxy)nicotinamide.

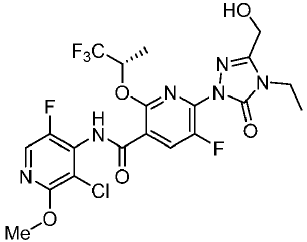

The title compound was prepared according to the representative procedure of Example 15, except substituting 3-chloro-5-fluoro-2-methoxypyridin-4-amine (Intermediate 4) for 5-bromoisothiazol-4-amine in Step A. MS (ESI): mass calcd. for C₂₀H₁₈ClF₅N₆O₅, 552.1; m/z found, 553 [M+H]⁺. ¹H NMR (400MHz, CDCl₃) δ = 9.33 (s, 1H), 8.55 (d, *J* = 9.5 Hz, 1H), 8.04 (d, *J* = 1.2 Hz, 1H), 5.98 (hept, *J* = 6.5 Hz, 1H), 4.70 (s, 2H), 4.03 (s, 3H), 3.92 (q, *J* = 7.2 Hz, 2H), 1.68 (d, *J* = 6.5 Hz, 3H), 1.43 (t, *J* = 7.2 Hz, 3H).

### Example 31: (S)-N-(4-Bromo-3-methylisothiazol-5-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

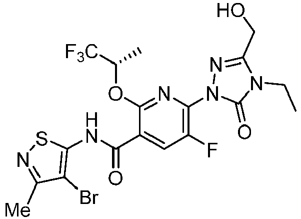

The title compound was prepared according to the representative procedure of Example 15, except substituting 4-bromo-3-methylisothiazol-5-amine for 5-bromoisothiazol-4-amine in Step A. MS (ESI): mass calcd. for C₁₈H₁₇BrF₄N₆O₄S, 568; m/z found, 569.1 [M+H]⁺. ¹H NMR (400MHz, CDCl₃) δ = 10.34 (s, 1H), 8.54 (d, *J* = 9.5 Hz, 1H), 6.16 (m, 1H), 4.70 (d, *J* = 6.0 Hz, 2H), 3.92 (d, *J* = 7.3 Hz, 2H), 2.50 (s, 3H), 2.12 (s, 1H), 1.74 (d, *J* = 6.6 Hz, 3H), 1.43 (t, *J* = 7.2 Hz, 3H).

### Example 32: (S)-6-(4-Ethyl-3-(hydroxymethyl)-5-oxo-4,5-dihydro-1H-1,2,4-triazol-1-yl)-5-fluoro-N-(5-methylthiazol-4-yl)-2-((1,1,1-trifluoropropan-2-yl)oxy)nicotinamide.

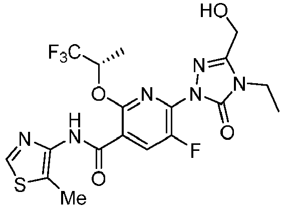

The title compound was prepared according to the representative procedure of Example 15, except substituting 5-methylthiazol-4-amine for 5-bromoisothiazol-4-amine in Step A. MS (ESI): mass calcd. for C₁₈H₁₈F₄N₆O₄S, 490.1; m/z found, 491 [M+H]⁺. ¹H NMR (400MHz, CDCl₃) δ = 9.31 (s, 1H), 8.58 (s, 1H), 8.53 (d, *J* = 9.6 Hz, 1H), 5.89 (m, 1H), 4.68 (s, 2H), 3.91 (q, *J* = 7.2 Hz, 2H), 2.65 (s, 1H), 2.41 (s, 3H), 1.75 - 1.61 (m, 3H), 1.42 (t, *J* = 7.2 Hz, 3H).

### Example 33: (S)-N-(5-Chloro-2-methoxypyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

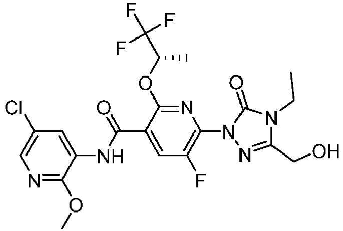

The title compound was prepared according to the representative procedures of Example 15, except substituting 5-chloro-2-methoxy-pyridin-3-ylamine for 5-bromoisothiazol-4-amine in Step A. MS (ESI): mass calcd. for C₂₀H₁₉ClF₄N₆O₅, 534.1; m/z found, 535.2 [M+H]⁺. ¹H NMR (400MHz, CDCl₃) δ ppm 9.91 (s, 1H) 8.88 (d, *J* = 2.45 Hz, 1H) 8.53 (d, *J* = 9.29 Hz, 1H) 7.87 (d, *J* = 2.45 Hz, 1H) 6.06 (s, 1H) 4.70 (d, *J* = 6.36 Hz, 2H) 4.05 (s, 3H) 3.92 (d, *J* = 7.34 Hz, 2H) 1.68 (d, *J* = 6.36 Hz, 3H) 1.43 (t, *J* = 7.34 Hz, 3H).

### Example 34: (S)-6-(4-Ethyl-3-(hydroxymethyl)-5-oxo-4,5-dihydro-1H-1,2,4-triazol-1-yl)-5-fluoro-N-(3-methoxypyrazin-2-yl)-2-((1,1,1-trifluoropropan-2-yl)oxy)nicotinamide.

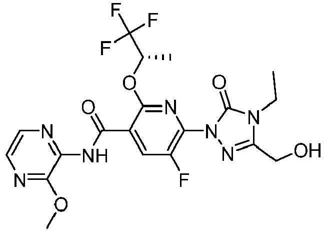

The title compound was prepared according to the representative procedures of Example 15, except substituting 3-methoxypyrazin-2-amine for 5-bromoisothiazol-4-amine in Step A. MS (ESI): mass calcd. for C₂₀H₁₆F₅N₇O₄, 501.14; m/z found, 502.2 [M+H]⁺. ¹H NMR (400MHz, CDCl₃) δ ppm 10.13 (s, 1H) 8.58 (d, *J* = 9.29 Hz, 1H) 8.01 - 8.15 (m, 1H) 7.88 (d, *J* = 2.93 Hz, 1H) 5.95 - 6.13 (m, 1H) 4.70 (d, *J* = 6.36 Hz, 2H) 4.08 (s, 3H) 3.92 (d, *J* = 7.34 Hz, 2H) 2.21 (t, *J* = 6.60 Hz, 1H) 1.68 (d, *J* = 6.85 Hz, 3H) 1.43 (t, *J* = 7.09 Hz, 4H).

### Example 35: (S)-N-(3,5-Dimethylisothiazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

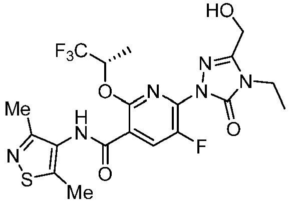

The title compound was prepared according to the representative procedure of Example 15, except substituting 3,5-dimethylisothiazol-4-amine for 5-bromoisothiazol-4-amine in Step A. MS (ESI): mass calcd. for C₁₉H₂₀F₄N₆O₄S, 504; m/z found, 505 [M+H]⁺. ¹H NMR (400MHz, CDCl₃) δ = 8.83 (s, 1H), 8.53 (d, *J* = 9.5 Hz, 1H), 5.91 (m, 1H), 4.69 (s, 2H), 3.92 (q, *J* = 7.2 Hz, 2H), 2.43 (s, 3H), 2.38 (s, 3H), 1.68 (d, *J* = 6.5 Hz, 3H), 1.43 (t, *J* = 7.2 Hz, 3H).

### Example 36: (S)-6-(4-Ethyl-3-(hydroxymethyl)-5-oxo-4,5-dihydro-1H-1,2,4-triazol-1-yl)-5-fluoro-N-(2-fluoro-5-methylpyridin-3-yl)-2-((1,1,1-trifluoropropan-2-yl)oxy)nicotinamide.

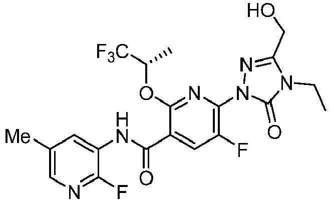

The title compound was prepared according to the representative procedure of Example 15, except substituting 2-fluoro-5-methylpyridin-3-amine for 5-bromoisothiazol-4-amine in Step A. MS (ESI): mass calcd. for C₂₀H₁₉F₅N₆O₄, 502.1; m/z found, 503 [M+H]⁺. ¹H NMR (400MHz, CDCl₃) δ = 9.82 (d, *J* = 2.8 Hz, 1H), 8.77 (dd, *J* = 9.5, 2.2 Hz, 1H), 8.53 (d, *J* = 9.5 Hz, 1H), 7.75 (s, 1H), 6.03 (hept, *J* = 6.3 Hz, 1H), 4.70 (d, *J* = 5.7 Hz, 2H), 3.92 (q, *J* = 7.2 Hz, 2H), 2.37 (s, 3H), 1.69 (d, *J* = 6.6 Hz, 3H), 1.43 (t, *J* = 7.2 Hz, 3H).

### Example 37: (S)-6-(4-Ethyl-3-(hydroxymethyl)-5-oxo-4,5-dihydro-1H-1,2,4-triazol-1-yl)-5-fluoro-N-(5-fluoro-2-methylpyridin-4-yl)-2-((1,1,1-trifluoropropan-2-yl)oxy)nicotinamide.

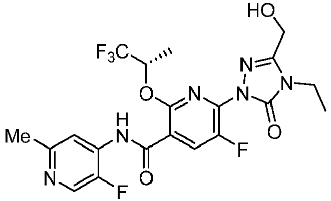

The title compound was prepared according to the representative procedure of Example 15, except substituting 5-fluoro-2-methylpyridin-4-amine for 5-bromoisothiazol-4-amine in Step A. MS (ESI): mass calcd. for C₂₀H₁₉F₅N₆O₄, 502.1; m/z found, 503 [M+H]⁺. ¹H NMR (400MHz, CDCl₃) δ = 9.95 (d, *J* = 3.2 Hz, 1H), 8.52 (d, *J* = 9.5 Hz, 1H), 8.48 - 8.20 (m, 2H), 6.02 (m, 1H), 4.69 (s, 2H), 3.93 (q, *J* = 7.2 Hz, 2H), 2.56 (s, 3H), 1.68 (d, *J* = 6.6 Hz, 3H), 1.43 (t, *J* = 7.2 Hz, 3H).

### Example 38: (S)-N-(4-Chloro-6-methylpyrimidin-5-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

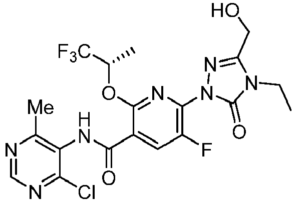

The title compound was prepared according to the representative procedure of Example 15, except substituting 4-chloro-6-methylpyrimidin-5-amine for 5-bromoisothiazol-4-amine in Step A. MS (ESI): mass calcd. for C₁₉H₁₈ClF₄N₇O₄, 519.1; m/z found, 502 [M+H]⁺. ¹H NMR (400MHz, CDCl₃) δ = 9.17 (s, 1H), 8.49 (d, *J* = 9.5 Hz, 1H), 8.02 (s, 1H), 5.92 (h, *J* = 6.5 Hz, 1H), 4.68 (s, 2H), 3.92 (q, *J* = 7.2 Hz, 2H), 2.37 (s, 3H), 1.69 (d, *J* = 6.5 Hz, 3H), 1.42 (t, *J* = 7.2 Hz, 3H).

### Example 39: (S)-6-(4-Ethyl-3-(hydroxymethyl)-5-oxo-4,5-dihydro-1H-1,2,4-triazol-1-yl)-5-fluoro-N-(3-fluoro-6-methylpyridin-2-yl)-2-((1,1,1-trifluoropropan-2-yl)oxy)nicotinamide.

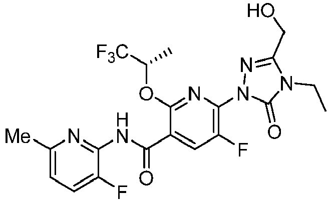

The title compound was prepared according to the representative procedure of Example 15, except substituting 3-fluoro-6-methylpyridin-2-amine for 5-bromoisothiazol-4-amine in Step A. MS (ESI): mass calcd. for C₂₀H₁₉F₅N₆O₄, 502.1; m/z found, 503 [M+H]⁺. ¹H NMR (400MHz, CDCl₃) δ = 9.69 (s, 1H), 8.56 (d, *J* = 9.7 Hz, 1H), 7.40 (t, *J* = 8.9 Hz, 1H), 7.03 (dd, *J* = 8.3, 3.4 Hz, 1H), 5.92 (m, 1H), 4.69 (s, 2H), 3.92 (q, *J* = 7.2 Hz, 2H), 2.56 (s, 3H), 1.67 (d, *J* = 6.5 Hz, 3H), 1.42 (t, *J* = 7.2 Hz, 3H).

### Example 40: (S)-N-(3-Chloro-2-methoxy-5-methylpyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

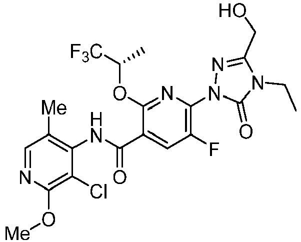

The title compound was prepared according to the representative procedure of Example 15, except substituting 3-chloro-2-methoxy-5-methylpyridin-4-amine (Intermediate 5) for 5-bromoisothiazol-4-amine in Step A. MS (ESI): mass calcd. for C₂₁H₂₁ClF₄N₆O₅, 548.1; m/z found, 549 [M+H]⁺. ¹H NMR (400MHz, CDCl₃) δ = 9.26 (s, 1H), 8.54 (d, *J* = 9.5 Hz, 1H), 7.98 (s, 1H), 6.04 - 5.95 (m, 1H), 4.69 (s, 2H), 4.03 (s, 3H), 3.92 (d, *J* = 7.2 Hz, 2H), 2.21 (s, 3H), 1.68 (d, *J* = 6.5 Hz, 3H), 1.43 (t, *J* = 7.2 Hz, 3H).

### Example 41: (S)-6-(4-Ethyl-3-(hydroxymethyl)-5-oxo-4,5-dihydro-1H-1,2,4-triazol-1-yl)-5-fluoro-N-(2-methoxy-5-methylpyridin-3-yl)-2-((1,1,1-trifluoropropan-2-yl)oxy)nicotinamide.

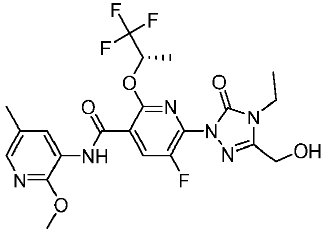

The title compound was prepared according to the representative procedures of Example 15, except substituting 2-methoxy-5-methylpyridin-3-amine for 5-bromoisothiazol-4-amine in Step A. MS (ESI): mass calcd. for C₂₁H₂₂F₄N₆O₅, 514.16; m/z found, 515.2 [M+H]⁺. ¹H NMR (400 MHz, CDCl₃) δ ppm 9.89 (s, 1H) 8.66 (d, *J* = 1.96 Hz, 1H) 8.54 (d, *J* = 9.29 Hz, 1H) 7.65 - 7.80 (m, 1H) 6.00 - 6.14 (m, 1H) 4.70 (d, *J* = 6.36 Hz, 2H) 4.03 (s, 3H) 3.92 (d, *J* = 7.34 Hz, 2H) 2.30 (s, 3H) 1.68 (d, *J* = 6.85 Hz, 3H) 1.43 (t, *J* = 7.09 Hz, 3H).

### Example 42: (S)-6-(4-Ethyl-3-(hydroxymethyl)-5-oxo-4,5-dihydro-1H-1,2,4-triazol-1-yl)-5-fluoro-N-(2-fluoro-4-methylpyridin-3-yl)-2-((1,1,1-trifluoropropan-2-yl)oxy)nicotinamide.

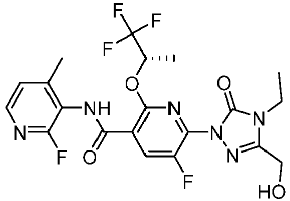

The title compound was prepared according to the representative procedures of Example 15, except substituting 2-fluoro-4-methyl-3-pyridinamine for 5-bromoisothiazol-4-amine in Step A. MS (ESI): mass calcd. for C₂₀H₁₉F₅N₆O₄, 502.14; m/z found, 503.1 [M+H]⁺. ¹H NMR (400 MHz, CDCl₃) δ ppm 9.03 (s, 1H) 8.53 (d, *J* = 9.78 Hz, 1H) 8.03 (d, *J* = 5.38 Hz, 1H) 7.13 (d, *J* = 4.89 Hz, 1H) 5.85 - 5.97 (m, 1H) 4.70 (d, *J* = 6.36 Hz, 2H) 3.92 (q, *J* = 7.34 Hz, 2H) 2.36 (s, 3H) 1.69 (d, *J* = 6.36 Hz, 3H) 1.43 (t, *J* = 7.34 Hz, 3H).

### Example 43: (S)-N-(5,6-Dimethylpyrimidin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

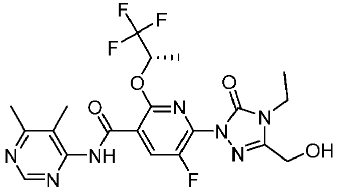

The title compound was prepared according to the representative procedures of Example 15, except substituting 5,6-dimethyl-4-pyrimidine for 5-bromoisothiazol-4-amine in Step A. MS (ESI): mass calcd. for C₂₀H₂₁F₄N₇O₄, 499.16; m/z found, 500.3 [M+H]⁺. ¹H NMR (400 MHz, CDCl₃) δ ppm 9.48 (s, 1H) 8.83 (s, 1H) 8.54 (d, *J* = 9.78 Hz, 1H) 5.87 - 6.05 (m, 1H) 4.70 (d, *J* = 6.36 Hz, 2H) 3.92 (d, *J* = 7.34 Hz, 2H) 2.58 (s, 3H) 2.23 (s, 3H) 1.68 (d, *J* = 6.36 Hz, 3H) 1.43 (t, *J* = 7.34 Hz, 3H).

### Example 44: (S)-N-(2-Chloro-4-methoxypyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

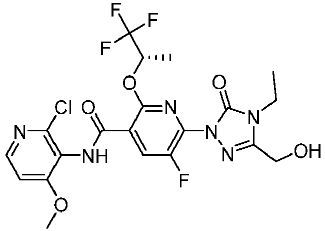

The title compound was prepared according to the representative procedures of Example 15, except substituting 2-chloro-4-methoxypyridin-3-amine for 5-bromoisothiazol-4-amine in Step A. MS (ESI): mass calcd. for C₂₀H₁₉ClF₄N₆O₅, 534.10; m/z found, 535.2 [M+H]⁺. ¹H NMR (400 MHz, CDCl₃) δ ppm 9.00 (s, 1H) 8.54 (d, *J* = 9.78 Hz, 1H) 8.27 (d, *J* = 5.38 Hz, 1H) 6.90 (d, *J* = 5.87 Hz, 1H) 5.92 (s, 1H) 4.70 (d, *J* = 6.36 Hz, 2H) 3.94 (s, 3H) 3.89 - 3.93 (m, 2H) 1.68 (d, *J* = 6.36 Hz, 3H) 1.43 (t, *J* = 7.09 Hz, 4H).

### Example 45: (S)-6-(4-Ethyl-3-(hydroxymethyl)-5-oxo-4,5-dihydro-1H-1,2,4-triazol-1-yl)-5-fluoro-N-(3-methylisoxazol-4-yl)-2-((1,1,1-trifluoropropan-2-yl)oxy)nicotinamide.

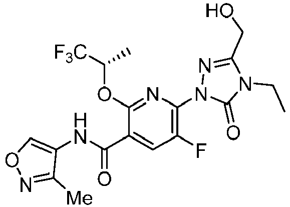

The title compound was prepared according to the representative procedure of Example 15, except substituting 3-methylisoxazol-4-amine for 5-bromoisothiazol-4-amine in Step A. MS (ESI): mass calcd. for C₁₈H₁₈F₄N₆O₅, 474.1; m/z found, 475 [M+H]⁺. ¹H NMR (400MHz, CDCl₃) δ = 9.17 (s, 1H), 9.08 (s, 1H), 8.53 (d, *J* = 9.6 Hz, 1H), 5.95 (m, 1H), 4.70 (d, *J* = 5.5 Hz, 2H), 3.92 (q, *J* = 7.2 Hz, 2H), 2.38 (s, 3H), 2.25 (s, 1H), 1.69 (d, *J* = 6.5 Hz, 3H), 1.43 (t, *J* = 7.2 Hz, 3H).

### Example 46: (S)-N-(3,5-Dimethylisoxazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

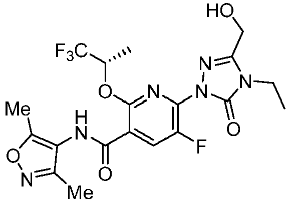

The title compound was prepared according to the representative procedure of Example 15, except substituting 3,5-dimethylisoxazol-4-amine for 5-bromoisothiazol-4-amine in Step A. MS (ESI): mass calcd. for C₁₉H₂₀F₄N₆O₅, 488.1; m/z found, 489 [M+H]⁺. ¹H NMR (400MHz, CDCl₃) δ = 8.64 (s, 1H), 8.51 (d, *J* = 9.6 Hz, 1H), 5.86 (hept, *J* = 6.5 Hz, 1H), 4.70 (d, *J* = 6.0 Hz, 2H), 3.92 (q, *J* = 7.2 Hz, 2H), 2.39 (s, 3H), 2.24 (s, 3H), 2.18 (s, 1H), 1.69 (d, *J* = 6.5 Hz, 3H), 1.43 (t, *J* = 7.2 Hz, 3H).

### Example 47: (S)-6-(4-Ethyl-3-(hydroxymethyl)-5-oxo-4,5-dihydro-1H-1,2,4-triazol-1-yl)-5-fluoro-N-(2-fluoro-5-methoxyphenyl)-2-((1,1,1-trifluoropropan-2-yl)oxy)nicotinamide.

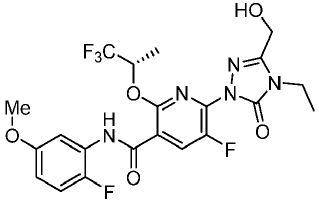

The title compound was prepared according to the representative procedure of Example 15, except substituting 2-fluoro-5-methoxyaniline for 5-bromoisothiazol-4-amine in Step A. MS (ESI): mass calcd. for C₂₁H₂₀F₅N₅O₅, 517.1; m/z found, 518 [M+H]⁺. ¹H NMR (400MHz, CDCl₃) δ = 9.85 (d, *J* = 3.4 Hz, 1H), 8.55 (d, *J* = 9.6 Hz, 1H), 8.20 (dd, *J* = 6.4, 3.1 Hz, 1H), 7.05 (dd, *J* = 10.4, 9.0 Hz, 1H), 6.63 (dt, *J* = 9.0, 3.6 Hz, 1H), 6.02 (hept, *J* = 6.4 Hz, 1H), 4.70 (d, *J* = 5.5 Hz, 2H), 3.92 (q, *J* = 7.2 Hz, 2H), 3.84 (s, 3H), 2.16 (s, 1H), 1.68 (d, *J* = 6.5 Hz, 3H).

### Example 48: (S)-N-(2-Chloro-5-methylphenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

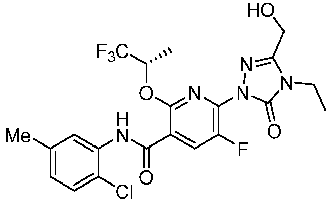

The title compound was prepared according to the representative procedure of Example 15, except substituting 2-chloro-5-methylaniline for 5-bromoisothiazol-4-amine in Step A. MS (ESI): mass calcd. for C₂₁H₂₀ClF₄N₅O₄, 517.1; m/z found, 518 [M+H]⁺. ¹H NMR (400MHz, CDCl₃) δ = 9.73 (s, 1H), 8.56 (d, *J* = 9.7 Hz, 1H), 8.30 (d, *J* = 2.0 Hz, 1H), 7.31 (d, *J* = 8.2 Hz, 1H), 6.94 (dd, *J* = 8.1, 2.0 Hz, 1H), 6.14 (hept, *J* = 6.5 Hz, 1H), 4.77 - 4.62 (m, 2H), 3.92 (q, *J* = 7.2 Hz, 2H), 2.38 (s, 3H), 2.24 (d, *J=* 15.7 Hz, 1H), 1.67 (d, *J* = 6.6 Hz, 3H), 1.43 (t, *J* = 7.2 Hz, 3H).

### Example 49: (S)-N-(3,5-Dimethyl-1H-pyrazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

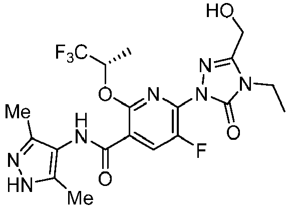

The title compound was prepared according to the representative procedure of Example 15, except substituting 3,5-dimethyl-1H-pyrazol-4-amine for 5-bromoisothiazol-4-amine in Step A. MS (ESI): mass calcd. for C₁₉H₂₁F₄N₇O₄, 487.2; m/z found, 488 [M+H]⁺. ¹H NMR (400MHz, CDCl₃) δ = 8.68 (s, 1H), 8.53 (d, *J* = 9.7 Hz, 1H), 5.86 (h, *J* = 6.5 Hz, 1H), 4.68 (s, 2H), 3.92 (q, *J* = 7.2 Hz, 2H), 2.22 (s, 6H), 1.67 (d, *J* = 6.5 Hz, 3H), 1.43 (t, *J* = 7.2 Hz, 3H).

### Example 50: (S)-N-(4-Chloro-6-methoxypyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

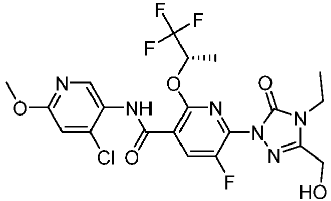

The title compound was prepared according to the representative procedures of Example 15, except substituting 4-chloro-6-methyoxypyridin-3-amine for 5-bromoisothiazol-4-amine in Step A . MS (ESI): mass calcd. For C₂₀H₁₉ClF₄N₆O₅, 534.10; m/z found, 535.2 [M+H]⁺. ¹H NMR (400 MHz, CDCl₃) δ ppm 9.48 (s, 1H) 9.10 (s, 1H) 8.56 (d, *J* = 9.78 Hz, 1H) 6.89 (s, 1H) 6.08 (dt, *J* = 13.21, 6.60 Hz, 1H) 4.70 (d, *J* = 6.85 Hz, 2H) 3.96 (s, 3H) 3.92 (d, *J* = 6.85 Hz, 2H) 1.67 (d, *J* = 6.36 Hz, 3H) 1.43 (t, *J* = 7.34 Hz, 3H).

### Example 51: (S)-N-(2,5-Dimethylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

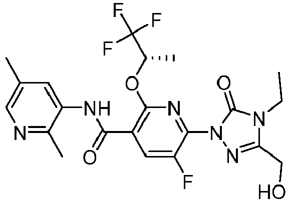

The title compound was prepared according to the representative procedures of Example 15, except substituting 2,5-dimethylpyridin-3-amine for 5-bromoisothiazol-4-amine in Step A. MS (ESI): mass calcd. For C₂₁H₂₂F₄N₆O₄, 498.16; m/z found, 499.2 [M+H]⁺. ¹H NMR (400 MHz, CDCl₃) δ ppm 9.15 (s, 1H) 8.57 (d, *J* = 9.29 Hz, 1H) 8.20 (d, *J* = 7.34 Hz, 2H) 6.07 (s, 1H) 4.70 (d, *J* = 6.36 Hz, 2H) 3.92 (q, *J* = 7.17 Hz, 2H) 2.54 (s, 3H) 2.36 (s, 3H) 1.68 (d, *J* = 6.36 Hz, 3H) 1.44 (t, *J* = 7.09 Hz, 3H).

### Example 52: (S)-N-(5-Chloro-2-methoxypyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

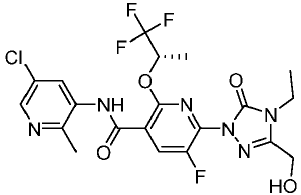

The title compound was prepared according to the representative procedures of Example 15, except substituting 3-amino-5-chloropicoline for 5-bromoisothiazol-4-amine in Step A. MS (ESI): mass calcd. for C₂₀H₁₉ClF₄N₆O₄, 518.11; m/z found, 519.2 [M+H]⁺. ¹H NMR(400 MHz, CDCl₃) δ ppm 9.21 (s, 1H) 8.51 - 8.70 (m, 2H) 8.31 (d, *J* = 1.96 Hz, 1H) 6.00 - 6.20 (m, 1H) 4.70 (d, *J* = 6.36 Hz, 2H) 3.92 (d, *J* = 7.34 Hz, 2H) 2.56 (s, 3H) 1.69 (d, *J* = 6.85 Hz, 3H) 1.44 (t, *J* = 7.09 Hz, 3H).

### Example 53: N-(1,4-Dimethyl-2-oxo-2,3-dihydro-114-pyridin-3-yl)-6-(4-ethyl-3-(hydroxymethyl)-5-oxo-4,5-dihydro-1H-1,2,4-triazol-1-yl)-5-fluoro-2-(((S)-1,1,1-trifluoropropan-2-yl)oxy)nicotinamide.

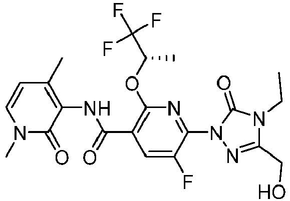

The title compound was prepared according to the representative procedures of Example 15, except substituting 3-amino-1,4-dimethyl-1,2-dihydropyridin-2-one for 5-bromoisothiazol-4-amine in Step A. MS (ESI): mass calcd. For C₂₁H₂₂F₄N₆O₅, 514.16; m/z found, 515.3 [M+H]⁺. ¹H NMR (400MHz, CDCl₃) δ ppm 9.28 (s, 1H) 8.50 (d, *J* = 9.29 Hz, 1H) 7.12 (d, *J* = 6.85 Hz, 1H) 6.13 (d, *J* = 6.85 Hz, 1H) 5.93 (quin, *J* = 6.48 Hz, 1H) 4.69 (s, 2H) 3.91 (d, *J* = 7.34 Hz, 2H) 3.57 (s, 3H) 2.18 - 2.18 (m, 1H) 2.19 (s, 3H) 1.70 (d, *J* = 6.36 Hz, 3H) 1.42 (t, *J* = 7.34 Hz, 3H).

### Example 54: (S)-N-(3-Chloropyridin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

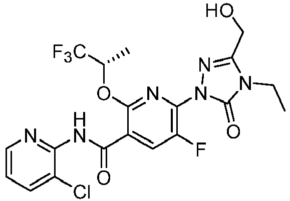

The title compound was prepared according to the representative procedure of Example 15, except substituting 3-chloropyridin-2-amine for 5-bromoisothiazol-4-amine in Step A. MS (ESI): mass calcd. for C₁₉H₁₇ClF₄N₆O₄, 504.1; m/z found, 505 [M+H]⁺. ¹H NMR (400MHz, CDCl₃) δ = 9.93 (s, 1H), 8.58 (d, *J* = 9.6 Hz, 1H), 8.53 (s, 1H), 7.80 (d, *J* = 7.9 Hz, 1H), 7.16 (dd, *J* = 8.0, 4.6 Hz, 1H), 6.06 (m, 1H), 4.70 (s, 2H), 3.92 (q, *J* = 7.2 Hz, 2H), 3.33 (s, 1H), 1.66 (d, *J* = 6.6 Hz, 3H), 1.41 (t, *J* = 7.2 Hz, 3H).

### Example 55: (S)-6-(4-Ethyl-3-(hydroxymethyl)-5-oxo-4,5-dihydro-1H-1,2,4-triazol-1-yl)-5-fluoro-2-((1,1,1-trifluoropropan-2-yl)oxy)-N-(1,3,5-trimethvl-1H-pyrazol-4-yl)nicotinamide.

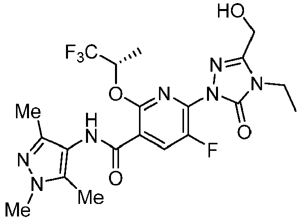

The title compound was prepared according to the representative procedure of Example 15, except substituting 1,3,5-trimethyl-1H-pyrazol-4-amine for 5-bromoisothiazol-4-amine in Step A. MS (ESI): mass calcd. for C₂₀H₂₃F₄N₇O₄, 501.2; m/z found, 502 [M+H]⁺. ¹H NMR (400MHz, CDCl₃) δ = 8.65 (s, 1H), 8.54 (d, *J* = 9.6 Hz, 1H), 5.86 (m, 1H), 4.70 (d, *J* = 6.3 Hz, 2H), 3.92 (q, *J* = 7.2 Hz, 2H), 3.74 (s, 3H), 2.18 (d, *J* = 5.4 Hz, 6H), 1.67 (d, *J* = 6.5 Hz, 3H), 1.43 (t, *J* = 7.2 Hz, 3H).

### Example 56: (S)-6-(4-Ethyl-3-(hydroxymethyl)-5-oxo-4,5-dihydro-1H-1,2,4-triazol-1-yl)-5-fluoro-N-(3-fluoro-6-methoxypyridin-2-yl)-2-((1,1,1-trifluoropropan-2-yl)oxy)nicotinamide.

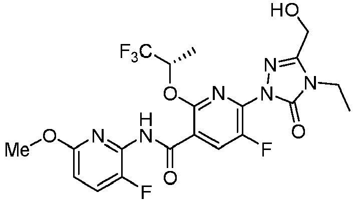

The title compound was prepared according to the representative procedure of Example 15, except substituting 3-fluoro-6-methoxypyridin-2-amine for 5-bromoisothiazol-4-amine in Step A. MS (ESI): mass calcd. for C₂₀H₁₉F₅N₆O₅, 518.1; m/z found, 519 [M+H]⁺. ¹H NMR (400MHz, CDCl₃) δ = 9.60 (s, 1H), 8.56 (d, *J* = 9.6 Hz, 1H), 7.44 (t, *J* = 8.9 Hz, 1H), 6.60 (dd, *J* = 8.8, 2.6 Hz, 1H), 5.90 (m, 1H), 4.70 (d, *J* = 6.0 Hz, 2H), 4.03 - 3.83 (m, 5H), 2.13 (t, *J* = 6.3 Hz, 1H), 1.68 (d, *J* = 6.5 Hz, 3H), 1.43 (t, *J* = 7.2 Hz, 3H).

### Example 57: (S)-6-(4-Ethyl-3-(hydroxymethyl)-5-oxo-4,5-dihydro-1H-1,2,4-triazol-1-yl)-5-fluoro-N-(3-methyl-1H-pyrazol-4-yl)-2-((1,1,1-trifluoropropan-2-yl)oxy)nicotinamide.

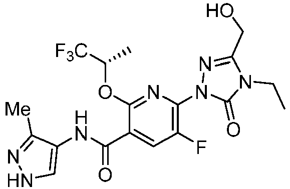

The title compound was prepared according to the representative procedure of Example 15, except substituting 3-methyl-1H-pyrazol-4-amine for 5-bromoisothiazol-4-amine in Step A. MS (ESI): mass calcd. for C₁₈H₁₉F₄N₇O₄, 473.1; m/z found, 474 [M+H]⁺. ¹H NMR (400MHz, CDCl₃) δ = 9.14 (s, 1H), 8.56 (d, *J* = 9.7 Hz, 1H), 8.24 (s, 1H), 5.96 (m, 1H), 4.69 (s, 2H), 3.92 (q, *J* = 7.2 Hz, 2H), 2.33 (s, 3H), 1.68 (d, *J* = 6.5 Hz, 3H), 1.43 (t, *J* = 7.2 Hz, 3H).

### Example 58: (S)-6-(4-Ethyl-3-(hydroxymethyl)-5-oxo-4,5-dihydro-1H-1,2,4-triazol-1-yl)-5-fluoro-N-(3-methoxy-1H-pyrazol-4-yl)-2-((1,1,1-trifluoropropan-2-yl)oxy)nicotinamide.

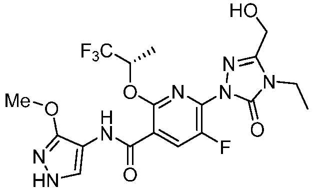

The title compound was prepared according to the representative procedure of Example 15, except substituting 3-methoxy-1H-pyrazol-4-amine for 5-bromoisothiazol-4-amine in Step A. MS (ESI): mass calcd. for C₁₈H₁₉F₄N₇O₅, 489.1; m/z found, 490 [M+H]⁺. ¹H NMR (400MHz, CDCl₃) δ = 9.44 (s, 1H), 8.91 (s, 1H), 8.52 (d, *J* = 9.7 Hz, 1H), 8.14 (s, 1H), 5.98 - 5.83 (m, 1H), 4.69 (s, 2H), 4.02 (s, 3H), 3.91 (q, *J* = 7.2 Hz, 2H), 1.68 (d, *J* = 6.5 Hz, 3H), 1.43 (t, *J* = 7.2 Hz, 3H).

### Example 59: (S)-6-(4-Ethyl-3-(hydroxymethyl)-5-oxo-4,5-dihydro-1H-1,2,4-triazol-1-yl)-5-fluoro-N-(3-isopropyl-1-methyl-1H-pyrazol-5-yl)-2-((1,1,1-trifluoropropan-2-yl)oxy)nicotinamide.

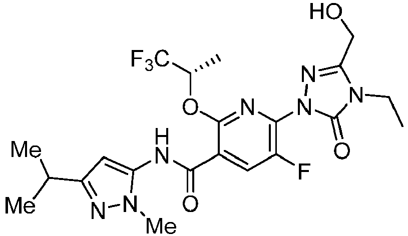

The title compound was prepared according to the representative procedure of Example 15, except substituting 3-isopropyl-1-methyl-1H-pyrazol-5-amine (Intermediate 6) for 5-bromoisothiazol-4-amine in Step A. MS (ESI): mass calcd. for C₂₁H₂₅F₄N₇O₄, 515.2; m/z found, 516 [M+H]⁺. ¹H NMR (400MHz, CDCl₃) δ = 9.27 (s, 1H), 8.53 (d, *J* = 9.5 Hz, 1H), 6.41 (s, 1H), 5.96 (m, 1H), 4.69 (d, *J* = 5.6 Hz, 2H), 3.92 (q, *J* = 7.2 Hz, 2H), 3.76 (s, 3H), 3.01 - 2.87 (m, 1H), 2.80 (d, *J* = 6.6 Hz, 1H), 1.68 (d, *J* = 6.1 Hz, 3H), 1.43 (t, *J* = 7.2 Hz, 3H), 1.28 (d, *J* = 6.9 Hz, 6H).

### Example 60: (S)-N-(5-Chloro-6-methylpyrimidin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

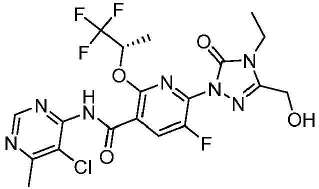

The title compound was prepared according to the representative procedures of Example 15, except substituting 5-chloro-6-methylpyrimidin-4-amine, for 5-bromoisothiazol-4-amine in Step A. MS (ESI): mass calcd. for C₁₉H₁₈ClF₄N₇O₄, 519.10; m/z found, 520.0 [M+H]+. ¹H NMR (400MHz, CDCl₃) δ = 10.08 (s, 1H) 8.88 (s, 1H) 8.56 (d, *J* = 9.78 Hz, 1H) 6.02 - 6.22 (m, 1H) 4.70 (d, *J* = 6.36 Hz, 2H) 3.92 (d, *J* = 7.34 Hz, 2H) 2.67 (s, 3H) 1.68 (d, *J* = 6.85 Hz, 3H) 1.43 (t, *J* = 7.34 Hz, 4H).

### Example 61: (S)-6-(4-Ethyl-3-(hydroxymethyl)-5-oxo-4,5-dihydro-1H-1,2,4-triazol-1-yl)-5-fluoro-N-(3-(methoxymethyl)-1-methyl-1H-pyrazol-4-yl)-2-((1,1,1-trifluoropropan-2-yl)oxy)nicotinamide.

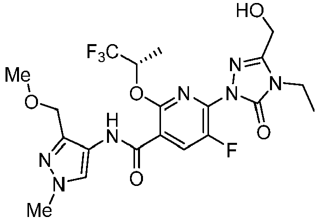

The title compound was prepared according to the representative procedure of Example 15, except substituting 3-(methoxymethyl)-1-methyl-1H-pyrazol-4-amine for 5-bromoisothiazol-4-amine in Step A. MS (ESI): mass calcd. for C₂₀H₂₃F₄N₇O₅, 517.2; m/z found, 518 [M+H]⁺. ¹H NMR (400MHz, CDCl₃) δ = 9.58 (s, 1H), 8.49 (d, *J=* 9.6 Hz, 1H), 8.21 (s, 1H), 6.09 (hept, *J* = 6.6 Hz, 1H), 4.66 (dd, *J* = 8.1, 4.8 Hz, 3H), 4.53 (d, *J* = 12.9 Hz, 1H), 4.02 - 3.78 (m, 5H), 3.32 (s, 3H), 3.23 (s, 1H), 1.68 (d, *J* = 6.6 Hz, 3H), 1.41 (t, *J* = 7.2 Hz, 3H).

### Example 62: (S)-N-(3-Cyclopropyl-1H-pyrazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

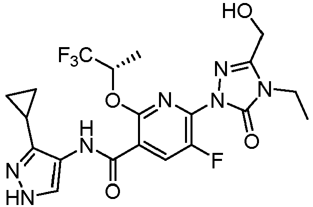

The title compound was prepared according to the representative procedure of Example 15, except substituting 3-cyclopropyl-1H-pyrazol-4-amine for 5-bromoisothiazol-4-amine in Step A. MS (ESI): mass calcd. for C₂₀H₂₁F₄N₇O₄, 499.1; m/z found, 500 [M+H]⁺. ¹H NMR (400MHz, DMSO-*d*₆) δ = 12.36 (s, 1H), 9.80 (s, 1H), 8.32 (d, *J* = 9.0 Hz, 1H), 7.85 (d, *J* = 113.0 Hz, 1H), 5.80 (td, *J* = 12.9, 6.3 Hz, 2H), 4.49 (d, *J* = 4.5 Hz, 2H), 3.79 (q, *J* = 7.1 Hz, 2H), 2.03 - 1.82 (m, 1H), 1.53 (d, *J* = 6.5 Hz, 3H), 1.28 (t, *J* = 7.1 Hz, 3H), 0.81 (d, *J* = 30.6 Hz, 4H).

### Example 64: (S)-N-(3-Chloro-1-methyl-6-oxo-1,6-dihydropyridin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

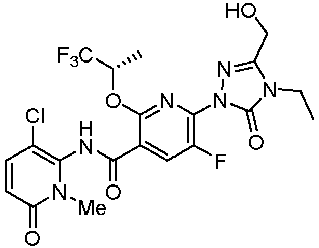

The title compound was prepared according to the representative procedure of Example 15, except substituting 6-amino-5-chloro-1-methylpyridin-2(1H)-one (Intermediate 7) for 5-bromoisothiazol-4-amine in Step A. MS (ESI): mass calcd. for C₂₀H₁₉ClF₄N₆O₅, 534.1; m/z found, 535 [M+H]⁺. ¹H NMR (400MHz, CDCl₃) δ = 9.20 (s, 1H), 8.49 (d, *J* = 9.4 Hz, 1H), 7.40 (d, *J* = 9.8 Hz, 1H), 6.62 (d, *J* = 9.8 Hz, 1H), 5.96 (hept, *J* = 6.5 Hz, 1H), 4.70 (s, 2H), 3.93 (q, *J* = 7.2 Hz, 2H), 3.53 (s, 3H), 1.69 (d, *J* = 6.5 Hz, 3H), 1.43 (t, *J* = 7.2 Hz, 3H).

### Example 65: (S)-6-(4-Ethyl-3-(hydroxymethyl)-5-oxo-4,5-dihydro-1H-1,2,4-triazol-1-yl)-5-fluoro-N-(5-fluoro-2-methoxypyridin-4-yl)-2-((1,1,1-trifluoropropan-2-yl)oxy)nicotinamide.

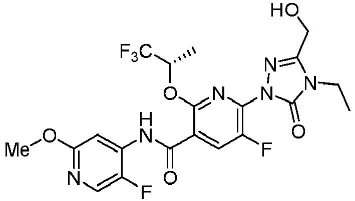

The title compound was prepared according to the representative procedure of Example 15, except substituting 5-fluoro-2-methoxypyridin-4-amine for 5-bromoisothiazol-4-amine in Step A. MS (ESI): mass calcd. for C₂₀H₁₉F₅N₆O₅, 518.1; m/z found, 519 [M+H]⁺. ¹H NMR (400MHz, CDCl₃) δ = 9.94 (d, *J* = 3.4 Hz, 1H), 8.53 (d, *J=* 9.6 Hz, 1H), 8.01 (d, *J* = 2.1 Hz, 1H), 7.96 (d, *J* = 5.2 Hz, 1H), 6.03 (m, 1H), 4.69 (d, *J* = 6.2 Hz, 2H), 3.91 (d, *J* = 4.9 Hz, 5H), 2.29 (t, *J* = 6.3 Hz, 1H), 1.68 (d, *J* = 6.5 Hz, 3H), 1.43 (t, *J* = 7.2 Hz, 3H).

### Example 66: (S)-6-(4-ethyl-3-(hydroxymethyl)-5-oxo-4,5-dihydro-1H-1,2,4-triazol-1-yl)-5-fluoro-N-(2-methoxy-5-methylpyridin-4-yl)-2-((1,1,1-trifluoropropan-2-yl)oxy)nicotinamide.

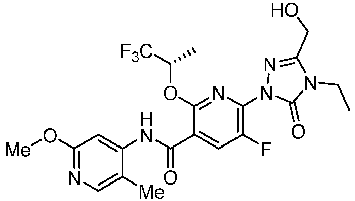

The title compound was prepared according to the representative procedure of Example 15, except substituting 2-methoxy-5-methylpyridin-4-amine for 5-bromoisothiazol-4-amine in Step A. MS (ESI): mass calcd. for C₂₁H₂₂F₄N₆O₅, 514.2; m/z found, 515 [M+H]⁺. ¹H NMR (400MHz, CDCl₃) δ = 9.21 (s, 1H), 8.57 (d, *J* = 9.7 Hz, 1H), 7.97 (s, 1H), 7.82 (s, 1H), 6.15 (m, 1H), 4.70 (d, *J* = 6.3 Hz, 2H), 3.92 (d, *J* = 9.1 Hz, 5H), 2.24 (s, 3H), 2.18 (t, *J* = 6.4 Hz, 1H), 1.67 (d, *J* = 6.6 Hz, 3H), 1.43 (t, *J* = 7.2 Hz, 3H).

### Example 67: (S)-N-(2-Chloro-5-methylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

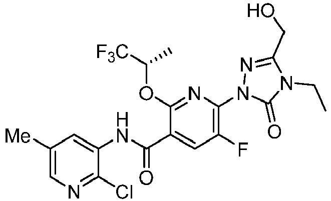

The title compound was prepared according to the representative procedure of Example 15, except substituting 2-chloro-5-methylpyridin-3-amine for 5-bromoisothiazol-4-amine in Step A. MS (ESI): mass calcd. for C₂₀H₁₉ClF₄N₆O₄, 518.1; m/z found, 519 [M+H]⁺. ¹H NMR (400MHz, CDCl₃) δ = 9.78 (s, 1H), 8.70 (d, *J* = 2.2 Hz, 1H), 8.53 (d, *J* = 9.6 Hz, 1H), 8.02 (d, *J* = 2.1 Hz, 1H), 6.17 (m, 1H), 4.70 (d, *J* = 5.9 Hz, 2H), 3.92 (q, *J* = 7.2 Hz, 2H), 2.39 (s, 3H), 2.29 (t, *J* = 6.3 Hz, 1H), 1.69 (d, *J* = 6.6 Hz, 3H), 1.43 (t, *J* = 7.2 Hz, 3H).

### Example 68: (S)-6-(4-Ethyl-3-(hydroxymethyl)-5-oxo-4,5-dihydro-1H-1,2,4-triazol-1-yl)-5-fluoro-N-(2-methoxy-4-methylpyridin-3-yl)-2-((1,1,1-trifluoropropan-2-yl)oxy)nicotinamide.

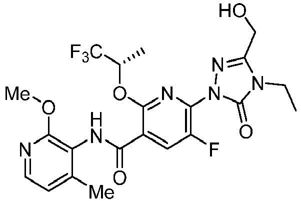

The title compound was prepared according to the representative procedure of Example 15, except substituting 2-methoxy-4-methylpyridin-3-amine for 5-bromoisothiazol-4-amine in Step A. MS (ESI): mass calcd. for C₂₁H₂₂F₄N₆O₅, 514.1; m/z found, 515 [M+H]⁺. ¹H NMR (400MHz, CDCl₃) δ = 9.10 (s, 1H), 8.53 (d, *J* = 9.6 Hz, 1H), 7.97 (d, *J* = 5.3 Hz, 1H), 6.83 (d, *J* = 5.2 Hz, 1H), 6.02 - 5.83 (m, 1H), 4.69 (d, *J* = 6.2 Hz, 2H), 3.96 (s, 3H), 3.91 (d, *J* = 7.2 Hz, 2H), 2.28 (s, 3H), 2.24 (s, 1H), 1.68 (d, *J* = 6.5 Hz, 3H), 1.43 (t, *J* = 7.2 Hz, 3H).

### Example 69: (S)-N-(2,4-Dichloropyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

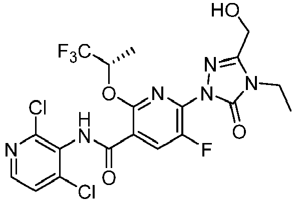

The title compound was prepared according to the representative procedure of Example 15, except substituting 2,4-dichloropyridin-3-amine for 5-bromoisothiazol-4-amine in Step A. MS (ESI): mass calcd. for C₁₉H₁₆Cl₂F₄N₆O₄, 538.1; m/z found, 539 [M+H]⁺. ¹H NMR (400MHz, DMSO-*d*₆) δ = 10.52 (s, 1H), 8.40 (d, *J* = 5.3 Hz, 1H), 8.29 (d, *J* = 8.7 Hz, 1H), 7.79 (d, *J* = 5.3 Hz, 1H), 5.81 (dt, *J* = 17.0, 6.2 Hz, 2H), 4.50 (d, *J* = 5.7 Hz, 2H), 3.80 (q, *J* = 7.1 Hz, 2H), 1.54 (d, *J* = 6.5 Hz, 3H), 1.29 (t, *J* = 7.1 Hz, 3H).

### Example 70: (S)-N-(2-Ethoxy-5-fluoropyrimidin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

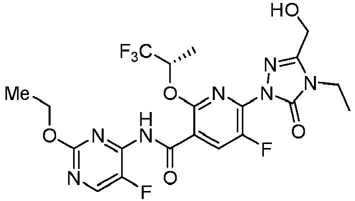

The title compound was prepared according to the representative procedure of Example 15, except substituting 2-ethoxy-5-fluoropyrimidin-4-amine for 5-bromoisothiazol-4-amine in Step A. MS (ESI): mass calcd. for C₂₀H₂₀F₅N₇O₅, 533.1; m/z found, 534 [M+H]⁺. ¹H NMR (400MHz, DMSO-d) δ = 11.32 (s, 1H), 8.56 (d, *J* = 2.6 Hz, 1H), 8.36 (d, *J* = 8.9 Hz, 1H), 5.83 (t, *J* = 5.8 Hz, 1H), 5.64 (h, *J* = 6.5 Hz, 1H), 4.48 (d, *J* = 5.7 Hz, 2H), 3.88 (q, *J* = 7.0 Hz, 2H), 3.78 (q, *J* = 7.2 Hz, 2H), 1.37 - 1.24 (m, 6H), 1.17 (t, *J* = 7.0 Hz, 3H).

### Example 71: (S)-6-(4-Ethyl-3-(hydroxymethyl)-5-oxo-4,5-dihydro-1H-1,2,4-triazol-1-yl)-5-fluoro-N-(3-fluoro-5-methylpyridin-4-yl)-2-((1,1,1-trifluoropropan-2-yl)oxy)nicotinamide.

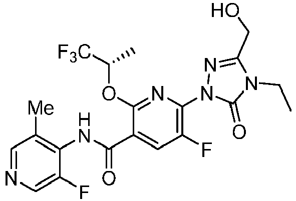

The title compound was prepared according to the representative procedure of Example 15, except substituting 3-fluoro-5-methylpyridin-4-amine for 5-bromoisothiazol-4-amine in Step A. MS (ESI): mass calcd. for C₂₀H₁₉F₅N₆O₄, 502.1; m/z found, 503 [M+H]⁺. ¹H NMR (400MHz, CDCl₃) δ = 9.17 (s, 1H), 8.53 (d, *J* = 9.5 Hz, 1H), 8.38 (d, *J* = 23.2 Hz, 2H), 5.94 (hept, *J* = 6.5 Hz, 1H), 4.68 (s, 2H), 3.93 (q, *J* = 7.2 Hz, 2H), 3.19 (s, 1H), 2.32 (s, 3H), 1.69 (d, *J* = 6.5 Hz, 3H), 1.43 (t, *J* = 7.2 Hz, 3H).

### Example 72: (S)-N-(2-Chloro-4,6-dimethylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

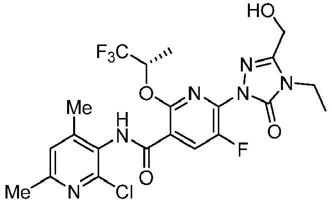

The title compound was prepared according to the representative procedure of Example 15, except substituting 2-chloro-4,6-dimethylpyridin-3-amine for 5-bromoisothiazol-4-amine in Step A. MS (ESI): mass calcd. for C₂₁H₂₁ClF₄N₆O₄, 532.1; m/z found, 533 [M+H]⁺. ¹H NMR (400MHz, CDCl₃) δ = 9.12 (s, 1H), 8.51 (d, *J* = 9.5 Hz, 1H), 7.06 (s, 1H), 5.96 (hept, *J* = 6.5 Hz, 1H), 4.67 (d, *J* = 5.6 Hz, 2H), 3.92 (q, *J* = 7.2 Hz, 2H), 3.16 (t, *J* = 6.1 Hz, 1H), 2.52 (s, 3H), 2.29 (s, 3H), 1.67 (d, *J* = 6.5 Hz, 3H), 1.42 (t, *J=* 7.2 Hz, 3H).

### Example 73: (S)-N-(1-Cyclopropyl-1H-imidazol-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-vl)oxy)nicotinamide.

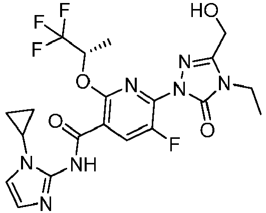

The title compound was prepared according to the representative procedure of Example 15, except substituting 1-cyclopropyl-1H-imidazol-2-amine for 5-bromoisothiazol-4-amine in Step A. MS (ESI): mass calcd. for C₂₀H₂₁F₄N₇O₄, 499.2; m/z found, 500 [M+H]⁺. ¹H NMR (400MHz, DMSO-*d*₆) δ = 8.29 (d, *J* = 9.1 Hz, 1H), 7.00 (s, 1H), 6.79 (d, *J* = 2.1 Hz, 1H), 5.96 - 5.71 (m, 2H), 4.49 (s, 2H), 3.79 (q, *J* = 7.1 Hz, 2H), 3.44 (s, 1H), 1.48 (d, *J* = 6.5 Hz, 3H), 1.28 (t, *J* = 7.1 Hz, 3H), 0.94 (d, *J* = 6.0 Hz, 4H).

### Example 74: (S)-N-(2-Chloro-5-cyanophenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

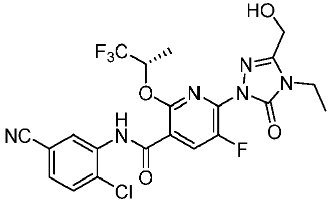

The title compound was prepared according to the representative procedure of Example 15, except substituting 3-amino-4-chlorobenzonitrile for 5-bromoisothiazol-4-amine in Step A. MS (ESI): mass calcd. for C₂₁H₁₇ClF₄N₆O₄, 528.2; m/z found, 529 [M+H]⁺. ¹H NMR (400MHz, CDCl₃) δ = 9.88 (s, 1H), 8.94 (d, *J* = 1.9 Hz, 1H), 8.56 (d, *J* = 9.6 Hz, 1H), 7.56 (d, *J* = 8.3 Hz, 1H), 7.40 (dd, *J* = 8.3, 1.9 Hz, 1H), 6.16 (hept, *J* = 6.6 Hz, 1H), 4.70 (d, *J* = 6.1 Hz, 2H), 3.92 (q, *J* = 7.2 Hz, 2H), 2.21 (t, *J* = 6.3 Hz, 1H), 1.68 (d, *J*= 6.6 Hz, 3H), 1.43 (t, *J* = 7.2 Hz, 3H).

### Example 75: (S)-N-(2-Chloro-5-cyanopyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

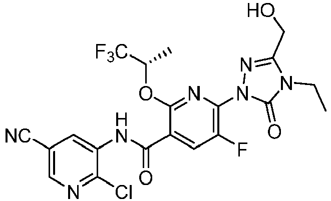

The title compound was prepared according to the representative procedure of Example 15, except substituting 5-amino-6-chloronicotinonitrile for 5-bromoisothiazol-4-amine in Step A. MS (ESI): mass calcd. for C₂₀H₁₆ClF₄N₇O₄, 529.1; m/z found, 530 [M+H]⁺. ¹H NMR (400MHz, CDCl₃) δ = 9.97 (s, 1H), 9.26 (d, *J* = 2.0 Hz, 1H), 8.55 (d, *J* = 9.6 Hz, 1H), 8.45 (d, *J* = 2.0 Hz, 1H), 6.20 (hept, *J* = 6.5 Hz, 1H), 4.70 (d, *J* = 6.0 Hz, 2H), 3.92 (q, *J* = 7.2 Hz, 2H), 2.35 (t, *J* = 6.3 Hz, 1H), 1.70 (d, *J* = 6.6 Hz, 3H), 1.43 (t, *J* = 7.2 Hz, 3H).

### Example 76: (S)-N-(4-(Dimethylamino)-2-methoxypyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide.

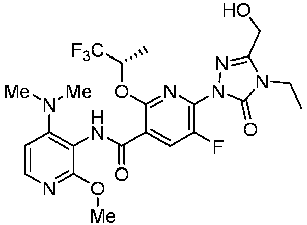

The title compound was prepared according to the representative procedure of Example 15, except substituting 2-methoxy-N4,N4-dimethylpyridine-3,4-diamine for 5-bromoisothiazol-4-amine in Step A. MS (ESI): mass calcd. for C₂₂H₂₅F₄N₇O₅, 543.2; m/z found, 544 [M+H]⁺. ¹H NMR (400MHz, CDCl₃) δ = 8.82 (s, 1H), 8.52 (d, *J* = 9.5 Hz, 1H), 7.89 (d, *J* = 6.2 Hz, 1H), 6.50 (d, *J* = 6.2 Hz, 1H), 5.90 (m, 1H), 4.70 (s, 2H), 3.98 (s, 3H), 3.92 (q, *J* = 7.2 Hz, 2H), 2.95 (s, 6H), 1.67 (d, *J* = 6.5 Hz, 3H), 1.43 (t, *J* = 7.2 Hz, 3H).

### Example 77: N-(2-Chloro-6-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

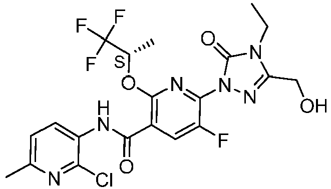

The title compound was prepared according to the representative procedures of Example 15, except substituting 3-amino-2-chloro-6-methylpyridine for 5-bromoisothiazol-4-amine in Step A. MS (ESI): mass calcd. for C₂₀H₁₉ClF₄N₆O₄, 518.11; m/z found, 519.2 [M+H]⁺. ¹H NMR (400 MHz, CDCl₃) δ ppm 9.76 (s, 1H) 8.71 (d, *J* = 8.31 Hz, 1H) 8.54 (d, *J* = 9.78 Hz, 1H) 7.17 (d, *J* = 8.31 Hz, 1H) 6.10 - 6.22 (m, 1H) 4.70 (d, *J* = 6.36 Hz, 2H) 3.92 (d, *J* = 6.85 Hz, 2H) 2.54 (s, 3H) 1.69 (d, *J* = 6.36 Hz, 3H) 1.43 (t, *J* = 7.09 Hz, 3H).

### Example 78: N-(2-Chloro-5-fluoro-3-pyridyl)-6-(4-ethyl-3-(hydroxymethyl)-5-oxo-1,2,4-triazol-1-yl]-5-fluoro-2-((1S)-2,2,2-trifluoro-1-methyl-ethoxylpyridine-3-carboxamide.

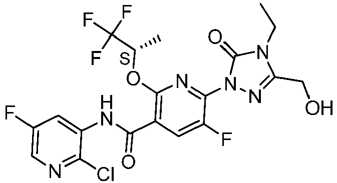

The title compound was prepared according to the representative procedures of Example 15, except substituting 2-chloro-5-fluoropyridin-3-amine for 5-bromoisothiazol-4-amine in Step A. MS (ESI): mass calcd. for C₁₉H₁₆ClF₅N₆O₄, 522.08; m/z found, 523.2 [M+H]⁺. ¹H NMR (400 MHz, CDCl₃) δ ppm 9.90 (s, 1H) 8.72 - 8.92 (m, 1H) 8.53 (d, *J* = 9.29 Hz, 1H) 8.08 (d, *J* = 2.45 Hz, 1H) 6.09 - 6.32 (m, 1H) 4.70 (d, *J* = 6.36 Hz, 2H) 3.92 (d, *J* = 7.34 Hz, 2H) 1.70 (d, *J* = 6.85 Hz, 3H) 1.43 (t, *J* = 7.34 Hz, 3H).

### Example 79: N-(4-Chloro-2-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

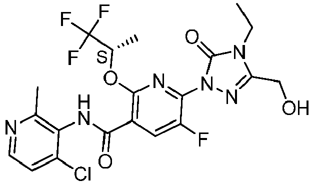

The title compound was prepared according to the representative procedure of Example 15, except substituting 4-chloro-2-methylpyridin-3-amine for 5-bromoisothiazol-4-amine in Step A. MS (ESI): mass calcd. for C₂₀H₁₉ClF₄N₆O₄, 518.1; m/z found, 519 [M+H]⁺. ¹H NMR (400MHz, CDCl₃) δ = 9.10 (s, 1H), 8.54 (d, *J* = 9.5 Hz, 1H), 8.38 (d, *J* = 5.3 Hz, 1H), 7.31 (d, *J* = 5.3 Hz, 1H), 6.00 - 5.90 (m, 1H), 4.70 (d, *J* = 6.1 Hz, 2H), 3.92 (q, *J* = 7.2 Hz, 2H), 2.56 (s, 3H), 2.31 (d, *J* = 6.3 Hz, 1H), 1.68 (d, *J* = 6.5 Hz, 3H), 1.43 (t, *J* = 7.2 Hz, 3H).

### Example 80: N-(3-Chloro-1-methyl-pyrazol-4-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

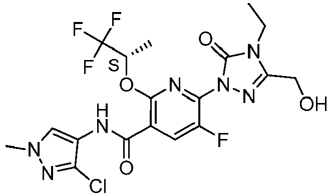

The title compound was prepared according to the representative procedure of Example 15, except substituting 3-chloro-1-methyl-1H-pyrazol-4-amine for 5-bromoisothiazol-4-amine in Step A. MS (ESI): mass calcd. for C₁₈H₁₈ClF₄N₇O₄, 507.1; m/z found, 508 [M+H]⁺. ¹H NMR (400MHz, CDCl₃) δ = 9.32 (s, 1H), 8.55 (d, *J* = 9.6 Hz, 1H), 7.38 (s, 1H), 5.97 (m, 1H), 4.68 (s, 2H), 3.91 (d, *J* = 10.2 Hz, 5H), 2.71 - 2.54 (m, 1H), 1.67 (d, *J* = 6.5 Hz, 3H), 1.42 (t, *J* = 7.2 Hz, 3H).

### Example 81: N-[2-(Difluoromethyl)-5-fluoro-4-pyridyl]-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

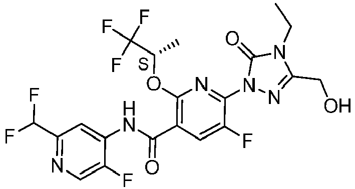

The title compound was prepared according to the representative procedure of Example 15, except substituting 2-(difluoromethyl)-5-fluoropyridin-4-amine for 5-bromoisothiazol-4-amine in Step A. MS (ESI): mass calcd. for C₂₀H₁₇F₇N₆O₄, 538.1; m/z found, 539 [M+H]⁺. ¹H NMR (400MHz, CDCl₃) δ = 10.09 (d, *J* = 3.4 Hz, 1H), 8.91 (d, *J* = 5.9 Hz, 1H), 8.54 (d, *J* = 9.5 Hz, 2H), 6.64 (t, *J* = 55.3 Hz, 1H), 6.03 (hept, *J* = 6.4 Hz, 1H), 4.70 (s, 2H), 3.92 (q, *J* = 7.2 Hz, 2H), 2.55 (s, 1H), 1.70 (d, *J* = 6.5 Hz, 3H), 1.43 (t, *J* = 7.2 Hz, 3H).

### Example 82: 6-[4-Ethyl-3-(hydroxymethyl)-5-oxo-1,2,4-triazol-1-yl]-5-fluoro-N-(5-fluoro-2-methoxy-pyrimidin-4-yl)-2-[(1S)-2,2,2-trifluoro-1-methyl-ethoxy]pyridine-3-carboxamide.

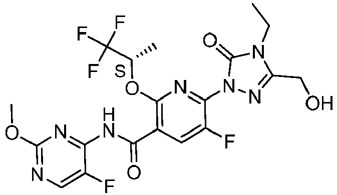

The title compound was prepared according to the representative procedure of Example 15, except substituting 5-fluoro-2-methoxypyrimidin-4-amine for 5-bromoisothiazol-4-amine in Step A. MS (ESI): mass calcd. for C₁₉H₁₈F₅N₇O₅, 519.1; m/z found, 520 [M+H]⁺. ¹H NMR (400MHz, CDCl₃) δ = 9.92 (s, 1H), 8.52 (d, *J* = 9.5 Hz, 1H), 8.34 (d, *J* = 2.1 Hz, 1H), 5.95 (m, 1H), 4.69 (s, 2H), 4.01 (s, 3H), 3.92 (q, *J* = 7.2 Hz, 2H), 2.37 (s, 1H), 1.68 (d, *J* = 6.5 Hz, 3H), 1.42 (t, *J* = 7.2 Hz, 3H).

### Example 83: N-(6-Chloro-2.3-dimethyl-phepyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

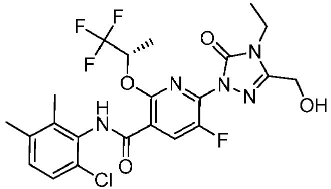

The title compound was prepared according to the representative procedure of Example 15, except substituting 6-chloro-2,3-dimethylaniline for 5-bromoisothiazol-4-amine in Step A. MS (ESI): mass calcd. for C₂₂H₂₂ClF₄N₅O₄, 531.1; m/z found, 532 [M+H]⁺. ¹H NMR (400MHz, CDCl₃) δ = 9.06 (s, 1H), 8.54 (d, *J* = 9.6 Hz, 1H), 7.23 (d, *J* = 8.2 Hz, 1H), 7.09 (d, *J* = 8.2 Hz, 1H), 5.95 (hept, *J* = 6.6 Hz, 1H), 4.68 (s, 2H), 3.91 (q, *J* = 7.2 Hz, 2H), 2.31 (s, 3H), 2.20 (s, 3H), 1.67 (d, *J* = 6.5 Hz, 3H), 1.43 (t, *J* = 7.2 Hz, 3H).

### Example 84: N-(2-Chloro-4-methyl-3-pyridyl)-5-fluoro-6-[3-(hydroxymethyl)-5-oxo-4-propyl-1,2,4-triazol-1-yl]-2-[(1S)-2,2,2-trifluoro-1-methyl-ethoxy]pyridine-3-carboxamide.

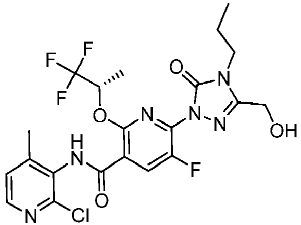

Step A: Isopropyl 6-(4-allyl-3-((benzyloxy)methyl)-5-oxo-4,5-dihydro-1H-1,2,4-triazol-1-yl)-2-chloro-5-fluoronicotinate . The title compound was prepared according to the representative procedure of Example 1, Steps A to C, except substituting 4-allyl-5-((benzyloxy)methyl)-2,4-dihydro-3H-1,2,4-triazol-3-one (Intermediate 9) in Step C. MS (ESI): mass calcd. for C₂₂H₂₂ClFN₄O₄, 460.1; m/z found, 461.1 [M+H]⁺. ¹H NMR (400 MHz, CDCl₃) δ 8.10 (d, *J* = 8.80 Hz, 1H), 7.30-7.40 (m, 5H), 5.80-5.92 (m, 1H), 5.17-5.33 (m, 3H), 4.58 (s, 2H), 4.51 (s, 2H), 4.40-4.44 (m, 2H), 1.41 (d, *J* =6.36 Hz, 6H).

Step B: Isopropyl (S)-6-(4-allyl-3-((benzyloxy)methyl)-5-oxo-4,5-dihydro-1H-1,2,4-triazol-1-yl)-5-fluoro-2-((1,1,1-trifluoropropan-2-yl)oxy)nicotinate. A solution of (S)-1,1,1-trifluoro-2-propanol (0.116 mL, 1.302 mmol) in THF (1 mL) was stirred at -10 °C under argon for 30 min. This solution was added to a solution of isopropyl 6-(4-allyl-3-((benzyloxy)methyl)-5-oxo-4,5-dihydro-1H-1,2,4-triazol-1-yl)-2-chloro-5-fluoronicotinate in THF (2.5 mL) at -78 °C which was stirred at -78 °C for 2h. The reaction mixture was poured into 1N HCl. The aqueous layer was extracted with ethyl acetate. The combined organic phase was washed with brine, dried over anhydrous MgSO₄, filtered and evaporated in vacuum. The residue was purified by column chromatography (SiO₂, Heptane 100% to Heptane 60% Ethyl acetate 40%) to afford desired product (260 mg, yield 44%). MS (ESI): mass calcd. for C₂₅H₂₆F₄N₄O₅, 538.2; m/z found, 539.3 [M+H]⁺.

Step C: Isopropyl (S)-5-fluoro-6-(3-(hydroxymethyl)-5-oxo-4-propyl-4,5-dihydro-1H-1,2,4-triazol-1-yl)-2-((1,1,1-trifluoropropan-2-yl)oxy)nicotinate. A solution of isopropyl (S)-6-(4-allyl-3-((benzyloxy)methyl)-5-oxo-4,5-dihydro-1H-1,2,4-triazol-1-yl)-5-fluoro-2-((1,1,1-trifluoropropan-2-yl)oxy)nicotinate (Example 84 product from Step B, 50 mg, 0.0929 mmol) in MeOH (0.57 mL) with Pd/C (10%) as catalyst was hydrogenated at room temperature under atmospheric pressure for 2h. The reaction mixture was filtered over Celite^{®}. The filtrate was washed with DCM/ MeOH. The solvent was evaporated until dryness to give (50 mg, quantitative yield). MS (ESI): mass calcd. for C₁₈H₂₂F₄N₄O₅, 450.2; m/z found, 451.2 [M+H]⁺.

Step D: N-(2-Chloro-4-methyl-3-pyridyl)-5-fluoro-6-(3-(hydroxymethyl)-5-oxo-4-propyl-1,2,4-triazol-1-yl]-2-[(1S)-2,2,2-trifluoro-1-methyl-ethoxy]pyridine-3-carboxamide. The title compound was prepared according to the representative procedure of Example 1, Step E, except substituting 3-amino-2-chloro-4-methylpyridine for 2-chloro-6-fluoroaniline and isopropyl (S)-5-fluoro-6-(3-(hydroxymethyl)-5-oxo-4-propyl-4,5-dihydro-1H-1,2,4-triazol-1-yl)-2-((1,1,1-trifluoropropan-2-yl)oxy)nicotinate for isopropyl (S)-6-(3-((benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinate. MS (ESI): mass calcd. for C₂₁H₂₁ClF₄N₆O₄, 532.12; m/z found, 533.2 [M+H]⁺. ¹H NMR (400 MHz, CDCl₃) δ ppm 9.21 (s, 1H) 8.54 (d, *J* = 9.29 Hz, 1H) 8.23 (d, *J* = 4.89 Hz, 1H) 7.20 (d, *J* = 5.38 Hz, 1H) 5.86 - 6.09 (m, 1H) 4.69 (d, *J* = 6.36 Hz, 2H) 3.74 - 3.93 (m, 2H) 2.34 (s, 3H) 1.78 - 1.93 (m, 2H) 1.69 (d, *J* = 6.36 Hz, 3H) 1.03 (t, *J* = 7.34 Hz, 3H).

### Example 85: 6-[4-Ethyl-3-(hydroxymethyl)-5-oxo-1,2,4-triazol-1-yl]-5-fluoro-N-(2-methoxy-3,5-dimethyl-4-pyridyl)-2-[(1S)-2,2,2-trifluoro-1-methyl-ethoxy]pyridine-3-carboxamide.

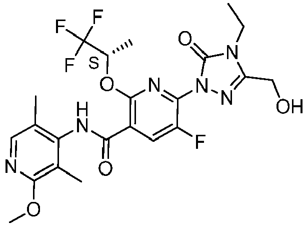

The title compound was prepared according to the representative procedure of Example 15, except using 2-methoxy-3,5-dimethylpyridin-4-amine instead of 5-bromoisothiazol-4-amine in Step A. MS (ESI): mass calcd. for C₁₉H₁₆ClF₅N₆O₄, 532.12; m/z found, 533.2 [M+H]⁺. ¹H NMR (400 MHz, CDCl₃) δ ppm 8.92 (s, 1H) 8.55 (d, *J* = 9.78 Hz, 1H) 7.92 (s, 1H) 5.85 - 6.04 (m, 1H) 4.70 (d, *J* = 6.36 Hz, 2H) 3.96 (s, 3H) 3.91 (s, 1H) 2.16 (s, 3H) 2.11 (s, 3H) 1.67 (d, *J* = 6.36 Hz, 3H) 1.43 (t, *J* = 7.09 Hz, 3H).

### Example 86: (S)-2-(5-(((2-Chloro-4-methylpyridin-3-yl)-12-azaneyl)carbonyl)-3-fluoro-6-((1,1,1-trifluoropropan-2-yl)oxy)pyridin-2-yl)-5-(fluoromethyl)-4-isopropyl-2,4-dihydro-3H-1,2,4-triazol-3-one.

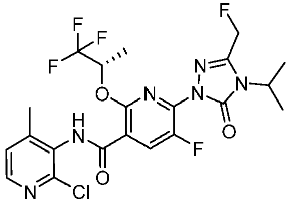

Step A: (S)-(1-(5-((2-Chloro-4-methylpyridin-3-yl)carbamoyl)-3-fluoro-6-((1,1,1-trifluoropropan-2-yl)oxy)pyridin-2-yl)-4-isopropyl-5-oxo-4,5-dihydro-1H-1,2,4-triazol-3-yl)methyl methanesulfonate. To a solution of N-(2-chloro-4-methyl-3-pyridyl)-5-fluoro-6-[3-(hydroxymethyl)-4-isopropyl-5-oxo-1,2,4-triazol-1-yl]-2-[(1S)-2,2,2-trifluoro-1-methyl-ethoxy]pyridine-3-carboxamide (Example 89, 55 mg, 0.103 mmol), in DCM (0.4 mL) at 0°C was added NEts (28.7 µL, 0.206 mmol) and methanesulfonyl chloride (8.0 µL, 0.103 mmol). The reaction was warmed to room temperature and stirred for 3 hours. The reaction was diluted with ethyl acetate and H₂O. The organic layer was washed with H₂O and brine, dried over MgSO₄, filtered and concentrated under reduced pressure. The residue would be used crude in the next step without further purification. MS (ESI): mass calcd. for C₂₂H₂₃ClF₄N₆O₆S, 610.10; m/z found, 611.1 [M+H]⁺.

Step B: (S)-2-(5-(((2-Chloro-4-methylpyridin-3-yl)-l2-azaneyl)carbonyl)-3-fluoro-6-((1,1,1-trifluoropropan-2-yl)oxy)pyridin-2-yl)-5-(fluoromethyl)-4-isopropyl-2,4-dihydro-3H-1,2,4-triazol-3-one. To a solution of (S)-(1-(5-((2-chloro-4-methylpyridin-3-yl)carbamoyl)-3-fluoro-6-((1,1,1-trifluoropropan-2-yl)oxy)pyridin-2-yl)-4-isopropyl-5-oxo-4,5-dihydro-1H-1,2,4-triazol-3-yl)methyl methane sulfonate in THF (1.1 mL) at 0°C was added tetra-n-butylammonium fluoride (TBAF) (1.0 M in THF, 0.36 mL, 0.362 mmol). The reaction was warmed to room temperature and was subsequently heated to 50°C for 20 mins. The reaction was cooled to room temperature, concentrated under reduced pressure and purified with flash column chromatography to provide the title compound as a white solid (6.8 mg, 12% yield). MS (ESI): mass calcd. for C₂₁H₂₀ClF₅N₆O₃, 534.12; m/z found, 535.2 [M+H]⁺. ¹H NMR (400 MHz, CDCl₃) δ = 9.20 (s, 1H), 8.55 (d, *J* = 9.29 Hz, 1H), 8.23 (d, *J* = 4.89 Hz, 1H), 7.20 (d, *J* = 5.16 Hz, 1H), 5.97 (td, *J* = 6.42, 13.08 Hz, 1H), 5.87-6.01 (m, 1H), 5.18-5.49 (m, 1H), 4.45 (td, *J* = 6.85, 13.69 Hz, 1H), 2.34 (s, 3H), 1.69 (d, *J* = 6.36 Hz, 3H), 1.59-1.63 (m, 6H).

### Example 87: N-(2-Chloro-4-methyl-3-pyridyl)-6-[4-ethyl-3-(fluoromethyl)-5-oxo-1,2,4-triazol-1-yl]-5-fluoro-2-[(1S)-2,2,2-trifluoro-1-methyl-ethoxylpyridine-3-carboxamide.

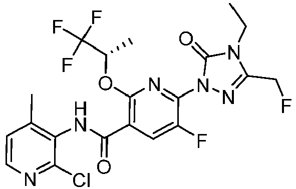

Step A: Isopropyl (S)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinate. A mixture of isopropyl (S)-6-(3-((benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinate (Example 1, product from Step D, 400 mg, 0.76 mmol) and trifluoroacetic acid (TFA) (15 mL, 0.76 mmol) was stirred at 65 °C for 12 h. The reaction mixture was filtered and concentrated under reduced pressure. The residue was taken up in DCM, washed with 1N NaOH and brine, dried over Na₂SO₄, filtered, and concentrated under reduced pressure. The residue was purified by flash column chromatography to afford the title compound (260 mg, 78% yield) as a tan solid. MS (ESI): mass calcd. for C₁₇H₂₀F₄N₄O₅, 436.1; m/z found, 437.1 [M+H]+. ¹H NMR (400 MHz, CDCl₃) δ = 1.34 - 1.38 (m, 6 H) 1.39 - 1.44 (m, 3 H) 1.53 - 1.57 (m, 3 H) 2.14 (t, *J* = 6.60 Hz, 1 H) 3.90 (q, *J* = 7.01 Hz, 2 H) 4.68 (d, *J* = 6.36 Hz, 2 H) 5.19 - 5.30 (m, 1 H) 5.66 (m, 1 H) 8.13 (d, *J* = 9.29 Hz, 1 H).

Step B: Isopropyl (S)-6-(4-ethyl-3-(fluoromethyl)-5-oxo-4,5-dihydro-1H-1,2,4-triazol-1-yl)-5-fluoro-2-((1,1,1-trifluoropropan-2-yl)oxy)nicotinate. To a mixture of Isopropyl (S)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinate (120 mg, 0.275 mmol) in DCM (4.4 mL) at - 78°C was added DAST (0.168 mL, 1.375 mmol). The reaction was slowly warmed to room temperature and let stir for 12 hours. The reaction was quenched with MeOH and diluted with DCM. The aqueous layer was extracted (3x) with DCM and the combined organics were washed with brine, dried over Na2SO₄, filtered and concentrated under reduced pressure. The residue was purified by flash column chromatography (SiO₂, eluting with a gradient of 0 to 50% ethyl acetate in heptane) to afford the title compound as a clear oil (107 mg, 0.244 mmol, 89% yield). MS (ESI): mass calcd. for C₁₇H₁₉F₅N₄O₄, 438.1; m/z found, 439.2 [M+H]⁺. ¹H NMR (400 MHz, CDCl₃) δ = 8.14 (d, *J* = 8.80 Hz, 1H), 5.65 (td, *J* = 6.54, 12.84 Hz, 1H), 5.42 (s, 1H), 5.22-5.34 (m, 2H), 3.90 (q, *J* = 7.34 Hz, 2H), 1.54-1.59 (m, 5H), 1.30-1.46 (m, 10H).

Step C: N-(2-Chloro-4-methyl-3-pyridyl)-6-[4-ethyl-3-(fluoromethyl)-5-oxo-1,2,4-triazol-1-yl]-5-fluoro-2-[(1S)-2,2,2-trifluoro-1-methyl-ethoxylpyridine-3-carboxamide. The title compound was prepared according to the representative procedure of Example 4, Step A, except using 3-amino-2-chloro-4-methylpyridine instead of 3-amino-2-chloropyridine. MS (ESI): mass calcd. for C₂₀H₁₈ClF₅N₆O₃, 520.10; m/z found, 521.1 [M+H]⁺. ¹H NMR (400 MHz, CDCl₃) δ = 9.20 (s, 1H), 8.56 (d, *J* = 9.29 Hz, 1H), 8.23 (d, *J* = 4.89 Hz, 1H), 7.18-7.24 (m, 1H), 5.96 (td, *J* = 6.54, 12.84 Hz, 1H), 5.44 (s, 1H), 5.32 (s, 1H), 3.92 (q, *J* = 7.17 Hz, 2H), 2.35 (s, 3H), 1.67-1.72 (m, 3H), 1.42-1.47 (m, 3H).

### Example 88: N-(3-Chloro-2-methoxy-5-methyl-4-pyridyl)-6-[4-ethyl-3-(hydroxymethyl)-5-oxo-1,2,4-triazol-1-yl]-5-fluoro-2-isopropoxy-pyridine-3-carboxamide.

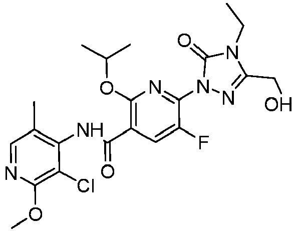

The title compound was prepared according to the representative procedure of Example 15, steps A-B except substituting 3-chloro-2-methoxy-5-methylpyridin-4-amine (Intermediate 5) for 5-bromoisothiazol-4-amine and 6-(3-((benzyloxy)methyl)-4-ethyl-5-oxo-4,5-dihydro-1H-1,2,4-triazol-1-yl)-5-fluoro-2-isopropoxynicotinic acid (Intermediate 20) for isopropyl (S)-6-(3-((benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinate. MS (ESI): mass calcd. for C₂₈H₃₀ClFN₆O₅, 584.2; m/z found, 585 [M+H]⁺. ¹H NMR (CDCl₃, 400 MHz) δ 9.91 (s, 1H), 8.48 (d, 1H, *J* = 9.3 Hz), 7.97 (s, 1H), 7.3-7.4 (m, 5H), 5.63 (m, 1H), 4.62 (s, 2H), 4.53 (s, 2H), 4.03 (s, 3H), 3.8-3.9 (m, 2H), 2.22 (s, 3H), 1.51 (d, 6H, *J* = 5.9 Hz), 1.4-1.4 (m, 3H).

### Example 89: N-(2-Chloro-4-methyl-3-pyridyl)-5-fluoro-6-[3-(hydroxymethyl)-4-isopropyl-5-oxo-1,2,4-triazol-1-yl]-2-[(1S)-2,2,2-trifluoro-1-methyl-ethoxy]pyridine-3-carboxamide.

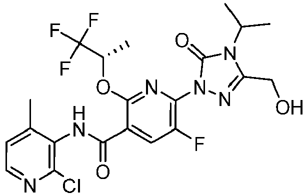

The title compound was prepared according to the representative procedure of Example 90, except using 5-((benzyloxy)methyl)-4-isopropyl-2,4-dihydro-3H-1,2,4-triazol-3-one (Intermediate 10) in Step A. MS (ESI): mass calcd. for C₂₁H₂₁ClF₄N₆O₄, 532.12; m/z found, 533.1 [M+H]⁺. ¹H NMR (400 MHz, CDCl₃) δ = 1.58 - 1.63 (m, 6 H) 1.68 (d, *J* = 6.36 Hz, 3 H) 2.34 (s, 3 H) 2.37 - 2.43 (m, 1 H) 4.47 (dt, *J* = 13.69, 6.85 Hz, 1 H) 4.69 (s, 2 H) 5.98 (dt, *J* = 12.72, 6.36 Hz, 1 H) 7.17 - 7.23 (m, 1 H) 8.23 (d, *J* = 4.89 Hz, 1 H) 8.53 (d, *J* = 9.29 Hz, 1 H) 9.21 (s, 1 H).

### Example 90: N-(2-Chloro-4-methyl-3-pyridyl)-6-[4-(2,2-difluoroethyl)-3-(hydroxymethyl)-5-oxo-1,2,4-triazol-1-yl]-5-fluoro-2-[(1S)-2,2,2-trifluoro-1-methyl-ethoxy]pyridine-3-carboxamide.

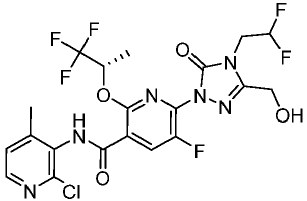

Step A: (S)-6-(3-((Benzyloxy)methyl)-4-(2,2-difluoroethyl)-5-oxo-4,5-dihydro-1H-1,2,4-triazol-1-yl)-N-(2-chloro-4-methylpyridin-3-yl)-5-fluoro-2-((1,1,1-trifluoropropan-2-yl)oxy)nicotinamide. The title compound was prepared according to the representative procedure of Example 1, Steps A to E, except substituting 5-((benzyloxy)methyl)-4-(2,2-difluoroethyl)-2,4-dihydro-3H-1,2,4-triazol-3-one (Intermediate 11) in Step C and substituting 3-amino-2-chloro-4-methylpyridine for 2-chloro-6-fluoroaniline in Step E. MS (ESI): mass calcd. for C₂₇H₂₃ClF₆N₆O₄, 644.14; m/z found, 645.2 [M+H]⁺. ¹H NMR (400 MHz, CDCl₃) δ = 1.68 - 1.72 (m, 3 H) 2.34 (s, 3 H) 4.15 - 4.29 (m, 2 H) 4.58 - 4.61 (m, 2 H) 4.61 - 4.65 (m, 2 H) 5.91 - 6.00 (m, 1 H) 6.05 - 6.24 (m, 1 H) 7.18 - 7.23 (m, 1 H) 7.34 - 7.41 (m, 5 H) 8.23 (d, *J* = 4.89 Hz, 1 H) 8.56 (d, *J* = 9.29 Hz, 1 H) 9.20 (s, 1 H).

Step B: N-(2-Chloro-4-methyl-3-pyridyl)-6-[4-(2,2-difluoroethyl)-3-(hydroxymethyl)-5-oxo-1,2,4-triazol-1-yl]-5-fluoro-2-[(1S)-2,2,2-trifluoro-1-methyl-ethoxy]pyridine-3-carboxamide. A mixture of (S)-6-(3-((benzyloxy)methyl)-4-(2,2-difluoroethyl)-5-oxo-4,5-dihydro-1H-1,2,4-triazol-1-yl)-N-(2-chloro-4-methylpyridin-3-yl)-5-fluoro-2-((1,1,1-trifluoropropan-2-yl)oxy)nicotinamide (70 mg, 0.11 mmol) and trifluoroacetic acid (TFA) (2.7 mL, 0.5 mmol) was stirred at 70 °C for 12 h. The reaction mixture was filtered and concentrated under reduced pressure. The residue was purified by prep-HPLC (Method F) to afford the title compound (6 mg, 10% yield) as a white solid. MS (ESI): mass calcd. for C₂₀H₁₇ClF₆N₆O₄, 554.09; m/z found, 555.0 [M+H]⁺. ¹H NMR (400 MHz, CDCl₃) δ = 9.20 (s, 1H), 8.55 (d, *J* = 9.29 Hz, 1H), 8.23 (d, *J* = 4.89 Hz, 1H), 7.17-7.25 (m, 1H), 6.15 (t, *J* = 4.16 Hz, 1H), 5.91-6.03 (m, 1H), 4.76 (d, *J* = 5.87 Hz, 2H), 4.31 (dt, *J* = 4.16, 13.82 Hz, 2H), 2.35 (s, 3H), 1.63-1.75 (m, 4H).

### Example 91: N-(5-Chloro-3-methyl-1H-pyrazol-4-yl)-5-fluoro-6-[3-(hydroxymethyl)-5-oxo-4-propyl-1,2,4-triazol-1-yl]-2-[(1S)-2,2,2-trifluoro-1-methyl-ethoxylpyridine-3-carboxamide.

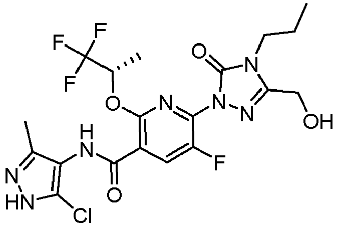

Step A: Isopropyl (S)-5-fluoro-6-(3-(hydroxymethyl)-5-oxo-4-propyl-4,5-dihydro-1H-1,2,4-triazol-1-yl)-2-((1,1,1-trifluoropropan-2-yl)oxy)nicotinate. A solution of isopropyl (S)-6-(4-allyl-3-((benzyloxy)methyl)-5-oxo-4,5-dihydro-1H-1,2,4-triazol-1-yl)-5-fluoro-2-((1,1,1-trifluoropropan-2-yl)oxy)nicotinate (Example 84 product from Step B, 160 mg, 0.297 mmol) in MeOH (1.80 mL) with Pd/C (10%) (31 mg) as catalyst was hydrogenated at room temperature under atmospheric pressure for 2h The reaction mixture was filtered over Celite^{®}. The filtrate was washed with DCM/ MeOH. The solvent was concentrated under reduced pressure to afford the title compound (82 mg, yield 82.1%) and used without purification. MS (ESI): mass calcd. for C₁₈H₂₂F₄N₄O₅, 450.2; m/z found, 451.2 [M+H]⁺. ¹H NMR (400 MHz, CDCl₃) δ = 8.13 (d, *J* = 8.80 Hz, 1H), 5.56 - 5.77 (m, 1H), 5.12 - 5.33 (m, 1H), 4.67 (d, *J* = 5.87 Hz, 2H), 3.65 - 3.88 (m, 2H), 1.75 - 1.94 (m, 3H), 1.56 (d, *J* = 6.36 Hz, 3H), 1.36 (dd, *J* = 6.11, 3.67 Hz, 7H), 1.01 (t, *J* = 7.58 Hz, 4 H).

Step B: N-(5-Chloro-3-methyl-1H-pyrazol-4-yl)-5-fluoro-6-[3-(hydroxymethyl)-5-oxo-4-propyl-1,2,4-triazol-1-yl]-2-[(1S)-2,2,2-trifluoro-1-methyl-ethoxy]pyridine-3-carboxamide. The title compound was prepared according to the representative procedure of Example 1, Step E, except substituting 5-chloro-3-methyl-4-amino-1H-pyrazole for 2-chloro-6-fluoroaniline and isopropyl (S)-5-fluoro-6-(3-(hydroxymethyl)-5-oxo-4-propyl-4,5-dihydro-1H-1,2,4-triazol-1-yl)-2-((1,1,1-trifluoropropan-2-yl)oxy)nicotinate for isopropyl (S)-6-(3-((benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinate (33 mg, yield 25.9%). MS (ESI): mass calcd. for C₁₉H₂₀ClF₄N₇O₄, 521.1; m/z found, 522.2 [M+H]⁺. ¹H NMR (400 MHz, CDCl₃) δ = 8.83 (s, 1H), 8.53 (d, *J* = 9.78 Hz, 1H), 5.92 (s, 1H), 4.69 (s, 2H), 3.67 - 3.93 (m, 2H), 2.34 (s, 3H), 1.77 - 1.93 (m, 3H), 1.66 - 1.70 (m, 4H), 1.03 (t, *J* = 7.34 Hz, 3 H).

### Example 93: N-(2,4-Dimethoxy-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

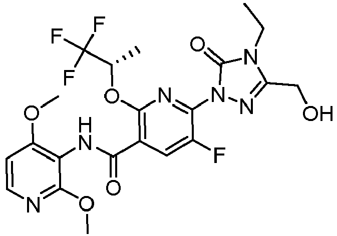

The title compound was prepared according to the representative procedure of Example 15, except using 2,4-dimethoxypyridin-3-amine instead of 5-bromoisothiazol-4-amine in Step A. MS (ESI): mass calcd. for C₂₁H₂₂F₄N₆O₆, 530.44; m/z found, 531.2 [M+H]⁺. ¹H NMR (400 MHz, CDCl₃) δ ppm 8.88 (s, 1H), 8.51 (d, *J* = 9.6 Hz, 1H), 8.04 (d, *J* = 5.9 Hz, 1H), 6.64 (d, *J* = 5.9 Hz, 1H), 5.89 (hept, *J* = 6.5 Hz, 1H), 4.63 (s, 2H), 3.98 (s, 3H), 3.90 (d, *J* = 8.7 Hz, 5H), 1.66 (d, *J* = 6.5 Hz, 3H), 1.40 (t, *J* = 7.2 Hz, 3H).

### Example 94: N-(4,6-Dimethoxypyrimidin-5-yl)-6-r4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

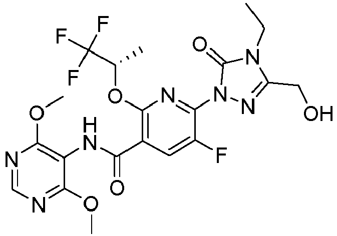

The title compound was prepared according to the representative procedure of Example 15, except using 4,6-dimethoxypyrimidin-5-amine instead of 5-bromoisothiazol-4-amine in Step A. MS (ESI): mass calcd. for C₂₀H₂₁F₄N₇O₆, 531.42; m/z found, 532.2 [M+H]⁺. ¹H NMR (400 MHz, DMSO-*d*₆) δ ppm 9.50 (s, 1H), 8.46 (s, 1H), 8.25 (d, *J* = 8.9 Hz, 1H), 5.79 (dt, *J* = 25.2, 6.2 Hz, 2H), 4.49 (d, *J* = 5.8 Hz, 2H), 3.94 (s, 6H), 3.79 (q, *J* = 7.1 Hz, 2H), 1.54 (d, *J* = 6.5 Hz, 3H), 1.28 (t, *J* = 7.1 Hz, 3H).

### Example 95: 6-[4-Ethyl-3-(hydroxymethyl)-5-oxo-1,2,4-triazol-1-yl]-5-fluoro-2-[(1S)-2,2,2-trifluoro-1-methyl-ethoxy]-N-[3-(trifluoromethyl)-1H-pyrazol-4-yl]pyridine-3-carboxamide.

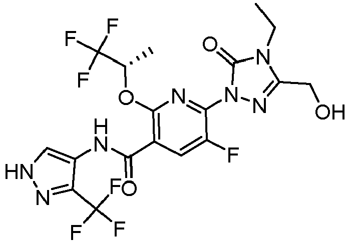

The title compound was prepared according to the representative procedure of Example 15, except using 3-(trifluoromethyl)-1H-pyrazol-4-amine instead of 5-bromoisothiazol-4-amine in Step A. MS (ESI): mass calcd. for C₁₈H₁₆F₇N₇O₄, 527.36; m/z found, 528.2 [M+H]⁺. ¹H NMR (400 MHz, DMSO-*d*₆) δ ppm 9.82 (s, 1H), 8.50 - 8.29 (m, 2H), 5.99 - 5.74 (m, *J* = 9.0, 7.8 Hz, 2H), 4.49 (s, 2H), 3.79 (q, *J* = 7.1 Hz, 2H), 1.54 (d, *J* = 6.6 Hz, 3H), 1.28 (t, *J* = 7.1 Hz, 3H).

### Example 96: N-(5-Chloro-3-methyl-1H-pyrazol-4-yl)-6-[4-ethyl-3-(fluoromethyl)-5-oxo-1,2,4-triazol-1-yl]-5-fluoro-2-[(1S)-2,2,2-trifluoro-1-methyl-ethoxy]pyridine-3-carboxamide.

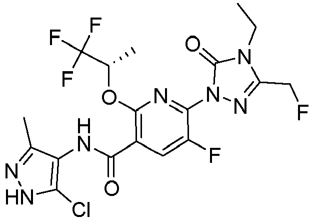

The title compound was prepared according to the representative procedure of Example 87, except using 5-chloro-3-methyl-1H-pyrazol-4-amine instead of 2-chloro-4-methylpyridin-3-amine in Step C. MS (ESI): mass calcd. for C₁₈H₁₇ClF₅N₇O₃, 509.1; m/z found, 510.1 [M+H]⁺. ¹H NMR (400 MHz, CDCl₃) δ = 10.05 (br s, 1H), 8.82 (s, 1H), 8.55 (d, *J* = 9.29 Hz, 1H), 5.91 (quin, *J* = 6.48 Hz, 1H), 5.27-5.49 (m, 2H), 3.91 (q, *J* = 7.34 Hz, 2H), 2.33 (s, 3H), 1.63-1.74 (m, 3H), 1.44 (t, *J* = 7.34 Hz, 3H).

### Example 97: N-(2-Chloro-4-methyl-3-pyridyl)-6-[4-ethyl-3-(fluoromethyl)-5-oxo-1,2,4-triazol-1-yl]-5-fluoro-2-isopropoxy-pyridine-3-carboxamide.

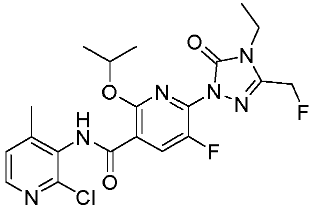

The title compound was prepared according to the representative procedure of Example 87, Steps A-C, except using isopropyl 6-(4-ethyl-3-(hydroxymethyl)-5-oxo-4,5-dihydro-1H-1,2,4-triazol-1-yl)-5-fluoro-2-isopropoxynicotinate (Intermediate 21) instead of isopropyl 6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinate in step C. MS (ESI): mass calcd. for C₂₀H₂₁ClF₂N₆O₃, 466.1; m/z found, 467 [M+H]⁺. ¹H NMR (CDCl₃, 400 MHz) δ 9.84 (s, 1H), 8.49 (d, 1H, *J* = 9.4 Hz), 8.21 (d, 1H, *J* = 4.9 Hz), 7.20 (d, 1H, *J* = 5.1 Hz), 5.63 (td, 1H, *J* = 6.1, 12.2 Hz), 5.31 (s, 1H), 5.43 (d, 1H, *J* = 46.5 Hz), 3.91 (q, 2H, *J* = 7.0 Hz), 2.35 (s, 3H), 1.52 (d, 6H, *J* = 6.4 Hz), 1.44 (t, 3H, *J* = 7.3 Hz).

### Example 98: N-(5-Chloro-2-methoxy-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

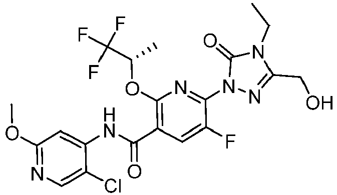

The title compound was prepared according to the representative procedure of Example 15, except using 5-chloro-2-methoxypyridin-4-amine instead of 5-bromoisothiazol-4-amine in Step A. MS (ESI): mass calcd. for C₂₀H₁₉ClF₄N₆O₅, 534.1; m/z found, 535.0 [M+H]⁺. ¹H NMR (400 MHz, CDCl₃) δ = 9.86 (s, 1H), 8.53 (d, *J* = 9.78 Hz, 1H), 8.14 (s, 1H), 8.04 (s, 1H), 6.19 (s, 1H), 4.70 (d, *J* = 6.36 Hz, 2H), 3.94 (s, 3H), 3.92 (d, *J* = 7.34 Hz, 2H), 1.68 (d, *J* = 6.85 Hz, 3H), 1.43 (t, *J* = 7.09 Hz, 3H).

### Example 99: N-(5-Chloro-3-methyl-1H-pyrazol-4-yl)-5-fluoro-6-[3-(fluoromethyl)-5-oxo-4-propyl-1,2,4-triazol-1-yl]-2-[(1S)-2,2,2-trifluoro-1-methyl-ethoxy]pyridine-3-carboxamide.

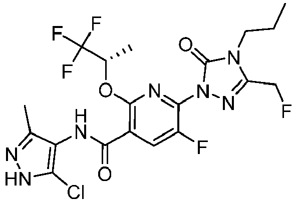

The title compound was prepared according to the representative procedure of Example 1, Step E, except using 5-chloro-3-methyl-4-amino-1H-pyrazole instead of 2-chloro-6-fluoroaniline and isopropyl (S)-5-fluoro-6-(3-(fluoromethyl)-5-oxo-4-propyl-4,5-dihydro-1H-1,2,4-triazol-1-yl)-2-((1,1,1-trifluoropropan-2-yl)oxy)nicotinate (Example 92, product from Step A) for isopropyl (S)-6-(3-((benzyloxy)methyl)-4-ethyl-5-oxo-4,5-dihydro-1H-1,2,4-triazol-1-yl)-5-fluoro-2-((1,1,1-trifluoropropan-2-yl)oxy)nicotinate (24 mg, yield 40.6%). MS (ESI): mass calcd. for C₁₉H₁₉ClF₅N₇O₃, 523.1; m/z found, 524.1 [M+H]⁺. ¹H NMR (400 MHz, CDCl₃) δ = 9.57 - 9.73 (m, 1H),8.83 (s, 1H), 8.54 (d, *J* = 9.29 Hz, 1H), 5.81 - 6.00 (m, 1H), 5.42 (s, 1H), 5.31 (s, 1H), 3.70 - 3.90 (m, 2H), 2.34 (s, 3H), 1.76 - 1.96 (m, 2H), 1.68 (d, *J* = 6.85 Hz, 3H), 1.04 (t, *J* = 7.34 Hz, 3 H).

### Example 100: N-(3-Cyano-1H-pyrazol-4-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

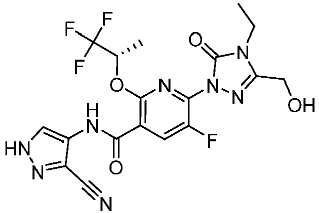

The title compound was prepared according to the representative procedure of Example 15, except using 4-amino-1H-pyrazole-3-carbonitrile instead of 5-bromoisothiazol-4-amine in Step A. MS (ESI): mass calcd. for C₁₈H₁₆F₄N₈O₄, 484.12; m/z found, 485.1 [M+H]⁺. ¹H NMR (400 MHz, DMSO-*d*₆) δ ppm 10.55 (s, 1H), 8.40 - 8.26 (m, 2H), 5.79 (m, 1H), 4.49 (s, 2H), 3.79 (q, *J* = 7.1 Hz, 2H), 1.55 (d, *J* = 6.5 Hz, 3H), 1.28 (t, *J*= 7.1 Hz, 3H).

### Example 101: N-(2-Chloro-4-methyl-3-pyridyl)-5-fluoro-6-[3-(fluoromethyl)-4-(3-hydroxypropyl)-5-oxo-1,2,4-triazol-1-yl]-2-[(1S)-2,2,2-trifluoro-1-methyl-ethoxy]pyridine-3-carboxamide.

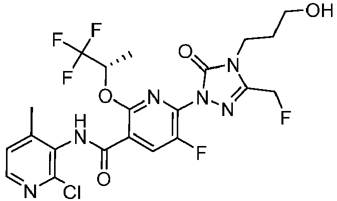

Step A: Isopropyl (S)-6-(4-allyl-3-(fluoromethyl)-5-oxo-4,5-dihydro-1H-1,2,4-triazol-1-yl)-5-fluoro-2-((1,1,1-trifluoropropan-2-yl)oxy)nicotinate. The title compound was prepared according to the representative procedure of Example 87, Steps A - B, except using isopropyl (S)-6-(4-allyl-3-((benzyloxy)methyl)-5-oxo-4,5-dihydro-1H-1,2,4-triazol-1-yl)-5-fluoro-2-((1,1,1-trifluoropropan-2-yl)oxy)nicotinate (Example 84 product from Step B) in Step A. MS (ESI): mass calcd. for C₁₈H₁₉F₅N₄O₄, 450.13; m/z found, 451.2 [M+H]⁺. ¹H NMR (400 MHz, CDCl₃) δ = 8.15 (dd, *J* = 7.34, 8.80 Hz, 1H), 5.85-6.02 (m, 1H), 5.66 (m, 1H), 5.20-5.38 (m, 4H), 4.39-4.54 (m, 2H), 1.52-1.59 (m, 4H), 1.34-1.39 (m, 6H).

Step B: Isopropyl (S)-5-fluoro-6-(3-(fluoromethyl)-4-(3-hydroxypropyl)-5-oxo-4,5-dihydro-1H-1,2,4-triazol-1-yl)-2-((1,1,1-trifluoropropan-2-yl)oxy)nicotinate. To a solution of isopropyl (S)-6-(4-allyl-3-(fluoromethyl)-5-oxo-4,5-dihydro-1H-1,2,4-triazol-1-yl)-5-fluoro-2-((1,1,1-trifluoropropan-2-yl)oxy)nicotinate (67 mg, 0.149 mmol) in THF (1.5 mL) was added 9-BBN (9-borabicyclo[3.3.1]nonane) (0.5 M in THF, 0.446 mL, 0.223 mmol) at 0 °C. Reaction was allowed to warm to room temperature and was stirred for 4 hours. The reaction was quenched with 1N NaOH (2.7 mL, 2.7 mmol) and aq. H₂O₂ (30% solution, 0.5 mL, 4.22 mmol). The layers were separated, and the aqueous phase was extracted with ether. The combined organic layers were dried over MgSO₄, filtered and concentrated under reduced pressure. Residue was purified with flash column chromatography (SiO₂, eluting with a gradient of 0-60% ethyl acetate in heptane) to provide the title compound as a clear oil (70 mg, 0.09 mmol, 57% yield). MS (ESI): mass calcd. for C₁₈H₂₁F₅N₄O₅, 468.14; m/z found, 469.2 [M+H]⁺. ¹H NMR (400 MHz, CDCl₃) δ = 8.11-8.19 (m, 1H), 5.64 (td, *J* = 6.54, 12.84 Hz, 1H), 5.32-5.46 (m, 2H), 5.20-5.28 (m, 1H), 3.99-4.06 (m, 2H), 3.65-3.75 (m, 2H), 2.71-2.81 (m, 1H), 1.96-2.04 (m, 2H), 1.54-1.57 (m, 3H), 1.34-1.38 (m, 6H).

Step C: N-(2-Chloro-4-methyl-3-pyridyl)-5-fluoro-6-[3-(fluoromethyl)-4-(3-hydroxypropyl)-5-oxo-1,2,4-triazol-1-yl]-2-[(1S)-2,2,2-trifluoro-1-methyl-ethoxy]pyridine-3-carboxamide. To a solution of 2-chloro-4-methylpyridin-3-amine (49 mg, 346 µmol) in dichloromethane (DCM) (0.7 mL) was added dropwise trimethylaluminum (AlMe₃) (2 M, 173 µL) at 0 °C, then added solution of Isopropyl (S)-5-fluoro-6-(3-(fluoromethyl)-4-(3-hydroxypropyl)-5-oxo-4,5-dihydro-1H-1,2,4-triazol-1-yl)-2-((1,1,1-trifluoropropan-2-yl)oxy)nicotinate (54 mg, 115 µmol) in DCM (1mL), The resulting mixture was stirred at 50 °C for 12 hr. The reaction mixture was quenched with MeOH (1 mL) and diluted with DCM (30 mL). The combined organic layers were washed with brine (20 mL * 3), dried over Na₂SO₄, filtered and concentrated under reduced pressure. The residue was purified by prep-HPLC (Method F) to afford the title compound as a white solid (25 mg, 40% yield). MS (ESI): mass calcd. for C₂₁H₂₀ClF₅N₆O₄, 550.12; m/z found, 551.1 [M+H]⁺. ¹H NMR (400 MHz, CDCl₃) δ = 9.20 (s, 1H), 8.57 (d, *J* = 9.29 Hz, 1H), 8.23 (d, *J* = 4.89 Hz, 1H), 7.18-7.24 (m, 1H), 5.94 (td, *J* = 6.36, 12.72 Hz, 1H), 5.47 (s, 1H), 5.35 (s, 1H), 4.05 (t, *J* = 6.36 Hz, 2H), 3.71 (q, *J* = 5.38 Hz, 2H), 2.70 (br t, *J* = 6.11 Hz, 1H), 2.34 (s, 3H), 2.02 (quin, *J* = 5.87 Hz, 2H), 1.69 (d, *J* = 6.36 Hz, 3H).

### Example 102: N-(2-Chloro-4,5-dimethyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

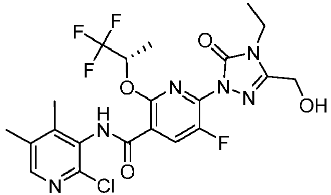

The title compound was prepared according to the representative procedure of Example 15, except using 2-chloro-4,5-dimethylpyridin-3-amine instead of 5-bromoisothiazol-4-amine in Step A. MS (ESI): mass calcd. for C₂₁H₂₁ClF₄N₆O₄, 532.12; m/z found, 533.1 [M+H]⁺. ¹H NMR (400 MHz, CDCl₃) δ ppm 9.19 (s, 1H), 8.53 (d, *J* = 9.4 Hz, 1H), 8.12 (s, 1H), 5.97 (m, 1H), 4.69 (d, *J* = 5.8 Hz, 2H), 3.92 (q, *J* = 7.2 Hz, 2H), 2.51 (t, *J* = 6.3 Hz, 1H), 2.31 (s, 3H), 2.23 (s, 3H), 1.68 (d, *J* = 6.5 Hz, 3H), 1.43 (t, *J* = 7.2 Hz, 3H).

### Example 103: 6-[4-ethyl-3-(hydroxymethyl)-5-oxo-1,2,4-triazol-1-yl]-5-fluoro-N-(4-methoxy-2,6-dimethyl-3-pyridyl)-2-[(1S)-2,2,2-trifluoro-1-methyl-ethoxy]pyridine-3-carboxamide.

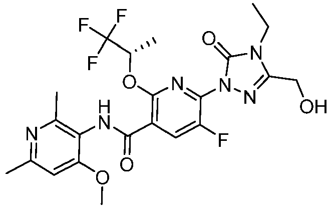

The title compound was prepared according to the representative procedure of Example 15, except using 4-methoxy-2,6-dimethylpyridin-3-amine instead of 5-bromoisothiazol-4-amine in Step A. MS (ESI): mass calcd. for C₂₂H₂₄F₄N₆O₅, 528.17; m/z found, 529.2 [M+H]⁺. ¹H NMR (400 MHz, CDCl₃) δ ppm 8.90 (s, 1H), 8.51 (d, *J* = 9.6 Hz, 1H), 6.63 (s, 1H), 5.90 (m, 1H), 4.67 (s, 2H), 3.93 (q, *J* = 7.2 Hz, 2H), 3.86 (s, 3H), 2.52 (s, 3H), 2.43 (s, 3H), 1.66 (d, *J* = 6.5 Hz, 3H), 1.43 (t, *J* = 7.2 Hz, 3H).

### Example 104: N-(3-Chloro-4-methoxy-2-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

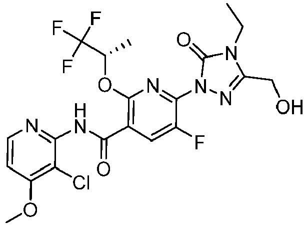

The title compound was prepared according to the representative procedure of Example 15, except using 3-chloro-4-methoxypyridin-2-amine (Intermediate 12) instead of 3-amino-2-chloropyridine in Step A. MS (ESI): mass calcd. for C₂₀H₁₉ClF₄N₆O₅, 534.1; m/z found, 535.2 [M+H]⁺. ¹H NMR (400 MHz, CDCl₃) δ = 9.87 (s, 1H), 8.59 (d, *J* = 9.78 Hz, 1H), 8.38 (d, *J* = 5.38 Hz, 1H), 6.78 (d, *J* = 5.87 Hz, 1H), 6.68 - 6.85 (m, 1H), 5.94 - 6.20 (m, 1H), 4.70 (s, 3H), 4.00 (s, 3H), 3.92 (d, *J* = 7.34 Hz, 2H), 1.66 (d, *J* = 6.36 Hz, 4H), 1.43 (t, *J* = 7.34 Hz, 4 H).

### Example 105: N-(3-Chloro-4-ethoxy-2-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

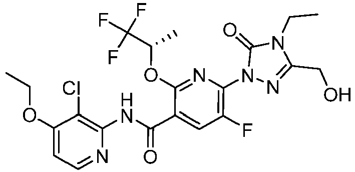

The title compound was prepared according to the representative procedure of Example 15, except using 3-chloro-4-ethoxypyridin-2-amine instead of 3-amino-2-chloropyridine in Step A. MS (ESI): mass calcd. for C₂₁H₂₁ClF₄N₆O₅, 548.1; m/z found, 549.2 [M+H]⁺. ¹H NMR (400 MHz, CDCl₃) δ = 9.88 (s, 1H), 8.59 (d, *J* = 9.78 Hz, 1H), 8.34 (d, *J* = 5.38 Hz, 1H), 6.74 (d, *J* = 5.87 Hz, 1H), 5.98 - 6.15 (m, 1H), 4.70 (d, *J* = 6.36 Hz, 2H), 4.21 (q, *J=* 7.01 Hz, 2H), 3.92 (q, *J* = 7.17 Hz, 2H), 1.66 (d, *J* = 6.36 Hz, 3H), 1.52 (t, *J* = 7.09 Hz, 4H), 1.43 (t, *J* = 7.34 Hz, 3 H).

### Example 106: N-(2-Chloro-6-methoxy-4-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

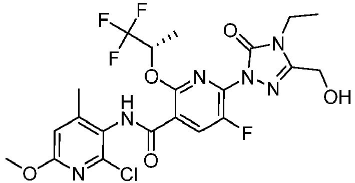

The title compound was prepared according to the representative procedure of Example 15, except using 2-chloro-6-methoxy-4-methylpyridin-3-amine (Intermediate 14) instead of 5-bromoisothiazol-4-amine in Step A. MS (ESI): mass calcd. for C₂₁H₂₁ClF₄N₆O₅, 548.12; m/z found, 549 [M+H]⁺. ¹H NMR (400 MHz, CDCl₃) δ ppm 9.23 (s, 1H), 8.54 (d, *J* = 9.5 Hz, 1H), 8.08 (s, 1H), 5.97 (hept, *J* = 6.5 Hz, 1H), 4.69 (d, *J* = 4.6 Hz, 2H), 3.97 (s, 3H), 3.92 (q, *J* = 7.2 Hz, 2H), 2.33 (d, *J* = 6.6 Hz, 1H), 2.12 (s, 3H), 1.68 (d, *J* = 6.5 Hz, 3H), 1.43 (t, *J* = 7.2 Hz, 3H).

### Example 107: N-(3-Chloro-5-methyl-1H-pyrazol-4-yl)-5-fluoro-6-[3-(fluoromethyl)-4-(3-hydroxypropyl)-5-oxo-1,2,4-triazol-1-yl]-2-[(1S)-2,2,2-trifluoro-1-methyl-ethoxy]pyridine-3-carboxamide.

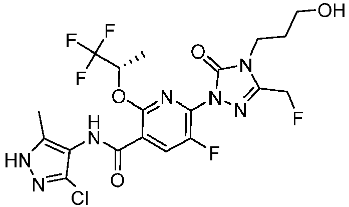

The title compound was prepared according to the representative procedure of Example 101, Steps A-C, except using 5-chloro-3-methyl-1H-pyrazol-4-amine instead of 2-chloro-4-methylpyridin-3-amine for in Step C. MS (ESI): mass calcd. for C₁₉H₁₉ClF₅N₇O₄, 539.11; m/z found, 540.2 [M+H]⁺. ¹H NMR (400 MHz, CDCl₃) δ = 10.01 (br s, 1H), 8.82 (s, 1H), 8.56 (d, *J* = 9.29 Hz, 1H), 5.89 (td, *J* = 6.54, 12.84 Hz, 1H), 5.47 (s, 1H), 5.35 (s, 1H), 4.04 (t, *J* = 6.36 Hz, 2H), 3.71 (br d, *J* = 4.89 Hz, 2H), 2.77 (br s, 1H), 2.33 (s, 3H), 1.95-2.06 (m, 2H), 1.64-1.73 (m, 4H).

### Example 108: N-(5-Chloro-3-methyl-1H-pyrazol-4-yl)-6-[4-ethyl-3-(fluoromethyl)-5-oxo-1,2,4-triazol-1-yl]-5-fluoro-2-isopropoxy-pyridine-3-carboxamide.

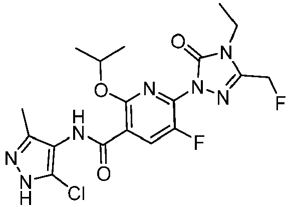

The title compound was prepared according to the representative procedure of Example 96, Step A-B, except using isopropyl 6-(4-ethyl-3-(hydroxymethyl)-5-oxo-4,5-dihydro-1H-1,2,4-triazol-1-yl)-5-fluoro-2-isopropoxynicotinate (Intermediate 21) instead of isopropyl 6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinate. MS (ESI): mass calcd. for C₁₈H₂₀ClF₂N₇O₃, 455.1; m/z found, 456 [M+H]⁺. ¹H NMR (CDCl₃, 400 MHz) δ 9.44 (s, 1H), 8.48 (d, 1H, *J* = 9.8 Hz), 5.58 (td, 1H, *J* = 6.1, 12.2 Hz), 5.43 (s, 1H), 5.31 (s, 1H), 3.91 (q, 2H, *J* = 7.3 Hz), 2.37 (s, 3H), 1.67 (br d, 1H, *J* = 6.8 Hz), 1.52 (d, 6H, *J* = 6.4 Hz), 1.43 (t, 3H, *J* = 7.1 Hz).

### Example 109: 6-[4-Ethyl-3-(hydroxymethyl)-5-oxo-1,2,4-triazol-1-yl]-5-fluoro-N-(3-methoxypyridazin-4-yl)-2-[(1S)-2,2,2-trifluoro-1-methyl-ethoxy]pyridine-3-carboxamide.

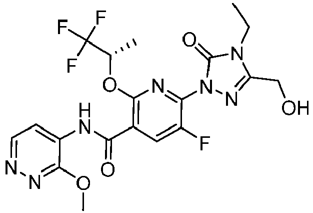

The title compound was prepared according to the representative procedure of Example 15, except using 3-methoxypyridazin-4-amine instead of 5-bromoisothiazol-4-amine in Step A. MS (ESI): mass calcd. for C₁₉H₁₉F₄N₇O₅, 501.14; m/z found, 502.2 [M+H]⁺. ¹H NMR (400 MHz, CDCl₃) δ ppm 10.05 (s, 1H), 8.78 (d, *J* = 5.3 Hz, 1H), 8.60 - 8.40 (m, 2H), 6.08 (hept, *J* = 6.5 Hz, 1H), 4.70 (s, 2H), 4.26 (s, 3H), 3.93 (q, *J* = 7.2 Hz, 2H), 1.70 (d, *J* = 6.5 Hz, 3H), 1.43 (t, *J* = 7.2 Hz, 3H).

### Example 110: N-(5-Chloro-2-methoxy-3-methyl-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

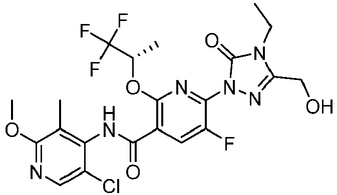

The title compound was prepared according to the representative procedure of Example 15, except using 5-chloro-2-methoxy-3-methylpyridin-4-amine (Intermediate 15) instead of 5-bromoisothiazol-4-amine in Step A. MS (ESI): mass calcd. for C₂₁H₂₁ClF₄N₆O₅, 548.12; m/z found, 549.2 [M+H]⁺. ¹H NMR (400 MHz, CDCl₃) δ ppm 8.95 (s, 1H), 8.61 - 8.48 (m, 1H), 6.63 (s, 1H), 5.93 (m, 1H), 4.70 (d, *J* = 6.2 Hz, 2H), 4.02 - 3.83 (m, 5H), 2.27 (s, 3H), 2.15 (t, *J* = 6.4 Hz, 1H), 1.68 (d, *J* = 6.5 Hz, 3H), 1.51 - 1.38 (m, 3H).

### Example 111: 6-[4-Ethyl-3-(hydroxymethyl)-5-oxo-1,2,4-triazol-1-yl]-5-fluoro-N-[3-methyl-5-(trifluoromethyl)-1H-pyrazol-4-yl]-2-[(1S)-222-trifluoro-1-methyl-ethoxy]pyridine-3-carboxamide.

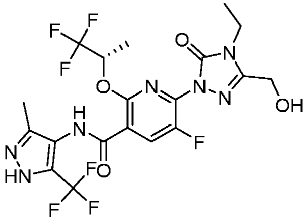

The title compound was prepared according to the representative procedure of Example 15, except using 3-methyl-5-(trifluoromethyl)-1H-pyrazol-4-amine instead of 5-bromoisothiazol-4-amine in Step A. MS (ESI): mass calcd. for C₁₉H₁₈F₇N₇O₄, 541.13; m/z found, 542.1 [M+H]⁺. ¹H NMR (400 MHz, CDCl₃) δ ppm 10.36 (s, 1H), 8.95 (s, 1H), 8.53 (d, *J* = 9.6 Hz, 1H), 5.95 (hept, *J* = 6.5 Hz, 1H), 4.74 - 4.64 (m, 2H), 3.92 (q, *J* = 7.2 Hz, 2H), 2.31 (s, 3H), 1.65 (d, *J* = 6.5 Hz, 3H), 1.43 (t, *J* = 7.2 Hz, 3H).

### Example 112: N-[3-Chloro-5-(trifluoromethyl)-1H-pyrazol-4-yl]-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

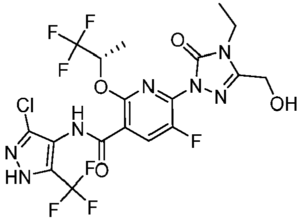

The title compound was prepared according to the representative procedure of Example 15, except using 3-chloro-5-(trifluoromethyl)-1H-pyrazol-4-amine instead of 5-bromoisothiazol-4-amine in Step A. MS (ESI): mass calcd. for C₁₈H₁₅ClF₇N₇O₄, 561.08; m/z found, 562 [M+H]⁺. ¹H NMR (400 MHz, CDCl₃) δ ppm 8.89 (s, 1H), 8.53 (d, *J* = 9.6 Hz, 1H), 5.92 (m, 1H), 4.70 (s, 2H), 3.92 (q, *J* = 7.2 Hz, 2H), 1.66 (d, *J* = 6.5 Hz, 3H), 1.43 (t, *J* = 7.2 Hz, 3H).

### Example 113: N-(6-Chloro-2-methoxy-4-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

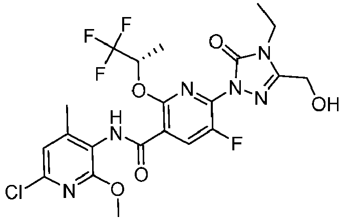

The title compound was prepared according to the representative procedure of Example 15, except using 6-chloro-2-methoxy-4-methylpyridin-3-amine (Intermediate 16) instead of 5-bromoisothiazol-4-amine in Step A. MS (ESI): mass calcd. for C₂₁H₂₁ClF₄N₆O₅, 548.12; m/z found, 549.2 [M+H]⁺. ¹H NMR (400 MHz, CDCl₃) δ ppm 9.04 (s, 1H), 8.50 (d, *J* = 9.6 Hz, 1H), 6.88 (s, 1H), 5.92 (m, 1H), 4.65 (d, *J* = 5.4 Hz, 2H), 3.97 (s, 3H), 3.90 (q, *J* = 7.2 Hz, 2H), 3.05 (t, *J* = 6.2 Hz, 1H), 2.25 (s, 3H), 1.67 (d, *J* = 6.5 Hz, 3H), 1.41 (t, *J* = 7.2 Hz, 3H).

### Example 114: 6-[4-Ethyl-3-(hydroxymethyl)-5-oxo-1,2,4-triazol-1-yl]-5-fluoro-N-(2-methoxy-4,6-dimethyl-3-pyridyl)-2-[(1S)-2,2,2-trifluoro-1-methyl-ethoxy]pyridine-3-carboxamide.

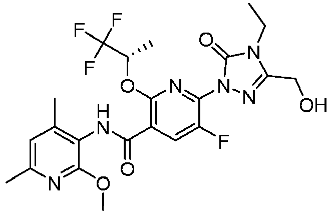

The title compound was prepared according to the representative procedure of Example 15, except using 2-methoxy-4,6-dimethylpyridin-3-amine instead of 5-bromoisothiazol-4-amine in Step A. MS (ESI): mass calcd. for C₂₂H₂₄F₄N₆O₅, 528.17; m/z found, 529.2 [M+H]⁺. ¹H NMR (400 MHz, CDCl₃) δ ppm 9.02 (s, 1H), 8.51 (d, *J* = 9.6 Hz, 1H), 6.67 (s, 1H), 5.92 (m, 1H), 4.63 (s, 2H), 3.94 (s, 3H), 3.89 (q, *J* = 7.2 Hz, 2H), 3.12 (s, 1H), 2.42 (s, 3H), 2.22 (s, 3H), 1.66 (d, *J* = 6.5 Hz, 3H), 1.40 (t, *J* = 7.2 Hz, 3H).

### Example 115: N-(5-Chloro-3-methyl-1H-pyrazol-4-yl)-5-fluoro-6-[3-(fluoromethyl)-4-(2-hydroxyethyl)-5-oxo-1,2,4-triazol-1-yl]-2-[(1S)-2,2,2-trifluoro-1-methyl-ethoxy]pyridine-3-carboxamide.

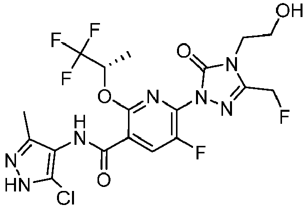

Step A: Isoprogvl (S)-5-fluoro-6-(3-(fluoromethyl)-5-oxo-4-(2-oxoethyl)-4,5-dihydro-1H-1,2,4-triazol-1-yl)-2-((1,1,1-trifluoropropan-2-yl)oxy)nicotinate. A mixture of isopropyl (S)-6-(4-allyl-3-(fluoromethyl)-5-oxo-4,5-dihydro-1H-1,2,4-triazol-1-yl)-5-fluoro-2-((1,1,1-trifluoropropan-2-yl)oxy)nicotinate (Example 97, Step A, 200 mg, 0.44 mmol), sodium periodate (190 mg, 0.88 mmol), potassium osmate(VI) dihydrate (16.4 mg, 0.04 mmol) in a 2:1 mixture of dioxane/water (2.4 mL) was stirred at room temperature for 16 hours. The reaction mixture was diluted with ethyl acetate and H₂O and organic layer separated. The aqueous phase was extracted (3x) with ethyl acetate and combined organics were washed with brine, dried over Na₂SO₄, filtered, and concentrated under reduced pressure. The crude reaction was used in the next step without further purification.

Step B: Isopropyl (S)-5-fluoro-6-(3-(fluoromethyl)-4-(2-hydroxyethyl)-5-oxo-4,5-dihydro-1H-1,2,4-triazol-1-yl)-2-((1,1,1-trifluoropropan-2-yl)oxy)nicotinate. To a solution of isopropyl (S)-5-fluoro-6-(3-(fluoromethyl)-5-oxo-4-(2-oxoethyl)-4,5-dihydro-1H-1,2,4-triazol-1-yl)-2-((1,1,1-trifluoropropan-2-yl)oxy)nicotinate (170 mg, 0.376 mmol) in MeOH (1.5 mL) at 0°C was added NaBH₄ (36 mg, 0.94 mmol). The reaction was allowed to warm to room temperature and stir for 2 hours. The reaction was quenched with NH₄Cl and extracted with ethyl acetate (3x). The combined organic layers were washed with brine, dried over Na₂SO₄, filtered and concentrated under reduced pressure. The residue was purified with flash column chromatography (SiO₂, eluting with a gradient 40 - 100% ethyl acetate in heptane) to provide the title compound as a clear oil (125 mg, 0.275 mmol). MS (ESI): mass calcd. for C₁₇H₁₉F₅N₄O₅, 454.13; m/z found, 455.1 [M+H]⁺. ¹H NMR (400 MHz, CDCl₃) δ = 8.15 (d, *J* = 9.29 Hz, 1H), 5.66 (quin, *J* = 6.48 Hz, 1H), 5.51 (s, 1H), 5.36-5.41 (m, 1H), 5.21-5.28 (m, 1H), 3.93-4.08 (m, 4H), 2.16-2.22 (m, 1H), 1.56 (d, *J* = 6.36 Hz, 3H), 1.36 (dd, *J* = 3.91, 6.36 Hz, 6H).

Step C: N-(5-Chloro-3-methyl-1H-pyrazol-4-yl)-5-fluoro-6-[3-(fluoromethyl)-4-(2-hydroxyethyl)-5-oxo-1,2,4-triazol-1-yl]-2-[(1S)-2,2,2-trifluoro-1-methyl-ethoxy]pyridine-3-carboxamide. The title compound was prepared according to the representative procedure of Example 4, Step A, except using 5-chloro-3-methyl-1H-pyrazol-4-amine instead of 3-amino-2-chloropyridine. MS (ESI): mass calcd. for C₁₈H₁₇ClF₅N₇O₄, 525.10; m/z found, 526.2 [M+H]⁺. ¹H NMR (400 MHz, CDCl₃) δ = 9.99 (br s, 1H), 8.82 (s, 1H), 8.55 (d, *J* = 9.78 Hz, 1H), 5.91 (td, *J* = 6.54, 12.84 Hz, 1H), 5.53 (s, 1H), 5.41 (s, 1H), 4.03-4.07 (m, 2H), 4.00 (br s, 2H), 2.32-2.35 (m, 3H), 2.27-2.30 (m, 1H), 1.67-1.71 (m, 3H).

### Example 116: N-(2-Chloro-4-methyl-3-pyridyl)-5-fluoro-6-[3-(fluoromethyl)-4-(2-hydroxyethyl)-5-oxo-1,2,4-triazol-1-yl]-2-[(1S)-2,2,2-trifluoro-1-methyl-ethoxy]pyridine-3-carboxamide.

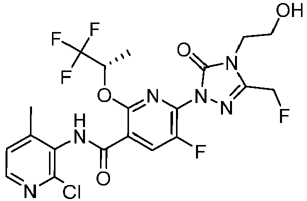

The title compound was prepared according to the representative procedure of Example 115, Steps A-C, except using 2-chloro-4-methylpyridin-3-amine in Step C. MS (ESI): mass calcd. for C₂₀H₁₈ClF₅N₆O₄, 536.10; m/z found, 537.1 [M+H]⁺. ¹H NMR (400 MHz, CDCl₃) δ = 9.20 (s, 1H), 8.56 (d, *J* = 9.29 Hz, 1H), 8.23 (d, *J* = 4.89 Hz, 1H), 7.21 (d, *J* = 4.89 Hz, 1H), 5.96 (td, *J* = 6.36, 12.72 Hz, 1H), 5.54 (s, 1H), 5.42 (s, 1H), 3.94-4.11 (m, 4H), 2.35 (s, 3H), 2.16 (t, *J* = 4.65 Hz, 1H), 1.69 (d, *J* = 6.36 Hz, 3H).

### Example 117: N-(3-Cyano-5-fluoro-2-methoxy-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

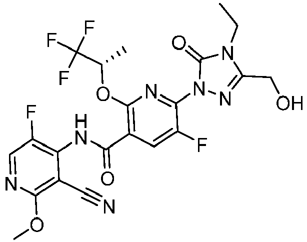

The title compound was prepared according to the representative procedure of Example 29, except using 4-amino-5-fluoro-2-methoxynicotinonitrile (Intermediate 17) instead of 5-bromoisothiazol-4-amine in Step A. MS (ESI): mass calcd. for C₂₁H₁₈F₅N₇O₅, 543.41; m/z found, 544 [M+H]⁺. ¹H NMR (400 MHz, CDCl₃) δ ppm 9.70 (s, 1H), 8.58 (d, *J* = 9.5 Hz, 1H), 8.25 (d, *J* = 1.3 Hz, 1H), 5.97 (m, 1H), 4.69 (d, *J* = 5.4 Hz, 2H), 4.06 (s, 3H), 3.92 (q, *J* = 7.2 Hz, 2H), 2.31 (t, *J* = 6.3 Hz, 1H), 1.70 (d, *J* = 6.5 Hz, 3H), 1.43 (t, *J* = 7.2 Hz, 3H).

### Example 118: N-(3-Chloro-5-methoxy-2-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

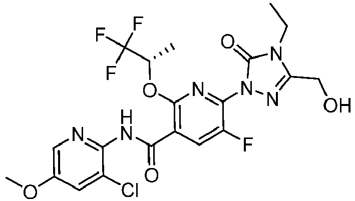

The title compound was prepared according to the representative procedure of Example 15, except using 3-chloro-5-methoxypyridin-2-amine instead of 5-bromoisothiazol-4-amine in Step A. MS (ESI): mass calcd. for C₂₀H₁₉ClF₄N₆O₅, 534.10; m/z found, 535.2 [M+H]⁺. ¹H NMR (400 MHz, CDC13) δ ppm 9.67 (s, 1H), 8.57 (d, *J* = 9.6 Hz, 1H), 8.18 (s, 1H), 7.36 (d, *J* = 2.5 Hz, 1H), 6.00 (m, 1H), 4.69 (s, 2H), 4.04 - 3.81 (m, 5H), 1.66 (d, *J* = 6.6 Hz, 3H), 1.45 - 1.39 (m, 3H).

### Example 119: N-(2,6-Dimethoxyphenyl)-6-[4-ethyl-3-(hydroxymethyl)-5-oxo-1,2,4-triazol-1-yl]-5-fluoro-2-((1S)-2,2,2-trifluoro-1-methyl-ethoxy]pyridine-3-carboxamide.

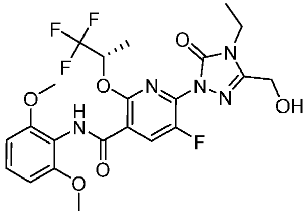

The title compound was prepared according to the representative procedure of Example 15, except using 2,6-dimethoxyaniline instead of 5-bromoisothiazol-4-amine in Step A. MS (ESI): mass calcd. for C₂₂H₂₃F₄N₅O₆, 529.19; m/z found, 530.3 [M+H]⁺. ¹H NMR (400 MHz, CDCl₃) δ ppm 8.95 (s, 1H), 8.54 (d, *J* = 9.7 Hz, 1H), 7.23 (d, *J* = 8.4 Hz, 1H), 6.64 (d, *J* = 8.4 Hz, 2H), 5.89 (h, *J* = 6.5 Hz, 1H), 4.64 (d, *J* = 5.2 Hz, 2H), 3.96 - 3.78 (m, 8H), 2.85 - 2.76 (m, 1H), 1.66 (d, *J* = 4.4 Hz, 3H), 1.40 (t, *J* = 7.2 Hz, 3H).

### Example 120: 6-[4-Ethyl-3-(hydroxymethyl)-5-oxo-1,2,4-triazol-1-yl]-5-fluoro-N-(o-tolyl)-2-[(1S)-2,2,2-trifluoro-1-methyl-ethoxy]pyridine-3-carboxamide.

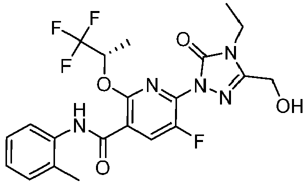

The title compound was prepared according to the representative procedure of Example 15, except using o-toluidine instead of 5-bromoisothiazol-4-amine in Step A. MS (ESI): mass calcd. for C₂₁H₂₁F₄N₅O₄, 483.15; m/z found, 484.2 [M+H]⁺. ¹H NMR (400 MHz, CDCl₃) δ ppm 9.14 (s, 1H), 8.58 (d, J = 9.7 Hz, 1H), 7.93 (d, J = 8.0 Hz, 1H), 7.32 - 7.24 (m, 2H), 7.15 (t, J = 7.4 Hz, 1H), 6.05 (hept, J = 6.6 Hz, 1H), 4.66 (d, J = 6.1 Hz, 2H), 3.90 (q, J = 7.2 Hz, 2H), 2.65 (t, J = 6.3 Hz, 1H), 2.34 (s, 3H), 1.67 (s, 3H), 1.41 (t, J = 7.2 Hz, 3H).

### Example 121: 6-[4-Ethyl-3-(hydroxymethyl)-5-oxo-1,2,4-triazol-1-yl]-5-fluoro-N-(3-methoxy-5-methyl-1H-pyrazol-4-yl)-2-[(1S)-2,2,2-trifluoro-1-methyl-ethoxy]pyridine-3-carboxamide.

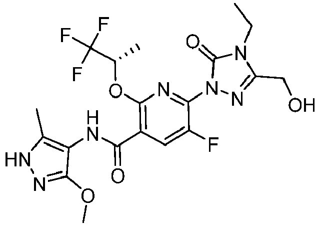

The title compound was prepared according to the representative procedure of Example 15, except using 3-methoxy-5-methyl-1H-pyrazol-4-amine instead of 5-bromoisothiazol-4-amine in Step A. MS (ESI): mass calcd. for C₁₉H₂₁F₄N₇O₅, 503.2; m/z found, 504.1 [M+H]⁺. ¹H NMR (400 MHz, CDCl₃) δ = 8.81 (br s, 1H), 8.69 (s, 1H), 8.52 (d, *J* = 9.78 Hz, 1H), 5.81 - 6.02 (m, 1H), 4.68 (br d, *J* = 3.91 Hz, 2H), 3.94 (s, 3H), 3.91 (d, *J* = 7.34 Hz, 2H), 2.28 (s, 3H), 1.66 (d, *J* = 6.36 Hz, 3H), 1.43 (t, *J* = 7.34 Hz, 3 H).

### Example 122: N-(3,5-Dimethoxy-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

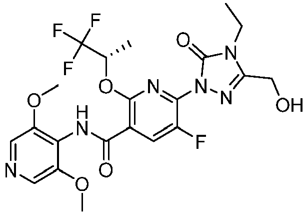

The title compound was prepared according to the representative procedure of Example 15, except using 3,5-dimethoxypyridin-4-amine instead of 5-bromoisothiazol-4-amine in Step A. MS (ESI): mass calcd. for C₂₁H₂₂F₄N₆O₆, 530.15; m/z found, 531.2 [M+H]⁺. ¹H NMR (400 MHz, CDCl₃) δ ppm 9.11 (s, 1H), 8.54 (dd, *J* = 9.6, 1.0 Hz, 1H), 8.10 (s, 2H), 5.90 (m, 1H), 4.69 (s, 2H), 3.98 (d, *J* = 1.1 Hz, 6H), 3.92 (q, *J* = 7.2 Hz, 2H), 1.66 (d, *J* = 6.5 Hz, 3H), 1.46 - 1.39 (m, 3H).

### Example 123: N-(3,5-Dichloro-1H-pyrazol-4-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

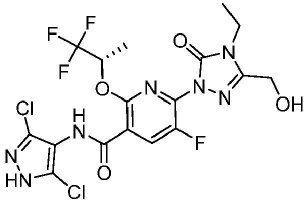

Step A: 3-Chloro-4-nitro-1-(tetrahydro-2H-pyran-2-yl)-1H-pyrazole. To a solution of 3-chloro-4-nitro-1H-pyrazole (150 mg, 1.017 mmol) in EtOAc (1.5 mL) were added 4-methylbenzenesulfonic acid (TsOH) (9.671 mg, 0.0508 mmol) and 3,4-dihydro-2H-pyran [DHP] (139.149 µL, 0.922 g/mL, 1.525 mmol) at room temperature. The reaction was stirred at rt overnight. The reaction mixture was quenched by addition of water + K₂CO₃ solid, then washed with brine. The organic layer was dried over MgSO₄, filtered and concentrated under reduced pressure. The residue was purified by prep-HPLC (Isco-normal phase, from Heptane/Ethyl acetate=100/0 to Heptane/Ethyl acetate= 40/60) to afford the title compound (220 mg, yield 93.4%) as colorless oil. ¹H NMR (400 MHz, CDCl₃) δ = 8.41 (s, 1H), 5.35 (dd, *J* = 8.80, 2.45 Hz, 1H), 4.08 (br d, *J* = 11.74 Hz, 1H), 3.73 (ddd, *J* = 11.74, 7.58, 4.65 Hz, 1H), 2.13 - 2.30 (m, 1H), 1.88 - 2.08 (m, 2H), 1.65 - 1.75 (m, 3H), 1.18 - 1.37 (m, 3H), 0.88 (t, *J* = 6.60 Hz, 2 H).

Step B: 3,5-Dichloro-4-nitro-1-(tetrahydro-2H-pyran-2-yl)-1H-pyrazole. To a solution of 3-chloro-4-nitro-1-(tetrahydro-2H-pyran-2-yl)-1H-pyrazole (145 mg, 0.626 mmol) in THF (1.1 mL) was added LiHMDS (529.672 µL, 1.3 M, 0.689 mmol) at -78 °C under N2.The reaction was stirred at -78 °C for 45 min and then a solution of hexachloroethane (444.5 mg, 1.878 mmol) in THF (1 mL) was added dropwise. The reaction was stirred at rt overnight. The reaction mixture was quenched by addition of an aqueous saturated NH4Cl solution, then washed with brine. The organic layer was dried over MgSO₄, filtered and concentrated under reduced pressure. The residue was purified by prep-HPLC (Isco-normal phase, from Heptane/Ethyl acetate=100/0 to Heptane/Ethyl acetate= 40/60) to afford the title compound (48 mg, 28.8%).

Step C: 3,5-Dichloro-1-(tetrahydro-2H-pyran-2-yl)-1H-pyrazol-4-amine. A solution of 3,5-dichloro-4-nitro-1-(tetrahydro-2H-pyran-2-yl)-1H-pyrazole (40 mg, 0.15 mmol), iron (41.977 mg, 0.752 mmol), NHaCl (40.206 mg, 0.752 mmol) in MeOH (0.4 mL), THF (0.4 mL) and water, distilled (0.4 mL) was stirred at 70°C for 1.5 hours. The reaction mixture was filtered through a pad of Celite and washed with EtOAc. The filtrate was washed with NaHCOs aq. and extracted with EtOAc. The organic layer was dried over MgSO₄, filtered and concentrated under reduced pressure to afford the title compound (35 mg, yield 64.1%) and used without purification. MS (ESI): mass calcd. for C₈H₁₁Cl₂N₃O, 235.0; m/z found, 236.1.

Step D: 6-(3-((Benzyloxy)methyl)-4-ethyl-5-oxo-4,5-dihydro-1H-1,2,4-triazol-1-yl)-N-(3,5-dichloro-1-(tetrahydro-2H-pyran-2-yl)-1H-pyrazol-4-yl)-5-fluoro-2-(((S)-1,1,1-trifluoropropan-2-vl)oxy)nicotinamide. The title compound was prepared according to the representative procedure of Example 15, except using 3,5-dichloro-1-(tetrahydro-2H-pyran-2-yl)-1H-pyrazol-4-amine instead of 5-bromoisothiazol-4-amine in Step A. MS (ESI): mass calcd. for C₂₉H₂₉Cl₂F₄N₇O₅, 701.1; m/z found, 702.0 [M+H]⁺.

Step E: (S)-6-(3-((Benzyloxy)methyl)-4-ethyl-5-oxo-4,5-dihydro-1H-1,2,4-triazol-1-yl)-N-(3,5-dichloro-1H-pyrazol-4-yl)-5-fluoro-2-((1,1,1-trifluoropropan-2-yl)oxy)nicotinamide. A solution of 6-(3-((benzyloxy)methyl)-4-ethyl-5-oxo-4,5-dihydro-1H-1,2,4-triazol-1-yl)-N-(3,5-dichloro-1-(tetrahydro-2H-pyran-2-yl)-1H-pyrazol-4-yl)-5-fluoro-2-(((S)-1,1,1-trifluoropropan-2-yl)oxy)nicotinamide (42 mg, 0.0598 mmol) in HCl (4M in dioxane) (298.936 µL, 4 M, 1.196 mmol) and DCM (500 µL, 7.806 mmol). The reaction mixture was stirred at 70°C for 3 hrs and at rt overnight. The reaction mixture was quenched by addition of water, then washed with brine. The organic layer was dried over MgSO₄, filtered and concentrated under reduced pressure to afford the title compound (55 mg, quantitative yield) and used without purification. MS (ESI): mass calcd. for C₂₄H₂₁Cl₂F₄N₇O₄, 617.1; m/z found, 618.2.

Step F: N-(3,5-Dichloro-1H-pyrazol-4-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide. The title compound was prepared according to the representative procedure of Example 15, except using 3,5-dichloro-1H-pyrazol-4-amine instead of 5-bromoisothiazol-4-amine in Step A. MS (ESI): mass calcd. for C₁₇H₁₅Cl₂F₄N₇O₄, 527.1; m/z found, 528.0 [M+H]⁺. ¹H NMR (400 MHz, CDCl₃) δ = 8.78 (s, 1H), 8.53 (d, *J* = 9.29 Hz, 1H), 5.85 - 5.97 (m, 1H), 4.70 (br s, 2H), 3.92 (d, *J* = 7.34 Hz, 2H), 1.68 (d, *J* = 6.36 Hz, 3H), 1.43 (t, *J* = 7.34 Hz, 4 H).

### Example 124: N-[3-(Difluoromethyl)-5-methyl-1H-pyrazol-4-yl]-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

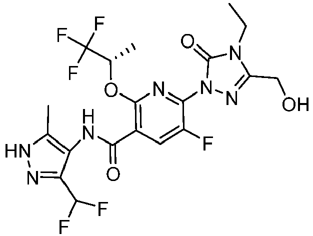

The title compound was prepared according to the representative procedure of Example 15, except using 3-(difluoromethyl)-5-methyl-1H-pyrazol-4-amine instead of 5-bromoisothiazol-4-amine in Step A. MS (ESI): mass calcd. for C₁₉H₁₉F₆N₇O₄, 523.14; m/z found, 524.1 [M+H]⁺. ¹H NMR (400 MHz, CDCl₃) δ ppm 9.02 (s, 1H), 8.50 (dd, *J* = 9.5, 1.1 Hz, 1H), 6.78 (t, *J* = 54.6 Hz, 1H), 5.94 (hept, *J* = 6.5 Hz, 1H), 4.67 (d, *J* = 1.1 Hz, 2H), 3.91 (q, *J* = 7.2 Hz, 2H), 2.27 (d, *J* = 1.1 Hz, 3H), 1.64 (d, *J* = 6.5 Hz, 3H), 1.54 - 1.38 (m, 3H).

### Example 125: 6-[4-Ethyl-3-(hydroxymethyl)-5-oxo-1,2,4-triazol-1-yl]-5-fluoro-N-(4-fluoro-2-methoxy-3-pyridyl)-2-[(1S)-2,2,2-trifluoro-1-methyl-ethoxy]pyridine-3-carboxamide.

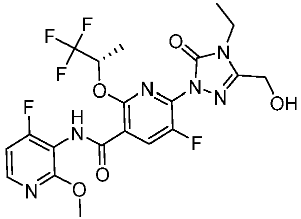

The title compound was prepared according to the representative procedure of Example 4, Steps A and B, except using 4-fluoro-2-methoxypyridin-3-amine (Intermediate 18) in Step A. MS (ESI): mass calcd. for C₂₀H₁₉F₅N₆O₅, 518.13; m/z found, 519.1 [M+H]⁺. ¹H NMR (400 MHz, CDCl₃) δ = ppm 9.06 (s, 1H), 8.51-8.57 (m, 1H), 8.00-8.08 (m, 1H), 6.79-6.86 (m, 1H), 5.88-5.97 (m, 1H), 4.66-4.71 (m, 2H), 3.99-4.04 (m, 3H), 3.88-3.96 (m, 2H), 2.33-2.40 (m, 1H), 1.67-1.71 (m, 3H), 1.40-1.47 (m, 3H).

### Example 126: N-(4-Chloro-2-methoxy-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide.

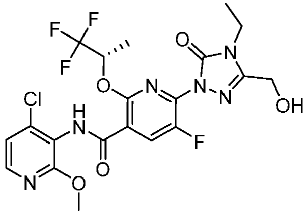

The title compound was prepared according to the representative procedure of Example 15, except using 4-chloro-2-methoxypyridin-3-amine (Intermediate 19) in Step A. MS (ESI): mass calcd. for C₂₀H₁₉ClF₄N₆O₅, 534.10; m/z found, 535.1 [M+H]⁺. ¹H NMR (400 MHz, CDCl₃) δ = 9.06 (s, 1H), 8.54 (d, *J* = 9.78 Hz, 1H), 8.01 (d, *J* = 5.87 Hz, 1H), 7.04 (d, *J* = 5.38 Hz, 1H), 5.92 (td, *J* = 6.24, 12.96 Hz, 1H), 4.69 (d, *J* = 6.36 Hz, 2H), 4.00 (s, 3H), 3.86-3.97 (m, 2H), 2.25 (t, *J* = 6.60 Hz, 1H), 1.66-1.70 (m, 3H), 1.40-1.46 (m, 3H).

### Example 127: (Racemic)-6-(4-Ethyl-3-(1-hydroxyethyl)-5-oxo-1,2,4-triazol-1-yl]-5-fluoro-N-[3-methyl-5-(trifluoromethyl)-1H-pyrazol-4-yl]-2-(2,2,2-trifluoro-1-methyl-ethoxy)pyridine-3-carboxamide.

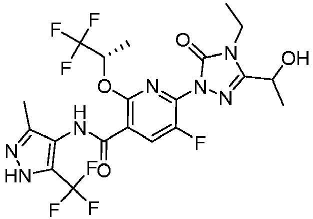

Step A: (S)-4-Ethyl-1-(3-fluoro-5-((3-methyl-5-(trifluoromethyl)-1H-pyrazol-4-yl)carbamoyl)-6-((1,1,1-trifluoropropan-2-yl)oxy)pyridin-2-yl)-5-oxo-4,5-dihydro-1H-1,2,4-triazole-3-carboxylic acid. To a solution of (S)-6-(4-ethyl-3-(hydroxymethyl)-5-oxo-4,5-dihydro-1H-1,2,4-triazol-1-yl)-5-fluoro-N-(3-methyl-5-(trifluoromethyl)-1H-pyrazol-4-yl)-2-((1,1,1-trifluoropropan-2-yl)oxy)nicotinamide (Example 111, 157 mg, 0.29 mmol) in acetone (2.0 mL) was added potassium permanganate (229.1 mg, 1.45 mmol) at 0°C. Reaction was warmed to room temperature and let stir for 30 mins. Following completion, reaction is cooled to 0°C and quenched with 10% aqueous solution of Na₂S₂O₃. Reaction was filtered through Celite^{®}. H₂O was added to the filtrate and was subsequently extracted (2x) with ethyl acetate. The combined organic layers were washed with brine, dried over sodium sulfate, and concentrated under reduced pressure to provide the tile compound as a white solid (121 mg, 0.22 mmol, 75% yield). MS (ESI): mass calcd. for C₁₉H₁₆F₇N₇O₅, 555.1; m/z found, 556.1 [M+H]⁺. ¹H NMR (400 MHz, METHANOL-d₄) δ = 8.25 (d, *J* = 8.80 Hz, 1H), 6.02 (td, *J* = 6.72, 12.96 Hz, 1H), 4.19 (q, *J* = 6.85 Hz, 2H), 2.28 (s, 3H), 1.60 (d, *J* = 6.36 Hz, 3H), 1.33-1.38 (m, 3H).

Step B: (S)-6-(4-ethyl-3-(methoxy(methyl)carbamoyl)-5-oxo-4,5-dihydro-1H-1,2,4-triazol-1-yl)-5-fluoro-N-(3-methyl-5-(trifluoromethyl)-1H-pyrazol-4-yl)-2-((1,1,1-trifluoropropan-2-yl)oxy)nicotinamide. To a solution of (S)-4-ethyl-1-(3-fluoro-5-((3-methyl-5-(trifluoromethyl)-1H-pyrazol-4-yl)carbamoyl)-6-((1,1,1-trifluoropropan-2-yl)oxy)pyridin-2-yl)-5-oxo-4,5-dihydro-1H-1,2,4-triazole-3-carboxylic acid in DCM (1.5 mL) was added N,O-dimethylhydroxylamine hydrochloride (18.8 mg, 0.19 mmol), DIPEA (0.08 mL, 0.481 mmol), and HATU (73 mg, 0.19 mmol) and the reaction stirred at room temperature for 16 hours. The reaction was diluted in DCM and H₂O and the layers were separated. The aqueous phase was extracted (3x) with DCM and the combined organics were washed with brine, dried over Na₂SO₄, and concentrated under reduced pressure. The residue was purified by flash column chromatography (SiO₂, eluting in 0 - 80% ethyl acetate in heptane) to provide the title compound as a clear oil (78 mg, 0.13 mmol, 81% yield). MS (ESI): mass calcd. for C₂₁H₂₁F₇N₈O₅, 598.15; m/z found, 599.2 [M+H]⁺. ¹H NMR (400 MHz, CDCl₃) δ = 10.78 (br s, 1H), 8.94 (s, 1H), 8.54 (d, *J* = 9.78 Hz, 1H), 5.94 (td, *J* = 6.54, 12.84 Hz, 1H), 3.93-4.06 (m, 2H), 3.88 (s, 3H), 3.30-3.52 (m, 3H), 2.30 (s, 3H), 1.66 (d, *J* = 6.36 Hz, 3H), 1.39 (t, *J* = 7.34 Hz, 3H).

Step C: (S)-6-(3-Acelyl-4-ethyl-5-oxo-4,5-dihydro-1H-1,2,4-triazol-1-yl)-5-fluoro-N-(3-methyl-5-(trifluoromethyl)-1H-pyrazol-4-yl)-2-((1,1,1-trifluoropropan-2-yl)oxy)nicotinamide. To a solution of (S)-6-(4-ethyl-3-(methoxy(methyl)carbamoyl)-5-oxo-4,5-dihydro-1H-1,2,4-triazol-1-yl)-5-fluoro-N-(3-methyl-5-(trifluoromethyl)-1H-pyrazol-4-yl)-2-((1,1,1-trifluoropropan-2-yl)oxy)nicotinamide (55 mg, 0.09 mmol) in THF (0.5 mL) at -78°C was added methylmagnesium bromide (3M solution, 0.034 mL, 0.101 mmol), dropwise. Reaction stirred at -78°C for one hour, then was warmed to room temperature. The reaction was quenched with NH₄Cl and extracted with ethyl acetate (3x). The combined organics were washed with brine, dried over Na₂SO₄, filtered and concentrated under reduced pressure. Purification with flash column chromatography (SiO₂, eluting with a gradient of 0 - 50 ethyl acetate in heptane) provided the title compound (30 mg, 0.054 mmol, 59% yield). MS (ESI): mass calcd. for C₂₀H₁₈F₇N₇O₄, 553.13; m/z found, 554.1 [M+H]⁺. ¹H NMR (400 MHz, CDCl₃) δ = 10.97 (br s, 1H), 8.95 (s, 1H), 8.58 (d, *J* = 9.29 Hz, 1H), 5.93 (td, *J* = 6.36, 12.72 Hz, 1H), 4.18 (q, *J* = 6.85 Hz, 2H), 2.64 (s, 3H), 2.30 (s, 3H), 1.65-1.69 (m, 3H), 1.37 (t, *J* = 7.09 Hz, 3H).

Step D: (Racemic)-6-[4-Ethyl-3-(1-hydroxyethyl)-5-oxo-1,2,4-triazol-1-yl]-5-fluoro-N-[3-methyl-5-(trifluoromethyl)-1H-pyrazol-4-yl]-2-(2,2,2-trifluoro-1-methyl-ethoxy)pyridine-3-carboxamide. To a solution of (S)-6-(3-acetyl-4-ethyl-5-oxo-4,5-dihydro-1H-1,2,4-triazol-1-yl)-5-fluoro-N-(3-methyl-5-(trifluoromethyl)-1H-pyrazol-4-yl)-2-((1,1,1-trifluoropropan-2-yl)oxy)nicotinamide (28 mg, 0.051 mmol) in MeOH (0.25 mL) at 0°C was added NaBH₄ (3.8 mg, 0.10 mmol). The reaction was warmed to room temperature and stirred at for 5 minutes. The reaction was concentrated, and the residue was taken up in DCM, washed with H₂O and brine, dried over Na₂SO₄, filtered and concentrated under reduced pressure. Purification by reverse phase chromatography (Phenomenex Gemini-NX, C18, 150x30mm, 5 um; 30 mL/min; Buffer A: 20 mM NH4OH/water Buffer B: MeCN; gradient: 20% B for 2 min then linear gradient to 1000 % B over 10 min) provided the title compound as a white solid (20 mg, 0.036 mmol, 71% yield). MS (ESI): mass calcd. for C₂₀H₂₀F₇N₇O₄, 555.15; m/z found, 556.2 [M+H]⁺. ¹H NMR (400 MHz, CDCl₃) δ = 10.79 (br s, 1H), 8.94 (s, 1H), 8.51 (d, *J* = 9.78 Hz, 1H), 5.94 (td, *J* = 6.36, 12.72 Hz, 1H), 4.89 (br t, *J* = 6.36 Hz, 1H), 3.95 (m, 2H), 2.33-2.40 (m, 1H), 2.29 (s, 3H), 1.70 (d, *J* = 6.36 Hz, 3H), 1.64 (s, 3H), 1.42 (t, *J* = 7.09 Hz, 3H).

### Example 128: 6-[4-Ethyl-3-(1-hydroxy-1-methyl-ethyl)-5-oxo-1,2,4-triazol-1-yl]-5-fluoro-N-[3-methyl-5-(trifluoromethyl)-1H-pyrazol-4-yl]-2-[(1S)-2,2,2-trifluoro-1-methyl-ethoxylpyridine-3-carboxamide.

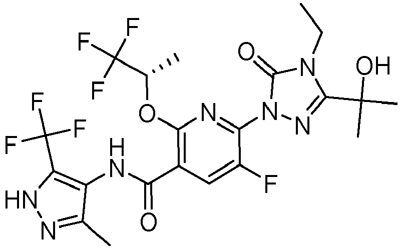

The title compound was prepared according to the representative procedure of Example 101, Step D, substituting 6-(4-ethyl-3-(1-hydroxyethyl)-5-oxo-4,5-dihydro-1H-1,2,4-triazol-1-yl)-5-fluoro-N-(3-methyl-5-(trifluoromethyl)-1H-pyrazol-4-yl)-2-(((S)-1,1,1-trifluoropropan-2-yl)oxy)nicotinamide (Example 127, product from Step C). MS (ESI): mass calcd. for C₂₁H₂₂F₇N₇O₄, 569.16; m/z found, 570.2 [M+H]⁺. ¹H NMR (400 MHz, CDCl₃) δ = 10.37 (br s, 1H), 8.95 (s, 1H), 8.50 (d, *J* = 9.29 Hz, 1H), 5.94 (m, 1H), 4.09 (q, *J* = 7.34 Hz, 2H), 2.31 (s, 3H), 2.10 (s, 1H), 1.69-1.74 (m, 6H), 1.65-1.67 (m, 3H), 1.40-1.45 (m, 3H).

Examples 130 and 131 may be prepared according to the procedures as described below.

### Example 130: 5-Fluoro-6-(3-(1-hydroxyethyl)-4-methyl-5-oxo-4,5-dihydro-1H-1,2,4-triazol-1-yl)-N-(5-methyl-3-(trifluoromethyl)-1H-pyrazol-4-yl)-2-(((S)-1,1,1-trifluoropropan-2-yl)oxy)nicotinamide

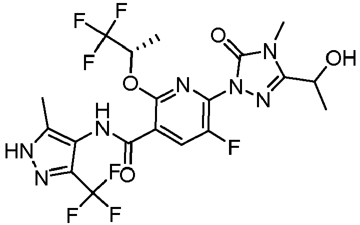

The title compound may be prepared in a manner analogous to N-(4-chloro-2-methoxy-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide (Example 127), using 5-((benzyloxy)methyl)-4-methyl-2,4-dihydro-3H-1,2,4-triazol-3-one instead of 5-((benzyloxy)methyl)-4-ethyl-2,4-dihydro-3H-1,2,4-triazol-3-one.

### Example 131: (S)-5-Fluoro-6-(3-(2-hydroxypropan-2-yl)-4-methyl-5-oxo-4,5-dihydro-1H-1,2,4-triazol-1-yl)-N-(5-methyl-3-(trifluoromethyl)-1H-pyrazol-4-yl)-2-((1,1,1-trifluoropropan-2-yl)oxy)nicotinamide.

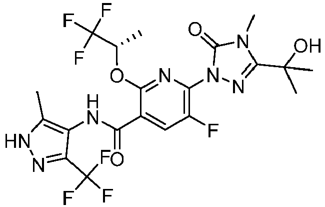

The title compound may be prepared in a manner analogous to 6-[4-ethyl-3-(1-hydroxy-1-methyl-ethyl)-5-oxo-1,2,4-triazol-1-yl]-5-fluoro-N-[3-methyl-5-(trifluoromethyl)-1H-pyrazol-4-yl]-2-[(1S)-2,2,2-trifluoro-1-methyl-ethoxy]pyridine-3-carboxamide (Example 5 128), using 5-((benzyloxy)methyl)-4-methyl-2,4-dihydro-3H-1,2,4-triazol-3-one instead of 5-((benzyloxy)methyl)-4-ethyl-2,4-dihydro-3H-1,2,4-triazol-3-one.

### Biological Data

DHODH inhibitory activities of the compounds of Examples 1-128 were assessed using the following assays. The half maximal effective concentration values (EC50) are summarized in Table 2.

### BIOLOGICAL ASSAYS

### In vitro Assay: DHODH enzymatic assay

To detect DHODH enzyme activities, dichloroindophenol (DCIP) is added as the final electron acceptor in the assay. DCIP can accept electrons from the reduced coenzyme Q generated in the assay, or from dihydroorotate (DHO) via FMN by binding presumably to the ubiquinone pocket. DCIP solutions are blue, with an intense absorbance around 600 nm, but becomes colorless upon reduction (J. Biol. Chem. (1986) 261, 11386). The assay buffer contained 50 nM HEPES, pH 7.5, 150 mM NaCl, 0.5 mM EDTA, and 0.1% Triton X-100 in MilliQ water. Substrate consisting of 20 mM DHO, 5mM CoQ₆, and 1mM DCIP in assay buffer, initiates the reaction. The assay is run in end-point mode by quenching the reaction with the potent DHODH inhibitor brequinar. Absorbance measurements were obtained using the BMG Phera Star plate-reading spectrophotomer. Purified human DHODH was purchased from Proteros (cat. No. PR-0044). Chemicals were purchased from Sigma-Aldrich, Teknova, and Avanti Polar Lipids. Liquid handling was performed using Labcyte Echo and Formulatrix Tempest.

### In vitro Assay: MOLM-13 Cellular Assay

MOLM-13 cells were obtained from DSMZ and were maintained in RPMI 1640 + Glutamax + 25mM HEPES (Invitrogen, catalog number 72400) supplemented with 10% heat inactivated fetal bovine serum (FBS; Invitrogen, catalog number 16140). The day prior to assay set-up, cells were pelleted, resuspended in fresh media, counted, and cells were plated at 0.4 × 10⁶ cell/mL in a T150 flask. On the day of the assay, cells were pelleted, resuspend in fresh media, counted and seeded at 5,000 cells/well in white opaque 96-well tissue culture treated microplates (Perkin Elmer, catalog number 6005680). Cells were exposed to different concentrations of test compounds at 37 °C, 5% CO₂ for 72 hours immediately after seeding. Cell viability was acquired on a Perkin Elmer Envision 2104 multilabel reader using the CellTiter-Glo assay (Promega) according to the manufacturer's instructions.

**Table 2.**

| Example # | DHODH Enzymatic Assay EC50 (nM) | MOLM-13 Cellular Assay EC50 (nM) |
|---|---|---|
| 1 | 0.237 | 0.156 |
| 2 | 6.1 | 5.2 |
| 3 | 0.245 | 0.037 |
| 4 | 2.8 | 6.5 |
| 5 | 1.2 | 2.0 |
| 6 | 0.369 | 1.47 |
| 7 | 0.5 | 0.219 |
| 8 | 22.1 | 15.7 |
| 9 | 31.8 | 18.5 |
| 10 | 22.9 | 60.9 |
| 11 | <1.69 | 0.5 |
| 12 | 324 | 672 |
| 13 | 22 | 3.3 |
| 14 | <1.69 | 0.2 |
| 15 | 3.1 | 2.5 |
| 16 | 734 | >1000 |
| 17 | 30.9 | 87.9 |
| 18 | 3.7 | 12.8 |
| 19 | 40.4 | 51.3 |
| 20 | 4.3 | 108 |
| 21 | 1.2 | 1.8 |
| 22 | 1.5 | 445 |
| 23 | 13.6 | 90 |
| 24 | 10.8 | 10.1 |
| 25 | 0.84 | 3.0 |
| 26 | 108 | >100 |
| 27 | 4.7 | 7.7 |
| 28 | 2.3 | 4.7 |
| 29 | 2.5 | 4.4 |
| 30 | 1.2 | 1.3 |
| 31 | 233 | >100 |
| 32 | 13.6 | 25.3 |
| 33 | 52.5 | >100 |
| 34 | 360 | >100 |
| 35 | 1.9 | 6.1 |
| 36 | 58.6 | >100 |
| 37 | 200 | >100 |
| 38 | 2394 | >100 |
| 39 | 14.1 | 16.5 |
| 40 | 0.31 | 0.3 |
| 41 | 17.4 | 52.4 |
| 42 | 2.4 | 4.3 |
| 43 | 815 | >100 |
| 44 | 3.9 | 10.7 |
| 45 | 142 | >100 |
| 46 | 12.7 | 32.5 |
| 47 | 13.5 | 6.9 |
| 48 | 0.761 | 0.553 |
| 49 | 6.4 | 21.1 |
| 50 | 0.852 | 1.83 |
| 51 | 6.4 | 52.4 |
| 52 | 13 | 68.9 |
| 53 | 144 | >100 |
| 54 | 5.2 | 6.9 |
| 55 | 17.3 | >100 |
| 56 | 4.6 | 5.2 |
| 57 | 1.7 | 20.2 |
| 58 | 5.8 | 23.2 |
| 59 | 98.1 | >100 |
| 60 | 254 | >100 |
| 61 | 365 | >100 |
| 62 | 2.7 | 8.5 |
| 63 | 865 | >100 |
| 64 | 101 | >100 |
| 65 | 202 | >100 |
| 66 | 9.9 | 19.9 |
| 67 | 5.7 | 46.5 |
| 68 | 0.87 | 0.886 |
| 69 | 0.611 | 0.673 |
| 70 | 489 | >100 |
| 71 | 5.6 | 21.1 |
| 72 | 0.895 | 1.4 |
| 73 | 95.2 | >100 |
| 74 | 1.7 | 13 |
| 75 | 19.2 | >100 |
| 76 | 3.6 | 27.9 |
| 77 | 14.0 | 13.2 |
| 78 | 12.3 | 25.0 |
| 79 | 1.9 | 5.3 |
| 80 | 7.2 | 12.9 |
| 81 | 568 | >100 |
| 82 | 729 | >100 |
| 83 | 0.69 | 0.73 |
| 84 | 5.1 | 4.8 |
| 85 | 0.91 | 1.1 |
| 86 | 3.90 | 1.80 |
| 87 | 0.448 | 1.136 |
| 88 | 1.16 | 2.343 |
| 89 | 10.7 | 4.09 |
| 90 | 23.1 | 14.901 |
| 91 | 3.856 | 2.04 |
| 92 | 0.639 | 0.435 |
| 93 | 13.8 | 4.14 |
| 94 | >33 | 48.0 |
| 95 | 2.09 | 1.23 |
| 96 | 0.382 | 0.320 |
| 97 | 1.15 | 1.06 |
| 98 | 91.4 | 41.6 |
| 99 | 0.975 | 0.380 |
| 100 | >100 | 19.0 |
| 101 | 39.4 | 19.4 |
| 102 | 20.1 | 1.39 |
| 103 | >100 | 17.3 |
| 104 | 6.62 | 7.59 |
| 105 | 3.40 | 4.60 |
| 106 | 2.90 | 0.792 |
| 107 | 18.1 | 1.50 |
| 108 | 0.851 | 0.170 |
| 109 | >100 | >100 |
| 110 | 0.367 | 0.282 |
| 111 | 1.93 | 0.345 |
| 112 | 4.12 | 0.303 |
| 113 | 1.07 | 0.416 |
| 114 | 0.415 | 0.368 |
| 115 | 4.10 | 1.25 |
| 116 | 20.5 | 7.23 |
| 117 | 1.03 | 15.6 |
| 118 | 4.64 | 6.38 |
| 119 | 2.25 | 1.48 |
| 120 | 0.246 | <0.169 |
| 121 | 23.0 | 2.47 |
| 122 | >100 | 9.10 |
| 123 | 2.46 | 0.208 |
| 124 | 3.31 | 0.371 |
| 125 | 3.69 | 1.11 |
| 126 | 0.312 | 0.328 |
| 127 | 4.22 | 0.938 |
| 128 | 9.55 | 4.46 |

## Claims

1. A compound having the structure of Formula (I): 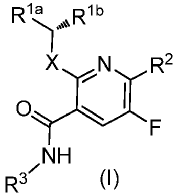 wherein
X is selected from the group consisting of: O, S and NR^{a};
when X is NR^{a}; R^{a} is H or CH₃; or R^{a} and R^{1a} come together to form a heterocyclic ring selected from the group consisting of: 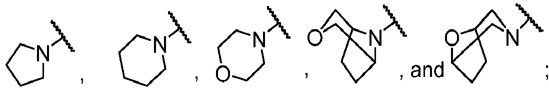 wherein the heterocyclic ring is substituted with one or two members each independently selected from the group consisting of: C₁₋₆alkyl, C₁₋₆haloalkyl, and OC₁₋₆alkyl;
R^{1a} is selected from the group consisting of: C₁₋₆alkyl; C₁₋₆alkyl substituted with OH, or OCH₃; C₁₋₆haloalkyl; C₁₋₆haloalkyl substituted with OH, or OCH₃; and C₃₋₆cycloalkyl;
R^{1b} is CH₃ or CHF₂; or R^{1a} and R^{1b} come together to form C₃₋₆cycloalkyl; C₃₋₆cycloalkyl independently substituted with one, two, three or four members each independently selected from the group consisting of: halo, OH, C₁₋₆alkyl, and C₁₋₆haloalkyl; oxetanyl; tetrahydrofuranyl; and tetrahydropyranyl;
R² is 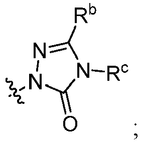 where
R^{b} is C₁₋₆alkyl substituted with a member selected from the group consisting of: OH, halo, CN, OC₁₋₆alkyl, OC₁₋₆haloalkyl and OC₃₋₆cycloalkyl;
R^{c} is selected from the group consisting of: C₁₋₆alkyl, C₁₋₆alkyl substituted with OH, allyl, C₁₋₆haloalkyl, and C₃₋₆cycloalkyl; and
R³ is selected from the group consisting of: 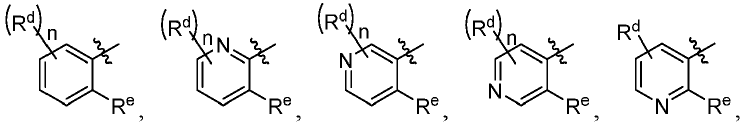 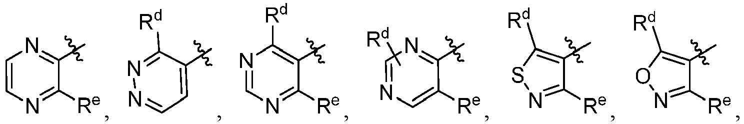 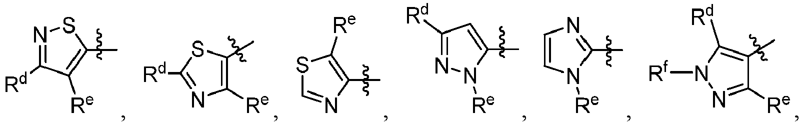 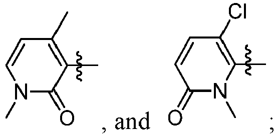
R^{d} is selected from the group consisting of: H; halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; N(CH₃)₂; OH; CN and OC₁₋₆alkyl;
R^{e} is selected from the group consisting of: halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; OH; OC₁₋₆alkyl;and C₃₋₆cycloalkyl;
R^{f} is selected from the group consisting of: H; C₁₋₆alkyl; C₁₋₆alkyl substituted with a member selected from the group consisting of: OH, OCH₃, SCH₃, and OCF₃; C₁₋₆haloalkyl; and C₁₋₆haloalkyl substituted with a member selected from the group consisting of: OH, and OCH₃; and
n is 1, or 2;
or a pharmaceutically acceptable salt, solvate, stereoisomer, tautomer, isotopic variant, or N-oxide thereof.

2. The compound according to claim 1, wherein:
(a) X is O; or a pharmaceutically acceptable salt, solvate, stereoisomer, tautomer, isotopic variant, or N-oxide thereof; or
(b) X is S; or a pharmaceutically acceptable salt, solvate, stereoisomer, tautomer, isotopic variant, or N-oxide thereof.

3. The compound according to claim 1, wherein:
(a) X is NR^{a}, and R^{a} is H; or a pharmaceutically acceptable salt, solvate, stereoisomer, tautomer, isotopic variant, or N-oxide thereof;
(b) X is NR^{a}, and R^{a} is CH₃; or a pharmaceutically acceptable salt, solvate, stereoisomer, tautomer, isotopic variant, or N-oxide thereof; or
(c) X is NR^{a}, where R^{a} and R^{1a} come together to form a heterocyclic ring selected from: 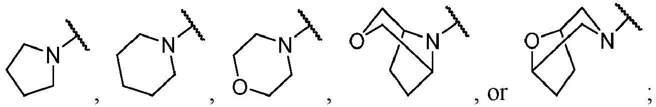 wherein the heterocyclic ring is substituted with one or two members each independently selected from the group consisting of: C₁₋₄alkyl, C₁₋₄haloalkyl, and OC₁₋₄alkyl; or a pharmaceutically acceptable salt, solvate, stereoisomer, tautomer, isotopic variant, or N-oxide thereof.

4. The compound according to any of claims 1-3, wherein:
(a) R^{1a} is C₁₋₄alkyl; C₁₋₄alkyl substituted with OH, or OCH₃; C₁₋₄haloalkyl; C₁₋₄haloalkyl substituted with OH, or OCH₃; or C₃₋₆cycloalkyl; or a pharmaceutically acceptable salt, solvate, stereoisomer, tautomer, isotopic variant, or N-oxide thereof; or
(b) R^{1a} is CF₃; or a pharmaceutically acceptable salt, solvate, stereoisomer, tautomer, isotopic variant, or N-oxide thereof.

5. The compound according to any of claims 1-4, wherein:
(a) R^{1b} is CH₃ or CHF₂; or a pharmaceutically acceptable salt, solvate, stereoisomer, tautomer, isotopic variant, or N-oxide thereof; or
(b) R^{1b} is CH₃; or a pharmaceutically acceptable salt, solvate, stereoisomer, tautomer, isotopic variant, or N-oxide thereof.

6. The compound according to any of claims 1-3, wherein R^{1a} and R^{1b} come together to form cyclopropyl, cyclobutyl, cyclopentyl, or cyclohexyl; cyclopropyl, cyclobutyl, cyclopentyl, or cyclohexyl each independently substituted with one, two, three or four members selected from the group consisting of: halo, OH, C₁₋₄alkyl, and C₁₋₄haloalkyl; oxetanyl; tetrahydrofuranyl; and tetrahydropyranyl; or a pharmaceutically acceptable salt, solvate, stereoisomer, tautomer, isotopic variant, or N-oxide thereof.

7. The compound according to any of claims 1-6, wherein:
(a) R² is 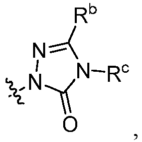 where
R^{b} is C₁₋₄alkyl substituted with OH, halo, CN, OC₁₋₄alkyl, OC₁₋₄haloalkyl or OC₃₋₆cycloalkyl; and
R^{c} is C₁₋₄alkyl, C₁₋₄haloalkyl, C₁₋₄alkyl substituted with OH, allyl, or C₃₋₆cycloalkyl; or a pharmaceutically acceptable salt, solvate, stereoisomer, tautomer, isotopic variant, or N-oxide thereof; or
(b) R² is 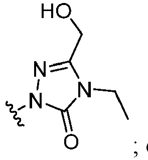 or a pharmaceutically acceptable salt, solvate, stereoisomer, tautomer, isotopic variant, or N-oxide thereof.

8. The compound according to any of claims 1-7, wherein:
(a) R³ is 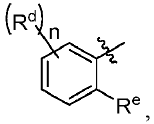 where
R^{d} is H; halo; C₁₋₄alkyl; C₁₋₄alkyl substituted with OH, OCH₃, SCH₃, or OCF₃; C₁₋₄haloalkyl; C₁₋₄haloalkyl substituted with OH, or OCH₃; CN; or OC₁₋₄alkyl;
R^{e} is halo; C₁₋₄alkyl; C₁₋₄alkyl substituted with OH, OCH₃, SCH₃, or OCF₃; C₁₋₄haloalkyl; or C₁₋₄haloalkyl substituted with OH, or OCH₃; and
n is 1 or 2;
or a pharmaceutically acceptable salt, solvate, stereoisomer, tautomer, isotopic variant, or N-oxide thereof;
(b) R³ is 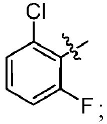 or a pharmaceutically acceptable salt, solvate, stereoisomer, tautomer, isotopic variant, or N-oxide thereof;
(c) R³ is 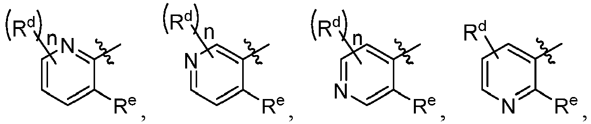 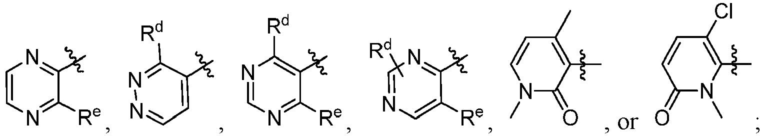 where
R^{d} is H; halo; C₁₋₄alkyl; C₁₋₄alkyl substituted with OH, OCH₃, SCH₃, or OCF₃; C₁₋₄haloalkyl; C₁₋₄haloalkyl substituted with OH, or OCH₃; N(CH₃)₂; OH; CN; or OC₁₋₄alkyl;
R^{e} is halo; OH; C₁₋₄alkyl; C₁₋₄alkyl substituted with OH, OCH₃, SCH₃, or OCF₃; C₁₋₄haloalkyl; OC₁₋₄alkyl; or C₁₋₄haloalkyl substituted with OH, or OCH₃; and
n is 1 or 2;
or a pharmaceutically acceptable salt, solvate, stereoisomer, tautomer, isotopic variant, or N-oxide thereof; or
(d) R³ is 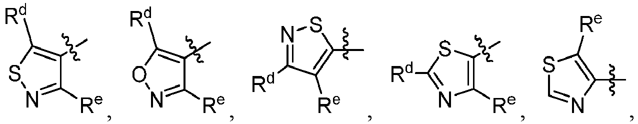 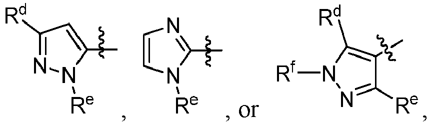 where
R^{d} is H; halo; C₁₋₄alkyl; C₁₋₄alkyl substituted with OH, OCH₃, SCH₃, or OCF₃; C₁₋₄haloalkyl; C₁₋₄haloalkyl substituted with OH, or OCH₃; or OC₁₋₄alkyl;
R^{e} is halo; C₁₋₄alkyl; C₁₋₄alkyl substituted with OH, OCH₃, SCH₃, or OCF₃; C₁₋₄haloalkyl; C₃₋₆cycloalkyl; or C₁₋₄haloalkyl substituted with OH, or OCH₃; and
R^{f} is H; C₁₋₄alkyl; C₁₋₄alkyl substituted with OH, OCH₃, SCH₃, or OCF₃; C₁₋₄haloalkyl; or C₁₋₄haloalkyl substituted with OH, or OCH₃;
or a pharmaceutically acceptable salt, solvate, stereoisomer, tautomer, isotopic variant, or N-oxide thereof.

9. The compound according to claim 1, having the structure of:
(a) Formula (IA): 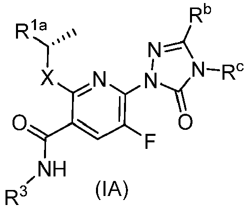 wherein
X is O;
R^{1a} is selected from the group consisting of: C₁₋₆alkyl; C₁₋₆alkyl substituted with OH, or OCH₃; C₁₋₆haloalkyl; C₁₋₆haloalkyl substituted with OH, or OCH₃; and C₃₋₆cycloalkyl;
R^{b} is C₁₋₆alkyl substituted with a member selected from the group consisting of: OH, halo, CN, OC₁₋₆alkyl, OC₁₋₆haloalkyl and OC₃₋₆cycloalkyl;
R^{c} is selected from the group consisting of: C₁₋₆alkyl, C₁₋₆alkyl substituted with OH, allyl, C₁₋₆haloalkyl, and C₃₋₆cycloalkyl; and
R³ is selected from the group consisting of: 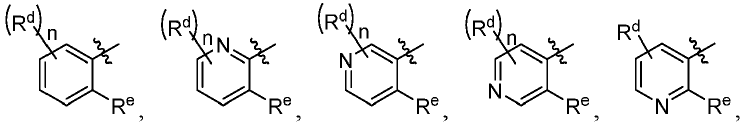 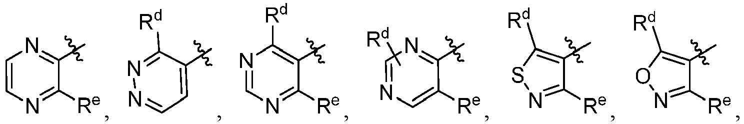 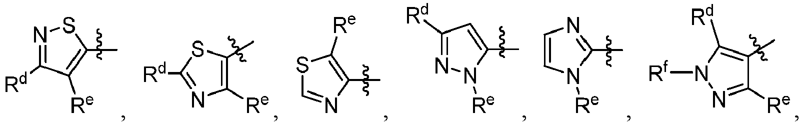 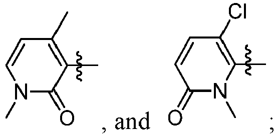
R^{d} is selected from the group consisting of: H; halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; N(CH₃)₂; OH; CN and OC₁₋₆alkyl;
R^{e} is selected from the group consisting of: halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; OH; OC₁₋₆alkyl;and C₃₋₆cycloalkyl;
R^{f} is selected from the group consisting of: H; C₁₋₆alkyl; C₁₋₆alkyl substituted with a member selected from the group consisting of: OH, OCH₃, SCH₃, and OCF₃; C₁₋₆haloalkyl; and C₁₋₆haloalkyl substituted with a member selected from the group consisting of: OH, and OCH₃; and
n is 1, or 2;
or a pharmaceutically acceptable salt, solvate, stereoisomer, tautomer, isotopic variant, or N-oxide thereof;
for example wherein R^{1a} is selected from the group consisting of: C₁₋₄alkyl; C₁₋₄alkyl substituted with OH, or OCH₃; C₁₋₄haloalkyl; C₁₋₄haloalkyl substituted with OH, or OCH₃; or C₃₋₆cycloalkyl;
(b) Formula (IB): 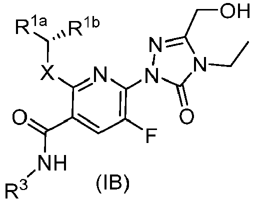 wherein
X is selected from the group consisting of: O, S and NR^{a};
when X is NR^{a}; R^{a} is H or CH₃; or R^{a} and R^{1a} come together to form a heterocyclic ring selected from the group consisting of: 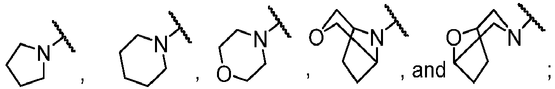 wherein the heterocyclic ring is substituted with one or two members each independently selected from the group consisting of: C₁₋₆alkyl, C₁₋₆haloalkyl, and OC₁₋₆alkyl;
R^{1a} is selected from the group consisting of: C₁₋₆alkyl; C₁₋₆alkyl substituted with OH, or OCH₃; C₁₋₆haloalkyl; C₁₋₆haloalkyl substituted with OH, or OCH₃; and C₃₋₆cycloalkyl;
R^{1b} is CH₃ or CHF₂; and
R³ is selected from the group consisting of: 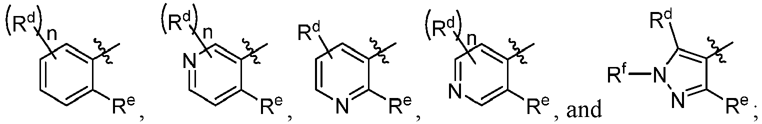
where
R^{d} is selected from the group consisting of: H; halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; N(CH₃)₂; OH; CN and OC₁₋₆alkyl;
R^{e} is selected from the group consisting of: halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; OH; OC₁₋₆alkyl;and C₃₋₆cycloalkyl;
R^{f} is selected from the group consisting of: H; C₁₋₆alkyl; C₁₋₆alkyl substituted with a member selected from the group consisting of: OH, OCH₃, SCH₃, and OCF₃; C₁₋₆haloalkyl; and C₁₋₆haloalkyl substituted with a member selected from the group consisting of: OH, and OCH₃; and
n is 1, or 2;
or a pharmaceutically acceptable salt, solvate, stereoisomer, tautomer, isotopic variant, or N-oxide thereof; for example wherein R^{d} and R^{e} are each independently halo, C₁₋₄alkyl, and OC₁₋₄alkyl; or
(c) Formula (IC): 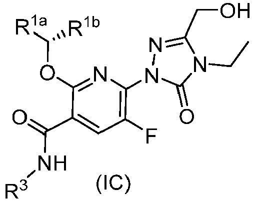
R^{1a} is selected from the group consisting of: C₁₋₆alkyl; C₁₋₆alkyl substituted with OH, or OCH₃; C₁₋₆haloalkyl; C₁₋₆haloalkyl substituted with OH, or OCH₃; and C₃₋₆cycloalkyl;
R^{1b} is CH₃ or CHF₂; or R^{1a} and R^{1b} come together to form C₃₋₆cycloalkyl; C₃₋₆cycloalkyl independently substituted with one, two, three or four members selected from the group consisting of: halo, OH, C₁₋₆alkyl, and C₁₋₆haloalkyl; oxetanyl; tetrahydrofuranyl; and tetrahydropyranyl; and
R³ is selected from the group consisting of: 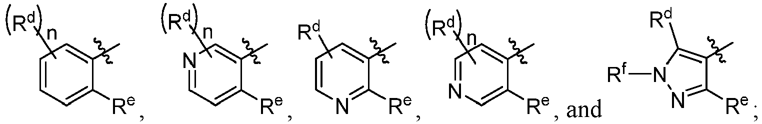
where
R^{d} is selected from the group consisting of: H; halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; N(CH₃)₂; OH; CN and OC₁₋₆alkyl;
R^{e} is selected from the group consisting of: halo; C₁₋₆alkyl; C₁₋₆alkyl substituted with a member selected from the group consisting of: OH, OCH₃, SCH₃, and OCF₃; C₁₋₆haloalkyl; C₁₋₆haloalkyl substituted with a member selected from the group consisting of: OH, and OCH₃; OH; OC₁₋₆alkyl;and C₃₋₆cycloalkyl;
R^{f} is selected from the group consisting of: H; C₁₋₆alkyl; C₁₋₆alkyl substituted with a member selected from the group consisting of: OH, OCH₃, SCH₃, and OCF₃; C₁₋₆haloalkyl; and C₁₋₆haloalkyl substituted with a member selected from the group consisting of: OH, and OCH₃; and
n is 1, or 2;
or a pharmaceutically acceptable salt, solvate, stereoisomer, tautomer, isotopic variant, or N-oxide thereof; for example wherein R^{1a} and R^{1b} come together to form C₃₋₆cycloalkyl; C₃₋₆cycloalkyl independently substituted with one, two, three or four members selected from the group consisting of: F, OH, C₁₋₆alkyl, and C₁₋₆haloalkyl; oxetanyl; tetrahydrofuranyl; or tetrahydropyranyl.

10. A compound according to claim 1 selected from the group consisting of:
(S)-N-(2-Chloro-6-fluorophenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-fluorophenyl)-2-((1,1,1-trifluoropropan-2-yl)oxy)nicotinamide;
(S)-N-(2-Chloro-6-fluorophenyl)-6-(4-ethyl-3-(fluoromethyl)-5-oxo-4,5-dihydro-1H-1,2,4-triazol-1-yl)-5-fluoro-2-((1,1,1-trifluoropropan-2-yl)oxy)nicotinamide;
(S)-N-(2-Chloropyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(2-Chloro-4-methylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(2-Chloro-6-fluorophenyl)-6-(3-(chloromethyl)-4-ethyl-5-oxo-4,5-dihydro-1H-1,2,4-triazol-1-yl)-5-fluoro-2-((1,1,1-trifluoropropan-2-yl)oxy)nicotinamide;
N-(2-Chloro-6-fluorophenyl)-6-(4-ethyl-3-(hydroxymethyl)-5-oxo-4,5-dihydro-1H-1,2,4-triazol-1-yl)-5-fluoro-2-isopropoxynicotinamide;
N-(2-Chloro-6-fluorophenyl)-6-(4-ethyl-3-(hydroxymethyl)-5-oxo-4,5-dihydro-1H-1,2,4-triazol-1-yl)-5-fluoro-2-(2,2,3,3-tetrafluorocyclobutoxy)nicotinamide;
Racemic-N-(2-Chloro-6-fluorophenyl)-6-(4-ethyl-3-(hydroxymethyl)-5-oxo-4,5-dihydro-1H-1,2,4-triazol-1-yl)-5-fluoro-2-((tetrahydrofuran-3 -
yl)oxy)nicotinamide; (S)-N-(3-Chloropyridin-4-yl)-6-(4-ethyl-3-(hydroxymethyl)-5-oxo-4,5-dihydro-1H-
1,2,4-triazol-1-yl)-5-fluoro-2-((1,1,1-trifluoropropan-2-yl)oxy)nicotinamide; (S)-6-(4-Ethyl-3-(hydroxymethyl)-5-oxo-4,5-dihydro-1H-1,2,4-triazol-1-yl)-5-
fluoro-N-(2-fluoro-5-methylphenyl)-2-((1,1,1-trifluoropropan-2-yl)oxy)nicotinamide;
(S)-N-(2-(Difluoromethyl)-4-methylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(2-Chloro-6-fluorophenyl)-6-(4-ethyl-3-(methoxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Chloropyridazin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-methylisothiazol-4-yl)-2-((1,1,1-trifluoropropan-2-yl)oxy)nicotinamide;
(S)-N-(2,4-Dimethylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Chloropyrazin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-chloro-6-methoxypyridin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Chloro-2-methoxypyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(5-Chloro-3-methyl-1H-pyrazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(4-Chloro-3-methylisothiazol-5-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(4-Chloropyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Chloro-5-fluoropyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Cyano-5-fluoropyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Chloro-5-fluoro-2-methoxypyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(4-Bromo-3-methylisothiazol-5-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(5-methylthiazol-4-yl)-2-((1,1,1-trifluoropropan-2-yl)oxy)nicotinamide;
(S)-N-(5-Chloro-2-methoxypyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-methoxypyrazin-2-yl)-2-((1,1,1-trifluoropropan-2-yl)oxy)nicotinamide;
(S)-N-(3,5-Dimethylisothiazol-4-yl)-6-(4-ethyl-3-(hydroxymethyl)-5-oxo-4,5-dihydro-1H-1,2,4-triazol-1-yl)-5-fluoro-2-((1,1, 1 -trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-fluoro-5-methylpyridin-3-yl)-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(5-fluoro-2-methylpyridin-4-yl)-2-((1,1,1-trifluoropropan-2-yl)oxy)nicotinamide;
(S)-N-(4-Chloro-6-methylpyrimidin-5-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-fluoro-6-methylpyridin-2-yl)-2-((1,1,1-trifluoropropan-2-yl)oxy)nicotinamide;
(S)-N-(3-Chloro-2-methoxy-5-methylpyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-methoxy-5-methylpyridin-3-yl)-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-fluoro-4-methylpyridin-3-yl)-2-((1,1,1-trifluoropropan-2-yl)oxy)nicotinamide;
(S)-N-(5,6-Dimethylpyrimidin-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(2-Chloro-4-methoxypyridin-3-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-methylisoxazol-4-yl)-2-((1,1,1-trifluoropropan-2-yl)oxy)nicotinamide;
(S)-N-(3,5-Dimethylisoxazol-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-fluoro-5-methoxyphenyl)-2-((1,1,1-trifluoropropan-2-yl)oxy)nicotinamide;
(S)-N-(2-Chloro-5-methylphenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3,5-Dimethyl-1H-pyrazol-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(4-Chloro-6-methoxypyridin-3-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(2,5-Dimethylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(5-Chloro-2-methoxypyridin-3-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
N-(1,4-Dimethyl-2-oxo-2,3-dihydro-114-pyridin-3-yl)-6-(4-ethyl-3-(hydroxymethyl)-5-oxo-4,5-dihydro-1H-1,2,4-triazol-1-yl)-5-fluoro-2-(((S)-1,1,1-trifluoropropan-2-yl)oxy)nicotinamide;
(S)-N-(3-Chloropyridin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-2-((1,1,1-trifluoropropan-2-yl)oxy)-N-(1,3,5-trimethyl-1H-pyrazol-4-yl)nicotinamide;
(S)-6-(4-Ethyl-3-(hydroxymethyl)-5-oxo-4,5-dihydro-1H-1,2,4-triazol-1-yl)-5-fluoro-N-(3-fluoro-6-methoxypyridin-2-yl)-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-methyl-1H-pyrazol-4-yl)-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-methoxy-1H-pyrazol-4-yl)-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-isopropyl-1-methyl-1H-pyrazol-5-yl)-2-((1,1,1-trifluoropropan-2-yl)oxy)nicotinamide;
(S)-N-(5-Chloro-6-methylpyrimidin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-(methoxymethyl)-1-methyl-1H-pyrazol-4-yl)-2-((1,1,1-trifluoropropan-2-yl)oxy)nicotinamide;
(S)-N-(3-Cyclopropyl-1H-pyrazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Chloro-1-methyl-6-oxo-1,6-dihydropyridin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(5-fluoro-2-methoxypyridin-4-yl)-2-((1,1,1-trifluoropropan-2-yl)oxy)nicotinamide;
(S)-6-(4-ethyl-3-(hydroxymethyl)-5-oxo-4, 5-dihydro-1H-1,2,4-triazol-1-yl)-5-fluoro-N-(2-methoxy-5-methylpyridin-4-yl)-2-((1,1,1-trifluoropropan-2-yl)oxy)nicotinamide;
(S)-N-(2-Chloro-5-methylpyridin-3-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-methoxy-4-methylpyridin-3-yl)-2-((1,1,1-trifluoropropan-2-yl)oxy)nicotinamide;
(S)-N-(2,4-Dichloropyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(2-Ethoxy-5-fluoropyrimidin-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-fluoro-5-methylpyridin-4-yl)-2-((1,1,1-trifluoropropan-2-yl)oxy)nicotinamide;
(S)-N-(2-Chloro-4,6-dimethylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(1-Cyclopropyl-1H-imidazol-2-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(2-Chloro-5-cyanophenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(2-Chloro-5-cyanopyridin-3-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(4-(Dimethylamino)-2-methoxypyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
N-(2-Chloro-6-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(2-Chloro-5-fluoro-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(4-Chloro-2-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(3-Chloro-1-methyl-pyrazol-4-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-[2-(Difluoromethyl)-5-fluoro-4-pyridyl]-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-N-(5-fluoro-2-methoxy-pyrimidin-4-yl)-2-[(1S)-2,2,2-trifluoro-1-methyl-ethoxy]pyridine-3-carboxamide;
N-(6-Chloro-2,3-dimethyl-phenyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(2-Chloro-4-methyl-3-pyridyl)-5-fluoro-6-[3-(hydroxymethyl)-5-oxo-4-propyl-1,2,4-triazol-1-yl]-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-N-(2-methoxy-3,5-dimethyl-4-pyridyl)-2-[(1S)-2,2,2-trifluoro-1-methyl-ethoxy]pyridine-3-carboxamide;
(S)-2-(5-(((2-Chloro-4-methylpyridin-3-yl)-12-azaneyl)carbonyl)-3-fluoro-6-((1,1,1-trifluoropropan-2-yl)oxy)pyridin-2-yl)-5-(fluoromethyl)-4-isopropyl-2,4-dihydro-3H-1,2,4-triazol-3-one;
N-(2-Chloro-4-methyl-3-pyridyl)-6-[4-ethyl-3-(fluoromethyl)-5-oxo-1,2,4-triazol-1-yl]-5-fluoro-2-[(1S)-2,2,2-trifluoro-1-methyl-ethoxy]pyridine-3-carboxamide;
N-(3-Chloro-2-methoxy-5-methyl-4-pyridyl)-6-[4-ethyl-3-(hydroxymethyl)-5-oxo-1,2,4-triazol-1-yl]-5-fluoro-2-isopropoxy-pyridine-3-carboxamide;
N-(2-Chloro-4-methyl-3-pyridyl)-5-fluoro-6-[3-(hydroxymethyl)-4-isopropyl-5-oxo-1,2,4-triazol-1-yl]-2-[(1S)-2,2,2-trifluoro-1-methyl-ethoxy]pyridine-3-carboxamide;
N-(2-Chloro-4-methyl-3-pyridyl)-6-[4-(2,2-difluoroethyl)-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(5-Chloro-3-methyl-1H-pyrazol-4-yl)-5-fluoro-6-[3-(hydroxymethyl)-5-oxo-4-propyl-1,2,4-triazol-1-yl]-2-[(1S)-2,2,2-trifluoro-1-methyl-ethoxy]pyridine-3-carboxamide;
N-(2,4-Dimethoxy-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(4,6-Dimethoxypyrimidin-5-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-2-[(1 S)-2,2,2-trifluoro-1-methyl-ethoxy]-N-[3-(trifluoromethyl)-1H-pyrazol-4-yl]pyridine-3-carboxamide;
N-(5-Chloro-3-methyl-1H-pyrazol-4-yl)-6-[4-ethyl-3-(fluoromethyl)-5-oxo-1,2,4-triazol-1-yl]-5-fluoro-2-[(1S)-2,2,2-trifluoro-1-methyl-ethoxy]pyridine-3-carboxamide;
N-(2-Chloro-4-methyl-3-pyridyl)-6-[4-ethyl-3-(fluoromethyl)-5-oxo-1,2,4-triazol-1-yl]-5-fluoro-2-isopropoxy-pyridine-3-carboxamide;
N-(5-Chloro-2-methoxy-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(5-Chloro-3-methyl-1H-pyrazol-4-yl)-5-fluoro-6-[3-(fluoromethyl)-5-oxo-4-propyl-1,2,4-triazol-1-yl]-2-[(1S)-2,2,2-trifluoro-1-methyl-ethoxy]pyridine-3-carboxamide;
N-(3-Cyano-1H-pyrazol-4-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(2-Chloro-4-methyl-3-pyridyl)-5-fluoro-6-[3-(fluoromethyl)-4-(3-hydroxypropyl)-5-oxo-1,2,4-triazol-1-yl]-2-[(1S)-2,2,2-trifluoro-1-methyl-ethoxy]pyridine-3-carboxamide;
N-(2-chloro-4,5-dimethyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-ethyl-3-(hydroxymethyl)-5-oxo-1,2,4-triazol-1-yl]-5-fluoro-N-(4-methoxy-2,6-dimethyl-3-pyridyl)-2-[(1S)-2,2,2-trifluoro-1-methyl-ethoxy]pyridine-3-carboxamide;
N-(3-Chloro-4-methoxy-2-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(3-Chloro-4-ethoxy-2-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(2-Chloro-6-methoxy-4-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(3-Chloro-5-methyl-1H-pyrazol-4-yl)-5-fluoro-6-[3-(fluoromethyl)-4-(3-hydroxypropyl)-5-oxo-1,2,4-triazol-1-yl]-2-[(1S)-2,2,2-trifluoro-1-methyl-ethoxy]pyridine-3-carboxamide;
N-(5-Chloro-3-methyl-1H-pyrazol-4-yl)-6-[4-ethyl-3-(fluoromethyl)-5-oxo-1,2,4-triazol-1-yl]-5-fluoro-2-isopropoxy-pyridine-3-carboxamide;
6-[4-Ethyl-3-(hydroxymethyl)-5-oxo-1,2,4-triazol-1-yl]-5-fluoro-N-(3-methoxypyridazin-4-yl)-2-[(1S)-2,2,2-trifluoro-1-methyl-ethoxy]pyridine-3-carboxamide;
N-(5-Chloro-2-methoxy-3-methyl-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-N-[3-methyl-5-(trifluoromethyl)-1H-pyrazol-4-yl]-2-[(1S)-2,2,2-trifluoro-1-methyl-ethoxy]pyridine-3-carboxamide;
N-[3-Chloro-5-(trifluoromethyl)-1H-pyrazol-4-yl]-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(6-Chloro-2-methoxy-4-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-N-(2-methoxy-4,6-dimethyl-3-pyridyl)-2-[(1S)-2,2,2-trifluoro-1-methyl-ethoxy]pyridine-3-carboxamide;
N-(5-Chloro-3-methyl-1H-pyrazol-4-yl)-5-fluoro-6-[3-(fluoromethyl)-4-(2-hydroxyethyl)-5-oxo-1,2,4-triazol-1-yl]-2-[(1S)-2,2,2-trifluoro-1-methyl-ethoxy]pyridine-3-carboxamide;
N-(2-Chloro-4-methyl-3-pyridyl)-5-fluoro-6-[3-(fluoromethyl)-4-(2-hydroxyethyl)-5-oxo-1,2,4-triazol-1-yl]-2-[(1S)-2,2,2-trifluoro-1-methyl-ethoxy]pyridine-3-carboxamide;
N-(3-Cyano-5-fluoro-2-methoxy-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(3-Chloro-5-methoxy-2-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(2,6-Dimethoxyphenyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-N-(o-tolyl)-2-[(1S)-2,2,2-trifluoro-1-methyl-ethoxy]pyridine-3 -carboxamide;
6-[4-Ethyl-3-(hydroxymethyl)-5-oxo-1,2,4-triazol-1-yl]-5-fluoro-N-(3-methoxy-5-methyl-1H-pyrazol-4-yl)-2-[(1S)-2,2,2-trifluoro-1-methyl-ethoxy]pyridine-3-carboxamide;
N-(3,5-Dimethoxy-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(3,5-Dichloro-1H-pyrazol-4-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-[3-(Difluoromethyl)-5-methyl-1H-pyrazol-4-yl]-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-N-(4-fluoro-2-methoxy-3-pyridyl)-2-[(1S)-2,2,2-trifluoro-1-methyl-ethoxy]pyridine-3-carboxamide;
N-(4-Chloro-2-methoxy-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
(Racemic)-6-[4-Ethyl-3-(1-hydroxyethyl)-5-oxo-1,2,4-triazol-1-yl]-5-fluoro-N-[3-methyl-5-(trifluoromethyl)-1H-pyrazol-4-yl]-2-(2,2,2-trifluoro-1-methyl-ethoxy)pyridine-3-carboxamide; and
6-[4-Ethyl-3-(1-hydroxy-1-methyl-ethyl)-5-oxo-1,2,4-triazol-1-yl]-5-fluoro-N-[3-methyl-5-(trifluoromethyl)-1H-pyrazol-4-yl]-2-[(1S)-2,2,2-trifluoro-1-methyl-ethoxy]pyridine-3-carboxamide;
or a pharmaceutically acceptable salt, solvate, stereoisomer, tautomer, isotopic variant, or N-oxide thereof.

11. A compound according to claim 1 selected from the group consisting of:
(S)-N-(2-Chloro-4-methylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(5-Chloro-3-methyl-1H-pyrazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Chloro-2-methoxy-5-methylpyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-methoxy-4-methylpyridin-3-yl)-2-((1,1,1-trifluoropropan-2-yl)oxy)nicotinamide;
6-[4-Ethyl-3-(hydroxymethyl)-5-oxo-1,2,4-triazol-1-yl]-5-fluoro-N-[3-methyl-5-(trifluoromethyl)-1H-pyrazol-4-yl]-2-[(1S)-2,2,2-trifluoro-1-methyl-ethoxy]pyridine-3-carboxamide; and
6-[4-Ethyl-3-(hydroxymethyl)-5-oxo-1,2,4-triazol-1-yl]-5-fluoro-N-(2-methoxy-3,5-dimethyl-4-pyridyl)-2-[(1S)-2,2,2-trifluoro-1-methyl-ethoxy]pyridine-3-carboxamide;
or a pharmaceutically acceptable salt, solvate, stereoisomer, tautomer, isotopic variant, or N-oxide thereof.

12. A pharmaceutical composition comprising: (A) an effective amount of a compound according to any of claims 1-11, or a pharmaceutically acceptable salt, solvate, stereoisomer, tautomer, isotopic variant, or N-oxide thereof; and (B) at least one pharmaceutically acceptable excipient.

13. At least one compound or a pharmaceutically acceptable salt, solvate, stereoisomer, tautomer, isotopic variant, or N-oxide thereof according to any of claims 1-11 for use in treating a subject suffering from or diagnosed with a disease, disorder, or medical condition comprising inhibiting or altering dihydroorotate oxygenase enzyme activity in the subject, wherein the disease, disorder, or medical condition is an inflammatory disorder, an autoimmune disorder, or cancer.

14. At least one compound or a pharmaceutically acceptable salt, solvate, stereoisomer, tautomer, isotopic variant, or N-oxide thereof for use according to claim 13, wherein the disorder, disease or medical condition is selected from the group consisting of: lymphomas, leukemias, carcinomas, and sarcomas.

15. At least one compound or a pharmaceutically acceptable salt, solvate, stereoisomer, tautomer, isotopic variant, or N-oxide thereof for use according to claim 13, wherein the disorder, disease or medical condition is selected from the group consisting of: acute lymphoblastic leukemia, acute myeloid leukemia, (acute) T-cell leukemia, acute lymphoblastic leukemia, acute lymphocytic leukemia, acute monocytic leukemia, acute promyelocytic leukemia, bisphenotypic B myelomonocytic leukemia, chronic lymphocytic leukemia, chronic myelogenous leukemia, chronic myeloid leukemia, chronic myelomonocytic leukemia, large granular lymphocytic leukemia, plasma cell leukemia, and also myelodysplastic syndrome, which can develop into an acute myeloid leukemia.

16. At least one compound or a pharmaceutically acceptable salt, solvate, stereoisomer, tautomer, isotopic variant, or N-oxide thereof for use according to claim 13, wherein the disorder, disease or medical condition is acute myeloid leukemia.

17. At least one compound or a pharmaceutically acceptable salt, solvate, stereoisomer, tautomer, isotopic variant, or N-oxide thereof for use according to any of claims 13-16, wherein the at least one compound comprises a compound selected from the group consisting of:
(S)-N-(2-Chloro-6-fluorophenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-fluorophenyl)-2-((1,1,1-trifluoropropan-2-yl)oxy)nicotinamide;
(S)-N-(2-Chloro-6-fluorophenyl)-6-(4-ethyl-3-(fluoromethyl)-5-oxo-4,5-dihydro-1H-1,2,4-triazol-1-yl)-5-fluoro-2-((1,1,1-trifluoropropan-2-yl)oxy)nicotinamide;
(S)-N-(2-Chloropyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(2-Chloro-4-methylpyridin-3-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(2-Chloro-6-fluorophenyl)-6-(3-(chloromethyl)-4-ethyl-5-oxo-4,5-dihydro-1H-1,2,4-triazol-1-yl)-5-fluoro-2-((1,1,1-trifluoropropan-2-yl)oxy)nicotinamide;
N-(2-Chloro-6-fluorophenyl)-6-(4-ethyl-3-(hydroxymethyl)-5-oxo-4,5-dihydro-1H-1,2,4-triazol-1-yl)-5-fluoro-2-isopropoxynicotinamide;
N-(2-Chloro-6-fluorophenyl)-6-(4-ethyl-3-(hydroxymethyl)-5-oxo-4,5-dihydro-1H-1,2,4-triazol-1-yl)-5-fluoro-2-(2,2,3,3-tetrafluorocyclobutoxy)nicotinamide;
Racemic-N-(2-Chloro-6-fluorophenyl)-6-(4-ethyl-3-(hydroxymethyl)-5-oxo-4,5-dihydro-1H-1,2,4-triazol-1-yl)-5-fluoro-2-((tetrahydrofuran-3 - yl)oxy)nicotinamide;
(S)-N-(3-Chloropyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-fluoro-5 -methylphenyl)-2-((1,1,1 -trifluoropropan-2-yl)oxy)nicotinamide;
(S)-N-(2-(Difluoromethyl)-4-methylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(2-Chloro-6-fluorophenyl)-6-(4-ethyl-3-(methoxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Chloropyridazin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-methylisothiazol-4-yl)-2-((1,1,1-trifluoropropan-2-yl)oxy)nicotinamide;
(S)-N-(2,4-Dimethylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Chloropyrazin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide; (S)-N-(3-chloro-6-methoxypyridin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Chloro-2-methoxypyridin-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(5-Chloro-3-methyl-1H-pyrazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(4-Chloro-3-methylisothiazol-5-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(4-Chloropyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Chloro-5-fluoropyridin-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(3-Cyano-5-fluoropyridin-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(3-Chloro-5-fluoro-2-methoxypyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(4-Bromo-3-methylisothiazol-5-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(5-methylthiazol-4-yl)-2-((1,1,1-trifluoropropan-2-yl)oxy)nicotinamide;
(S)-N-(5-Chloro-2-methoxypyridin-3-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-methoxypyrazin-2-yl)-2-((1,1,1-trifluoropropan-2-yl)oxy)nicotinamide;
(S)-N-(3,5-Dimethylisothiazol-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-fluoro-5 -methylpyridin-3-yl)-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(5-fluoro-2-methylpyridin-4-yl)-2-((1,1,1-trifluoropropan-2-yl)oxy)nicotinamide;
(S)-N-(4-Chloro-6-methylpyrimidin-5-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-fluoro-6-methylpyridin-2-yl)-2-((1, 1, 1-trifluoropropan-2-yl)oxy)nicotinamide;
(S)-N-(3-Chloro-2-methoxy-5-methylpyridin-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-methoxy-5-methylpyridin-3-yl)-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-fluoro-4-methylpyridin-3-yl)-2-((1,1,1-trifluoropropan-2-yl)oxy)nicotinamide;
(S)-N-(5,6-Dimethylpyrimidin-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(2-Chloro-4-methoxypyridin-3-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-methylisoxazol-4-yl)-2-((1,1,1 -trifluoropropan-2-yl)oxy)nicotinamide;
(S)-N-(3,5-Dimethylisoxazol-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-fluoro-5-methoxyphenyl)-2-((1,1, 1-trifluoropropan-2-yl)oxy)nicotinamide;
(S)-N-(2-Chloro-5-methylphenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3,5-Dimethyl-1H-pyrazol-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(4-Chloro-6-methoxypyridin-3-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(2,5-Dimethylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(5-Chloro-2-methoxypyridin-3-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
N-(1,4-Dimethyl-2-oxo-2,3-dihydro-114-pyridin-3-yl)-6-(4-ethyl-3-(hydroxymethyl)-5-oxo-4,5-dihydro-1H-1,2,4-triazol-1-yl)-5-fluoro-2-(((S)-1,1,1-trifluoropropan-2-yl)oxy)nicotinamide;
(S)-N-(3-Chloropyridin-2-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-2-((1,1,1 -trifluoropropan-2-yl)oxy)-N-(1,3,5-trimethyl-1H-pyrazol-4-yl)nicotinamide;
(S)-6-(4-Ethyl-3-(hydroxymethyl)-5-oxo-4,5-dihydro-1H-1,2,4-triazol-1-yl)-5-fluoro-N-(3-fluoro-6-methoxypyridin-2-yl)-2-((1, 1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3 -methyl-1H-pyrazol-4-yl)-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3 -methoxy-1H-pyrazol-4-yl)-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3-isopropyl-1-methyl-1H-pyrazol-5-yl)-2-((1,1,1-trifluoropropan-2-yl)oxy)nicotinamide;
(S)-N-(5-Chloro-6-methylpyrimidin-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3 -(methoxymethyl)-1-methyl-1H-pyrazol-4-yl)-2-((1,1,1-trifluoropropan-2-yl)oxy)nicotinamide;
(S)-N-(3-Cyclopropyl-1H-pyrazol-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(3-Chloro-1-methyl-6-oxo-1,6-dihydropyridin-2-yl)-6-(4-ethyl-3-(hydroxymethyl)-5-oxo-4,5-dihydro- 1H-1,2,4-triazol-1-yl)-5-fluoro-2-((1, 1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(5-fluoro-2-methoxypyridin-4-yl)-2-((1,1,1-trifluoropropan-2-yl)oxy)nicotinamide;
(S)-6-(4-ethyl-3-(hydroxymethyl)-5-oxo-4, 5-dihydro-1H-1,2,4-triazol-1-yl)-5-fluoro-N-(2-methoxy-5-methylpyridin-4-yl)-2-((1,1,1-trifluoropropan-2-yl)oxy)nicotinamide;
(S)-N-(2-Chloro-5-methylpyridin-3-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-methoxy-4-methylpyridin-3 -yl)-2-((1,1,1-trifluoropropan-2-yl)oxy)nicotinamide;
(S)-N-(2,4-Dichloropyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(2-Ethoxy-5-fluoropyrimidin-4-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(3 -fluoro-5-methylpyridin-4-yl)-2-((1,1,1-trifluoropropan-2-yl)oxy)nicotinamide;
(S)-N-(2-Chloro-4,6-dimethylpyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(1-Cyclopropyl-1H-imidazol-2-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(2-Chloro-5-cyanophenyl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(2-Chloro-5-cyanopyridin-3-yl)-6-(4-ethyl-3-(hydroxymethyl)-5-oxo-4,5-dihydro-1H-1,2,4-triazol-1-yl)-5-fluoro-2-((1,1, 1-trifluoropropan-2-yl)oxy)nicotinamide;
(S)-N-(4-(Dimethylamino)-2-methoxypyridin-3-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
N-(2-Chloro-6-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(2-Chloro-5-fluoro-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(4-Chloro-2-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(3-Chloro-1-methyl-pyrazol-4-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-[2-(Difluoromethyl)-5-fluoro-4-pyridyl]-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-N-(5-fluoro-2-methoxy-pyrimidin-4-yl)-2-[(1S)-2,2,2-trifluoro-1-methyl-ethoxy]pyridine-3-carboxamide;
N-(6-Chloro-2,3-dimethyl-phenyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(2-Chloro-4-methyl-3-pyridyl)-5-fluoro-6-[3-(hydroxymethyl)-5-oxo-4-propyl-1,2,4-triazol-1-yl]-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-N-(2-methoxy-3,5-dimethyl-4-pyridyl)-2-[(1S)-2,2,2-trifluoro-1-methyl-ethoxy]pyridine-3-carboxamide;
(S)-2-(5-(((2-Chloro-4-methylpyridin-3-yl)-12-azaneyl)carbonyl)-3-fluoro-6-((1,1,1-trifluoropropan-2-yl)oxy)pyridin-2-yl)-5-(fluoromethyl)-4-isopropyl-2,4-dihydro-3H-1,2,4-triazol-3-one;
N-(2-Chloro-4-methyl-3-pyridyl)-6-[4-ethyl-3-(fluoromethyl)-5-oxo-1,2,4-triazol-1-yl]-5-fluoro-2-[(1S)-2,2,2-trifluoro-1-methyl-ethoxy]pyridine-3-carboxamide;
N-(3-Chloro-2-methoxy-5-methyl-4-pyridyl)-6-[4-ethyl-3-(hydroxymethyl)-5-oxo-1,2,4-triazol-1-yl]-5-fluoro-2-isopropoxy-pyridine-3-carboxamide;
N-(2-Chloro-4-methyl-3-pyridyl)-5-fluoro-6-[3-(hydroxymethyl)-4-isopropyl-5-oxo-1,2,4-triazol-1-yl]-2-[(1S)-2,2,2-trifluoro-1-methyl-ethoxy]pyridine-3 - carboxamide;
N-(2-Chloro-4-methyl-3-pyridyl)-6-[4-(2,2-difluoroethyl)-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(5-Chloro-3-methyl-1H-pyrazol-4-yl)-5-fluoro-6-[3-(hydroxymethyl)-5-oxo-4-propyl-1,2,4-triazol-1-yl]-2-[(1S)-2,2,2-trifluoro-1-methyl-ethoxy]pyridine-3-carboxamide;
N-(2,4-Dimethoxy-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(4,6-Dimethoxypyrimidin-5-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-2-[(1S)-2,2,2-trifluoro-1-methyl-ethoxy]-N-[3-(trifluoromethyl)-1H-pyrazol-4-yl]pyridine-3-carboxamide;
N-(5-Chloro-3-methyl-1H-pyrazol-4-yl)-6-[4-ethyl-3-(fluoromethyl)-5-oxo-1,2,4-triazol-1-yl]-5-fluoro-2-[(1S)-2,2,2-trifluoro-1-methyl-ethoxy]pyridine-3-carboxamide;
N-(2-Chloro-4-methyl-3-pyridyl)-6-[4-ethyl-3-(fluoromethyl)-5-oxo-1,2,4-triazol-1-yl]-5-fluoro-2-isopropoxy-pyridine-3-carboxamide;
N-(5-Chloro-2-methoxy-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(5-Chloro-3-methyl-1H-pyrazol-4-yl)-5-fluoro-6-[3-(fluoromethyl)-5-oxo-4-propyl-1,2,4-triazol-1-yl]-2-[(1S)-2,2,2-trifluoro-1-methyl-ethoxy]pyridine-3-carboxamide;
N-(3-Cyano-1H-pyrazol-4-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(2-Chloro-4-methyl-3-pyridyl)-5-fluoro-6-[3-(fluoromethyl)-4-(3-hydroxypropyl)-5-oxo-1,2,4-triazol-1-yl]-2-[(1S)-2,2,2-trifluoro-1-methyl-ethoxy]pyridine-3-carboxamide;
N-(2-chloro-4,5-dimethyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-ethyl-3-(hydroxymethyl)-5-oxo-1,2,4-triazol-1-yl]-5-fluoro-N-(4-methoxy-2,6-dimethyl-3-pyridyl)-2-[(1S)-2,2,2-trifluoro-1-methyl-ethoxy]pyridine-3-carboxamide;
N-(3-Chloro-4-methoxy-2-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(3-Chloro-4-ethoxy-2-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(2-Chloro-6-methoxy-4-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(3-Chloro-5-methyl-1H-pyrazol-4-yl)-5-fluoro-6-[3-(fluoromethyl)-4-(3-hydroxypropyl)-5-oxo-1,2,4-triazol-1-yl]-2-[(1S)-2,2,2-trifluoro-1-methyl-ethoxy]pyridine-3-carboxamide;
N-(5-Chloro-3-methyl-1H-pyrazol-4-yl)-6-[4-ethyl-3-(fluoromethyl)-5-oxo-1,2,4-triazol-1-yl]-5-fluoro-2-isopropoxy-pyridine-3-carboxamide;
6-[4-Ethyl-3-(hydroxymethyl)-5-oxo-1,2,4-triazol-1-yl]-5-fluoro-N-(3-methoxypyridazin-4-yl)-2-[(1S)-2,2,2-trifluoro-1-methyl-ethoxy]pyridine-3-carboxamide;
N-(5-Chloro-2-methoxy-3-methyl-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-N-[3-methyl-5-(trifluoromethyl)-1H-pyrazol-4-yl]-2-[(1S)-2,2,2-trifluoro-1-methyl-ethoxy]pyridine-3-carboxamide;
N-[3-Chloro-5-(trifluoromethyl)-1H-pyrazol-4-yl]-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(6-Chloro-2-methoxy-4-methyl-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-N-(2-methoxy-4,6-dimethyl-3-pyridyl)-2-[(1S)-2,2,2-trifluoro-1-methyl-ethoxy]pyridine-3-carboxamide;
N-(5-Chloro-3-methyl-1H-pyrazol-4-yl)-5-fluoro-6-[3-(fluoromethyl)-4-(2-hydroxyethyl)-5-oxo-1,2,4-triazol-1-yl]-2-[(1S)-2,2,2-trifluoro-1-methyl-ethoxy]pyridine-3-carboxamide;
N-(2-Chloro-4-methyl-3-pyridyl)-5-fluoro-6-[3-(fluoromethyl)-4-(2-hydroxyethyl)-5-oxo-1,2,4-triazol-1-yl]-2-[(1S)-2,2,2-trifluoro-1-methyl-ethoxy]pyridine-3-carboxamide;
N-(3-Cyano-5-fluoro-2-methoxy-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(3-Chloro-5-methoxy-2-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(2,6-Dimethoxyphenyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-N-(o-tolyl)-2-[(1S)-2,2,2-trifluoro-1-methyl-ethoxy]pyridine-3 -carboxamide;
6-[4-Ethyl-3-(hydroxymethyl)-5-oxo-1,2,4-triazol-1-yl]-5-fluoro-N-(3-methoxy-5-methyl-1H-pyrazol-4-yl)-2-[(1S)-2,2,2-trifluoro-1-methyl-ethoxy]pyridine-3-carboxamide;
N-(3, 5-Dimethoxy-4-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-(3, 5-Dichloro-1H-pyrazol-4-yl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
N-[3-(Difluoromethyl)-5-methyl-1H-pyrazol-4-yl]-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
6-[4-Ethyl-3-(hydroxymethyl)-5-oxo-1,2,4-triazol-1-yl]-5-fluoro-N-(4-fluoro-2-methoxy-3-pyridyl)-2-[(1S)-2,2,2-trifluoro-1-methyl-ethoxy]pyridine-3-carboxamide;
N-(4-Chloro-2-methoxy-3-pyridyl)-6-[4-ethyl-3-(hydroxymethyl)-5-oxo-1,2,4-triazol-1-yl]-5-fluoro-2-[(1S)-2,2,2-trifluoro-1-methyl-ethoxy]pyridine-3-carboxamide;
(Racemic)-6-[4-Ethyl-3-(1-hydroxyethyl)-5-oxo-1,2,4-triazol-1-yl]-5-fluoro-N-[3-methyl-5-(trifluoromethyl)-1H-pyrazol-4-yl]-2-(2,2,2-trifluoro-1-methyl-ethoxy)pyridine-3-carboxamide; and
6-[4-Ethyl-3-(1-hydroxy-1-methyl-ethyl)-5-oxo-1,2,4-triazol-1-yl]-5-fluoro-N-[3-methyl-5-(trifluoromethyl)-1H-pyrazol-4-yl]-2-[(1S)-2,2,2-trifluoro-1-methyl-ethoxy]pyridine-3-carboxamide;
or a pharmaceutically acceptable salt, solvate, stereoisomer, tautomer, isotopic variant, or N-oxide thereof.

18. At least one compound or a pharmaceutically acceptable salt, solvate, stereoisomer, tautomer, isotopic variant, or N-oxide thereof for use according to any of claims 13-16, wherein the at least one compound comprises a compound selected from the group consisting of:
(S)-N-(2-Chloro-4-methylpyridin-3-yl)-6-(4-ethyl-3-(hydroxymethyl)-5-oxo-4,5-dihydro-1H-1,2,4-triazol-1-yl)-5-fluoro-2-((1, 1, 1-trifluoropropan-2-yl)oxy)nicotinamide;
(S)-N-(5-Chloro-3-methyl-1H-pyrazol-4-yl)-6-(4-ethyl-3-(hydroxymethyl)-5-oxo-4,5-dihydro-1H-1,2,4-triazol-1-yl)-5-fluoro-2-((1,1,1-trifluoropropan-2-yl)oxy)nicotinamide;
(S)-N-(3-Chloro-2-methoxy-5-methylpyridin-4-yl)-6-(4-ethyl-3-(hydroxymethyl)-5-oxo-4,5-dihydro-1H-1,2,4-triazol-1-y1)-5-fluoro-2-((1,1,1-trifluoropropan-2-yl)oxy)nicotinamide;
(S)-6-(4-Ethyl-3-(hydroxymethyl)-5-oxo-4,5-dihydro-1H-1,2,4-triazol-1-yl)-5-fluoro-N-(2-methoxy-4-methylpyridin-3-yl)-2-((1,1,1-trifluoropropan-2-yl)oxy)nicotinamide;
6-[4-Ethyl-3-(hydroxymethyl)-5-oxo-1,2,4-triazol-1-yl]-5-fluoro-N-[3-methyl-5-(trifluoromethyl)-1H-pyrazol-4-yl]-2-[(1S)-2,2,2-trifluoro-1-methyl-ethoxy]pyridine-3-carboxamide; and
6-[4-Ethyl-3-(hydroxymethyl)-5-oxo-1,2,4-triazol-1-yl]-5-fluoro-N-(2-methoxy-3,5-dimethyl-4-pyridyl)-2-[(1S)-2,2,2-trifluoro-1-methyl-ethoxy]pyridine-3-carboxamide;
or a pharmaceutically acceptable salt, solvate, stereoisomer, tautomer, isotopic variant, or N-oxide thereof.

19. A compound according to claim 1 selected from: 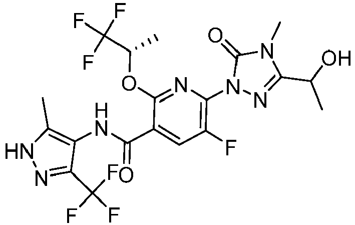 and 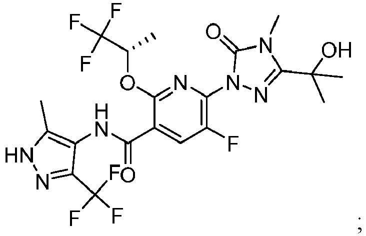 or a pharmaceutically acceptable salt, solvate, stereoisomer, tautomer, isotopic variant, or N-oxide thereof.

20. The compound according to any of claims 1-6, wherein R² is 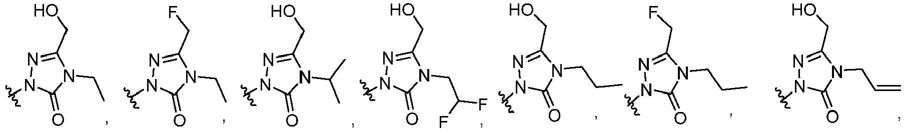 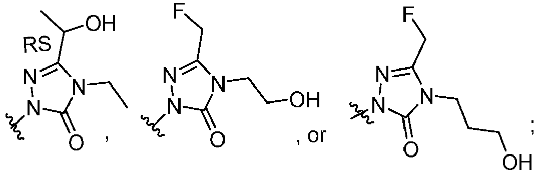 or a pharmaceutically acceptable salt, solvate, stereoisomer, tautomer, isotopic variant, or N-oxide thereof.

21. The compound according to any of claims 1-3, wherein R^{1a} is CH₃; or a pharmaceutically acceptable salt, solvate, stereoisomer, tautomer, isotopic variant, or N-oxide thereof.

## Patentansprüche

1. Verbindung mit der Struktur der Formel (I): 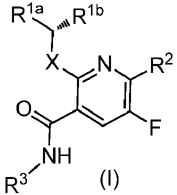 wobei
X aus der Gruppe bestehend aus O, S und NR^{a} ausgewählt ist;
dann, wenn X für NR^{a} steht, R^{a} für H oder CH₃ steht oder R^{a} und R^{1a} zusammen einen heterocyclischen Ring bilden, der aus der Gruppe bestehend aus 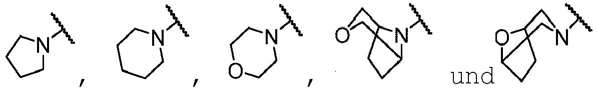 ausgewählt ist;
wobei der heterocyclische Ring durch ein oder zwei Glieder, die unabhängig aus der Gruppe bestehend aus C₁₋₆-Alkyl, C₁₋₆-Halogenalkyl und O-C₁₋₆-Alkyl ausgewählt sind, substituiert ist;
R^{1a} aus der Gruppe bestehend aus C₁₋₆-Alkyl; C₁₋₆-Alkyl, das durch OH oder OCH₃ substituiert ist; C₁₋₆-Halogenalkyl; C₁₋₆-Halogenalkyl, das durch OH oder OCH₃ substituiert ist; und C₃₋₆-Cycloalkyl ausgewählt ist;
R^{1b} für CH₃ oder CHF₂ steht; oder R^{1a} und R^{1b} zusammen C₃₋₆-Cycloalkyl; C₃₋₆-Cycloalkyl, das unabhängig durch ein, zwei, drei oder vier Glieder, die jeweils unabhängig aus der Gruppe bestehend aus Halogen, OH, C₁₋₆-Alkyl und C₁₋₆-Halogenalkyl ausgewählt sind, substituiert ist;
Oxetanyl; Tetrahydrofuranyl und Tetrahydropyranyl bilden;
R² für 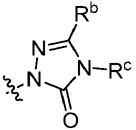 steht; wobei
R^{b} für C₁₋₆-Alkyl, das durch ein Mitglied aus der Gruppe bestehend aus OH, Halogen, CN, O-C₁₋₆-Alkyl, O-C₁₋₆-Halogenalkyl und O-C₃₋₆-Cycloalkyl substituiert ist, steht;
R^{c} aus der Gruppe bestehend aus C₁₋₆-Alkyl, C₁₋₆-Alkyl, das durch OH substituiert ist, Allyl, C₁₋₆-Halogenalkyl und C₃₋₆-Cycloalkyl ausgewählt ist; und
R³ aus der Gruppe bestehend aus 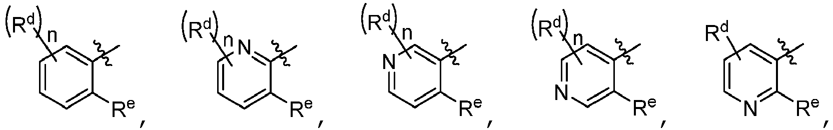 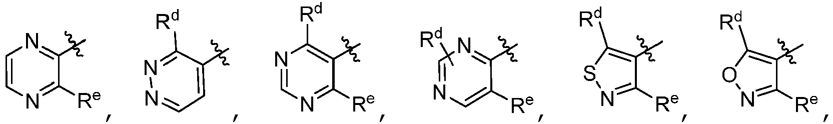 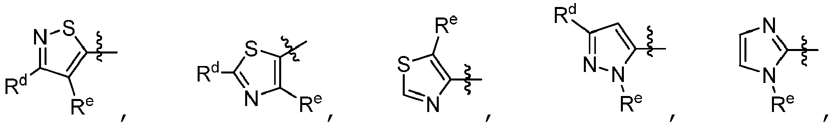 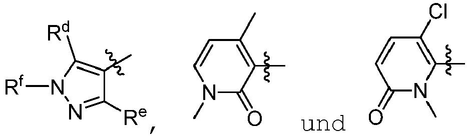 ausgewählt ist;
R^{d} aus der Gruppe bestehend aus H; Halogen; C₁₋₆-Alkyl; C₁₋₆-Alkyl, das durch ein Mitglied aus der Gruppe bestehend aus OH, OCH₃, SCH₃ und OCF₃ substituiert ist; C₁₋₆-Halogenalkyl; C₁₋₆-Halogenalkyl, das durch ein Mitglied aus der Gruppe bestehend aus OH und OCH₃ substituiert ist; N(CH₃)₂; OH; CN und O-C₁₋₆-Alkyl ausgewählt ist;
R^{e} aus der Gruppe bestehend aus Halogen; C₁₋₆-Alkyl; C₁₋₆-Alkyl, das durch ein Mitglied aus der Gruppe bestehend aus OH, OCH₃, SCH₃ und OCF₃ substituiert ist; C₁₋₆-Halogenalkyl; C₁₋₆-Halogenalkyl, das durch ein Mitglied aus der Gruppe bestehend aus OH und OCH₃ substituiert ist; OH; O-C₁₋₆-Alkyl; und C₃₋₆-Cycloalkyl ausgewählt ist; R^{f} aus der Gruppe bestehend aus H; C₁₋₆-Alkyl; C₁₋₆-Alkyl, das durch ein Mitglied aus der Gruppe bestehend aus OH, OCH₃, SCH₃ und OCF₃ substituiert ist; C₁₋₆-Halogenalkyl; und C₁₋₆-Halogenalkyl, das durch ein Mitglied aus der Gruppe bestehend aus OH und OCH₃ substituiert ist; ausgewählt ist; und
n für 1 oder 2 steht;
oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

2. Verbindung nach Anspruch 1, wobei:
(a) X für O steht; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon; oder
(b) X für S steht; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

3. Verbindung nach Anspruch 1, wobei:
(a) X für NR^{a} steht und R^{a} für H steht; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon;
(b) X für NR^{a} steht und R^{a} für CH₃ steht; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon; oder
(c) X für NR^{a} steht, wobei R^{a} und R^{1a} zusammen einen heterocyclischen Ring bilden, der aus: 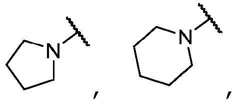 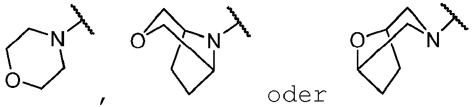 ausgewählt ist;
wobei der heterocyclische Ring durch ein oder zwei Glieder, die jeweils unabhängig aus der Gruppe bestehend aus C₁₋₄-Alkyl, C₁₋₄-Halogenalkyl und O-C₁₋₄-Alkyl ausgewählt sind, substituiert ist; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

4. Verbindung nach einem der Ansprüche 1-3, wobei:
(a) R^{1a} für C₁₋₄-Alkyl; C₁₋₄-Alkyl, das durch OH oder OCH₃ substituiert ist; C₁₋₄-Halogenalkyl; C₁₋₄-Halogenalkyl, das durch OH oder OCH₃ substituiert ist; oder C₃₋₆-Cycloalkyl steht; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon; oder
(b) R^{1a} für CF₃ steht; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

5. Verbindung nach einem der Ansprüche 1-4, wobei:
(a) R^{1b} für CH₃ oder CHF₂ steht; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon; oder
(b) R^{1b} für CH₃ steht; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

6. Verbindung nach einem der Ansprüche 1-3, wobei R^{1a} und R^{1b} zusammen Cyclopropyl, Cyclobutyl, Cyclopentyl, oder Cyclohexyl; Cyclopropyl, Cyclobutyl, Cyclopentyl oder Cyclohexyl, das jeweils unabhängig durch ein, zwei, drei oder vier Glieder, die jeweils aus der Gruppe bestehend aus Halogen, OH, C₁₋₄-Alkyl und C₁₋₄-Halogenalkyl substituiert ist; Oxetanyl; Tetrahydrofuranyl und Tetrahydropyranyl bilden; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

7. Verbindung nach einem der Ansprüche 1-6, wobei:
(a) R² für 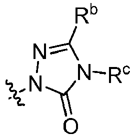 steht, wobei
R^{b} für C₁₋₆-Alkyl, das durch OH, Halogen, CN, O-C₁₋₄-Alkyl, O-C₁₋₄-Halogenalkyl oder O-C₃₋₆-Cycloalkyl substituiert ist, steht; und
R^{c} für C₁₋₄-Alkyl, C₁₋₄-Halogenalkyl, C₁₋₄-Alkyl, das durch OH, Allyl oder C₃₋₆-Cycloalkyl substituiert ist, steht; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon; oder
(b) R² für 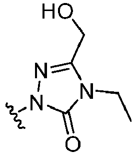 steht; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

8. Verbindung nach einem der Ansprüche 1-7, wobei:
(a) R³ für 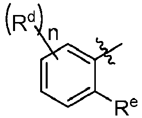 steht, wobei
R^{d} für H; Halogen; C₁₋₄-Alkyl; C₁₋₄-Alkyl, das durch OH, OCH₃, SCH₃ oder OCF₃ substituiert ist; C₁₋₄-Halogenalkyl; C₁₋₄-Halogenalkyl, das durch OH oder OCH₃ substituiert ist; CN oder OC₁₋₄-Alkyl steht;
R^{e} für Halogen; C₁₋₄-Alkyl; C₁₋₄-Alkyl, das durch OH, OCH₃, SCH₃ oder OCF₃ substituiert ist; C₁₋₄-Halogenalkyl oder C₁₋₄-Halogenalkyl, das durch OH oder OCH₃ substituiert ist, steht; und
n für 1 oder 2 steht;
oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon;
(b) R³ für 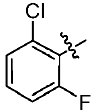 steht; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon;
(c) R³ für 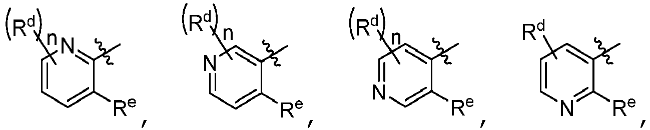 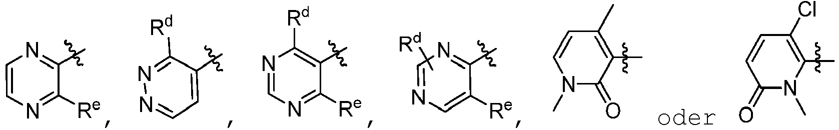 steht; wobei
R^{d} für H; Halogen; C₁₋₄-Alkyl; C₁₋₄-Alkyl, das durch OH, OCH₃, SCH₃ oder OCF₃ substituiert ist; C₁₋₄-Halogenalkyl; C₁₋₄-Halogenalkyl, das durch OH oder OCH₃ substituiert ist; N(CH₃)₂; OH; CN oder OC₁₋₄-Alkyl steht;
R^{e} für Halogen; OH; C₁₋₄-Alkyl; C₁₋₄-Alkyl, das durch OH, OCH₃, SCH₃ oder OCF₃ substituiert ist; C₁₋₄-Halogenalkyl; OC₁₋₄-Alkyl; oder C₁₋₄-Halogenalkyl, das durch OH oder OCH₃ substituiert ist; steht; und
n für 1 oder 2 steht;
oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon; oder
(d) R³ für 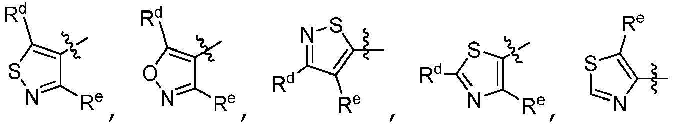 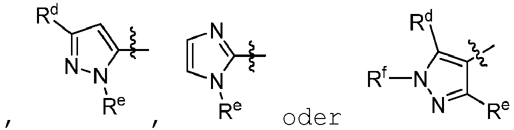 steht, wobei
R^{d} für H; Halogen; C₁₋₄-Alkyl; C₁₋₄-Alkyl, das durch OH, OCH₃, SCH₃ oder OCF₃ substituiert ist; C₁₋₄-Halogenalkyl; C₁₋₄-Halogenalkyl, das durch OH oder OCH₃ substituiert ist; oder OC₁₋₄-Alkyl steht;
R^{e} für Halogen; C₁₋₄-Alkyl; C₁₋₄-Alkyl, das durch OH, OCH₃, SCH₃ oder OCF₃ substituiert ist; C₁₋₄-Halogenalkyl; C₃₋₆-Cycloalkyl; oder C₁₋₄-Halogenalkyl, das durch OH oder OCH₃ substituiert ist; und
R^{f} für H; C₁₋₄-Alkyl; C₁₋₄-Alkyl, das durch OH, OCH₃, SCH₃ oder OCF₃ substituiert ist C₁₋₄-Halogenalkyl oder C₁₋₄-Halogenalkyl, das durch OH oder OCH₃ substituiert ist; steht;
oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

9. Verbindung nach Anspruch 1 mit der Struktur von:
(a) Formel (IA): 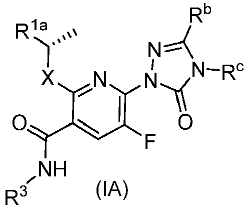 wobei
X für O steht;
R^{1a} aus der Gruppe bestehend aus C₁₋₆-Alkyl; C₁₋₆-Alkyl, das durch OH oder OCH₃ substituiert ist; C₁₋₆-Halogenalkyl; C₁₋₆-Halogenalkyl, das durch OH oder OCH₃ substituiert ist; und C₃₋₆-Cycloalkyl ausgewählt ist;
R^{b} für C₁₋₆-Alkyl, das durch ein Mitglied aus der Gruppe bestehend aus OH, Halogen, CN, O-C₁₋₆-Alkyl, O-C₁₋₆-Halogenalkyl und O-C₃₋₆-Cycloalkyl substituiert ist, steht;
R^{c} aus der Gruppe bestehend aus C₁₋₆-Alkyl, C₁₋₆-Alkyl, das durch OH substituiert ist, Allyl, C₁₋₆-Halogenalkyl und C₃₋₆-Cycloalkyl ausgewählt ist; und
R³ aus der Gruppe bestehend aus 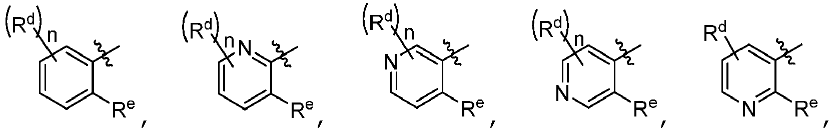 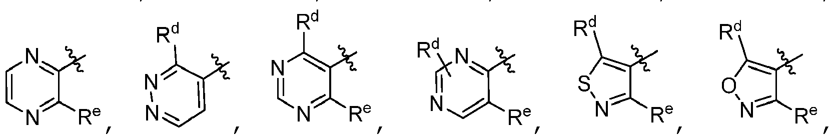 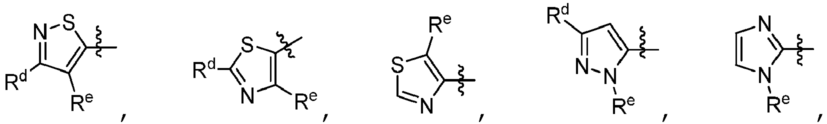 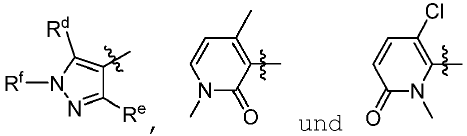 ausgewählt ist;
R^{d} aus der Gruppe bestehend aus H; Halogen; C₁₋₆-Alkyl; C₁₋₆-Alkyl, das durch ein Mitglied aus der Gruppe bestehend aus OH, OCH₃, SCH₃ und OCF₃ substituiert ist; C₁₋₆-Halogenalkyl; C₁₋₆-Halogenalkyl, das durch ein Mitglied aus der Gruppe bestehend aus OH und OCH₃ substituiert ist; N(CH₃)₂; OH; CN und O-C₁₋₆-Alkyl ausgewählt ist;
R^{e} aus der Gruppe bestehend aus Halogen; C₁₋₆-Alkyl; C₁₋₆-Alkyl, das durch ein Mitglied aus der Gruppe bestehend aus OH, OCH₃, SCH₃ und OCF₃ substituiert ist; C₁₋₆-Halogenalkyl; C₁₋₆-Halogenalkyl, das durch ein Mitglied aus der Gruppe bestehend aus OH und OCH₃ substituiert ist; OH; O-C₁₋₆-Alkyl; und C₃₋₆-Cycloalkyl ausgewählt ist; R^{f} aus der Gruppe bestehend aus H; C₁₋₆-Alkyl; C₁₋₆-Alkyl, das durch ein Mitglied aus der Gruppe bestehend aus OH, OCH₃, SCH₃ und OCF₃ substituiert ist; C₁₋₆-Halogenalkyl; und C₁₋₆-Halogenalkyl, das durch ein Mitglied aus der Gruppe bestehend aus OH und OCH₃ substituiert ist; ausgewählt ist; und
n für 1 oder 2 steht;
oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon;
beispielsweise wobei R^{1a} aus der Gruppe bestehend aus: C₁₋₄-Alkyl; C₁₋₄-Alkyl, das durch OH oder OCH₃ substituiert ist; C₁₋₄-Halogenalkyl; C₁₋₄-Halogenalkyl, das durch OH oder OCH₃ substituiert ist; oder C₃₋₆-Cycloalkyl ausgewählt ist;
(b) Formel (IB): 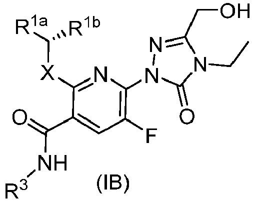 wobei
X aus der Gruppe bestehend aus O, S und NR^{a} ausgewählt ist;
dann, wenn X für NR^{a} steht, R^{a} für H oder CH₃ steht oder R^{a} und R^{1a} zusammen einen heterocyclischen Ring bilden, der aus der Gruppe bestehend aus 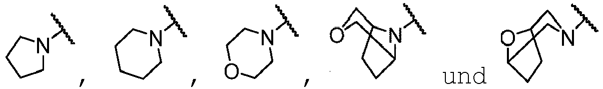 ausgewählt ist;
wobei der heterocyclische Ring durch ein oder zwei Glieder, die unabhängig aus der Gruppe bestehend aus C₁₋₆-Alkyl, C₁₋₆-Halogenalkyl und O-C₁₋₆-Alkyl ausgewählt sind, substituiert ist;
R^{1a} aus der Gruppe bestehend aus C₁₋₆-Alkyl; C₁₋₆-Alkyl, das durch OH oder OCH₃ substituiert ist; C₁₋₆-Halogenalkyl; C₁₋₆-Halogenalkyl, das durch OH oder OCH₃ substituiert ist; und C₃₋₆-Cycloalkyl ausgewählt ist;
R^{1b} für CH₃ oder CHF₂ steht; und
R³ aus der Gruppe bestehend aus 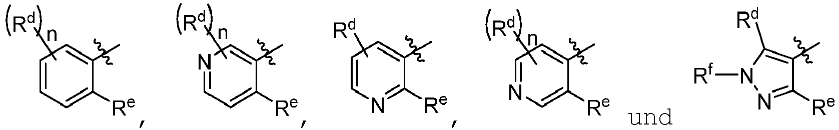 ausgewählt ist;
wobei
R^{d} aus der Gruppe bestehend aus H; Halogen; C₁₋₆-Alkyl; C₁₋₆-Alkyl, das durch ein Mitglied aus der Gruppe bestehend aus OH, OCH₃, SCH₃ und OCF₃ substituiert ist; C₁₋₆-Halogenalkyl; C₁₋₆-Halogenalkyl, das durch ein Mitglied aus der Gruppe bestehend aus OH und OCH₃ substituiert ist; N(CH₃)₂; OH; CN und O-C₁₋₆-Alkyl ausgewählt ist;
R^{e} aus der Gruppe bestehend aus Halogen; C₁₋₆-Alkyl; C₁₋₆-Alkyl, das durch ein Mitglied aus der Gruppe bestehend aus OH, OCH₃, SCH₃ und OCF₃ substituiert ist; C₁₋₆-Halogenalkyl; C₁₋₆-Halogenalkyl, das durch ein Mitglied aus der Gruppe bestehend aus OH und OCH₃ substituiert ist; OH; O-C₁₋₆-Alkyl; und C₃₋₆-Cycloalkyl ausgewählt ist; R^{f} aus der Gruppe bestehend aus H; C₁₋₆-Alkyl; C₁₋₆-Alkyl, das durch ein Mitglied aus der Gruppe bestehend aus OH, OCH₃, SCH₃ und OCF₃ substituiert ist; C₁₋₆-Halogenalkyl; und C₁₋₆-Halogenalkyl, das durch ein Mitglied aus der Gruppe bestehend aus OH und OCH₃ substituiert ist; ausgewählt ist; und
n für 1 oder 2 steht;
oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon; beispielsweise wobei R^{d} und R^{e} jeweils unabhängig für Halogen, C₁₋₄-Alkyl und O-C₁₋₄-Alkyl stehen; oder
(c) Formel (IC): 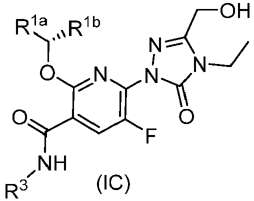
R^{1a} aus der Gruppe bestehend aus C₁₋₆-Alkyl; C₁₋₆-Alkyl, das durch OH oder OCH₃ substituiert ist; C₁₋₆-Halogenalkyl; C₁₋₆-Halogenalkyl, das durch OH oder OCH₃ substituiert ist; und C₃₋₆-Cycloalkyl ausgewählt ist;
R^{1b} für CH₃ oder CHF₂ steht; oder R^{1a} und R^{1b} zusammen C₃₋₆-Cycloalkyl; C₃₋₆-Cycloalkyl, das unabhängig durch ein, zwei, drei oder vier Glieder, die jeweils aus der Gruppe bestehend aus Halogen, OH, C₁₋₆-Alkyl und C₁₋₆-Halogenalkyl ausgewählt sind, substituiert ist; Oxetanyl; Tetrahydrofuranyl und Tetrahydropyranyl bilden; und
R³ aus der Gruppe bestehend aus 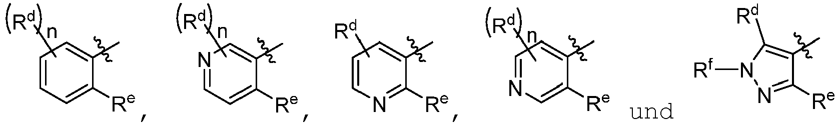 ausgewählt ist;
wobei
R^{d} aus der Gruppe bestehend aus H; Halogen; C₁₋₆-Alkyl; C₁₋₆-Alkyl, das durch ein Mitglied aus der Gruppe bestehend aus OH, OCH₃, SCH₃ und OCF₃ substituiert ist; C₁₋₆-Halogenalkyl; C₁₋₆-Halogenalkyl, das durch ein Mitglied aus der Gruppe bestehend aus OH und OCH₃ substituiert ist; N(CH₃)₂; OH; CN und O-C₁₋₆-Alkyl ausgewählt ist;
R^{e} aus der Gruppe bestehend aus Halogen; C₁₋₆-Alkyl; C₁₋₆-Alkyl, das durch ein Mitglied aus der Gruppe bestehend aus OH, OCH₃, SCH₃ und OCF₃ substituiert ist; C₁₋₆-Halogenalkyl; C₁₋₆-Halogenalkyl, das durch ein Mitglied aus der Gruppe bestehend aus OH und OCH₃ substituiert ist; OH; O-C₁₋₆-Alkyl; und C₃₋₆-Cycloalkyl ausgewählt ist; R^{f} aus der Gruppe bestehend aus H; C₁₋₆-Alkyl; C₁₋₆-Alkyl, das durch ein Mitglied aus der Gruppe bestehend aus OH, OCH₃, SCH₃ und OCF₃ substituiert ist; C₁₋₆-Halogenalkyl; und C₁₋₆-Halogenalkyl, das durch ein Mitglied aus der Gruppe bestehend aus OH und OCH₃ substituiert ist; ausgewählt ist; und
n für 1 oder 2 steht;
oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon; beispielsweise wobei R^{1a} und R^{1b} zusammen C₃₋₆-Cycloalkyl; C₃₋₆-Cycloalkyl, das unabhängig durch ein, zwei, drei oder vier Glieder, die jeweils aus der Gruppe bestehend aus F, OH, C₁₋₆-Alkyl und C₁₋₆-Halogenalkyl ausgewählt sind, substituiert ist; Oxetanyl; Tetrahydrofuranyl oder Tetrahydropyranyl bilden.

10. Verbindung nach Anspruch 1, ausgewählt aus der Gruppe bestehend aus:
(S)-N-(2-Chlor-6-fluorphenyl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-fluorphenyl)-2-((1,1,1-trifluorpropan-2-yl)oxy)nicotinamid;
(S)-N-(2-Chlor-6-fluorphenyl)-6-(4-ethyl-3-(fluormethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlorpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlor-4-methylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlor-6-fluorphenyl)-6-(3-(chlormethyl)-4-ethyl-5-oxo-4,5-dihydro-1H-1,2,4-triazol-1-yl)-5-fluor-2-((1,1,1-trifluorpropan-2-yl)oxy)nicotinamid;
N-(2-Chlor-6-fluorphenyl)-6-(4-ethyl-3-(hydroxymethyl)-5-oxo-4,5-dihydro-1H-1,2,4-triazol-1-yl)-5-fluor-2-isopropoxynicotinamid;
N-(2-Chlor-6-fluorphenyl)-6-(4-ethyl-3-(hydroxymethyl)-5-oxo-4,5-dihydro-1H-1,2,4-triazol-1-yl)-5-fluor-2-(2,2,3,3-tetrafluorcyclobutoxy)nicotinamid;
racemischem N-(2-Chlor-6-fluorphenyl)-6-(4-ethyl-3-(hydroxymethyl)-5-oxo-4,5-dihydro-1H-1,2,4-triazol-1-yl)-5-fluor-2-((tetrahydrofuran-3-yl)oxy)nicotinamid;
(S)-N-(3-Chlorpyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-fluor-5-methylphenyl)-2-((1,1,1-trifluorpropan-2-yl)oxy)nicotinamid;
(S)-N-(2-(Difluormethyl)-4-methylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlor-6-fluorphenyl)-6-(4-ethyl-3-(methoxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlorpyridazin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-methylisothiazol-4-yl)-2-((1,1,1-trifluorpropan-2-yl)oxy)nicotinamid;
(S)-N-(2,4-Dimethylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlorpyrazin-2-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlor-6-methoxypyridin-2-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlor-2-methoxypyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(5-Chlor-3-methyl-1H-pyrazol-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(4-Chlor-3-methylisothiazol-5-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(4-Chlorpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlor-5-fluorpyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Cyano-5-fluorpyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlor-5-fluor-2-methoxypyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(4-Brom-3-methylisothiazol-5-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(5-methylthiazol-4-yl)-2-((1,1,1-trifluorpropan-2-yl)oxy)nicotinamid;
(S)-N-(5-Chlor-2-methoxypyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-methoxypyrazin-2-yl)-2-((1,1,1-trifluorpropan-2-yl)oxy)nicotinamid;
(S)-N-(3,5-Dimethylisothiazol-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-fluor-5-methylpyridin-3-yl)-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(5-fluor-2-methylpyridin-4-yl)-2-((1,1,1-trifluorpropan-2-yl)oxy)nicotinamid;
(S)-N-(4-Chlor-6-methylpyrimidin-5-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-fluor-6-methylpyridin-2-yl)-2-((1,1,1-trifluorpropan-2-yl)oxy)nicotinamid;
(S)-N-(3-Chlor-2-methoxy-5-methylpyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-methoxy-5-methylpyridin-3-yl)-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-fluor-4-methylpyridin-3-yl)-2-((1,1,1-trifluorpropan-2-yl)oxy)nicotinamid;
(S)-N-(5,6-Dimethylpyrimidin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlor-4-methoxypyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-methylisoxazol-4-yl)-2-((1,1,1-trifluorpropan-2-yl)oxy)nicotinamid;
(S)-N-(3,5-Dimethylisoxazol-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-fluor-5-methoxyphenyl)-2-((1,1,1-trifluorpropan-2-yl)oxy)nicotinamid;
(S)-N-(2-Chlor-5-methylphenyl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3,5-Dimethyl-1H-pyrazol-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(4-Chlor-6-methoxypyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2,5-Dimethylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(5-Chlor-2-methoxypyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
N-(1,4-Dimethyl-2-oxo-2,3-dihydro-114-pyridin-3-yl)-6-(4-ethyl-3-(hydroxymethyl)-5-oxo-4,5-dihydro-1H-1,2,4-triazol-1-yl)-5-fluor-2-(((S)-1,1,1-trifluorpropan-2-yl)oxy)nicotinamid;
(S)-N-(3-Chlorpyridin-2-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-2-((1,1,1-trifluorpropan-2-yl)oxy)-N-(1,3,5-trimethyl-1H-pyrazol-4-yl)nicotinamid;
(S)-6-(4-Ethyl-3-(hydroxymethyl)-5-oxo-4,5-dihydro-1H-1,2,4-triazol-1-yl)-5-fluor-N-(3-fluor-6-methoxypyridin-2-yl)-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-methyl-1H-pyrazol-4-yl)-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-methoxy-1H-pyrazol-4-yl)-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-isopropyl-1-methyl-1H-pyrazol-5-yl)-2-((1,1,1-trifluorpropan-2-yl)oxy)nicotinamid;
(S)-N-(5-Chlor-6-methylpyrimidin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-(methoxymethyl)-1-methyl-1H-pyrazol-4-yl)-2-((1,1,1-trifluorpropan-2-yl)oxy)nicotinamid;
(S)-N-(3-Cyclopropyl-1H-pyrazol-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlor-1-methyl-6-oxo-1,6-dihydropyridin-2-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(5-fluor-2-methoxypyridin-4-yl)-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-methoxy-5-methylpyridin-4-yl)-2-((1,1,1-trifluorpropan-2-yl)oxy)nicotinamid;
(S)-N-(2-Chlor-5-methylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-methoxy-4-methylpyridin-3-yl)-2-((1,1,1-trifluorpropan-2-yl)oxy)nicotinamid;
(S)-N-(2,4-Dichlorpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Ethoxy-5-fluorpyrimidin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-fluor-5-methylpyridin-4-yl)-2-((1,1,1-trifluorpropan-2-yl)oxy)nicotinamid;
(S)-N-(2-Chlor-4,6-dimethylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(1-Cyclopropyl-1H-imidazol-2-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlor-5-cyanophenyl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlor-5-cyanopyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(4-(Dimethylamino)-2-methoxypyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
N-(2-Chlor-6-methyl-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(2-Chlor-5-fluor-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(4-Chlor-2-methyl-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(3-Chlor-1-methyl-pyrazol-4-yl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-[2-(Difluormethyl)-5-fluor-4-pyridyl]-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-(5-fluor-2-methoxy-pyrimidin-4-yl)-2-[(1S)-2,2,2-trifluor-1-methylethoxy]pyridin-3-carboxamid;
N-(6-Chlor-2,3-dimethyl-phenyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(2-Chlor-4-methyl-3-pyridyl)-5-fluor-6-[3-(hydroxymethyl)-5-oxo-4-propyl-1,2,4-triazol-1-yl]-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-(2-methoxy-3,5-dimethyl-4-pyridyl)-2-[(1S)-2,2,2-trifluor-1-methylethoxy]pyridin-3-carboxamid;
(S)-2-(5-(((2-Chlor-4-methylpyridin-3-yl)-12-azanyl)carbonyl)-3-fluor-6-((1,1,1-trifluorpropan-2-yl)oxy)pyridin-2-yl)-5-(fluormethyl)-4-isopropyl-2,4-dihydro-3H-1,2,4-triazol-3-on;
N-(2-Chlor-4-methyl-3-pyridyl)-6-[4-ethyl-3-(fluormethyl)-5-oxo-1,2,4-triazol-1-yl]-5-fluor-2-[(1S)-2,2,2-trifluor-1-methylethoxy]pyridin-3-carboxamid;
N-(3-Chlor-2-methoxy-5-methyl-4-pyridyl)-6-[4-ethyl-3-(hydroxymethyl)-5-oxo-1,2,4-triazol-1-yl]-5-fluor-2-isopropoxypyridin-3-carboxamid;
N-(2-Chlor-4-methyl-3-pyridyl)-5-fluor-6-[3-(hydroxymethyl)-4-isopropyl-5-oxo-1,2,4-triazol-1-yl]-2-[(1S)-2,2,2-trifluor-1-methylethoxy]pyridin-3-carboxamid;
N-(2-Chlor-4-methyl-3-pyridyl)-6-[4-(2,2-difluorethyl)-3-(hydroxymethyl)-5-oxo-1,2,4-triazol-1-yl]-5-fluor-2-[(1S)-2,2,2-trifluor-1-methylethoxy]pyridin-3-carboxamid;
N-(5-Chlor-3-methyl-1H-pyrazol-4-yl)-5-fluor-6-[3-(hydroxymethyl)-5-oxo-4-propyl-1,2,4-triazol-1-yl]-2-[(1S)-2,2,2-trifluor-1-methylethoxy]pyridin-3-carboxamid;
N-(2,4-Dimethoxy-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(4,6-Dimethoxypyrimidin-5-yl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-2-[(1S)-2,2,2-trifluor-1-methylethoxy]-N-[3-(trifluormethyl)-1H-pyrazol-4-yl]pyridin-3-carboxamid;
N-(5-Chlor-3-methyl-1H-pyrazol-4-yl)-6-[4-ethyl-3-(fluormethyl)-5-oxo-1,2,4-triazol-1-yl]-5-fluor-2-[(1S)-2,2,2-trifluor-1-methylethoxy]pyridin-3-carboxamid;
N-(2-Chlor-4-methyl-3-pyridyl)-6-[4-ethyl-3-(fluormethyl)-5-oxo-1,2,4-triazol-1-yl]-5-fluor-2-isopropoxypyridin-3-carboxamid;
N-(5-Chlor-2-methoxy-4-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(5-Chlor-3-methyl-1H-pyrazol-4-yl)-5-fluor-6-[3-(fluormethyl)-5-oxo-4-propyl-1,2,4-triazol-1-yl]-2-[(1S)-2,2,2-trifluor-1-methylethoxy]pyridin-3-carboxamid;
N-(3-Cyano-1H-pyrazol-4-yl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(2-Chlor-4-methyl-3-pyridyl)-5-fluor-6-[3-(fluormethyl)-4-(3-hydroxypropyl)-5-oxo-1,2,4-triazol-1-yl]-2-[(1S)-2,2,2-trifluor-1-methylethoxy]pyridin-3-carboxamid;
N-(2-Chlor-4,5-dimethyl-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-(4-methoxy-2,6-dimethyl-3-pyridyl)-2-[(1S)-2,2,2-trifluor-1-methylethoxy]pyridin-3-carboxamid;
N-(3-Chlor-4-methoxy-2-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(3-Chlor-4-ethoxy-2-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(2-Chlor-6-methoxy-4-methyl-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(3-Chlor-5-methyl-1H-pyrazol-4-yl)-5-fluor-6-[3-(fluormethyl)-4-(3-hydroxypropyl)-5-oxo-1,2,4-triazol-1-yl]-2-[(1S)-2,2,2-trifluor-1-methylethoxy]pyridin-3-carboxamid;
N-(5-Chlor-3-methyl-1H-pyrazol-4-yl)-6-[4-ethyl-3-(fluormethyl)-5-oxo-1,2,4-triazol-1-yl]-5-fluor-2-isopropoxypyridin-3-carboxamid;
6-[4-Ethyl-3-(hydroxymethyl)-5-oxo-1,2,4-triazol-1-yl]-5-fluor-N-(3-methoxypyridazin-4-yl)-2-[(1S)-2,2,2-trifluor-1-methylethoxy]pyridin-3-carboxamid;
N-(5-Chlor-2-methoxy-3-methyl-4-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-[3-methyl-5-(trifluormethyl)-1H-pyrazol-4-yl]-2-[(1S)-2,2,2-trifluor-1-methylethoxy]pyridin-3-carboxamid;
N-[3-Chlor-5-(trifluormethyl)-1H-pyrazol-4-yl]-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(6-Chlor-2-methoxy-4-methyl-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-(2-methoxy-4,6-dimethyl-3-pyridyl)-2-[(1S)-2,2,2-trifluor-1-methylethoxy]pyridin-3-carboxamid;
N-(5-Chlor-3-methyl-1H-pyrazol-4-yl)-5-fluor-6-[3-(fluormethyl)-4-(2-hydroxyethyl)-5-oxo-1,2,4-triazol-1-yl]-2-[(1S)-2,2,2-trifluor-1-methylethoxy]pyridin-3-carboxamid;
N-(2-Chlor-4-methyl-3-pyridyl)-5-fluor-6-[3-(fluormethyl)-4-(2-hydroxyethyl)-5-oxo-1,2,4-triazol-1-yl]-2-[(1S)-2,2,2-trifluor-1-methylethoxy]pyridin-3-carboxamid;
N-(3-Cyano-5-fluor-2-methoxy-4-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(3-Chlor-5-methoxy-2-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(2,6-Dimethoxyphenyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-(o-tolyl)-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-(3-methoxy-5-methyl-1H-pyrazol-4-yl)-2-[(1S)-2,2,2-trifluor-1-methylethoxy]pyridin-3-carboxamid;
N-(3,5-Dimethoxy-4-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(3,5-Dichlor-1H-pyrazol-4-yl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-[3-(Difluormethyl)-5-methyl-1H-pyrazol-4-yl]-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-(4-fluor-2-methoxy-3-pyridyl)-2-[(1S)-2,2,2-trifluor-1-methylethoxy]pyridin-3-carboxamid;
N-(4-Chlor-2-methoxy-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
(racemischem) 6-[4-Ethyl-3-(1-hydroxyethyl)-5-oxo-1,2,4-triazol-1-yl]-5-fluor-N-[3-methyl-5-(trifluormethyl)-1H-pyrazol-4-yl]-2-(2,2,2-trifluor-1-methylethoxy)pyridin-3-carboxamid und
6-[4-Ethyl-3-(1-hydroxy-1-methyl-ethyl)-5-oxo-1,2,4-triazol-1-yl]-5-fluor-N-[3-methyl-5-(trifluormethyl)-1H-pyrazol-4-yl]-2-[(1S)-2,2,2-trifluor-1-methylethoxy]pyridin-3-carboxamid;
oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

11. Verbindung nach Anspruch 1, ausgewählt aus der Gruppe bestehend aus:
(S)-N-(2-Chlor-4-methylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(5-Chlor-3-methyl-1H-pyrazol-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlor-2-methoxy-5-methylpyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-methoxy-4-methylpyridin-3-yl)-2-((1,1,1-trifluorpropan-2-yl)oxy)nicotinamid;
6-[4-Ethyl-3-(hydroxymethyl)-5-oxo-1,2,4-triazol-1-yl]-5-fluor-N-[3-methyl-5-(trifluormethyl)-1H-pyrazol-4-yl]-2-[(1S)-2,2,2-trifluor-1-methylethoxy]pyridin-3-carboxamid und
6-[4-Ethyl-3-(hydroxymethyl)-5-oxo-1,2,4-triazol-1-yl]-5-fluor-N-(2-methoxy-3,5-dimethyl-4-pyridyl)-2-[(1S)-2,2,2-trifluor-1-methylethoxy]pyridin-3-carboxamid;
oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

12. Pharmazeutische Zusammensetzung, umfassend: (A) eine wirksame Menge einer Verbindung nach einem der Ansprüche 1-11 oder eines pharmazeutisch unbedenklichen Salzes, eines pharmazeutisch unbedenklichen Solvats, eines pharmazeutisch unbedenklichen Stereoisomers, eines pharmazeutisch unbedenklichen Tautomers, einer pharmazeutisch unbedenklichen Isotopenvariante oder eines pharmazeutisch unbedenklichen N-Oxids davon; und (B) mindestens einen pharmazeutisch unbedenklichen Hilfsstoff.

13. Mindestens eine Verbindung oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon nach einem der Ansprüche 1-11 zur Verwendung bei der Behandlung eines Individuums, das an einer Erkrankung, einer Störung oder einem medizinischen Leiden leidet oder damit diagnostiziert ist, umfassend das Inhibierend oder Ändern von Dihydroorotatoxygenaseenzymaktivität bei dem Individuum, wobei es sich bei der Erkrankung, der Störung oder dem medizinischen Leiden um eine entzündliche Störung, eine Autoimmunstörung oder Krebs handelt.

14. Mindestens eine Verbindung oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon zur Verwendung nach Anspruch 13, wobei die Störung, die Erkrankung bzw. das medizinische Leiden aus der Gruppe bestehend aus Lymphomen, Leukämien, Karzinomen und Sarkomen ausgewählt ist.

15. Mindestens eine Verbindung oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon zur Verwendung nach Anspruch 13, wobei die Störung, die Erkrankung bzw. das medizinische Leiden aus der Gruppe bestehend aus akuter lymphoblastischer Leukämie, akuter myeloischer Leukämie, (akuter) T-Zell-Leukämie, akuter lymphoblastischer Leukämie, akuter lymphatischer Leukämie, akuter Monozytenleukämie, akuter Promyeolozytenleukämie, bisphänotypischer B-Myelomonozytenleukämie, chronischer lymphatischer Leukämie, chronischer myelogener Leukämie, chronischer myeloischer Leukämie, chronischer Myelomonozytenleukämie, großkörniger lymphatischer Leukämie, Plasmazellleukämie sowie myelodysplastischem Syndrom, das sich zu einer akuten myeloischen Leukämie entwickeln kann, ausgewählt ist.

16. Mindestens eine Verbindung oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon zur Verwendung nach Anspruch 13, wobei es sich bei der Störung, der Erkrankung bzw. dem medizinischen Leiden um akute myeloische Leukämie handelt.

17. Mindestens eine Verbindung oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon zur Verwendung nach einem der Ansprüche 13-16, wobei die mindestens eine Verbindung eine Verbindung umfasst, die ausgewählt ist aus der Gruppe bestehend aus:
(S)-N-(2-Chlor-6-fluorphenyl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-fluorphenyl)-2-((1,1,1-trifluorpropan-2-yl)oxy)nicotinamid;
(S)-N-(2-Chlor-6-fluorphenyl)-6-(4-ethyl-3-(fluormethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlorpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlor-4-methylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlor-6-fluorphenyl)-6-(3-(chlormethyl)-4-ethyl-5-oxo-4,5-dihydro-1H-1,2,4-triazol-1-yl)-5-fluor-2-((1,1,1-trifluorpropan-2-yl)oxy)nicotinamid;
N-(2-Chlor-6-fluorphenyl)-6-(4-ethyl-3-(hydroxymethyl)-5-oxo-4,5-dihydro-1H-1,2,4-triazol-1-yl)-5-fluor-2-isopropoxynicotinamid;
N-(2-Chlor-6-fluorphenyl)-6-(4-ethyl-3-(hydroxymethyl)-5-oxo-4,5-dihydro-1H-1,2,4-triazol-1-yl)-5-fluor-2-(2,2,3,3-tetrafluorcyclobutoxy)nicotinamid;
racemischem N-(2-Chlor-6-fluorphenyl)-6-(4-ethyl-3-(hydroxymethyl)-5-oxo-4,5-dihydro-1H-1,2,4-triazol-1-yl)-5-fluor-2-((tetrahydrofuran-3-yl)oxy)nicotinamid;
(S)-N-(3-Chlorpyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-fluor-5-methylphenyl)-2-((1,1,1-trifluorpropan-2-yl)oxy)nicotinamid;
(S)-N-(2-(Difluormethyl)-4-methylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlor-6-fluorphenyl)-6-(4-ethyl-3-(methoxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlorpyridazin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-methylisothiazol-4-yl)-2-((1,1,1-trifluorpropan-2-yl)oxy)nicotinamid;
(S)-N-(2,4-Dimethylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlorpyrazin-2-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlor-6-methoxypyridin-2-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlor-2-methoxypyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(5-Chlor-3-methyl-1H-pyrazol-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(4-Chlor-3-methylisothiazol-5-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(4-Chlorpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlor-5-fluorpyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Cyano-5-fluorpyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlor-5-fluor-2-methoxypyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(4-Brom-3-methylisothiazol-5-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(5-methylthiazol-4-yl)-2-((1,1,1-trifluorpropan-2-yl)oxy)nicotinamid;
(S)-N-(5-Chlor-2-methoxypyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-methoxypyrazin-2-yl)-2-((1,1,1-trifluorpropan-2-yl)oxy)nicotinamid;
(S)-N-(3,5-Dimethylisothiazol-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-fluor-5-methylpyridin-3-yl)-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(5-fluor-2-methylpyridin-4-yl)-2-((1,1,1-trifluorpropan-2-yl)oxy)nicotinamid;
(S)-N-(4-Chlor-6-methylpyrimidin-5-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-fluor-6-methylpyridin-2-yl)-2-((1,1,1-trifluorpropan-2-yl)oxy)nicotinamid;
(S)-N-(3-Chlor-2-methoxy-5-methylpyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-methoxy-5-methylpyridin-3-yl)-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-fluor-4-methylpyridin-3-yl)-2-((1,1,1-trifluorpropan-2-yl)oxy)nicotinamid;
(S)-N-(5,6-Dimethylpyrimidin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlor-4-methoxypyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-methylisoxazol-4-yl)-2-((1,1,1-trifluorpropan-2-yl)oxy)nicotinamid;
(S)-N-(3,5-Dimethylisoxazol-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-fluor-5-methoxyphenyl)-2-((1,1,1-trifluorpropan-2-yl)oxy)nicotinamid;
(S)-N-(2-Chlor-5-methylphenyl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3,5-Dimethyl-1H-pyrazol-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(4-Chlor-6-methoxypyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2,5-Dimethylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(5-Chlor-2-methoxypyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
N-(1,4-Dimethyl-2-oxo-2,3-dihydro-1l4-pyridin-3-yl)-6-(4-ethyl-3-(hydroxymethyl)-5-oxo-4,5-dihydro-1H-1,2,4-triazol-1-yl)-5-fluor-2-(((S)-1,1,1-trifluorpropan-2-yl)oxy)nicotinamid;
(S)-N-(3-Chlorpyridin-2-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-2-((1,1,1-trifluorpropan-2-yl)oxy)-N-(1,3,5-trimethyl-1H-pyrazol-4-yl)nicotinamid;
(S)-6-(4-Ethyl-3-(hydroxymethyl)-5-oxo-4,5-dihydro-1H-1,2,4-triazol-1-yl)-5-fluor-N-(3-fluor-6-methoxypyridin-2-yl)-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-methyl-1H-pyrazol-4-yl)-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-methoxy-1H-pyrazol-4-yl)-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-isopropyl-1-methyl-1H-pyrazol-5-yl)-2-((1,1,1-trifluorpropan-2-yl)oxy)nicotinamid;
(S)-N-(5-Chlor-6-methylpyrimidin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-(methoxymethyl)-1-methyl-1H-pyrazol-4-yl)-2-((1,1,1-trifluorpropan-2-yl)oxy)nicotinamid;
(S)-N-(3-Cyclopropyl-1H-pyrazol-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlor-1-methyl-6-oxo-1,6-dihydropyridin-2-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(5-fluor-2-methoxypyridin-4-yl)-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-methoxy-5-methylpyridin-4-yl)-2-((1,1,1-trifluorpropan-2-yl)oxy)nicotinamid;
(S)-N-(2-Chlor-5-methylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-methoxy-4-methylpyridin-3-yl)-2-((1,1,1-trifluorpropan-2-yl)oxy)nicotinamid;
(S)-N-(2,4-Dichlorpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Ethoxy-5-fluorpyrimidin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(3-fluor-5-methylpyridin-4-yl)-2-((1,1,1-trifluorpropan-2-yl)oxy)nicotinamid;
(S)-N-(2-Chlor-4,6-dimethylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(1-Cyclopropyl-1H-imidazol-2-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlor-5-cyanophenyl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(2-Chlor-5-cyanopyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(4-(Dimethylamino)-2-methoxypyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
N-(2-Chlor-6-methyl-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(2-Chlor-5-fluor-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(4-Chlor-2-methyl-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(3-Chlor-1-methyl-pyrazol-4-yl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-[2-(Difluormethyl)-5-fluor-4-pyridyl]-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-(5-fluor-2-methoxy-pyrimidin-4-yl)-2-[(1S)-2,2,2-trifluor-1-methylethoxy]pyridin-3-carboxamid;
N-(6-Chlor-2,3-dimethyl-phenyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(2-Chlor-4-methyl-3-pyridyl)-5-fluor-6-[3-(hydroxymethyl)-5-oxo-4-propyl-1,2,4-triazol-1-yl]-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-(2-methoxy-3,5-dimethyl-4-pyridyl)-2-[(1S)-2,2,2-trifluor-1-methylethoxy]pyridin-3-carboxamid;
(S)-2-(5-(((2-Chlor-4-methylpyridin-3-yl)-12-azanyl)carbonyl)-3-fluor-6-((1,1,1-trifluorpropan-2-yl)oxy)pyridin-2-yl)-5-(fluormethyl)-4-isopropyl-2,4-dihydro-3H-1,2,4-triazol-3-on;
N-(2-Chlor-4-methyl-3-pyridyl)-6-[4-ethyl-3-(fluormethyl)-5-oxo-1,2,4-triazol-1-yl]-5-fluor-2-[(1S)-2,2,2-trifluor-1-methylethoxy]pyridin-3-carboxamid;
N-(3-Chlor-2-methoxy-5-methyl-4-pyridyl)-6-[4-ethyl-3-(hydroxymethyl)-5-oxo-1,2,4-triazol-1-yl]-5-fluor-2-isopropoxypyridin-3-carboxamid;
N-(2-Chlor-4-methyl-3-pyridyl)-5-fluor-6-[3-(hydroxymethyl)-4-isopropyl-5-oxo-1,2,4-triazol-1-yl]-2-[(1S)-2,2,2-trifluor-1-methylethoxy]pyridin-3-carboxamid;
N-(2-Chlor-4-methyl-3-pyridyl)-6-[4-(2,2-difluorethyl)-3-(hydroxymethyl)-5-oxo-1,2,4-triazol-1-yl]-5-fluor-2-[(1S)-2,2,2-trifluor-1-methylethoxy]pyridin-3-carboxamid;
N-(5-Chlor-3-methyl-1H-pyrazol-4-yl)-5-fluor-6-[3-(hydroxymethyl)-5-oxo-4-propyl-1,2,4-triazol-1-yl]-2-[(1S)-2,2,2-trifluor-1-methylethoxy]pyridin-3-carboxamid;
N-(2,4-Dimethoxy-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(4,6-Dimethoxypyrimidin-5-yl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-2-[(1S)-2,2,2-trifluor-1-methylethoxy]-N-[3-(trifluormethyl)-1H-pyrazol-4-yl]pyridin-3-carboxamid;
N-(5-Chlor-3-methyl-1H-pyrazol-4-yl)-6-[4-ethyl-3-(fluormethyl)-5-oxo-1,2,4-triazol-1-yl]-5-fluor-2-[(1S)-2,2,2-trifluor-1-methylethoxy]pyridin-3-carboxamid;
N-(2-Chlor-4-methyl-3-pyridyl)-6-[4-ethyl-3-(fluormethyl)-5-oxo-1,2,4-triazol-1-yl]-5-fluor-2-isopropoxypyridin-3-carboxamid;
N-(5-Chlor-2-methoxy-4-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(5-Chlor-3-methyl-1H-pyrazol-4-yl)-5-fluor-6-[3-(fluormethyl)-5-oxo-4-propyl-1,2,4-triazol-1-yl]-2-[(1S)-2,2,2-trifluor-1-methylethoxy]pyridin-3-carboxamid;
N-(3-Cyano-1H-pyrazol-4-yl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(2-Chlor-4-methyl-3-pyridyl)-5-fluor-6-[3-(fluormethyl)-4-(3-hydroxypropyl)-5-oxo-1,2,4-triazol-1-yl]-2-[(1S)-2,2,2-trifluor-1-methylethoxy]pyridin-3-carboxamid;
N-(2-Chlor-4,5-dimethyl-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-ethyl-3-(hydroxymethyl)-5-oxo-1,2,4-triazol-1-yl]-5-fluor-N-(4-methoxy-2,6-dimethyl-3-pyridyl)-2-[(1S)-2,2,2-trifluor-1-methylethoxy]pyridin-3-carboxamid;
N-(3-Chlor-4-methoxy-2-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(3-Chlor-4-ethoxy-2-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(2-Chlor-6-methoxy-4-methyl-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(3-Chlor-5-methyl-1H-pyrazol-4-yl)-5-fluor-6-[3-(fluormethyl)-4-(3-hydroxypropyl)-5-oxo-1,2,4-triazol-1-yl]-2-[(1S)-2,2,2-trifluor-1-methylethoxy]pyridin-3-carboxamid;
N-(5-Chlor-3-methyl-1H-pyrazol-4-yl)-6-[4-ethyl-3-(fluormethyl)-5-oxo-1,2,4-triazol-1-yl]-5-fluor-2-isopropoxypyridin-3-carboxamid;
6-[4-Ethyl-3-(hydroxymethyl)-5-oxo-1,2,4-triazol-1-yl]-5-fluor-N-(3-methoxypyridazin-4-yl)-2-[(1S)-2,2,2-trifluor-1-methylethoxy]pyridin-3-carboxamid;
N-(5-Chlor-2-methoxy-3-methyl-4-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-[3-methyl-5-(trifluormethyl)-1H-pyrazol-4-yl]-2-[(1S)-2,2,2-trifluor-1-methylethoxy]pyridin-3-carboxamid;
N-[3-Chlor-5-(trifluormethyl)-1H-pyrazol-4-yl]-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(6-Chlor-2-methoxy-4-methyl-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-(2-methoxy-4,6-dimethyl-3-pyridyl)-2-[(1S)-2,2,2-trifluor-1-methylethoxy]pyridin-3-carboxamid;
N-(5-Chlor-3-methyl-1H-pyrazol-4-yl)-5-fluor-6-[3-(fluormethyl)-4-(2-hydroxyethyl)-5-oxo-1,2,4-triazol-1-yl]-2-[(1S)-2,2,2-trifluor-1-methylethoxy]pyridin-3-carboxamid;
N-(2-Chlor-4-methyl-3-pyridyl)-5-fluor-6-[3-(fluormethyl)-4-(2-hydroxyethyl)-5-oxo-1,2,4-triazol-1-yl]-2-[(1S)-2,2,2-trifluor-1-methylethoxy]pyridin-3-carboxamid;
N-(3-Cyano-5-fluor-2-methoxy-4-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(3-Chlor-5-methoxy-2-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(2,6-Dimethoxyphenyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-(o-tolyl)-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-(3-methoxy-5-methyl-1H-pyrazol-4-yl)-2-[(1S)-2,2,2-trifluor-1-methylethoxy]pyridin-3-carboxamid;
N-(3,5-Dimethoxy-4-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-(3,5-Dichlor-1H-pyrazol-4-yl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
N-[3-(Difluormethyl)-5-methyl-1H-pyrazol-4-yl]-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
6-[4-Ethyl-3-(hydroxymethyl)-5-oxo-1,2,4-triazol-1-yl]-5-fluor-N-(4-fluor-2-methoxy-3-pyridyl)-2-[(1S)-2,2,2-trifluor-1-methylethoxy]pyridin-3-carboxamid;
N-(4-Chlor-2-methoxy-3-pyridyl)-6-[4-ethyl-3-(hydroxymethyl)-5-oxo-1,2,4-triazol-1-yl]-5-fluor-2-[(1S)-2,2,2-trifluor-1-methylethoxy]pyridin-3-carboxamid;
(racemischem) 6-[4-Ethyl-3-(1-hydroxyethyl)-5-oxo-1,2,4-triazol-1-yl]-5-fluor-N-[3-methyl-5-(trifluormethyl)-1H-pyrazol-4-yl]-2-(2,2,2-trifluor-1-methylethoxy)pyridin-3-carboxamid und
6-[4-Ethyl-3-(1-hydroxy-1-methyl-ethyl)-5-oxo-1,2,4-triazol-1-yl]-5-fluor-N-[3-methyl-5-(trifluormethyl)-1H-pyrazol-4-yl]-2-[(1S)-2,2,2-trifluor-1-methylethoxy]pyridin-3-carboxamid;
oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

18. Mindestens eine Verbindung oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon zur Verwendung nach einem der Ansprüche 13-16, wobei die mindestens eine Verbindung eine Verbindung umfasst, die ausgewählt ist aus der Gruppe bestehend aus:
(S)-N-(2-Chlor-4-methylpyridin-3-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(5-Chlor-3-methyl-1H-pyrazol-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-N-(3-Chlor-2-methoxy-5-methylpyridin-4-yl)-6-(4-ethyl-3-(hydroxymethyl)-5-oxo-4,5-dihydro-1H-1,2,4-triazol-1-yl)-5-fluor-2-((1,1,1-trifluorpropan-2-yl)oxy)nicotinamid;
(S)-6-(4-Ethyl-3-(hydroxymethyl)-5-oxo-4,5-dihydro-1H-1,2,4-triazol-1-yl)-5-fluor-N-(2-methoxy-4-methylpyridin-3-yl)-2-((1,1,1-trifluorpropan-2-yl)oxy)nicotinamid;
6-[4-Ethyl-3-(hydroxymethyl)-5-oxo-1,2,4-triazol-1-yl]-5-fluor-N-[3-methyl-5-(trifluormethyl)-1H-pyrazol-4-yl]-2-[(1S)-2,2,2-trifluor-1-methylethoxy]pyridin-3-carboxamid; und
6-[4-Ethyl-3-(hydroxymethyl)-5-oxo-1,2,4-triazol-1-yl]-5-fluor-N-(2-methoxy-3,5-dimethyl-4-pyridyl)-2-[(1S)-2,2,2-trifluor-1-methylethoxy]pyridin-3-carboxamid;
oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

19. Verbindung nach Anspruch 1, ausgewählt aus: 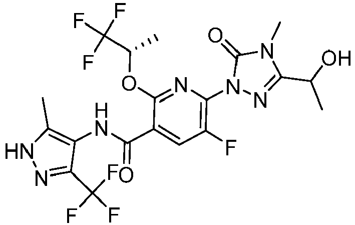 und 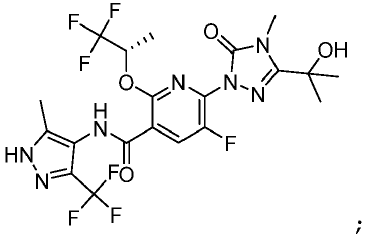 oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

20. Verbindung nach einem der Ansprüche 1-6, wobei R² für 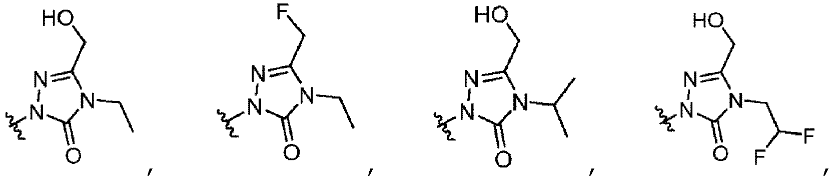 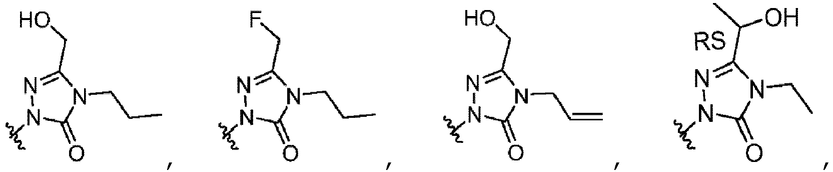 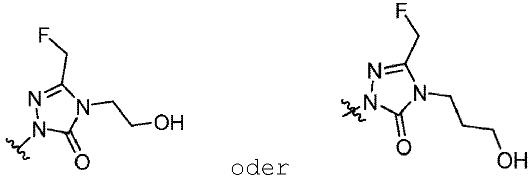 steht; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

21. Verbindung nach einem der Ansprüche 1-3, wobei R^{1a} für CH₃ steht; oder ein pharmazeutisch unbedenkliches Salz, ein pharmazeutisch unbedenkliches Solvat, ein pharmazeutisch unbedenkliches Stereoisomer, ein pharmazeutisch unbedenkliches Tautomer, eine pharmazeutisch unbedenkliche Isotopenvariante oder ein pharmazeutisch unbedenkliches N-Oxid davon.

## Revendications

1. Composé ayant la structure de formule (I) : 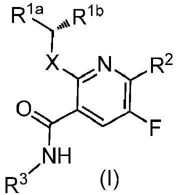
X étant choisi dans le groupe constitué par : O, S et NR^{a} ;
lorsque X est NR^{a} ; R^{a} est H ou CH₃ ; ou R^{a} et R^{1a} se joignent pour former un cycle hétérocyclique choisi dans le groupe constitué par : 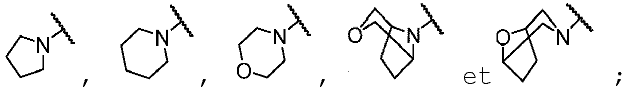 le cycle hétérocyclique étant substitué par un ou deux membres chacun indépendamment choisi dans le groupe constitué par : C₁₋₆alkyle, C₁₋₆halogénoalkyle et OC₁₋₆alkyle ;
R^{1a} étant choisi dans le groupe constitué par : C₁₋₆alkyle ; C₁₋₆alkyle substitué par OH ou OCH₃ ; C₁₋₆halogénoalkyle ; C₁₋₆halogénoalkyle substitué par OH ou OCH₃ ; et C₃₋₆cycloalkyle ;
R^{1b} étant CH₃ ou CHF₂ ; ou R^{1a} et R^{1b} se joignant pour former C₃₋₆cycloalkyle ; C₃₋₆cycloalkyle indépendamment substitué par un, deux, trois ou quatre membres chacun indépendamment choisi dans le groupe constitué par : halogéno, OH, C₁₋₆alkyle et C₁₋₆halogénoalkyle ; oxétanyle ; tétrahydrofuranyle ; et tétrahydropyranyle ;
R² étant 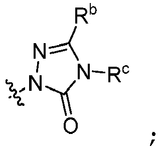
R^{b} étant C₁₋₆alkyle substitué par un membre choisi dans le groupe constitué par : OH, halogéno, CN, OC₁₋₆alkyle, OC₁₋₆halogénoalkyle et OC₃₋₆cycloalkyle ;
R^{c} étant choisi dans le groupe constitué par : C₁₋₆alkyle, C₁₋₆alkyle substitué par OH, allyle, C₁₋₆halogénoalkyle et C₃₋₆cycloalkyle ; et
R³ étant choisi dans le groupe constitué par : 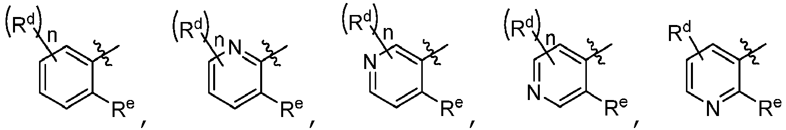 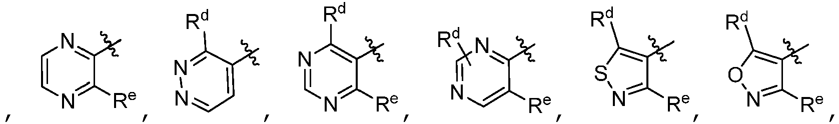 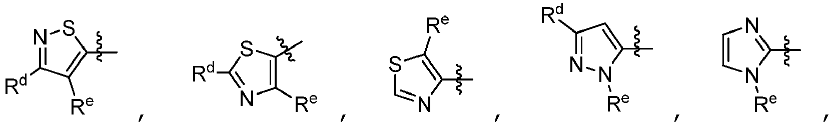 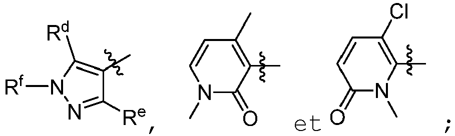
R^{d} étant choisi dans le groupe constitué par : H ; halogéno ; C₁₋₆alkyle ; C₁₋₆alkyle substitué par un membre choisi dans le groupe constitué par : OH, OCH₃, SCH₃ et OCF₃ ; C₁₋₆halogénoalkyle ; C₁₋₆halogénoalkyle substitué par un membre choisi dans le groupe constitué par : OH et OCH₃ ; N(CH₃)₂ ; OH ; CN et OC₁₋₆alkyle ;
R^{e} étant choisi dans le groupe constitué par : halogéno ; C₁₋₆alkyle ; C₁₋₆alkyle substitué par un membre choisi dans le groupe constitué par : OH, OCH₃, SCH₃ et OCF₃ ; C₁₋₆halogénoalkyle ; C₁₋₆halogénoalkyle substitué par un membre choisi dans le groupe constitué par : OH et OCH₃ ; OH ; OC₁₋₆alkyle ; et C₃₋₆cycloalkyle ;
R^{f} étant choisi dans le groupe constitué par : H ; C₁₋₆alkyle ; C₁₋₆alkyle substitué par un membre choisi dans le groupe constitué par : OH, OCH₃, SCH₃ et OCF₃ ; C₁₋₆halogénoalkyle ; et C₁₋₆halogénoalkyle substitué par un membre choisi dans le groupe constitué par : OH et OCH₃ ; et
n étant 1 ou 2 ;
ou sel, solvate, stéréoisomère, forme tautomère, variante isotopique ou N-oxyde pharmaceutiquement acceptable correspondant(e).

2. Composé selon la revendication 1,
(a) X étant O ; ou sel, solvate, stéréoisomère, forme tautomère, variante isotopique ou N-oxyde pharmaceutiquement acceptable correspondant(e) ; ou
(b) X étant S ; ou sel, solvate, stéréoisomère, forme tautomère, variante isotopique ou N-oxyde pharmaceutiquement acceptable correspondant(e).

3. Composé selon la revendication 1,
(a) X étant NR^{a}, et R^{a} étant H ; ou sel, solvate, stéréoisomère, forme tautomère, variante isotopique ou N-oxyde pharmaceutiquement acceptable correspondant(e) ;
(b) X étant NR^{a}, et R^{a} étant CH₃ ; ou sel, solvate, stéréoisomère, forme tautomère, variante isotopique ou N-oxyde pharmaceutiquement acceptable correspondant(e) ; ou
(c) X étant NR^{a}, où R^{a} et R^{1a} se joignent pour former un cycle hétérocyclique choisi parmi 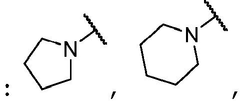 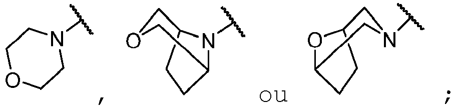
le cycle hétérocyclique étant substitué par un ou deux membres chacun indépendamment choisi dans le groupe constitué par : C₁₋₄alkyle, C₁₋₄halogénoalkyle et OC₁₋₄alkyle ; ou sel, solvate, stéréoisomère, forme tautomère, variante isotopique ou N-oxyde pharmaceutiquement acceptable correspondant(e).

4. Composé selon l'une quelconque des revendications 1 à 3,
(a) R^{1a} étant C₁₋₄alkyle ; C₁₋₄alkyle substitué par OH ou OCH₃ ; C₁₋₄halogénoalkyle ; C₁₋₄halogénoalkyle substitué par OH ou OCH₃ ; ou C₃₋₆cycloalkyle ; ou sel, solvate, stéréoisomère, forme tautomère, variante isotopique ou N-oxyde pharmaceutiquement acceptable correspondant(e) ; ou
(b) R^{1a} étant CF₃ ; ou sel, solvate, stéréoisomère, forme tautomère, variante isotopique ou N-oxyde pharmaceutiquement acceptable correspondant(e).

5. Composé selon l'une quelconque des revendications 1 à 4,
(a) R^{1b} étant CH₃ ou CHF₂ ; ou sel, solvate, stéréoisomère, forme tautomère, variante isotopique ou N-oxyde pharmaceutiquement acceptable correspondant(e) ; ou
(b) R^{1b} étant CH₃ ; ou sel, solvate, stéréoisomère, forme tautomère, variante isotopique ou N-oxyde pharmaceutiquement acceptable correspondant(e).

6. Composé selon l'une quelconque des revendications 1 à 3, R^{1a} et R^{1b} se joignant pour former cyclopropyle, cyclobutyle, cyclopentyle ou cyclohexyle ; cyclopropyle, cyclobutyle, cyclopentyle ou cyclohexyle étant chacun indépendamment substitué par un, deux, trois ou quatre membres choisis dans le groupe constitué par : halogène, OH, C₁₋₄alkyle et C₁₋₄halogénoalkyle ; oxétanyle ; tétrahydrofuranyle ; et tétrahydropyranyle ; ou sel, solvate, stéréoisomère, forme tautomère, variante isotopique ou N-oxyde pharmaceutiquement acceptable correspondant(e).

7. Composé selon l'une quelconque des revendications 1 à 6,
(a) R² étant 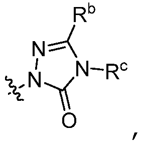
R^{b} étant C₁₋₄alkyle substitué par OH, halogéno, CN, OC₁₋₄alkyle, OC₁₋₄halogénoalkyle ou OC₃₋₆cycloalkyle ; et
R^{e} étant C₁₋₄alkyle, C₁₋₄halogénoalkyle, C₁₋₄alkyle substitué par OH, allyle ou C₃₋₆cycloalkyle ; ou sel, solvate, stéréoisomère, forme tautomère, variante isotopique ou N-oxyde pharmaceutiquement acceptable correspondant(e) ; ou
(b) R² étant 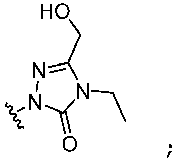 ou sel, solvate, stéréoisomère, forme tautomère, variante isotopique ou N-oxyde pharmaceutiquement acceptable correspondant(e).

8. Composé selon l'une quelconque des revendications 1 à 7,
(a) R³ étant 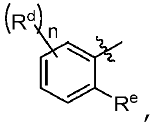
R^{d} étant H ; halogéno ; C₁₋₄alkyle ; C₁₋₄alkyle substitué par OH, OCH₃, SCH₃ ou OCF₃ ; C₁₋₄halogénoalkyle ; C₁₋₄halogénoalkyle substitué par OH ou OCH₃ ; CN ; ou OC₁₋₄alkyle ;
R^{e} étant halogéno ; C₁₋₄alkyle ; C₁₋₄alkyle substitué par OH, OCH₃, SCH₃ ou OCF₃ ; C₁₋₄halogénoalkyle ; ou C₁₋₄halogénoalkyle substitué par OH ou OCH₃ ; et
n étant 1 ou 2 ;
ou sel, solvate, stéréoisomère, forme tautomère, variante isotopique ou N-oxyde pharmaceutiquement acceptable correspondant(e) ;
(b) R³ étant 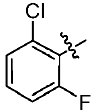 ; ou sel, solvate, stéréoisomère, forme tautomère, variante isotopique ou N-oxyde pharmaceutiquement acceptable correspondant(e) ;
(c) R³ étant 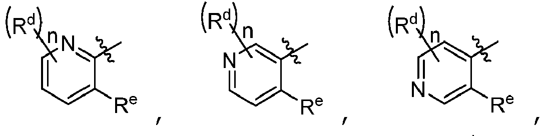 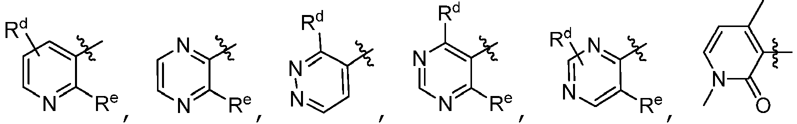 ou 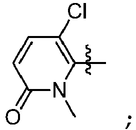
R^{d} étant H ; halogéno ; C₁₋₄alkyle ; C₁₋₄alkyle substitué par OH, OCH₃, SCH₃ ou OCF₃ ; C₁₋₄halogénoalkyle ; C₁₋₄halogénoalkyle substitué par OH ou OCH₃ ; N(CH₃)₂ ; OH ; CN ; ou OC₁₋₄alkyle ;
R^{e} étant halogéno ; OH ; C₁₋₄alkyle ; C₁₋₄alkyle substitué par OH, OCH₃, SCH₃ ou OCF₃ ; C₁₋₄halogénoalkyle ; OC₁₋₄alkyle ; ou C₁₋₄halogénoalkyle substitué par OH ou OCH₃ ; et
n étant 1 ou 2 ;
ou sel, solvate, stéréoisomère, forme tautomère, variante isotopique ou N-oxyde pharmaceutiquement acceptable correspondant(e) ; ou
(d) R³ étant 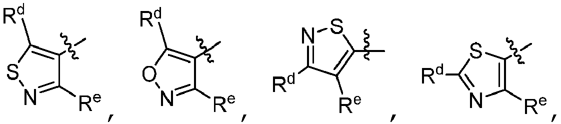 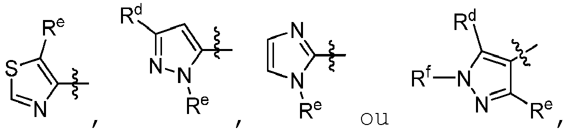
R^{d} étant H ; halogéno ; C₁₋₄alkyle ; C₁₋₄alkyle substitué par OH, OCH₃, SCH₃ ou OCF₃ ; C₁₋₄halogénoalkyle ; C₁₋₄halogénoalkyle substitué par OH ou OCH₃ ; ou OC₁₋₄alkyle ;
R^{e} étant halogéno ; C₁₋₄alkyle ; C₁₋₄alkyle substitué par OH, OCH₃, SCH₃ ou OCF₃ ; C₁₋₄halogénoalkyle ; C₃₋₆cycloalkyle ; ou C₁₋₄halogénoalkyle substitué par OH ou OCH₃ ; et
R^{f} étant H ; C₁₋₄alkyle ; C₁₋₄alkyle substitué par OH, OCH₃, SCH₃ ou OCF₃ ; C₁₋₄halogénoalkyle ; ou C₁₋₄halogénoalkyle substitué par OH ou OCH₃ ;
ou sel, solvate, stéréoisomère, forme tautomère, variante isotopique ou N-oxyde pharmaceutiquement acceptable correspondant(e).

9. Composé selon la revendication 1, ayant la structure de :
(a) Formule (IA) : 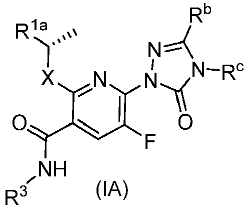
X étant O ;
R^{1a} étant choisi dans le groupe constitué par : C₁₋₆alkyle ; C₁₋₆alkyle substitué par OH ou OCH₃ ; C₁₋₆halogénoalkyle ; C₁₋₆halogénoalkyle substitué par OH ou OCH₃ ; et C₃₋₆cycloalkyle ;
R^{b} étant C₁₋₆alkyle substitué par un membre choisi dans le groupe constitué par : OH, halogéno, CN, OC₁₋₆alkyle, OC₁₋₆halogénoalkyle et OC₃₋₆cycloalkyle ;
R^{e} étant choisi dans le groupe constitué par : C₁₋₆alkyle, C₁₋₆alkyle substitué par OH, allyle, C₁₋₆halogénoalkyle et C₃₋₆cycloalkyle ; et
R³ étant choisi dans le groupe constitué par : 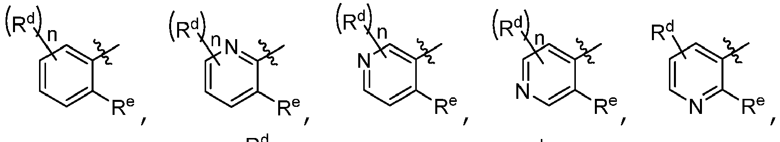 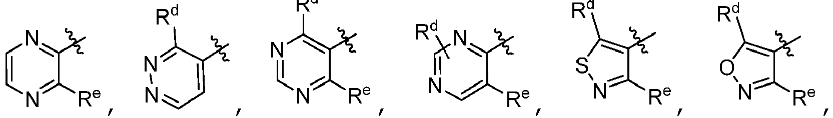 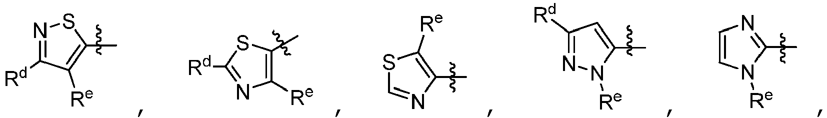 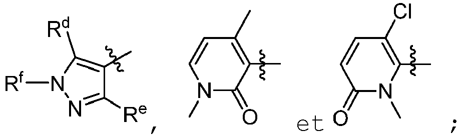
R^{d} étant choisi dans le groupe constitué par : H ; halogéno ; C₁₋₆alkyle ; C₁₋₆alkyle substitué par un membre choisi dans le groupe constitué par : OH, OCH₃, SCH₃ et OCF₃ ; C₁₋₆halogénoalkyle ; C₁₋₆halogénoalkyle substitué par un membre choisi dans le groupe constitué par : OH et OCH₃ ; N(CH₃)₂ ; OH ; CN et OC₁₋₆alkyle ;
R^{e} étant choisi dans le groupe constitué par : halogéno ; C₁₋₆alkyle ; C₁₋₆alkyle substitué par un membre choisi dans le groupe constitué par : OH, OCH₃, SCH₃ et OCF₃ ; C₁₋₆halogénoalkyle ; C₁₋₆halogénoalkyle substitué par un membre choisi dans le groupe constitué par : OH et OCH₃ ; OH ; OC₁₋₆alkyle ; et C₃₋₆cycloalkyle ;
R^{f} étant choisi dans le groupe constitué par : H ; C₁₋₆alkyle ; C₁₋₆alkyle substitué par un membre choisi dans le groupe constitué par : OH, OCH₃, SCH₃ et OCF₃ ; C₁₋₆halogénoalkyle ; et C₁₋₆halogénoalkyle substitué par un membre choisi dans le groupe constitué par : OH et OCH₃ ; et
n étant 1 ou 2 ;
ou sel, solvate, stéréoisomère, forme tautomère, variante isotopique ou N-oxyde pharmaceutiquement acceptable correspondant(e) ;
par exemple, R^{1a} étant choisi dans le groupe constitué par : C₁₋₄alkyle ; C₁₋₄alkyle substitué par OH ou OCH₃ ; C₁₋₄halogénoalkyle ; C₁₋₄halogénoalkyle substitué par OH ou OCH₃ ; ou C₃₋₆cycloalkyle ;
(b) Formule (IB) : 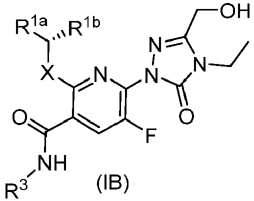
X étant choisi dans le groupe constitué par : O, S et NR^{a} ;
lorsque X est NR^{a} ; R^{a} est H ou CH₃ ; ou R^{a} et R^{1a} se joignent pour former un cycle hétérocyclique choisi dans le groupe constitué par : 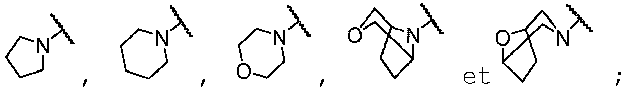 le cycle hétérocyclique étant substitué par un ou deux membres chacun indépendamment choisi dans le groupe constitué par : C₁₋₆alkyle, C₁₋₆halogénoalkyle et OC₁₋₆alkyle ;
R^{1a} étant choisi dans le groupe constitué par : C₁₋₆alkyle ; C₁₋₆alkyle substitué par OH ou OCH₃ ; C₁₋₆halogénoalkyle ; C₁₋₆halogénoalkyle substitué par OH ou OCH₃ ; et C₃₋₆cycloalkyle ;
R^{1b} étant CH₃ ou CHF₂ ; et
R³ étant choisi dans le groupe constitué par : 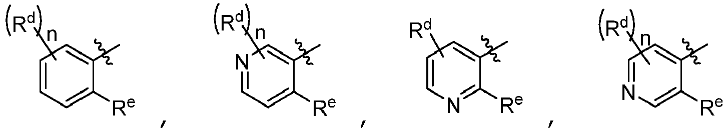 et 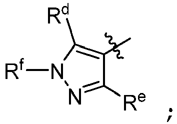
R^{d} étant choisi dans le groupe constitué par : H ; halogéno ; C₁₋₆alkyle ; C₁₋₆alkyle substitué par un membre choisi dans le groupe constitué par : OH, OCH₃, SCH₃ et OCF₃ ; C₁₋₆halogénoalkyle ; C₁₋₆halogénoalkyle substitué par un membre choisi dans le groupe constitué par : OH et OCH₃ ; N(CH₃)₂ ; OH ; CN et OC₁₋₆alkyle ;
R^{e} étant choisi dans le groupe constitué par : halogéno ; C₁₋₆alkyle ; C₁₋₆alkyle substitué par un membre choisi dans le groupe constitué par : OH, OCH₃, SCH₃ et OCF₃ ; C₁₋₆halogénoalkyle ; C₁₋₆halogénoalkyle substitué par un membre choisi dans le groupe constitué par : OH et OCH₃ ; OH ; OC₁₋₆alkyle ; et C₃₋₆cycloalkyle ;
R^{f} étant choisi dans le groupe constitué par : H ; C₁₋₆alkyle ; C₁₋₆alkyle substitué par un membre choisi dans le groupe constitué par : OH, OCH₃, SCH₃ et OCF₃ ; C₁₋₆halogénoalkyle ; et C₁₋₆halogénoalkyle substitué par un membre choisi dans le groupe constitué par : OH et OCH₃ ; et
n étant 1 ou 2 ;
ou sel, solvate, stéréoisomère, forme tautomère, variante isotopique ou N-oxyde pharmaceutiquement acceptable correspondant(e) ; par exemple, R^{d} et R^{e} étant chacun indépendamment halogéno, C₁₋₄alkyle et OC₁₋₄alkyle ; ou
(c) Formule (IC) : 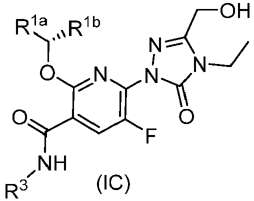
R^{1a} étant choisi dans le groupe constitué par : C₁₋₆alkyle ; C₁₋₆alkyle substitué par OH ou OCH₃ ; C₁₋₆halogénoalkyle ; C₁₋₆halogénoalkyle substitué par OH ou OCH₃ ; et C₃₋₆cycloalkyle ;
R^{1b} étant CH₃ ou CHF₂ ; ou R^{1a} et R^{1b} se joignant pour former C₃₋₆cycloalkyle ; C₃₋₆cycloalkyle indépendamment substitué par un, deux, trois ou quatre membres choisis dans le groupe constitué par : halogéno, OH, C₁₋₆alkyle et C₁₋₆halogénoalkyle ; oxétanyle ; tétrahydrofuranyle ; et tétrahydropyranyle ; et
R³ étant choisi dans le groupe constitué par : 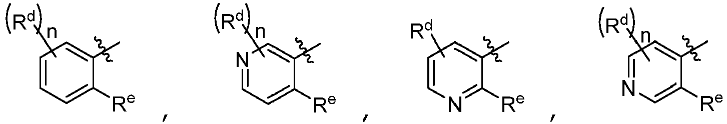 et 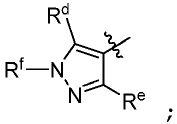
R^{d} étant choisi dans le groupe constitué par : H ; halogéno ; C₁₋₆alkyle ; C₁₋₆alkyle substitué par un membre choisi dans le groupe constitué par : OH, OCH₃, SCH₃ et OCF₃ ; C₁₋₆halogénoalkyle ; C₁₋₆halogénoalkyle substitué par un membre choisi dans le groupe constitué par : OH et OCH₃ ; N(CH₃)₂ ; OH ; CN et OC₁₋₆alkyle ;
R^{e} étant choisi dans le groupe constitué par : halogéno ; C₁₋₆alkyle ; C₁₋₆alkyle substitué par un membre choisi dans le groupe constitué par : OH, OCH₃, SCH₃ et OCF₃ ; C₁₋₆halogénoalkyle ; C₁₋₆halogénoalkyle substitué par un membre choisi dans le groupe constitué par : OH et OCH₃ ; OH ; OC₁₋₆alkyle ; et C₃₋₆cycloalkyle ;
R^{f} étant choisi dans le groupe constitué par : H ; C₁₋₆alkyle ; C₁₋₆alkyle substitué par un membre choisi dans le groupe constitué par : OH, OCH₃, SCH₃ et OCF₃ ; C₁₋₆halogénoalkyle ; et C₁₋₆halogénoalkyle substitué par un membre choisi dans le groupe constitué par : OH et OCH₃ ; et
n étant 1 ou 2 ;
ou sel, solvate, stéréoisomère, forme tautomère, variante isotopique ou N-oxyde pharmaceutiquement acceptable correspondant (e) ; par exemple, R^{1a} et R^{1b} se joignant pour former C₃₋₆cycloalkyle ; C₃₋₆cycloalkyle indépendamment substitué par un, deux, trois ou quatre membres choisis dans le groupe constitué par : F, OH, C₁₋₆alkyle et C₁₋₆halogénoalkyle ; oxétanyle ; tétrahydrofuranyle ; ou tétrahydropyranyle.

10. Composé selon la revendication 1 choisi dans le groupe constitué par :
(S)-N-(2-Chloro-6-fluorophényl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-fluorophényl)-2-((1,1,1-trifluoropropan-2-yl)oxy)nicotinamide ;
(S)-N-(2-Chloro-6-fluorophényl)-6-(4-éthyl-3-(fluorométhyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloropyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloro-4-méthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloro-6-fluorophényl)-6-(3-(chlorométhyl)-4-éthyl-5-oxo-4,5-dihydro-1H-1,2,4-triazol-1-yl)-5-fluoro-2-((1,1,1-trifluoropropan-2-yl)oxy)nicotinamide ;
N-(2-Chloro-6-fluorophényl)-6-(4-éthyl-3-(hydroxyméthyl)-5-oxo-4,5-dihydro-1H-1,2,4-triazol-1-yl)-5-fluoro-2-isopropoxynicotinamide ;
N-(2-Chloro-6-fluorophényl)-6-(4-éthyl-3-(hydroxyméthyl)-5-oxo-4,5-dihydro-1H-1,2,4-triazol-1-yl)-5-fluoro-2-(2,2,3,3-tétrafluorocyclobutoxy)nicotinamide ;
N-(2-Chloro-6-fluorophényl)-6-(4-éthyl-3-(hydroxyméthyl)-5-oxo-4,5-dihydro-1H-1,2,4-triazol-1-yl)-5-fluoro-2-((tétrahydrofuran-3-yl)oxy)nicotinamide racémique ;
(S)-N-(3-Chloropyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-fluoro-5-méthylphényl)-2-((1,1,1-trifluoropropan-2-yl)oxy)nicotinamide ;
(S)-N-(2-(Difluorométhyl)-4-méthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloro-6-fluorophényl)-6-(4-éthyl-3-(méthoxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloropyridazin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-méthylisothiazol-4-yl)-2-((1,1,1-trifluoropropan-2-yl)oxy)nicotinamide ;
(S)-N-(2,4-Diméthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloropyrazin-2-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-chloro-6-méthoxypyridin-2-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloro-2-méthoxypyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(5-Chloro-3-méthyl-1H-pyrazol-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(4-Chloro-3-méthylisothiazol-5-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(4-Chloropyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloro-5-fluoropyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Cyano-5-fluoropyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloro-5-fluoro-2-méthoxypyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(4-Bromo-3-méthylisothiazol-5-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(5-méthylthiazol-4-yl)-2-((1,1,1-trifluoropropan-2-yl)oxy)nicotinamide ;
(S)-N-(5-Chloro-2-méthoxypyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-méthoxypyrazin-2-yl)-2-((1,1,1-trifluoropropan-2-yl)oxy)nicotinamide ;
(S)-N-(3,5-Diméthylisothiazol-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-fluoro-5-méthylpyridin-3-yl)-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(5-fluoro-2-méthylpyridin-4-yl)-2-((1,1,1-trifluoropropan-2-yl)oxy)nicotinamide ;
(S)-N-(4-Chloro-6-méthylpyrimidin-5-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-fluoro-6-méthylpyridin-2-yl)-2-((1,1,1-trifluoropropan-2-yl)oxy)nicotinamide ;
(S)-N-(3-Chloro-2-méthoxy-5-méthylpyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-méthoxy-5-méthylpyridin-3-yl)-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-fluoro-4-méthylpyridin-3-yl)-2-((1,1,1-trifluoropropan-2-yl)oxy)nicotinamide ;
(S)-N-(5,6-Diméthylpyrimidin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloro-4-méthoxypyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-méthylisoxazol-4-yl)-2-((1,1,1-trifluoropropan-2-yl)oxy)nicotinamide ;
(S)-N-(3,5-Diméthylisoxazol-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-fluoro-5-méthoxyphényl)-2-((1,1,1-trifluoropropan-2-yl)oxy)nicotinamide ;
(S)-N-(2-Chloro-5-méthylphényl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3,5-Diméthyl-1H-pyrazol-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(4-Chloro-6-méthoxypyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2,5-Diméthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(5-Chloro-2-méthoxypyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
N-(1,4-Diméthyl-2-oxo-2,3-dihydro-1l4-pyridin-3-yl)-6-(4-éthyl-3-(hydroxyméthyl)-5-oxo-4,5-dihydro-1H-1,2,4-triazol-1-yl)-5-fluoro-2-(((S)-1,1,1-trifluoropropan-2-yl)oxy)nicotinamide ;
(S)-N-(3-Chloropyridin-2-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-2-((1,1,1-trifluoropropan-2-yl)oxy)-N-(1,3,5-triméthyl-1H-pyrazol-4-yl)nicotinamide ;
(S)-6-(4-Éthyl-3-(hydroxyméthyl)-5-oxo-4,5-dihydro-1H-1,2,4-triazol-1-yl)-5-fluoro-N-(3-fluoro-6-méthoxypyridin-2-yl)-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-méthyl-1H-pyrazol-4-yl)-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-méthoxy-1H-pyrazol-4-yl)-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-isopropyl-1-méthyl-1H-pyrazol-5-yl)-2-((1,1,1-trifluoropropan-2-yl)oxy)nicotinamide ;
(S)-N-(5-Chloro-6-méthylpyrimidin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-(méthoxyméthyl)-1-méthyl-1H-pyrazol-4-yl)-2-((1,1,1-trifluoropropan-2-yl)oxy)nicotinamide ;
(S)-N-(3-Cyclopropyl-1H-pyrazol-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloro-1-méthyl-6-oxo-1,6-dihydropyridin-2-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(5-fluoro-2-méthoxypyridin-4-yl)-2-((1,1,1-trifluoropropan-2-yl)oxy)nicotinamide ;
(S)-6-(4-éthyl-3-(hydroxyméthyl)-5-oxo-4,5-dihydro-1H-1,2,4-triazol-1-yl)-5-fluoro-N-(2-méthoxy-5-méthylpyridin-4-yl)-2-((1,1,1-trifluoropropan-2-yl)oxy)nicotinamide ;
(S)-N-(2-Chloro-5-méthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-méthoxy-4-méthylpyridin-3-yl)-2-((1,1,1-trifluoropropan-2-yl)oxy)nicotinamide ;
(S)-N-(2,4-Dichloropyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Éthoxy-5-fluoropyrimidin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-fluoro-5-méthylpyridin-4-yl)-2-((1,1,1-trifluoropropan-2-yl)oxy)nicotinamide ;
(S)-N-(2-Chloro-4,6-diméthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(1-Cyclopropyl-1H-imidazol-2-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloro-5-cyanophényl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloro-5-cyanopyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(4-(Diméthylamino)-2-méthoxypyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
N-(2-Chloro-6-méthyl-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(2-Chloro-5-fluoro-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(4-Chloro-2-méthyl-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(3-Chloro-1-méthyl-pyrazol-4-yl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-[2-(Difluorométhyl)-5-fluoro-4-pyridyl]-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-(5-fluoro-2-méthoxy-pyrimidin-4-yl)-2-[(1S)-2,2,2-trifluoro-1-méthyl-éthoxy]pyridine-3-carboxamide ;
N-(6-Chloro-2,3-diméthyl-phényl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(2-Chloro-4-méthyl-3-pyridyl)-5-fluoro-6-[3-(hydroxyméthyl)-5-oxo-4-propyl-1,2,4-triazol-1-yl]-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-(2-méthoxy-3,5-diméthyl-4-pyridyl)-2-[(1S)-2,2,2-trifluoro-1-méthyl-éthoxy]pyridine-3-carboxamide ;
(S)-2-(5-(((2-Chloro-4-méthylpyridin-3-yl)-l2-azaneyl)carbonyl)-3-fluoro-6-((1,1,1-trifluoropropan-2-yl)oxy)pyridin-2-yl)-5-(fluorométhyl)-4-isopropyl-2,4-dihydro-3H-1,2,4-triazol-3-one ;
N-(2-Chloro-4-méthyl-3-pyridyl)-6-[4-éthyl-3-(fluorométhyl)-5-oxo-1,2,4-triazol-1-yl]-5-fluoro-2-[(1S)-2,2,2-trifluoro-1-méthyl-éthoxy]pyridine-3-carboxamide ;
N-(3-Chloro-2-méthoxy-5-méthyl-4-pyridyl)-6-[4-éthyl-3-(hydroxyméthyl)-5-oxo-1,2,4-triazol-1-yl]-5-fluoro-2-isopropoxy-pyridine-3-carboxamide ;
N-(2-Chloro-4-méthyl-3-pyridyl)-5-fluoro-6-[3-(hydroxyméthyl)-4-isopropyl-5-oxo-1,2,4-triazol-1-yl]-2-[(1S)-2,2,2-trifluoro-1-méthyl-éthoxy]pyridine-3-carboxamide ;
N-(2-Chloro-4-méthyl-3-pyridyl)-6-[4-(2,2-difluoroéthyl)-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(5-Chloro-3-méthyl-1H-pyrazol-4-yl)-5-fluoro-6-[3-(hydroxyméthyl)-5-oxo-4-propyl-1,2,4-triazol-1-yl]-2-[(1S)-2,2,2-trifluoro-1-méthyl-éthoxy]pyridine-3-carboxamide ;
N-(2,4-Diméthoxy-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(4,6-Diméthoxypyrimidin-5-yl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-2-[(1S)-2,2,2-trifluoro-1-méthyl-éthoxy]-N-[3-(trifluorométhyl)-1H-pyrazol-4-yl]pyridine-3-carboxamide ;
N-(5-Chloro-3-méthyl-1H-pyrazol-4-yl)-6-[4-éthyl-3-(fluorométhyl)-5-oxo-1,2,4-triazol-1-yl]-5-fluoro-2-[(1S)-2,2,2-trifluoro-1-méthyl-éthoxy]pyridine-3-carboxamide ;
N-(2-Chloro-4-méthyl-3-pyridyl)-6-[4-éthyl-3-(fluorométhyl)-5-oxo-1,2,4-triazol-1-yl]-5-fluoro-2-isopropoxy-pyridine-3-carboxamide ;
N-(5-Chloro-2-méthoxy-4-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(5-Chloro-3-méthyl-1H-pyrazol-4-yl)-5-fluoro-6-[3-(fluorométhyl)-5-oxo-4-propyl-1,2,4-triazol-1-yl]-2-[(1S)-2,2,2-trifluoro-1-méthyl-éthoxy]pyridine-3-carboxamide ;
N-(3-Cyano-1H-pyrazol-4-yl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(2-Chloro-4-méthyl-3-pyridyl)-5-fluoro-6-[3-(fluorométhyl)-4-(3-hydroxypropyl)-5-oxo-1,2,4-triazol-1-yl]-2-[(1S)-2,2,2-trifluoro-1-méthyl-éthoxy]pyridine-3-carboxamide ;
N-(2-chloro-4,5-diméthyl-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-éthyl-3-(hydroxyméthyl)-5-oxo-1,2,4-triazol-1-yl]-5-fluoro-N-(4-méthoxy-2,6-diméthyl-3-pyridyl)-2-[(1S)-2,2,2-trifluoro-1-méthyl-éthoxy]pyridine-3-carboxamide ;
N-(3-Chloro-4-méthoxy-2-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(3-Chloro-4-éthoxy-2-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(2-Chloro-6-méthoxy-4-méthyl-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(3-Chloro-5-méthyl-1H-pyrazol-4-yl)-5-fluoro-6-[3-(fluorométhyl)-4-(3-hydroxypropyl)-5-oxo-1,2,4-triazol-1-yl]-2-[(1S)-2,2,2-trifluoro-1-méthyl-éthoxy]pyridine-3-carboxamide ;
N-(5-Chloro-3-méthyl-1H-pyrazol-4-yl)-6-[4-éthyl-3-(fluorométhyl)-5-oxo-1,2,4-triazol-1-yl]-5-fluoro-2-isopropoxy-pyridine-3-carboxamide ;
6-[4-Éthyl-3-(hydroxyméthyl)-5-oxo-1,2,4-triazol-1-yl]-5-fluoro-N-(3-méthoxypyridazin-4-yl)-2-[(1S)-2,2,2-trifluoro-1-méthyl-éthoxy]pyridine-3-carboxamide ;
N-(5-Chloro-2-méthoxy-3-méthyl-4-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-[3-méthyl-5-(trifluorométhyl)-1H-pyrazol-4-yl]-2-[(1S)-2,2,2-trifluoro-1-méthyl-éthoxy]pyridine-3-carboxamide ;
N-[3-Chloro-5-(trifluorométhyl)-1H-pyrazol-4-yl]-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(6-Chloro-2-méthoxy-4-méthyl-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-(2-méthoxy-4,6-diméthyl-3-pyridyl)-2-[(1S)-2,2,2-trifluoro-1-méthyl-éthoxy]pyridine-3-carboxamide ;
N-(5-Chloro-3-méthyl-1H-pyrazol-4-yl)-5-fluoro-6-[3-(fluorométhyl)-4-(2-hydroxyéthyl)-5-oxo-1,2,4-triazol-1-yl]-2-[(1S)-2,2,2-trifluoro-1-méthyl-éthoxy]pyridine-3-carboxamide ;
N-(2-Chloro-4-méthyl-3-pyridyl)-5-fluoro-6-[3-(fluorométhyl)-4-(2-hydroxyéthyl)-5-oxo-1,2,4-triazol-1-yl]-2-[(1S)-2,2,2-trifluoro-1-méthyl-éthoxy]pyridine-3-carboxamide ;
N-(3-Cyano-5-fluoro-2-méthoxy-4-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(3-Chloro-5-méthoxy-2-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(2,6-Diméthoxyphényl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-(o-tolyl)-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-(3-méthoxy-5-méthyl-1H-pyrazol-4-yl)-2-[(1S)-2,2,2-trifluoro-1-méthyl-éthoxy]pyridine-3-carboxamide ;
N-(3,5-Diméthoxy-4-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(3,5-Dichloro-1H-pyrazol-4-yl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-[3-(Difluorométhyl)-5-méthyl-1H-pyrazol-4-yl]-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-(4-fluoro-2-méthoxy-3-pyridyl)-2-[(1S)-2,2,2-trifluoro-1-méthyl-éthoxy]pyridine-3-carboxamide ;
N-(4-Chloro-2-méthoxy-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(1-hydroxyéthyl)-5-oxo-1,2,4-triazol-1-yl]-5-fluoro-N-[3-méthyl-5-(trifluorométhyl)-1H-pyrazol-4-yl]-2-(2,2,2-trifluoro-1-méthyl-éthoxy)pyridine-3-carboxamide (racémique) ; et
6-[4-Éthyl-3-(1-hydroxy-1-méthyl-éthyl)-5-oxo-1,2,4-triazol-1-yl]-5-fluoro-N-[3-méthyl-5-(trifluorométhyl)-1H-pyrazol-4-yl]-2-[(1S)-2,2,2-trifluoro-1-méthyl-éthoxy]pyridine-3-carboxamide ;
ou sel, solvate, stéréoisomère, forme tautomère, variante isotopique ou N-oxyde pharmaceutiquement acceptable correspondant(e).

11. Composé selon la revendication 1 choisi dans le groupe constitué par :
(S)-N-(2-Chloro-4-méthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(5-Chloro-3-méthyl-1H-pyrazol-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloro-2-méthoxy-5-méthylpyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-méthoxy-4-méthylpyridin-3-yl)-2-((1,1,1-trifluoropropan-2-yl)oxy)nicotinamide ;
6-[4-Éthyl-3-(hydroxyméthyl)-5-oxo-1,2,4-triazol-1-yl]-5-fluoro-N-[3-méthyl-5-(trifluorométhyl)-1H-pyrazol-4-yl]-2-[(1S)-2,2,2-trifluoro-1-méthyl-éthoxy]pyridine-3-carboxamide ; et
6-[4-Éthyl-3-(hydroxyméthyl)-5-oxo-1,2,4-triazol-1-yl]-5-fluoro-N-(2-méthoxy-3,5-diméthyl-4-pyridyl)-2-[(1S)-2,2,2-trifluoro-1-méthyl-éthoxy]pyridine-3-carboxamide ;
ou sel, solvate, stéréoisomère, forme tautomère, variante isotopique ou N-oxyde pharmaceutiquement acceptable correspondant(e).

12. Composition pharmaceutique comprenant : (A) une quantité efficace d'un composé selon l'une quelconque des revendications 1 à 11 ou un sel, un solvate, un stéréoisomère, une forme tautomère, une variante isotopique ou un N-oxyde pharmaceutiquement acceptable correspondant(e) ; et (B) au moins un excipient pharmaceutiquement acceptable.

13. Au moins un composé ou sel, solvate, stéréoisomère, forme tautomère, variante isotopique ou N-oxyde pharmaceutiquement acceptable correspondant(e) selon l'une quelconque des revendications 1 à 11 pour une utilisation dans le traitement d'un sujet souffrant ou diagnostiqué d'une maladie, d'un trouble ou d'une affection médicale comprenant l'inhibition ou la modification de l'activité de l'enzyme dihydroorotate oxygénase chez le sujet, la maladie, le trouble ou l'affection médicale étant un trouble inflammatoire, un trouble auto-immun ou un cancer.

14. Au moins un composé ou sel, solvate, stéréoisomère, forme tautomère, variante isotopique ou N-oxyde pharmaceutiquement acceptable correspondant(e) pour une utilisation selon la revendication 13, le trouble, la maladie ou l'affection médicale étant choisi(e) dans le groupe constitué par : lymphomes, leucémies, carcinomes et sarcomes.

15. Au moins un composé ou sel, solvate, stéréoisomère, forme tautomère, variante isotopique ou N-oxyde pharmaceutiquement acceptable correspondant(e) pour une utilisation selon la revendication 13, le trouble, la maladie ou l'affection médicale étant choisi(e) dans le groupe constitué par : leucémie lymphoblastique aiguë, leucémie myéloïde aiguë, leucémie à cellules T (aiguë), leucémie lymphoblastique aiguë, leucémie lymphocytaire aiguë, leucémie monocytaire aiguë, leucémie promyélocytaire aiguë, leucémie myélomonocytaire bisphénotypique B, leucémie lymphocytaire chronique, leucémie myélogène chronique, leucémie myéloïde chronique, leucémie myélomonocytaire chronique, leucémie lymphocytaire granulaire de grande taille, leucémie plasmocytaire et également syndrome myélodysplasique, qui peut se développer en leucémie myéloïde aiguë.

16. Au moins un composé ou sel, solvate, stéréoisomère, forme tautomère, variante isotopique ou N-oxyde pharmaceutiquement acceptable correspondant(e) pour une utilisation selon la revendication 13, le trouble, la maladie ou l'affection médicale étant une leucémie myéloïde aiguë.

17. Au moins un composé ou sel, solvate, stéréoisomère, forme tautomère, variante isotopique ou N-oxyde pharmaceutiquement acceptable correspondant(e) pour une utilisation selon l'une quelconque des revendications 13 à 16, l'au moins un composé comprenant un composé choisi dans le groupe constitué par :
(S)-N-(2-Chloro-6-fluorophényl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-fluorophényl)-2-((1,1,1-trifluoropropan-2-yl)oxy)nicotinamide ;
(S)-N-(2-Chloro-6-fluorophényl)-6-(4-éthyl-3-(fluorométhyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloropyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloro-4-méthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloro-6-fluorophényl)-6-(3-(chlorométhyl)-4-éthyl-5-oxo-4,5-dihydro-1H-1,2,4-triazol-1-yl)-5-fluoro-2-((1,1,1-trifluoropropan-2-yl)oxy)nicotinamide ;
N-(2-Chloro-6-fluorophényl)-6-(4-éthyl-3-(hydroxyméthyl)-5-oxo-4,5-dihydro-1H-1,2,4-triazol-1-yl)-5-fluoro-2-isopropoxynicotinamide ;
N-(2-Chloro-6-fluorophényl)-6-(4-éthyl-3-(hydroxyméthyl)-5-oxo-4,5-dihydro-1H-1,2,4-triazol-1-yl)-5-fluoro-2-(2,2,3,3-tétrafluorocyclobutoxy)nicotinamide ;
N-(2-Chloro-6-fluorophényl)-6-(4-éthyl-3-(hydroxyméthyl)-5-oxo-4,5-dihydro-1H-1,2,4-triazol-1-yl)-5-fluoro-2-((tétrahydrofuran-3-yl)oxy)nicotinamide racémique ;
(S)-N-(3-Chloropyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-fluoro-5-méthylphényl)-2-((1,1,1-trifluoropropan-2-yl)oxy)nicotinamide ;
(S)-N-(2-(Difluorométhyl)-4-méthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloro-6-fluorophényl)-6-(4-éthyl-3-(méthoxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloropyridazin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-méthylisothiazol-4-yl)-2-((1,1,1-trifluoropropan-2-yl)oxy)nicotinamide ;
(S)-N-(2,4-Diméthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloropyrazin-2-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-chloro-6-méthoxypyridin-2-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloro-2-méthoxypyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(5-Chloro-3-méthyl-1H-pyrazol-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(4-Chloro-3-méthylisothiazol-5-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(4-Chloropyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloro-5-fluoropyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Cyano-5-fluoropyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloro-5-fluoro-2-méthoxypyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(4-Bromo-3-méthylisothiazol-5-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(5-méthylthiazol-4-yl)-2-((1,1,1-trifluoropropan-2-yl)oxy)nicotinamide ;
(S)-N-(5-Chloro-2-méthoxypyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-méthoxypyrazin-2-yl)-2-((1,1,1-trifluoropropan-2-yl)oxy)nicotinamide ;
(S)-N-(3,5-Diméthylisothiazol-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-fluoro-5-méthylpyridin-3-yl)-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(5-fluoro-2-méthylpyridin-4-yl)-2-((1,1,1-trifluoropropan-2-yl)oxy)nicotinamide ;
(S)-N-(4-Chloro-6-méthylpyrimidin-5-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-fluoro-6-méthylpyridin-2-yl)-2-((1,1,1-trifluoropropan-2-yl)oxy)nicotinamide ;
(S)-N-(3-Chloro-2-méthoxy-5-méthylpyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-méthoxy-5-méthylpyridin-3-yl)-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-fluoro-4-méthylpyridin-3-yl)-2-((1,1,1-trifluoropropan-2-yl)oxy)nicotinamide ;
(S)-N-(5,6-Diméthylpyrimidin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloro-4-méthoxypyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-méthylisoxazol-4-yl)-2-((1,1,1-trifluoropropan-2-yl)oxy)nicotinamide ;
(S)-N-(3,5-Diméthylisoxazol-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-fluoro-5-méthoxyphényl)-2-((1,1,1-trifluoropropan-2-yl)oxy)nicotinamide ;
(S)-N-(2-Chloro-5-méthylphényl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3,5-Diméthyl-1H-pyrazol-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(4-Chloro-6-méthoxypyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2,5-Diméthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(5-Chloro-2-méthoxypyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
N-(1,4-Diméthyl-2-oxo-2,3-dihydro-1l4-pyridin-3-yl)-6-(4-éthyl-3-(hydroxyméthyl)-5-oxo-4,5-dihydro-1H-1,2,4-triazol-1-yl)-5-fluoro-2-(((S)-1,1,1-trifluoropropan-2-yl)oxy)nicotinamide ;
(S)-N-(3-Chloropyridin-2-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-2-((1,1,1-trifluoropropan-2-yl)oxy)-N-(1,3,5-triméthyl-1H-pyrazol-4-yl)nicotinamide ;
(S)-6-(4-Éthyl-3-(hydroxyméthyl)-5-oxo-4,5-dihydro-1H-1,2,4-triazol-1-yl)-5-fluoro-N-(3-fluoro-6-méthoxypyridin-2-yl)-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-méthyl-1H-pyrazol-4-yl)-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-méthoxy-1H-pyrazol-4-yl)-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-isopropyl-1-méthyl-1H-pyrazol-5-yl)-2-((1,1,1-trifluoropropan-2-yl)oxy)nicotinamide ;
(S)-N-(5-Chloro-6-méthylpyrimidin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-(méthoxyméthyl)-1-méthyl-1H-pyrazol-4-yl)-2-((1,1,1-trifluoropropan-2-yl)oxy)nicotinamide ;
(S)-N-(3-Cyclopropyl-1H-pyrazol-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloro-1-méthyl-6-oxo-1,6-dihydropyridin-2-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(5-fluoro-2-méthoxypyridin-4-yl)-2-((1,1,1-trifluoropropan-2-yl)oxy)nicotinamide ;
(S)-6-(4-éthyl-3-(hydroxyméthyl)-5-oxo-4,5-dihydro-1H-1,2,4-triazol-1-yl)-5-fluoro-N-(2-méthoxy-5-méthylpyridin-4-yl)-2-((1,1,1-trifluoropropan-2-yl)oxy)nicotinamide ;
(S)-N-(2-Chloro-5-méthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-méthoxy-4-méthylpyridin-3-yl)-2-((1,1,1-trifluoropropan-2-yl)oxy)nicotinamide ;
(S)-N-(2,4-Dichloropyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Éthoxy-5-fluoropyrimidin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(3-fluoro-5-méthylpyridin-4-yl)-2-((1,1,1-trifluoropropan-2-yl)oxy)nicotinamide ;
(S)-N-(2-Chloro-4,6-diméthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(1-Cyclopropyl-1H-imidazol-2-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloro-5-cyanophényl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(2-Chloro-5-cyanopyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(4-(Diméthylamino)-2-méthoxypyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
N-(2-Chloro-6-méthyl-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(2-Chloro-5-fluoro-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(4-Chloro-2-méthyl-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(3-Chloro-1-méthyl-pyrazol-4-yl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-[2-(Difluorométhyl)-5-fluoro-4-pyridyl]-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-(5-fluoro-2-méthoxy-pyrimidin-4-yl)-2-[(1S)-2,2,2-trifluoro-1-méthyl-éthoxy]pyridine-3-carboxamide ;
N-(6-Chloro-2,3-diméthyl-phényl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(2-Chloro-4-méthyl-3-pyridyl)-5-fluoro-6-[3-(hydroxyméthyl)-5-oxo-4-propyl-1,2,4-triazol-1-yl]-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-(2-méthoxy-3,5-diméthyl-4-pyridyl)-2-[(1S)-2,2,2-trifluoro-1-méthyl-éthoxy]pyridine-3-carboxamide ;
(S)-2-(5-(((2-Chloro-4-méthylpyridin-3-yl)-l2-azaneyl)carbonyl)-3-fluoro-6-((1,1,1-trifluoropropan-2-yl)oxy)pyridin-2-yl)-5-(fluorométhyl)-4-isopropyl-2,4-dihydro-3H-1,2,4-triazol-3-one ;
N-(2-Chloro-4-méthyl-3-pyridyl)-6-[4-éthyl-3-(fluorométhyl)-5-oxo-1,2,4-triazol-1-yl]-5-fluoro-2-[(1S)-2,2,2-trifluoro-1-méthyl-éthoxy]pyridine-3-carboxamide ;
N-(3-Chloro-2-méthoxy-5-méthyl-4-pyridyl)-6-[4-éthyl-3-(hydroxyméthyl)-5-oxo-1,2,4-triazol-1-yl]-5-fluoro-2-isopropoxy-pyridine-3-carboxamide ;
N-(2-Chloro-4-méthyl-3-pyridyl)-5-fluoro-6-[3-(hydroxyméthyl)-4-isopropyl-5-oxo-1,2,4-triazol-1-yl]-2-[(1S)-2,2,2-trifluoro-1-méthyl-éthoxy]pyridine-3-carboxamide ;
N-(2-Chloro-4-méthyl-3-pyridyl)-6-[4-(2,2-difluoroéthyl)-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(5-Chloro-3-méthyl-1H-pyrazol-4-yl)-5-fluoro-6-[3-(hydroxyméthyl)-5-oxo-4-propyl-1,2,4-triazol-1-yl]-2-[(1S)-2,2,2-trifluoro-1-méthyl-éthoxy]pyridine-3-carboxamide ;
N-(2,4-Diméthoxy-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(4,6-Diméthoxypyrimidin-5-yl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-2-[(1S)-2,2,2-trifluoro-1-méthyl-éthoxy]-N-[3-(trifluorométhyl)-1H-pyrazol-4-yl]pyridine-3-carboxamide ;
N-(5-Chloro-3-méthyl-1H-pyrazol-4-yl)-6-[4-éthyl-3-(fluorométhyl)-5-oxo-1,2,4-triazol-1-yl]-5-fluoro-2-[(1S)-2,2,2-trifluoro-1-méthyl-éthoxy]pyridine-3-carboxamide ;
N-(2-Chloro-4-méthyl-3-pyridyl)-6-[4-éthyl-3-(fluorométhyl)-5-oxo-1,2,4-triazol-1-yl]-5-fluoro-2-isopropoxy-pyridine-3-carboxamide ;
N-(5-Chloro-2-méthoxy-4-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(5-Chloro-3-méthyl-1H-pyrazol-4-yl)-5-fluoro-6-[3-(fluorométhyl)-5-oxo-4-propyl-1,2,4-triazol-1-yl]-2-[(1S)-2,2,2-trifluoro-1-méthyl-éthoxy]pyridine-3-carboxamide ;
N-(3-Cyano-1H-pyrazol-4-yl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(2-Chloro-4-méthyl-3-pyridyl)-5-fluoro-6-[3-(fluorométhyl)-4-(3-hydroxypropyl)-5-oxo-1,2,4-triazol-1-yl]-2-[(1S)-2,2,2-trifluoro-1-méthyl-éthoxy]pyridine-3-carboxamide ;
N-(2-chloro-4,5-diméthyl-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-éthyl-3-(hydroxyméthyl)-5-oxo-1,2,4-triazol-1-yl]-5-fluoro-N-(4-méthoxy-2,6-diméthyl-3-pyridyl)-2-[(1S)-2,2,2-trifluoro-1-méthyl-éthoxy]pyridine-3-carboxamide ;
N-(3-Chloro-4-méthoxy-2-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(3-Chloro-4-éthoxy-2-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(2-Chloro-6-méthoxy-4-méthyl-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(3-Chloro-5-méthyl-1H-pyrazol-4-yl)-5-fluoro-6-[3-(fluorométhyl)-4-(3-hydroxypropyl)-5-oxo-1,2,4-triazol-1-yl]-2-[(1S)-2,2,2-trifluoro-1-méthyl-éthoxy]pyridine-3-carboxamide ;
N-(5-Chloro-3-méthyl-1H-pyrazol-4-yl)-6-[4-éthyl-3-(fluorométhyl)-5-oxo-1,2,4-triazol-1-yl]-5-fluoro-2-isopropoxy-pyridine-3-carboxamide ;
6-[4-Éthyl-3-(hydroxyméthyl)-5-oxo-1,2,4-triazol-1-yl]-5-fluoro-N-(3-méthoxypyridazin-4-yl)-2-[(1S)-2,2,2-trifluoro-1-méthyl-éthoxy]pyridine-3-carboxamide ;
N-(5-Chloro-2-méthoxy-3-méthyl-4-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-[3-méthyl-5-(trifluorométhyl)-1H-pyrazol-4-yl]-2-[(1S)-2,2,2-trifluoro-1-méthyl-éthoxy]pyridine-3-carboxamide ;
N-[3-Chloro-5-(trifluorométhyl)-1H-pyrazol-4-yl]-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(6-Chloro-2-méthoxy-4-méthyl-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-(2-méthoxy-4,6-diméthyl-3-pyridyl)-2-[(1S)-2,2,2-trifluoro-1-méthyl-éthoxy]pyridine-3-carboxamide ;
N-(5-Chloro-3-méthyl-1H-pyrazol-4-yl)-5-fluoro-6-[3-(fluorométhyl)-4-(2-hydroxyéthyl)-5-oxo-1,2,4-triazol-1-yl]-2-[(1S)-2,2,2-trifluoro-1-méthyl-éthoxy]pyridine-3-carboxamide ;
N-(2-Chloro-4-méthyl-3-pyridyl)-5-fluoro-6-[3-(fluorométhyl)-4-(2-hydroxyéthyl)-5-oxo-1,2,4-triazol-1-yl]-2-[(1S)-2,2,2-trifluoro-1-méthyl-éthoxy]pyridine-3-carboxamide ;
N-(3-Cyano-5-fluoro-2-méthoxy-4-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(3-Chloro-5-méthoxy-2-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(2,6-Diméthoxyphényl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-(o-tolyl)-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-(3-méthoxy-5-méthyl-1H-pyrazol-4-yl)-2-[(1S)-2,2,2-trifluoro-1-méthyl-éthoxy]pyridine-3-carboxamide ;
N-(3,5-Diméthoxy-4-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-(3,5-Dichloro-1H-pyrazol-4-yl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
N-[3-(Difluorométhyl)-5-méthyl-1H-pyrazol-4-yl]-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(hydroxyméthyl)-5-oxo-1,2,4-triazol-1-yl]-5-fluoro-N-(4-fluoro-2-méthoxy-3-pyridyl)-2-[(1S)-2,2,2-trifluoro-1-méthyl-éthoxy]pyridine-3-carboxamide ;
N-(4-Chloro-2-méthoxy-3-pyridyl)-6-[4-éthyl-3-(hydroxyméthyl)-5-oxo-1,2,4-triazol-1-yl]-5-fluoro-2-[(1S)-2,2,2-trifluoro-1-méthyl-éthoxy]pyridine-3-carboxamide ;
6-[4-Éthyl-3-(1-hydroxyéthyl)-5-oxo-1,2,4-triazol-1-yl]-5-fluoro-N-[3-méthyl-5-(trifluorométhyl)-1H-pyrazol-4-yl]-2-(2,2,2-trifluoro-1-méthyl-éthoxy)pyridine-3-carboxamide (racémique) ; et
6-[4-Éthyl-3-(1-hydroxy-1-méthyl-éthyl)-5-oxo-1,2,4-triazol-1-yl]-5-fluoro-N-[3-méthyl-5-(trifluorométhyl)-1H-pyrazol-4-yl]-2-[(1S)-2,2,2-trifluoro-1-méthyl-éthoxy]pyridine-3-carboxamide ;
ou sel, solvate, stéréoisomère, forme tautomère, variante isotopique ou N-oxyde pharmaceutiquement acceptable correspondant(e).

18. Au moins un composé ou sel, solvate, stéréoisomère, forme tautomère, variante isotopique ou N-oxyde pharmaceutiquement acceptable correspondant(e) pour une utilisation selon l'une quelconque des revendications 13 à 16, l'au moins un composé comprenant un composé choisi dans le groupe constitué par :
(S)-N-(2-Chloro-4-méthylpyridin-3-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(5-Chloro-3-méthyl-1H-pyrazol-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-N-(3-Chloro-2-méthoxy-5-méthylpyridin-4-yl)-6-(4-éthyl-3-(hydroxyméthyl)-5-oxo-4,5-dihydro-1H-1,2,4-triazol-1-yl)-5-fluoro-2-((1,1,1-trifluoropropan-2-yl)oxy)nicotinamide ;
(S)-6-(4-Éthyl-3-(hydroxyméthyl)-5-oxo-4,5-dihydro-1H-1,2,4-triazol-1-yl)-5-fluoro-N-(2-méthoxy-4-méthylpyridin-3-yl)-2-((1,1,1-trifluoropropan-2-yl)oxy)nicotinamide ;
6-[4-Éthyl-3-(hydroxyméthyl)-5-oxo-1,2,4-triazol-1-yl]-5-fluoro-N-[3-méthyl-5-(trifluorométhyl)-1H-pyrazol-4-yl]-2-[(1S)-2,2,2-trifluoro-1-méthyl-éthoxy]pyridine-3-carboxamide ; et
6-[4-Éthyl-3-(hydroxyméthyl)-5-oxo-1,2,4-triazol-1-yl]-5-fluoro-N-(2-méthoxy-3,5-diméthyl-4-pyridyl)-2-[(1S)-2,2,2-trifluoro-1-méthyl-éthoxy]pyridine-3-carboxamide ;
ou sel, solvate, stéréoisomère, forme tautomère, variante isotopique ou N-oxyde pharmaceutiquement acceptable correspondant(e).

19. Composé selon la revendication 1 choisi parmi : 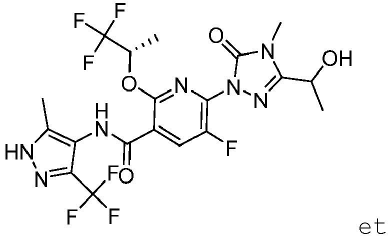 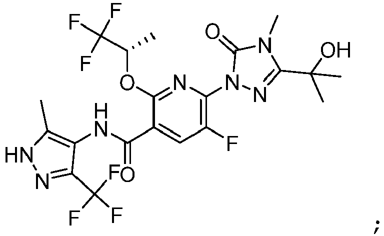 ou sel, solvate, stéréoisomère, forme tautomère, variante isotopique ou N-oxyde pharmaceutiquement acceptable correspondant(e).

20. Composé selon l'une quelconque des revendications 1 à 6, R² étant 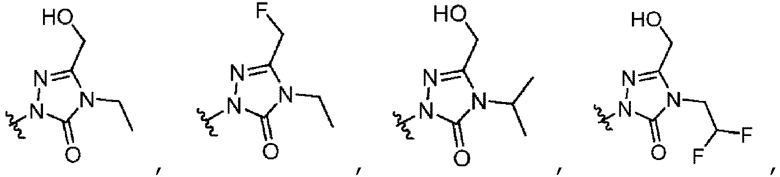 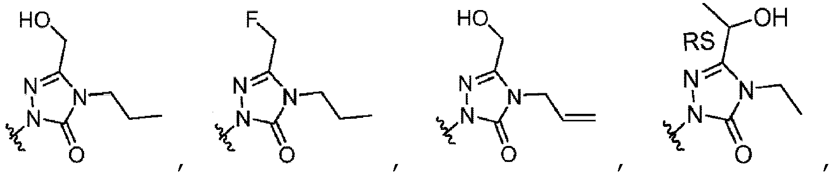 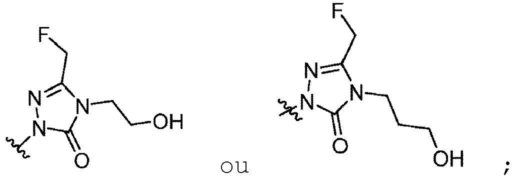 ou sel, solvate, stéréoisomère, forme tautomère, variante isotopique ou N-oxyde pharmaceutiquement acceptable correspondant(e).

21. Composé selon l'une quelconque des revendications 1 à 3, R^{1a} étant CH₃ ; ou sel, solvate, stéréoisomère, forme tautomère, variante isotopique ou N-oxyde pharmaceutiquement acceptable correspondant(e).
